(19) Europäisches Patentamt
European Patent Office
Office européen des brevets

(11) **EP 4 741 383 A1**

(12) **EUROPEAN PATENT APPLICATION**
published in accordance with Art. 153(4) EPC


(43) Date of publication:
**13.05.2026 Bulletin 2026/20**

(21) Application number: **24838733.4**

(22) Date of filing: **05.07.2024**

(51) International Patent Classification (IPC):
***C07D 291/08*** *(2006.01)* ***A61K 31/16*** *(2006.01)*
***A61K 31/33*** *(2006.01)* ***A61P 35/00*** *(2006.01)*

(52) Cooperative Patent Classification (CPC):
**A61K 31/16; A61K 31/33; A61P 35/00; C07D 291/08**

(86) International application number:
**PCT/CN2024/103967**

(87) International publication number:
**WO 2025/011480 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.07.2023 CN 202310836777**



(71) Applicant: **Tyligand Bioscience (Shanghai) Limited**
**Shanghai 201203 (CN)**

(72) Inventors:
- **ZHONG, Boyu**
  **Irving, TX 75038 (US)**
- **WANG, Qian**
  **Shanghai 200333 (CN)**
- **ZHANG, Tony Yantao**
  **Fishers (US)**
- **DONG, Chunlan**
  **Shanghai 201203 (CN)**
- **PANG, Wei**
  **Shanghai 200137 (CN)**
- **QIN, Quan**
  **Shanghai 200032 (CN)**
- **WANG, Zhiqiang**
  **Shanghai 201204 (CN)**
- **LU, Wanglong**
  **Shanghai 201315 (CN)**
- **WANG, Jifa**
  **Shanghai 201210 (CN)**

(74) Representative: **Vossius & Partner Patentanwälte Rechtsanwälte mbB**
**Siebertstraße 3**
**81675 München (DE)**


(54) **COMPOUND AND USE THEREOF AS ANTI-CANCER DRUG**


(57) The present invention provides a compound having a structure of formula (I) and can be used as an SHP2 inhibitor, a pharmaceutical composition comprising the compound, a method for preparing the compound, and a use of the compound in the treatment of cancer.


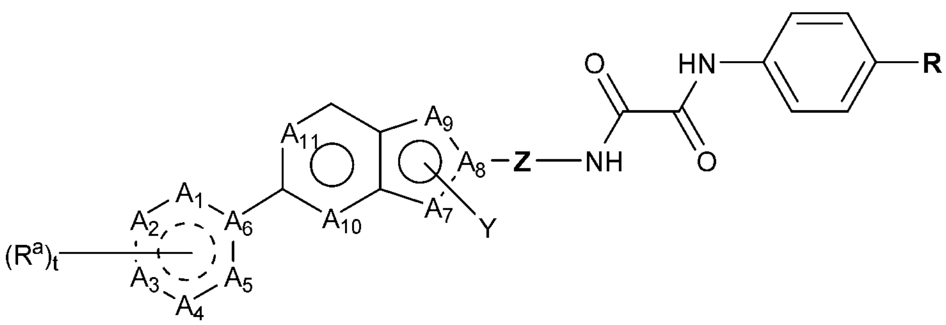

(I)

EP 4 741 383 A1

## Description

### Cross-reference

This application claims priority to Chinese invention patent application 2023108367770, filed on July 7, 2023.

### Technical Field

The present invention relates to the field of pharmaceutical chemistry. More specifically, the present invention relates to a class of compounds with novel structures that can be used as SHP2 inhibitors, pharmaceutical compositions comprising such compounds, methods of using such compounds to treat or prevent diseases mediated by SHP2, especially cancer or tumors, and the use of these compounds in the treatment or prevention of diseases mediated by SHP2, especially cancer or tumors.

### Background Technology

Dysregulation in the level of molecular phosphorylation in the body often leads to the development and progression of various diseases, such as cancer. Specifically, the phosphorylation state of tyrosine in signaling proteins plays a decisive role in the initiation, development, and termination of many cellular signaling cascades. Tyrosine phosphorylation is regulated by protein tyrosine kinase (PTK, phosphorylation) and protein tyrosine phosphatase (PTP, dephosphorylation). Abnormal regulation of these two enzymes leads to dysregulation of tyrosine phosphorylation, resulting in the development of diseases, including diabetes, cancer, and autoimmune diseases.

As a member of the PTP family, protein tyrosine phosphatase 2 (SHP2), containing Src homology-2, is a dephosphorylase with a full length of 593 amino acids encoded by the PTPN1 1 gene. SHP2 consists of two N-terminal SH2 domains, N-SH2 and C-SH2, a conserved PTP domain, and a flexible C-terminal tail. The N-SH2 and C-SH2 domains are primarily used for substrate recognition, enzyme activity regulation, and downstream signaling protein binding, while the PTP domain binds to phosphorylated tyrosine peptide fragments and catalyzes the hydrolysis of their phosphate ester groups. In the basal state of SHP2, N-SH2 and C-SH2 bind to PTP, masking its catalytic active center and forming a self-inhibiting, inactive "off" conformation. This inhibits the activity of the SHP2 protein and restricts substrate access to the catalytic site. Under the stimulation of appropriate signaling factors such as growth factors or cytokines, or in some pathogenic mutations, N-SH2 and C-SH2 leave PTP, opening the PTP active center and exposing the catalytic site. This enables the catalysis of dephosphorylation for phosphorylated substrates, such as receptors, kinases, and phospholipids, thereby regulating downstream signaling.

SHP2 is located at the intersection of multiple signaling pathways and is a crucial hub connecting various intracellular oncogenic signaling pathways. For example, it is a convergence node in multiple signaling pathways such as Ras-Raf-MEK-ERK, JAK-STAT, PI3K-AKT-mTOR, and PD-1/PD-L1, participating in the regulation of various signaling pathways in the body. Overactivated SHP2 can stimulate the activation of multiple signaling pathways, thereby driving the occurrence and development of various cancers. In other words, the occurrence of many cancers is often closely related to the overactivation of signaling pathways regulated by SHP2. Therefore, inhibiting SHP2 activity can suppress the survival and proliferation of tumor cells.

However, the development of SHP2 inhibitors has seen limited progress for a considerable period, even leading to a consensus within the industry that they are "untreatable". This is because early development of small-molecule SHP2 inhibitors focused on the catalytic active center. These active center inhibitors have low biological activity; the enzyme activity of the strongest inhibitor to date (the salicylic acid-based active center inhibitor 11a-1) only achieves an IC50 of about 0.2 $\mu$M, with cellular activity not exceeding an IC50 of 1 $\mu$M. Furthermore, their druggability is poor, with an oral bioavailability of only 0.07% (J. Med. Chem. 2014, 57, 6594-6609). Moreover, the catalytic domain of SHP2 is highly conserved, and inhibitors at catalytic sites generally lack selectivity, potentially leading to severe toxic reactions due to off-target effects.

SHP2 inhibitors designed to inhibit the transition of SHP2 to its "open" active conformation by stabilizing its basal state (inactive "off" conformation) are called "allosteric inhibitors". Allosteric inhibitors bind to the interaction regions of the three protein modules, rather than the catalytic active center. Currently, nine structurally very similar allosteric inhibitors, guided by the pioneer TNO155, have entered clinical trials. However, these allosteric inhibitors have exhibited numerous problems in clinical trials, including severe cardiotoxicity, low specificity, easy off-target toxicity, rapid development of resistance in solid tumors with mutations occurring on the N-SH2 and C-SH2 modules that affect the binding of the PTP module in the "off" conformation, and ineffectiveness in diseases where SHP2 only exists in the "open" conformation due to SHP2 mutations, such as NS, LS, and JMML.

In contrast, despite the aforementioned drawbacks of early and current SHP2 active center inhibitors, overcoming these shortcomings through structural optimization, coupled with the significant advantages of SHP2 active center

inhibitors over allosteric inhibitors in terms of their mode of action, a superior drug option will be provided. For example, active center inhibitors have a significantly altered structural backbone, exhibiting binding characteristics consistent with phosphorylated tyrosine peptide fragments. This not only eliminates the inherent toxic side effects of allosteric inhibitors, such as cardiotoxicity caused by hERG inhibition, but also demonstrates high protein binding specificity, and is less likely to produce unknown off-target effects. Furthermore, the low mutation rate of the SHP2 catalytic active center and its distant binding site compared to allosteric inhibitors result in a slower rate of resistance development, and can overcome the resistance of allosteric inhibitors. Additionally, they remain effective against diseases where only exist in the "open" conformation due to SHP2 mutations, such as NS, LS, and JMML.

Therefore, there is a great need for more compounds with different structural types to serve as SHP2 active center inhibitors. It is hoped that these compounds can provide further improved inhibitory activity and other superior properties compared to existing SHP2 active center inhibitors and allosteric inhibitors, fully leveraging the advantages of SHP2 active center inhibitors while overcoming the shortcomings of existing active center inhibitors and allosteric inhibitors, thereby providing more potent therapeutic drugs for clinical use.

Through in-depth research, the inventors have addressed the aforementioned needs. The present disclosure provides novel structural inhibitory compounds with SHP2 active center inhibitory activity. These inhibitors, due to their improved structural pattern, exhibit enhanced SHP2 protein-specific inhibitory activity and inhibitory activity against related tumors compared to existing SHP2 inhibitors, along with favorable pharmacokinetic properties (thus demonstrating good druggability), excellent selectivity for SHP2 among protein phosphatases, good cardiac safety such as reduced hERG toxicity, and a reduced risk of drug interactions, demonstrating promising application prospects.

## Summary of invention

The present disclosure provides compounds having structural formula (I) as defined herein,

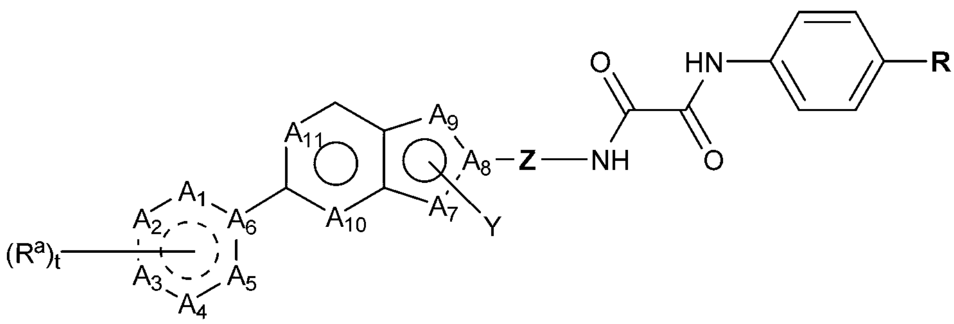

(I)

or a pharmaceutically acceptable salt, isomer, solvate, hydrate or stable isotopic variant thereof. wherein:

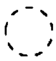 represents a saturated, partially unsaturated, or aromatic ring system;

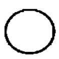 represents an aromatic ring system;

$A_1$, $A_2$, $A_3$, $A_4$, and $A_5$ are each independently selected from absence, C, N, O, and S, and $A_6$ is selected from C and N, provided that (1) at most two of $A_1$~$A_5$ are absent, and (2) at most four of $A_1$~$A_6$ are heteroatoms;

$R^a$ is selected from-H, =O, =S, =N-OH, -OH, $-NH_2$, halogen, -CN and $-C_{1-6}$ alkyl optionally substituted by halogen;

$A_7$, $A_8$ and $A_9$ are each independently selected from C, N, O, and S, provided that at least one of $A_7$, $A_8$ and $A_9$ is not C;

$A_{10}$ is selected from C-$R^b$ and N;

$A_{11}$ is selected from C-X and N;

$R^b$ is selected from H, halogen, CN and $-C_{1-6}$ alkyl substituted by halogen;

X is selected from H, halogen, CN, -OH, $-OC_{1-6}$ alkyl and $-C_{1-6}$ alkyl, wherein the $C_{1-6}$ alkyl is optionally substituted;

Y is an optionally substituted group selected from $-C_{1-6}$ alkyl, $-(CH_2)_t$-3-15 membered carbocyclyl, $-(CH_2)_t-C_{6-10}$ aryl, $-(CH_2)_t$-5-12 membered heteroaryl having one or more heteroatom(s) independently selected from N, O and S, and $-(CH_2)_t$-3-15 membered heterocyclyl having one or more heteroatom(s) independently selected from N, O and S;

Z is an optionally substituted divalent group selected from $-C_{6-10}$ aryl-, -3-10 membered carbocyclyl-, -5-12 membered heteroaryl having one or more heteroatom(s) independently selected from N, O and S-, and -3-15 membered heterocyclyl having one or more heteroatom(s) independently selected from N, O and S-;

R is an optionally substituted group selected from $-C_{6-10}$ aryl, -3-15 membered carbocyclyl, -5-12 membered heteroaryl having one or more heteroatom(s) independently selected from N, O, and S, and -3-15 membered heterocyclyl having one or more heteroatom(s) independently selected from N, O, and S; and

t is an integer from 0 to 3.

The present disclosure further provides a pharmaceutical composition comprising a compound of formula (I) or a pharmaceutically acceptable salt, isomer, solvate, hydrate or stable isotopic variant thereof, and optionally a pharmaceutically acceptable excipient or carrier.

The present disclosure further provides a compound of formula (I), or a pharmaceutically acceptable salt, isomer, solvate, hydrate, or stable isotopic variant thereof, for use as a medicament, e.g., as an SHP2 inhibitor, e.g., for the treatment and/or prevention of diseases mediated by SHP2.

The present invention further provides the use of the compound of formula (I) or a pharmaceutically acceptable salt, isomer, solvate, hydrate or stable isotopic variant thereof, or a pharmaceutical composition comprising thereof, for the treatment and/or prevention of diseases mediated by SHP2, especially those that benefit from SHP2 inhibition.

The present invention further provides the use of the compound of formula (I) or a pharmaceutically acceptable salt, isomer, solvate, hydrate or stable isotopic variant thereof, or a pharmaceutical composition comprising thereof, in the preparation of a medicament for the treatment and/or prevention of diseases mediated by SHP2, especially those that benefit from SHP2 inhibition.

The present invention further provides a method for treating and/or preventing diseases mediated by SHP2, especially those that benefit from SHP2 inhibition, comprising administering to a subject in need a therapeutically effective amount of a compound of formula (I) or a pharmaceutically acceptable salt, isomer, solvate, hydrate or stable isotopic variant thereof, or a pharmaceutical composition comprising thereof.

The present invention further provides a method for treating tumors or cancer, comprising administering to a patient in need an effective amount of a compound of formula (I) or a pharmaceutically acceptable salt, isomer, solvate, hydrate or stable isotopic variant thereof, or a pharmaceutical composition comprising thereof.

The present invention further provides a method for inhibiting SHP2 activity, comprising contacting an effective amount of a compound of formula (I) or a pharmaceutically acceptable salt, isomer, solvate, hydrate or stable isotopic variant thereof with a biological sample such as cells.

The present invention further provides the use of the compound of formula (I) or a pharmaceutically acceptable salt, isomer, solvate, hydrate or stable isotopic variant thereof as a research tool compound for an SHP2 inhibitor in studies.

The present invention further provides a pharmaceutical combination comprising a compound of formula (I) or a pharmaceutically acceptable salt, isomer, solvate, hydrate or stable isotopic variant thereof and one or more other pharmaceutically active agent(s).

The present invention further provides a method for preparing a compound of formula (I) or a pharmaceutically acceptable salt, isomer, solvate, hydrate or stable isotopic variant thereof.

## Detailed description of invention

### Definitions

Unless otherwise indicated, each term used in the specification and claims has the meanings set forth below. In the absence of a specific definition for a particular term or phrase, it should be understood in accordance with its ordinary meaning in the art. In case of conflict, the present specification (including the definitions) shalled.

In case of a conflict between the chemical structures and names of the compounds disclosed herein, the chemical structures shall prevail.

For any of the following chemical definitions, the number following the atom symbol represents the total number of atoms of that element present in a particular chemical part. Other atoms, such as H atoms or substituents, may be present as needed to satisfy the valence of the atoms.

As used herein, the term "comprising", "including", "having", and variations thereof are inclusive or open-ended,

meaning "including, but not limited to," and are not intended to exclude other additives, components, integers, or steps. When an element is described as including a plurality of components, steps, or conditions, the element may also be described as including any combination of the plurality of components, steps, or conditions, or "consisting of the plurality or combination of components, steps, or conditions," or "essentially consisting of the plurality or combination of components, steps, or conditions".

As used herein, the term "about" means within the range of +10% of the numerical value involved, preferably within the range of +5%, and more preferably within the range of +2%.

Unless otherwise specified, $C_{n-n+m}$ or $C_n$-$C_m$ in the definition of compounds of the present disclosure includes various situations from n to n+m carbons, for example, $C_{1-6}$ includes $C_1$, $C_2$, $C_3$, $C_4$, $C_5$ and $C_6$, and also includes any range from n to n+m, for example, $C_{1-6}$ includes $C_{1-2}$, $C_{1-3}$, $C_{1-4}$, $C_{2-6}$, $C_{3-6}$, etc. In the same way, n membered to n+m membered in the definition of compounds of the present disclosure means that the number of ring atoms is n to n+m. For example, 3-12 membered rings include 3 membered, 4 membered, 5 membered, 6 membered, 12 membered rings, etc., and also include any range from n to n+m membered rings, such as 3-6 membered, 3-8 membered, 4-7 membered, 4-10 membered, 5-6 membered, 6-8 membered, 6-10 membered, 6-12 membered, 8-10 membered rings, etc.

As used herein, the term "SHP2" refers to protein tyrosine phosphatase 2 containing Src homology-2, is a dephosphorylase with a full length of 593 amino acids encoded by the PTPN11 gene. For the purposes of the present disclosure, SHP2 can be wild-type or any mutant or variant of SHP2 containing one or more mutation(s) (e.g., conserved substitutions).

As used herein, the term "diseases mediated by SHP2" refers to a disease in which SHP2 activity promotes the occurrence and development of the disease. For the purposes of the present disclosure, "diseases mediated by SHP2" specifically refers to a disease in which inhibiting SHP2 activity reduces the incidence of the disease, inhibits, improves, alleviates or eliminates the disease and/or its symptoms, or is sensitive to or responsive to SHP2 inhibition, or benefits from SHP2 inhibition, including but not limited to proliferative diseases, metabolic diseases or hematologic diseases, especially cancer or neoplastic diseases.

As used herein, the terms "cancer" or "tumor" refer to abnormal cell growth and proliferation, whether malignant or benign, and all precancerous and cancerous cells and tissues. For all aspects of the present disclosure, cancers that can be treated with the compounds of the present disclosure are selected from, but are not limited to, the following: juvenile myelomonocytic leukemia (JMML), myelodysplastic syndrome (MDS), acute myeloid leukemia (AML), B-cell acute lymphoblastic leukemia, neuroblastoma, esophageal cancer, breast cancer (including triple-negative breast cancer), lung cancer (including small cell lung cancer, non-small cell lung cancer, bronchioloalveolar carcinoma), lung adenocarcinoma, colon cancer, rectal adenocarcinoma, adenoid cystic carcinoma, gastric cancer, gastrointestinal stromal tumor, head and neck cancer (e.g., squamous cell carcinoma of the head and neck), ovarian cancer, prostate cancer, melanoma, cutaneous or intraocular melanoma, soft tissue sarcoma; cancers of the oral cavity and pharynx (lip, tongue, mouth, throat, nasopharynx), stomach, small intestine, large intestine, colon, rectum, anal region, liver and biliary tract, pancreas, bone, connective tissue, skin (including epithelial cell carcinoma), vagina, vulva, cervix, uterus, endometrium, fallopian tubes, urethra, penis, testis, bladder, ureter, kidneys, and other urinary tissues, including renal cell carcinoma, renal pelvis carcinoma, and hepatocellular carcinoma; gastroesophageal cancer; and cancers of the eye, brain, spinal cord, and central and peripheral nervous systems and related structures, such as meningeal carcinoma, primary CNS lymphoma, oligodendroglioma, medulloblastoma, spinal tumors, brainstem glioma, or pituitary adenoma; cancers of the thyroid and other endocrine glands, Hodgkin's disease, non-Hodgkin's lymphoma, parathyroid carcinoma, adrenal carcinoma, multiple myeloma, medulloblastoma, and hematopoietic malignancies, including chronic or acute lymphoblastic leukemia, chronic or acute myeloid leukemia, chronic myelomonocytic leukemia, and lymphomas, including lymphocytic, granulocytic and monocytic, mantle cell lymphoma and histiocytic lymphoma.

As used herein, the term "treatment" refers to the administration of one or more of the compound(s) described herein, or a pharmaceutically acceptable salt, isomer, solvate, hydrate, or stable isotopic variant thereof to a subject, such as a mammal, such as a human, suffering from or having symptoms of the disease described, to cure, alleviate or reduce the disease or its symptoms. Preferably, treatment is curative or ameliorative.

As used herein, the term "prevention" refers to the administration of one or more of the compound(s) described herein, or a pharmaceutically acceptable salt, isomer, solvate, hydrate, or stable isotopic variant thereof to a subject, such as a mammal or human, suspected of developing or predisposing to diseases mediated by SHP2, especially cancer or tumors, as defined herein, to reduce the risk of developing the disease as defined, or to prevent the onset of the disease. The term "prevention" includes compounds of the present disclosure used prior to diagnosis or identification of any clinical and/or pathological symptoms.

As used herein, the terms "inhibit" and "reduce", or any variations thereof refer to the ability of a bioactive agent to reduce the signaling activity of the target of interest by interacting with the target, and refer to any measurable reduction or complete inhibition of activity of the target of interest. For example, compared to normal conditions, the reduction in activity (e.g., SHP2 activity) can be about, at most or at least about 5%, 10%, 15%, 20%, 25%, 30%, 35%, 40%, 45%, 50%, 55%, 60%, 65%, 70%, 75%, 80%, 85%, 90%, 95%, 99% or more, or any range derived therefrom.

As used herein, the term "selective inhibition" or "specific inhibition" refers to the ability of a bioactive agent to distinguish potential targets, preferentially binding to the target of interest in the presence of one or more competing alternative target(s), and preferentially reducing the signal transduction activity of the target of interest. In some embodiments, the compound of the present disclosure undetectably binds to the competing alternative targets under conditions that bind SHP2. In some embodiments, compared to the competing alternative targets, the compound of the present disclosure binds to SHP2 with a higher binding rate, a lower dissociation rate, increased affinity, decreased dissociation, and/or increased stability. Specifically, compared to other PTP phosphatases, such as SHP1, HePTP, Laforin, LMPTP, LYP, PTP1B, PRL2, SSU72, VHR, FAP1, STEP, CDC14A, CD45, MEG2, PP5, etc., the compound of the present disclosure has the ability to selectively inhibit SHP2 activity. For example, compared to the IC50 of another specific PTP phosphatase, the compound of the present disclosure exhibits an IC50 value for SHP2 that is at least about 1-fold lower, such as about 1-5-fold, 5-10-fold, 10-100-fold lower, even more than 100-fold lower.

As used herein, the terms "subject", "individual", "or" "patient" refer to a vertebrate. In some embodiments, the vertebrate is a mammal. Mammals include, but are not limited to, farm animals (e.g., cattle), sports animals, pets (e.g., guinea pigs, cats, dogs, rabbits, and horses), primates, mice, and rats. In some embodiments, the mammal is a human.

As used herein, the term "therapeutic effective amount" refers to an amount or dose sufficient to elicit a beneficial therapeutic response in a patient afflicted with a "disease mediated by SHP2", such as cancer or tumors. A person skilled in the art may determine the effective amount or dose of the active ingredient of the present disclosure using conventional methods, in combination with conventional influencing factors.

As used herein, the term "pharmaceutical combination" refers to the ability of the compound of the present disclosure may be combined with other active agents for carrying out the purposes of the present invention. These other active agents may be one or more additional compound(s) of the present disclosure, or may be a second or additional (e.g., a third) compound that is compatible with the compound of the present disclosure, meaning that they do not adversely affect each other or exhibit complementary activities. For example, these active agents are known to modulate other biological pathways, or modulate different components of the biological pathways involved by the compound of the present disclosure, or even overlap with the biological targets of the compound of the present disclosure. The other active agent(s) may be co-administered with the compound of the present disclosure in a single pharmaceutical composition or administered separately in distinct units. When administered separately, the administration may be simultaneous or sequential. Sequential administration may be closely spaced or separated by a longer interval.

As used herein, the term "pharmaceutically acceptable" refers to molecular entities and compositions that, when administered in appropriate amounts to an animal, such as a human, do not produce adverse, allergic, or other undesirable reactions.

As used herein, the term "pharmaceutically acceptable excipient" or "pharmaceutically acceptable carrier" refers to one or more compatible solid or liquid fillers or gelling substances suitable for human use and having sufficient purity and sufficiently low toxicity. Examples include, but are not limited to, cellulose and its derivatives (such as sodium carboxymethyl cellulose, cellulose acetate, etc.), gelatin, talc, solid lubricants (such as magnesium stearate), calcium sulfate, vegetable oils, polyols (such as propylene glycol, glycerin, mannitol, sorbitol, etc.), emulsifiers (such as Tweens), wetting agents (such as sodium dodecyl sulfate), colorants, flavoring agents, stabilizers, antioxidants, preservatives, etc.

As used herein, the term "pharmaceutically acceptable salt" refers to salts that retain the biological effectiveness of the parent compound and are not biologically or otherwise undesirable, including acid addition salts and base addition salts. "Pharmaceutically acceptable acid adducts" can be formed from compounds with basic groups with inorganic or organic acids, inorganic acids such as hydrochloric acid, hydrobromic acid, sulfuric acid, nitric acid, carbonic acid, phosphoric acid, etc., organic acids can be selected from aliphatic, aliphatic, aromatic, aromatic, heterocyclic, carboxylic, and sulfonic organic acids, such as formic acid, acetic acid, propionic acid, glycolic acid, gluconic acid, lactic acid, pyruvate, oxalic acid, apple acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, pamoate, phenylacetic acid, methanesulfonic acid, ethanesulfonic acid, benzenesulfonic acid, p-toluenesulfonic acid, salicylic acid, etc. "Pharmaceutically acceptable base addition salts" include those derived from inorganic bases such as salts of sodium, potassium, lithium, ammonium, calcium, magnesium, iron, zinc, copper, manganese, and aluminum, and salts derived from pharmaceutically acceptable organic non-toxic bases, including but not limited to primary, secondary and tertiary amines, substituted ammonium, including naturally occurring substituted amines, cyclic amines and basic ion exchange resins, such as ammonia, isopropylamine, trimethylamine, diethylamine, triethylamine, tripropylamine, ethanolamine, diethanolamine, 2-bis methyl aminoethanol, 2-diethylaminoethanol, tromethamine, dicyclohexylamine, lysine, arginine, histidine, caffeine, procaine, hybamine, choline, betaine, ethylenediamine, glucosamine, methylglucosamine, triethanolamine, theobromine, purine, piperazine, piperidine, N-ethylpiperidine, polyamine resin, etc.

As used herein, the term "isomer" refers to any stereoisomer, enantiomeric mixture, including racemate, diastereomeric mixture, geometric isomer, atropisomer, and/or tautomer that may exist in the structure of a compound. For example, some compounds of the present disclosure may contain at least one asymmetric center, thereby producing stereoisomers. Therefore, the present invention encompasses all possible isomeric forms of the compounds defined

herein, as well as their pharmaceutically acceptable salts or solvates, unless otherwise indicated. Methods for determining and separating the stereochemistry of said isomers are well known to those skilled in the art (SP Parker, Ed., McGraw-Hill Dictionary of Chemical Terms (1984), McGraw-Hill Book Company, New York; and Eliel, E. and Wilen, S., "Stereochemistry of Organic Compounds", John Wiley & Sons, Inc., New York, 1994).

"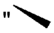" or "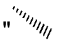" used in the structural formulae or structural fragments of the compounds herein represent the absolute configuration of the stereocenter, i.e., the chiral center. Correspondingly, in the naming of the compounds or intermediates provided in the present invention, R or S is used to represent the absolute configuration of the relevant chiral center.

It should be understood that when those skilled in the art can determine, based on the compound structure shown herein, that the compound has one and only one chiral isomer and can be easily resolved by conventional methods in the art, then the disclosure of the racemate of the compound herein (whether in the form of a structural formula or a chemical name) shall be regarded as having separately disclosed each isomer of the compound.

As used herein, the term "isotope variant" refers to a compound in which one or more atom(s) are replaced by atoms enriched with the corresponding isotope. Examples of isotopes that can be incorporated into compounds of formula (I) include isotopes of hydrogen, carbon, nitrogen, oxygen, fluorine, chlorine, and iodine, such as $^{2}H$, $^{3}H$, $^{11}C$, $^{13}C$, $^{14}C$, $^{15}N$, $^{18}O$, $^{17}O$, $^{35}S$, $^{18}F$, $^{37}Cl$, and $^{125}I$. Such isotope variants can be used as probes in biological assays, analytical tools, or therapeutic agents. In certain embodiments, the compounds of the present disclosure are provided in an unlabeled form. In certain embodiments, the compounds of the present disclosure also include isotope variants formed by replacing one or more atom(s) present in the compound with corresponding isotopes, for example, one or more hydrogen atom(s) replaced by $^{2}H$ or $^{3}H$, or one or more carbon atom(s) replaced by $^{13}C$or $^{14}C$-enriched carbon.

As used herein, the term "solvent" refers to a solvent addition form of a compound that contains stoichiometric or non-stoichiometric amounts of solvent. This includes any solvated form of the compounds of the present disclosure, such as solvates with water (e.g., hydrates) or with organic solvents (e.g., methanol, ethanol, or acetonitrile), referred to as methanolates, ethanolates, or acetonitrileates, respectively. It also includes solvates in any polymorphic form. It should be understood that such solvates of the compounds of the present disclosure also encompass solvates of pharmaceutically acceptable salts of the compounds of the present disclosure.

As used herein, the term "halogen" or "halogenated" refers to F, Cl, Br, or I. In addition, the term "halogen-substituted" group used in the definition of groups herein is intended to include monohalogenated or poly-halogenated groups, in which one or more identical or different halogen(s) substitute one or more hydrogen(s) in the corresponding group.

As used herein, the term "hydroxyl group" refers to the-OH group.

As used herein, the term "cyano" refers to the-CN group.

As used herein, the term "nitro" refers to the-$NO_2$ group.

As used herein, the term "alkyl" refers to refers to monovalent saturated hydrocarbon groups consisting of straight or branched chains of carbon and hydrogen atoms. Specifically, alkyl has 1-10, for example, 1 to 8, 1 to 6, 1 to 4, 1 to 3, or 1 to 2 carbon atoms. For example, as used herein, the term "$C_{1-6}$ alkyl" refers to saturated hydrocarbon groups having straight or branched chains of 1 to 6 carbon atoms, examples of which include, but are not limited to, methyl, ethyl, propyl (including n-propyl and isopropyl), butyl (including n-butyl, isobutyl, sec-butyl, or tert-butyl), pentyl (including n-pentyl, isopentyl, and neopentyl), n-hexyl, 2-methylpentyl, etc.

As used herein, the term "alkoxy" refers to an alkyl defined herein that is connected by an oxygen atom to the rest of the molecule. Specifically, the alkoxy group has 1 to 10, for example, 1 to 8, 1 to 6, 1 to 4, 1 to 3, or 1 to 2 carbon atoms. For example, as used herein, the term "$C_{1-6}$ alkoxy" or "-$OC_{1-6}$ alkyl" refers to a saturated hydrocarbon group having a straight or branched chain of 1 to 6 carbon atoms attached to the rest of the molecule by an oxygen atom, examples of which include, but are not limited to, -O-methyl, -O-ethyl, -O-propyl (including-O-n-propyl and -O-isopropyl), -O-butyl (including-O-n-butyl, -O-isobutyl, -O-sec-butyl, or -O-tert-butyl), -O-pentyl (including-O-n-pentyl, - O-isopentyl, -O-neopentyl), -O-n-hexyl, 2-methylpentyl-O-, etc.

As used herein, the term "$C_{1-6}$ alkyl optionally substituted by halogen or CN" refers to the $C_{1-6}$ alkyl described above, wherein one or more (e.g., 1, 2, 3, 4, or 5) hydrogen atom(s) are optionally replaced by halogen and/or CN. When there is more than one halogen substituent, the halogen may be the same or different, and may be located on the same or different C atoms. Examples of "$C_{1-6}$ alkyl optionally substituted by halogen or CN" include, but are not limited to, -$CH_2F$, -$CH_2Cl$, - $CH_2CN$, -$CHF_2$, -$CF_3$, -$CCl_3$, -$C_2F_5$, -$C_2Cl_5$, -$CH_2CF_3$, -$CH_2CH_2CN$, -$CH_2CH_2CF_3$, or -$CF(CF_3)_2$, etc.

As used herein, the term "carbocyclyl" refers to a monocyclic, fused polycyclic, bridged polycyclic, or spirocyclic saturated or partially unsaturated non-aromatic hydrocarbon ring structure having a specified number of ring carbon atoms, wherein a fully conjugated $\pi$-electron system is absent. Carbocyclyl can have 3 to 15 carbon atoms (i.e., $C_{3-15}$ carbocyclyl), for example, 3 to 10, 3 to 8, 3 to 6, 4 to 8, 5 to 10, 6 to 10, 6 to 11, 6 to 14, 6 to 15 carbon atoms, etc. The carbocyclyl in the compounds disclosed herein can be unsubstituted or substituted by one or more substituent(s) as defined.

In some embodiments, the term "carbocyclyl" as used herein refers to a "monocyclic saturated or partially unsaturated carbocyclyl" having a specified number of ring carbon atoms. "Monocyclic saturated carbocyclyl" is also called "monocyclic cycloalkyl," such as the 3-8 membered monocyclic saturated carbocyclyl or 3-6 membered monocyclic saturated carbocyclyl in the compounds disclosed herein, i.e., $C_{3-8}$ cycloalkyl or $C_{3-6}$ cycloalkyl, specific examples including but not limited to cyclopropyl, cyclobutyl, cyclopentyl, cyclohexyl, etc. "Monocyclic partially unsaturated carbocyclyl" is also called "monocyclic cycloalkenyl," such as the 3-8 membered monocyclic partially unsaturated carbocyclyl or 3-6 membered monocyclic partially unsaturated carbocyclyl, i.e., $C_{3-8}$ cycloalkenyl or $C_{3-6}$ cycloalkenyl, specific examples including but not limited to cyclopropenyl, cyclobutenyl, cyclopentenyl, cyclopentadienyl, cyclohexenyl, cyclohexadienyl, cycloheptadienyl, etc.

In some embodiments, the term "carbocyclyl" as used herein refers to a "bridged saturated or partially unsaturated carbocyclyl" having a specified number of ring carbon atoms. meaning a polycyclic carbocycle sharing two non-directly connected carbon atoms, called bridgehead carbons. The two bridgehead carbons can be connected by a carbon chain or a single bond, called a bridge. These groups may contain one or more double bond(s) but do not possess a fully conjugated π-electron system. They can be 5-14 membered, for example 6-12 membered, more preferably 5-10 membered. Based on the number of rings, they can be classified as bicyclic, tricyclic, or polycyclic bridged carbocycle, preferably bicyclic or tricyclic. For example, examples of 5-10 membered, such as 5-8 membered bridged saturated or partially unsaturated carbocycle in the compounds disclosed herein include, but are not limited to: bicyclo[1.1.0]butyl, bicyclo[1.1.1]pentyl, bicyclo[2.2.1]heptyl, bicyclo[3.1.1]hexyl, bicyclo[3.1.1]heptyl, or bicyclo[3.2.1]octyl, etc.

In some embodiments, the term "carbocyclyl" as used herein refers to a "spirocyclic saturated or partially unsaturated carbocyclyl" having a specified number of ring carbon atoms, meaning a polycyclic group sharing a single carbon atom (called a spiro atom) between monocyclic rings. It may contain one or more double bond(s) but does not have a fully conjugated π-electron system. It can be 6-15 membered, preferably 7-15 membered, and more preferably 7-11 membered. Based on the number of spiro atoms shared between rings, spiro cycloalkyl are classified into monospiro cycloalkyl (monospiro carbocyclyl), bispiro cycloalkyl (bispiro carbocyclyl), or polyspiro cycloalkyl (polyspiro carbocyclyl), preferably monospiro and bispiro carbocyclyl. The monospiro carbocyclyl can also be called a bicyclic spiro carbocyclyl; more preferably, 4 membered/4 membered, 4 membered/5 membered, 4 membered/6 membered, 5 membered/5 membered, 5 membered/6 membered, and 6 membered/6 membered monospirocarbocycle. The spiro carbocyclyl in the compounds disclosed herein can be a 6-15 membered monospiro carbocyclyl, i.e., a 6-15 membered bicyclic spirocarbocycle, preferably a 7-11 membered bicyclic spirocarbocycle. Non-limiting examples of spiro carbocyclyl include:

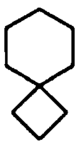 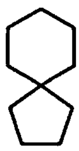 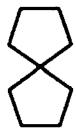 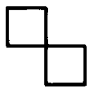 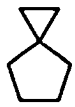 ,

etc.

In some embodiments, the term "carbocyclyl" as used herein refers to a "fused saturated or partially unsaturated carbocyclyl" having a specified number of ring carbon atoms, meaning a carbocyclyl group in which each ring shares a set of adjacent carbon atoms with other rings, and may contain one or more double bond(s) but does not have a fully conjugated π-electron system. It can be 6-15 membered, for example 5-14 membered, 6-12 membered, 7-14 membered, preferably 7-11 membered. Based on the number of rings, it can be classified as bicyclic, tricyclic, or polycyclic fused carbocyclyl, preferably 5 membered /5 membered or 5 membered /6 membered bicyclic fused carbocyclyl. Non-limiting examples of fused carbocyclyl include

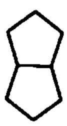 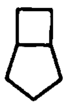 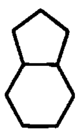 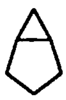 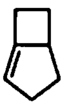 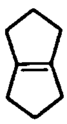 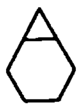 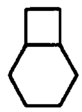 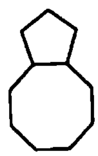 .

As used herein, the term "aryl" refers to a monocyclic or fused polycyclic aromatic ring structure having a specified number of ring atoms. Specifically, the term includes groups comprising, for example, 6 to 10, preferably 6, ring members. Specific aryl groups include phenyl and naphthyl, with the most specific aryl being phenyl. The aryl groups in the compounds disclosed herein may be unsubstituted or substituted by one or more substituent(s) as defined.

As used herein, the term "heterocyclyl" refers to a cyclic group comprising one or more heteroatom(s)

independently selected from O, N, and S, and a specified number of ring atoms. It may be saturated or partially unsaturated, and may be a monocyclic, fused, spirocyclic, or bridged polycyclic ring structure. The definitions of fused heterocyclyl, spiro heterocyclyl, or bridged heterocyclyl are similar to those defined above for "fused carbocyclyl", "spiro carbocyclyl" or "bridged carbocyclyl", except that each comprises one or more heteroatom(s) independently selected from O, N, and S, for example, 1 to 4, 1 to 3, or 1 to 2 of the aforementioned heteroatoms. In the case of polycyclic heterocyclyl, the ring structure attached to the remainder of the molecule need only be a non-aromatic ring. Even if the ring further fused, spiro-fused, or bridged with this non-aromatic ring is an aromatic ring, the heterocyclic system is still defined herein as a heterocyclyl. Heterocyclyl can have 3 to 15 ring members (referred to as 3-15 membered heterocyclyl), such as 3 to 10 ring members, 3 to 8 ring members, 4 to 8 ring members, 5 to 8 ring members, 5 to 10 ring members, 6 to 11 ring members, 6 to 14 ring members, 6 to 15 ring members, 7 to 15 ring members, 7 to 11 ring members, etc., for example, monocyclic heterocyclyl, such as 3-8 membered or 4-8 membered monocyclic saturated heterocyclyl, or bicyclic or tricyclic heterocyclyl, such as 7-14 membered or 7-11 membered spiro heterocyclyl (preferably 4 membered/4 membered, 4 membered/5 membered, 4 membered/6 membered, 5 membered/5 membered, 5 membered/6 membered, 6 membered/6 membered monospiro heterocyclyl), 5-14 membered or 5-10 membered or 5-8 membered bridged heterocyclyl (preferably bicyclic or tricyclic), 6-14 membered or 6-10 membered or 7-11 membered fused heterocyclyl (preferably a 5 membered /5 membered or 5 membered /6 membered bicyclic fused heterocyclyl). The heterocyclyl typically contains at least one and at most four (e.g., one, two, three, or four) heteroatoms, such as a 3-8 membered monocyclic heterocyclyl containing one to three heteroatoms independently selected from N, O, and S, or a bicyclic or tricyclic heterocyclyl containing one to three heteroatoms independently selected from N, O, and S, such as a 7-14 membered or 7-11 membered spiro heterocyclyl, a 5-14 membered or 5-10 membered or 5-8 membered bridged heterocyclyl, a 6-14 membered, 6-10 membered or 7-11 membered fused heterocyclyl. The heterocyclyl in the compounds disclosed herein may be unsubstituted or substituted by one or more substituent(s) as defined.

Examples of suitable heterocyclyl include, but are not limited to, azetidinyl, oxetanyl, thietanyl, pyrrolidinyl (e.g., 1-pyrrolidinyl, 2-pyrrolidinyl, and 3-pyrrolidinyl), tetrahydrofuryl (e.g., 1-tetrahydrofuryl, 2-tetrahydrofuryl, and 3-tetrahydrofuryl), tetrahydrothienyl (e.g., 1-tetrahydrothienyl, 2-tetrahydrothienyl and 3-tetrahydrothienyl), piperidinyl (e.g., 1-piperidinyl, 2-piperidinyl, 3-piperidinyl and 4-piperidinyl), tetrahydropyranyl (e.g., 4-tetrahydropyranyl), tetrahydrothianyl (e.g., 4-tetrahydrothianyl), morpholinyl (e.g., morpholino), thiomorpholinyl, dioxanyl, piperazinyl or azepanyl, diazepanyl e.g., 1,4-diazepanyl, 2-aza-bicyclo[2.2.1]heptyl, 2-aza-bicyclo[2.2.2]octyl, 3,6-diaza-bicyclo[3.1.1]heptyl, 3-aza-bicyclo[3.2.1]octyl, 1,7-dioxa-[4.5]decyl, 2-oxa-7-aza-spiro[4.4]nonyl, 7-oxa-spiro[3.5]nonyl, 5-oxa-spiro[2.4]heptyl, octahydropyrrolo[3,4-c]pyrrolyl, octahydro-1H-isoindolyl, octahydrobenzo[b][1,4]dioxinyl. The atom in the heterocycloalkyl that is attached to the rest of the compound can be either a carbon atom or a heteroatom, as long as it is chemically feasible. It should be understood that the structure with the asymmetric center encompasses its racemic and/or single enantiomeric forms, for example,

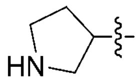

may represent

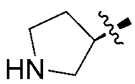

and/or

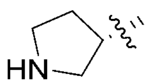

.

As used herein, the term "heteroaryl" refers to a monocyclic or polycyclic aromatic ring system having 5-12 ring atoms, including 1 to 4 heteroatoms selected from nitrogen, oxygen, and sulfur. This ring system can be attached to the heteroatoms or carbon atoms of the remaining part of the molecule. Specific embodiments include, for example, 5-6 membered monocyclic or 8-10 membered bicyclic heteroaryl. Examples of heteroaryl include, but are not limited to, pyrrole, furan, thiophene, pyrazole, imidazole, isoxazole, oxazole, isothiazole, thiazole, 1,2,3-triazole, 1,3,4-triazole, 1-oxa-2,3-diazole, 1-oxa-2,4-diazole, 1-oxa-2,5-diazole, 1-oxa-3,4-diazole, 1-thia-2,3-diazole, 1-thia-2,4-diazole, 1-thia-2,5-diazole, 1-thia-3,4-diazole, tetrazole, pyridine, pyridazine, pyrimidine, pyrazine, benzofuran, benzothiophene, indole, benzimidazole, indazole, benzotriazole, pyrrolo[2,3-b]pyridine, pyrrolo[2,3-c]pyridine, pyrrolo[3,2-c]pyridine, pyrrolo[3,2-b]pyridine, imidazo[4,5-b]pyridine, imidazo[4,5-c]pyridine, pyrazolo[4,3-d]pyridine, pyrazolo[4,3-c]pyridine, pyrazolo[3,4-c]pyridine, pyrazolo[3,4-b]pyridine, isoindole, purine, indolizine, imidazo[1,2-a]pyridine, imidazo[1,5-a]pyridine, pyrazolo[1,5-a]pyridazine, pyrrolo[1,2-b]pyrimidine, imidazo[1,2-c]pyrimidine, 5H-pyrrolo[3,2-b]pyrazine, 1H-pyrazolo[4,3-b]pyrazine, IH-pyrazolo[3,4-d]pyrimidine, 7H-pyrrolo[2,3-d]pyrimidine, quinoline, isoquinoline, cycloline, quinazoline, quinoxaline, phthalazine, 1,6-naphthidine, 1,7-naphthidine, 1,8-naphthidine, 1,5-naphthidine, 2,6-naphthidine, 2,7-naphthidine, pyrido[3,2-d]pyrimidine, pyrido[4,3-d]pyrimidine, pyrido[3,4-d]pyrimidine, pyrido[2,3-d]pyrimidine, pyrido[2,3-b]pyrazine, pyrido[3,4-b]pyrazine, pyrimido[5,4-d]pyrimidine, pyrazino[2,3-b]pyrazine, and pyrimido[4,5-d]pyrimidine. The heteroaryl in the compounds disclosed herein may be unsubstituted or substituted by one or more substituent(s) as defined.

As used herein, the term "optionally substituted" means that one or more hydrogen(s) in a specified moiety are unsubstituted or replaced by suitable substituents. An "optionally substituted" group may carry stable substituents at each substituted position of the group, for example

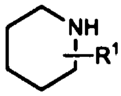,

at least

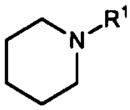, 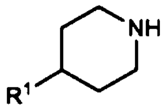, 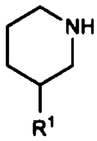 or 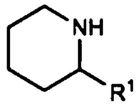;

and when more than one position in any specified structure can be substituted by more than one substituent selected from the specified group, the substituents may be the same or different at each position. The substituent combinations contemplated in the present invention are preferably those that result in the formation of stable or chemically viable compounds. In one embodiment, the optionally substituted group has one substituent. In another embodiment, the optionally substituted group has 2 identical or different substituents. In another embodiment, the optionally substituted group has 3 identical or different substituents. In yet another embodiment, the optionally substituted group has 4 identical or different substituents.

Many of the groups defined herein are optionally substituted. The list of substituents provided in this definition section is merely exemplary and is not intended to limit the substituents defined in other parts of the specification and the claims.

In the cyclic structural fragments discussed herein, a substituent depicted as spanning a chemical bond, such as $-(R_1)_t$ in the example provided, indicates that the one or more R1 substituent(s) may occupy any chemically feasible substitution site(s) on the ring, including position G.

The substituents shown across chemical bonds in cyclic structural fragments mentioned herein, such as

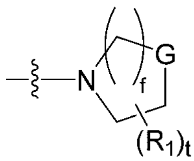 $-(R_1)_t$,

refer to one or more $R_1$ substituent(s) that can substitute one or more chemically feasible substitution site(s) in the ring, including G.

The "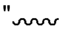" used in the structural fragments mentioned herein, such as  in

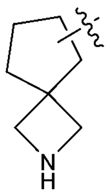,

represents that the fragment is attached to the rest of the molecule by any one of the chemically feasible ring atoms.

Those skilled in the art of organic synthesis will understand that all groups carried on the structure of the compounds of the present invention, whether unsubstituted or substituted by various defined substituents, are provided to make the compound molecule chemically feasible and stable, wherein the type and number of substituents are determined by the number of atoms and the chemical valence of the group.

## Technical problems to be solved

As mentioned above, compounds capable of inhibiting SHP2 activity can be used to treat or prevent SHP2-mediated diseases (such as cancer or tumors). Therefore, various structural types of SHP2 inhibitors have been developed in this field. However, existing SHP2 inhibitors still face challenges that need to be addressed, including unsatisfactory antitumor activity, poor drug resistance due to toxic side effects, insufficient pharmacokinetic properties to allow for convenient administration (i.e., poor "drugability"), or undesirable drug interactions due to inhibition of the cytochrome P450 enzyme system. Furthermore, even for SHP2 inhibitors with good antitumor activity, there remains a desire to further enhance their selective inhibitory activity against target proteins in vivo, improve their drug resistance (reduced toxicity or better safety), and optimize their pharmacokinetic properties through structural optimization, thereby providing more and better therapeutic options for clinical use.

## Technical solutions

Through extensive and in-depth research, the inventors have developed a group of compounds with excellent inhibitory activity against SHP2. Through structural modification and activity verification, the inventors discovered that by modifying specific sites on certain sites of the SHP2 inhibitor structure with specific types of fragment modifications, they obtained significantly improved inhibitory activity against SHP2 compared to existing inhibitors. These modified compounds can inhibit the proliferation of various tumor cells, and exhibit excellent selectivity for SHP2 among numerous PTP phosphatases. They also show significantly reduced hERG toxicity (i.e., good safety), reduced drug interaction risk, and good, or even further improved, pharmacokinetic properties, enabling convenient administration methods.

Accordingly, the present invention primarily provides effective selective SHP2 inhibitors; pharmaceutical compositions containing such compounds as active ingredients; said compounds as a medicament for treating or preventing diseases mediated or benefited by SHP2 inhibition, preferably tumors or cancers; methods of using said compounds for treating or preventing diseases mediated or benefited by SHP2 inhibition, preferably tumors or cancers; and the use of said compounds in the preparation of a medicament for treating or preventing diseases mediated or benefited by SHP2 inhibition, preferably tumors or cancers.

## Compounds of Disclosure

The terms "the compound disclosed" and "compound of the present disclosure" as used throughout this application, unless otherwise specified, encompass compounds as defined in the various embodiments herein and their preferred or exemplary embodiments, and also encompass compounds formed by any combination or subcombination of the various groups as generally, preferably, or exemplaryly defined, including pharmaceutically acceptable salts, isomers (including transisomers, enantiomer mixtures, particularly racemates, diastereomer mixtures, geometric isomers, tautomers), solvates, or isotopic variants of such compounds. However, they are preferably compounds of the present disclosure and/or pharmaceutically acceptable salts or solvates thereof.

The present disclosure also encompasses N-oxides of the compounds disclosed herein, as long as these compounds contain basic nitrogen atoms, such as those present in nitrogen-containing heterocycle, and are chemically and biologically feasible. Certain compounds of the present disclosure may exist in polymorphic or amorphous forms, and therefore they also fall within the scope of the present invention.

The present disclosure provides a compound of formula (I), or a pharmaceutically acceptable salt, isomer, solvate, hydrate, or stable isotopic variant thereof,

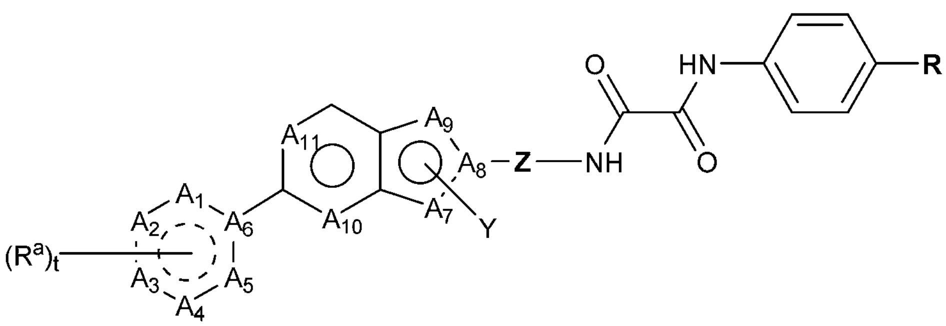

(I)

or a pharmaceutically acceptable salt, isomer, solvate, hydrate or stable isotopic variant thereof, wherein:

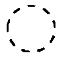

represents a saturated, partially unsaturated, or aromatic ring system;

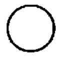

represents an aromatic ring system;

$A_1$, $A_2$, $A_3$, $A_4$, and $A_5$ are each independently selected from absence, C, N, O, and S, and $A_6$ is selected from C and N, provided that (1) at most two of $A_1$~$A_5$ are absent, and (2) at most four of $A_1$~A6 are heteroatoms;

$R^a$ is selected from-H, =O, =S, =N-OH, -OH, $-NH_2$, halogen, -CN and $-C_{1-6}$ alkyl optionally substituted by halogen;

$A_7$, $A_8$ and $A_9$ are each independently selected from C, N, O, and S, provided that at least one of $A_7$, $A_8$ and $A_9$ is not C;

$A_{10}$ is selected from C-$R^b$ and N;

$A_{11}$ is selected from C-X and N;

$R^b$ is selected from H, halogen, CN and $-C_{1-6}$ alkyl substituted by halogen;

X is selected from H, halogen, CN, -OH, $-OC_{1-6}$ alkyl and $-C_{1-6}$ alkyl, wherein the $C_{1-6}$ alkyl is optionally substituted;

Y is an optionally substituted group selected from $-C_{1-6}$ alkyl, $-(CH_2)_t$-3-15 membered carbocyclyl, $-(CH_2)_t-C_{6-10}$ aryl, $-(CH_2)_t$-5-12 membered heteroaryl having one or more heteroatom(s) independently selected from N, O and S, and $-(CH_2)_t$-3-15 membered heterocyclyl having one or more heteroatom(s) independently selected from N, O and S;

Z is an optionally substituted divalent group selected from $-C_{6-10}$ aryl-, -3-10 membered carbocyclyl-, -5-12 membered heteroaryl having one or more heteroatom(s) independently selected from N, O and S-, and -3-15 membered heterocyclyl having one or more heteroatom(s) independently selected from N, O and S-;

R is an optionally substituted group selected from $-C_{6-10}$ aryl, -3-15 membered carbocyclyl, -5-12 membered heteroaryl having one or more heteroatom(s) independently selected from N, O and S, and -3-15 membered heterocyclyl having one or more heteroatom(s) independently selected from N, O and S; and

t is each independently an integer from 0 to 3.

**In** a further embodiment of the present disclosure, in the compound of formula (I), Y is an optionally substituted group selected from:

- $C_{1-6}$ alkyl,
- $(CH_2)_t$-3-8 membered monocyclic saturated or partially unsaturated carbocycle,
- $(CH_2)_t$-5-10 membered bridged saturated or partially unsaturated carbocycle,
- $(CH_2)_t$-6-15 membered spiro or fused saturated or partially unsaturated carbocycle,
- $(CH2)_t-C_{6-10}$ aryl,
- $(CH_2)_t$-5-12 membered heteroaryl having 1-4 heteroatoms independently selected from N, O, and S,
- $(CH_2)_t$-3-8 membered monocyclic saturated or partially unsaturated heterocycle having 1 to 3 heteroatoms independently selected from N, O and S;
- $(CH_2)_t$-5-10 membered bridged saturated or partially unsaturated heterocycle having 1-3 heteroatoms independently

selected from N, O and S;

- $(CH_2)_t$-6-15 membered spiro or fused saturated or partially unsaturated heterocycle having 1-4 heteroatoms independently selected from N, O and S.

**In a further embodiment of the present disclosure,** in the compound of formula (I), Z is an optionally substituted divalent group selected from:

- $C_{6-10}$ aryl-
- 3-8 membered monocyclic saturated or partially unsaturated carbocycle-,
- 5-10 membered bridged saturated or partially unsaturated carbocycle-,
  5-12 membered heteroaryl having 1-4 heteroatoms independently selected from N, O, and S-,
- 3-8 membered monocyclic saturated or partially unsaturated heterocycle having 1-3 heteroatoms independently selected from N, O, and S-,
- 5-10 membered bridged saturated or partially unsaturated heterocycle having 1-3 heteroatoms independently selected from N, O, and S-,
- 6-15 membered spiro or fused saturated or partially unsaturated heterocycle having 1-4 heteroatoms independently selected from N, O and S-.

**In a further embodiment of the present disclosure,** in the compound of formula (I), R is an optionally substituted group selected from:

- $C_{6-10}$ aryl,
- 3-8 membered monocyclic saturated or partially unsaturated carbocycle,
- 5-10 membered bridged saturated or partially unsaturated carbocycle,
- 6-15 membered spiro or fused saturated or partially unsaturated carbocycle,
- 5-12 membered heteroaryl having 1-4 heteroatoms independently selected from N, O, and S,
- 3-8 membered monocyclic saturated or partially unsaturated heterocycle having 1-3 heteroatoms independently selected from N, O, and S,
- 5-10 membered bridged saturated or partially unsaturated heterocycle with 1-3 heteroatoms independently selected from N, O, and S,
- 6-15 membered spiro or fused saturated or partially unsaturated heterocycle having 1-4 heteroatoms independently selected from N, O and S.

**In some embodiments of the compound of formula (I),** the cyclic heteroatom

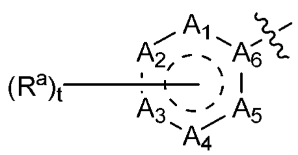

is a five-or six-membered saturated, partially unsaturated, or aromatic group, optionally substituted by 0-3 $R^a$, having 1-4 cyclic heteroatoms selected from N, O, and S; preferably a five-membered partially unsaturated or aromatic group, optionally substituted by 0-3 $R^a$, having 2-4 cyclic heteroatoms selected from N, O, and S; more preferably a five-membered partially unsaturated or aromatic group, optionally substituted by 0-1 $R^a$, having 3-4 cyclic heteroatoms

selected from N, O, and S; and most preferably a five-membered aromatic group, optionally substituted by 0-1 $R^a$, having 3-4 nitrogen heterocyclic atoms.

**In some embodiments of the compound of formula (I),** $A_6$ is C.

**In some embodiments of the compound of formula (I),** $R^a$ is H; in some embodiments, $R^a$ is =O or =S; in some embodiments, $R^a$ is -OH or -$NH_2$; in some embodiments, $R^a$ is halogen, -CN, or optionally -$C_{1-6}$ alkyl substituted by halogen. In some embodiments, $R^a$ is selected from -H, =O, =S, -OH, -$NH_2$, -CN, and -$C_{1-6}$ alkyl optionally substituted by halogen, and further selected from -H, - CN, halogen, =O, and -$C_{1-6}$ alkyl substituted by halogen. In some embodiments, $R^a$ is substituted on a ring carbon atom.

**In some embodiments of the compound of formula (I),** the ring formed by $A_1$-$A_6$ is triazolyl and $A_6$ is C, for example

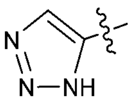 or 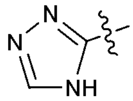 ,

substituted by 1 $R^a$, said $R^a$ is selected from H, halogen, CN and -$C_{1-6}$ alkyl optionally substituted by halogen, preferably selected from halogen, CN and -$C_{1-6}$ alkyl optionally substituted by halogen, most preferably -$C_{1-6}$ alkyl substituted by halogen, for example -$CF_3$.

**In some embodiments of the compound of formula (I),** the ring formed by $A_1$-$A_6$ is tetrazolyl

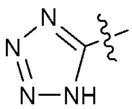 .

**In some embodiments of the compound of formula (I),** the ring formed by $A_1$-$A_6$ is a five-membered partially unsaturated ring having 3-4 cyclic heteroatoms selected from N, O and S, for example, but not limited to

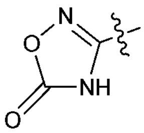 , 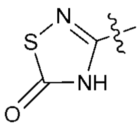 , 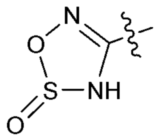 ,

preferably

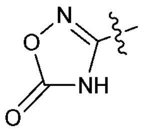 .

In this embodiment, exemplary

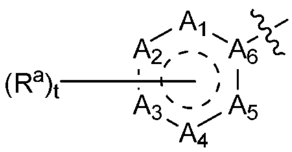

includes but is not limited to

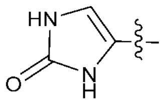 ,

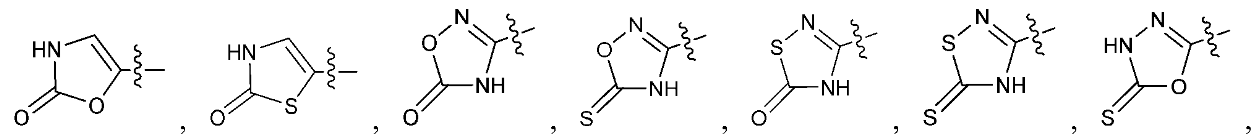

, , , , , , ,

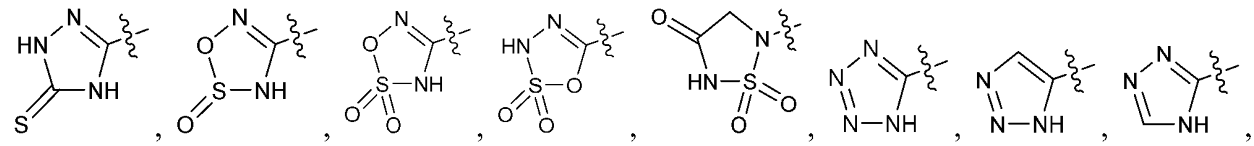

, , , , , , , ,

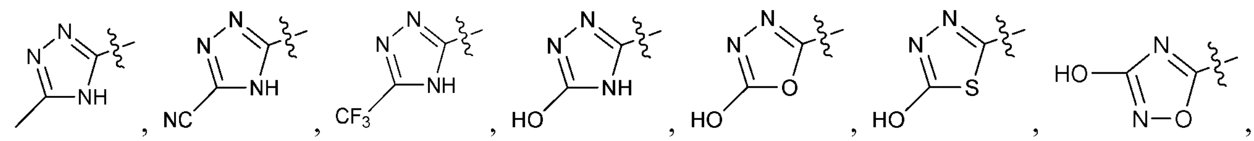

, , , , , , ,

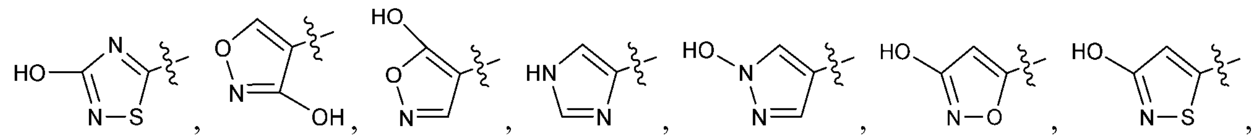

, , , , , , ,

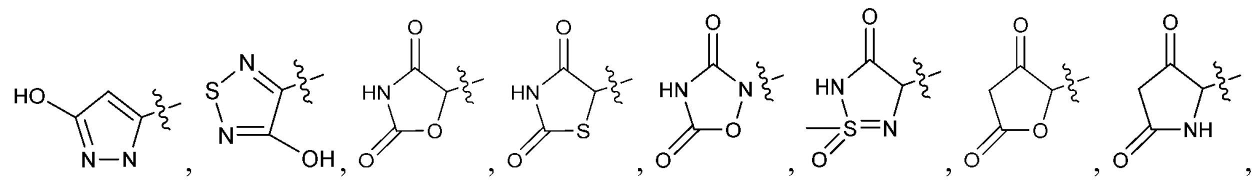

, , , , , , , ,

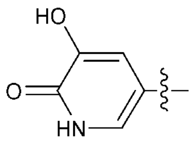
,
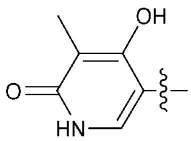
,
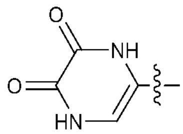
,
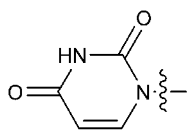
,
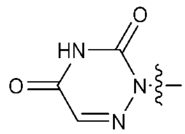
;

preferably

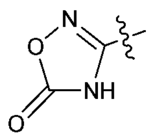
,

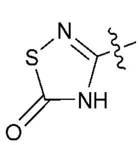
,
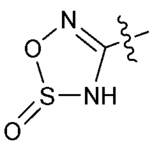
,
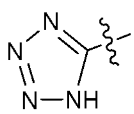
,
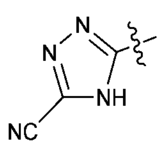
,
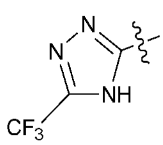
;

more preferably

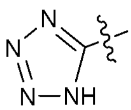
。

**In some embodiments of the compound of formula (I),**

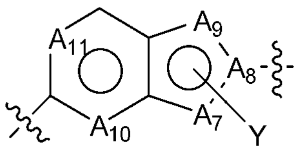

is a fused heteroaromatic ring substituted by Y and having 1-3 heterocyclic atoms selected from N, O and S.

In some embodiments, $A_{10}$ is C-$R^b$ and $A_{11}$ is C-X; in some embodiments, one of $A_{10}$ and $A_{11}$ is a nitrogen heteroatom and the other is a carbon atom, for example, $A_{10}$ is N and $A_{11}$ is C-X; in some embodiments, both $A_{10}$ and $A_{11}$ are nitrogen atoms.

In some embodiments, one of $A_7$ and $A_9$ is a heteroatom selected from N, O and S, and the other is a carbon atom, and $A_8$ is a carbon atom; preferably $A_7$ is selected from C-Y and N-Y, $A_9$ is selected from C, O and S, and only one of $A_7$ and $A_9$ is a heteroatom, and $A_8$ is a carbon atom; further more preferably Y is attached to the ring atom of $A_7$.

In this embodiment, exemplary

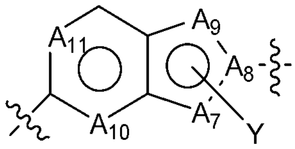

includes, but is not limited to

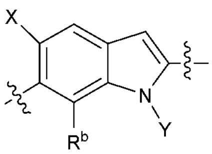 , 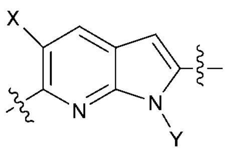 , 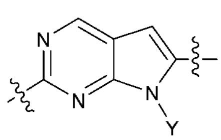 , 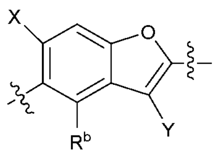 , 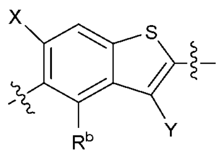 ,

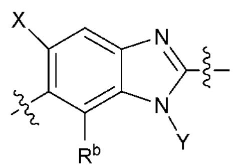 ;

preferably

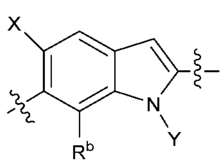 , 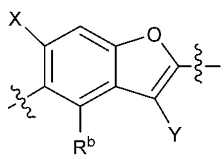 , 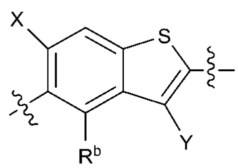 ,

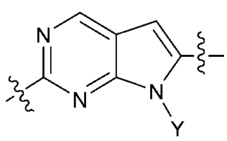 , 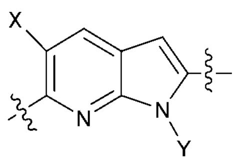 ;

more preferably

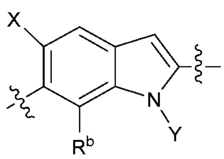 .

In some embodiments of the compound of formula (I), $R^b$ is H; in some embodiments, $R^b$ is halogen, preferably F; in some embodiments, $R^b$ is CN; in some embodiments, $R^b$ is a $C_{1-6}$ alkyl optionally substituted by halogen, such as $-CH_2F$, $-CH_2Cl$, $-CHF_2$, $-CF_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C_2F_5$, $-CF(CF_3)_2$, preferably $-CF_3$.

**In some embodiments of the compound of formula (I)**, X is H.

In some embodiments of the compound of formula (I), X is halogen selected from F, Cl, Br, and I; preferably F.

In some embodiments of the compound of formula (I), X is CN.

In some embodiments of the compound of formula (I), X is -OH.

In some embodiments of compounds of formula (I), X is $-OC_{1-6}$ alkyl, optionally substituted by halogen or CN; for example, but not limited to $-O-CH_3$, $-O-CH_2CH_3$, $-O-CH_2CH_2CH_3$, $-O-CH(CH_3)_2$, $-O-CH_2F$, $-O-CH_2Cl$, $-O-CHF_2$, $-O-CF_3$, $-O-CH_2CH_2F$, $-O-CH_2CHF_2$, $-O-CH_2CF_3$, $-O-CH_2CN$, $-O-CH_2CH_2CN$.

In some embodiments of compounds of formula (I), X is $-C_{1-6}$ alkyl, optionally substituted by halogen or CN; for example, but not limited to $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2-CN$, $-CH_2CH_2-CN$, $-CH_2F$, $-CH_2Cl$, $-CHF_2$, $-CF_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C_2F_5$, $-CF(CF_3)_2$.

In a preferred embodiment, X is selected from H, halogen, and -OH, more preferably F.

**In some embodiments of the compound of formula (I),** Y is $-C_{1-6}$ alkyl, optionally substituted by halogen or CN and wherein any carbon atom is optionally substituted by NR', O or S, preferably $-C_{1-6}$ alkyl substituted by halogen or CN, more preferably $-C_{1-6}$ alkyl substituted by halogen; for example, but not limited to $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2-CN$, $-CH_2CH_2-CN$, $-CH_2F$, $-CH_2Cl$, $-CHF_2$, $-CF_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C_2F_5$, $-CF(CF_3)_2$, $-CH_2-OCH_3$, $-CH_2-O-CH_2CH_3$, $-CH_2CH_2-O-CH_3$, $-CH_2CH_2-O-CH_2CH_3$, $-CH_2-SCH_3$, $-CH_2-S-CH_2CH_3$, $-CH_2CH_2-S-CH_3$, $-CH_2CH_2-S-CH_2CH_3$, $-CH_2-NH-CH_3$, $-CH_2-N(CH_3)-CH_3$, $-CH_2-NH-CH_2CH_3$, $-CH_2CH_2-NH-CH_3$, $-CH_2CH_2-NH-CH_2CH_3$, $-CH_2CH_2-N(CH_3)-CH_2CH_3$.

In some embodiments of the compound of formula (I), Y is a $-(CH_2)_t$-3-15 membered carbocyclyl, specifically a $-(CH_2)_t$-3-8 membered monocyclic saturated or partially unsaturated carbocycle, more specifically a $-(CH_2)_t$-3-6 membered monocyclic saturated carbocycle, optionally substituted by $-C_{1-6}$ alkyl, -OH or $-OC_{1-6}$ alkyl, wherein t is preferably 0 or 1, wherein the $-C_{1-6}$ alkyl is optionally substituted by halogen or CN; for example, but not limited to

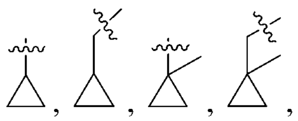

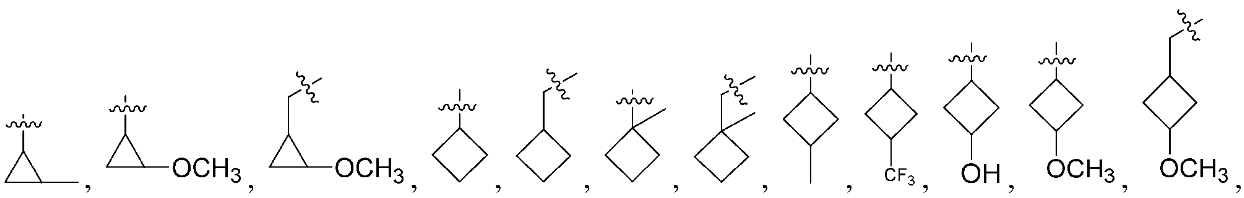

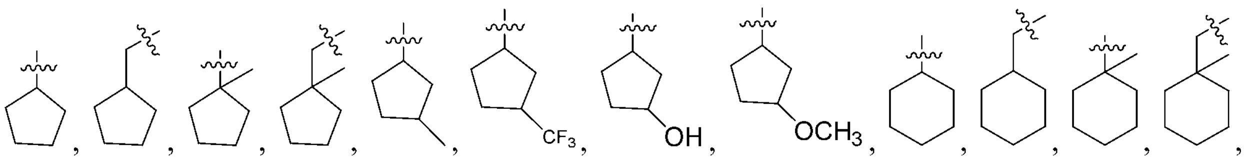

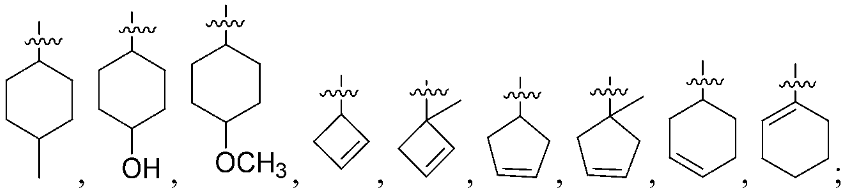;

preferably

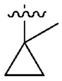, 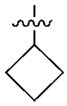 and 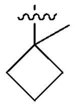.

In some embodiments of the compound of formula (I), Y is a $-(CH_2)_t$-3-15 membered carbocyclyl, specifically a $-(CH_2)_t$-5-10 membered bridged saturated or partially unsaturated carbocycle, more specifically a $-(CH_2)_t$-5-8 membered bicyclic bridged saturated carbocycle, optionally substituted by $-C_{1-6}$ alkyl, -OH or $-OC_{1-6}$ alkyl, wherein t is preferably 0 or 1, wherein the $-C_{1-6}$ alkyl is optionally substituted by halogen or CN; wherein the bridged carbocycle is, for example, but not limited to

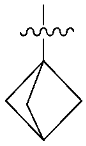, 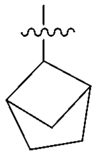,, 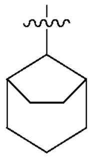, 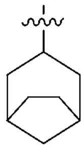, 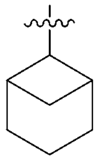, 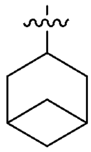, 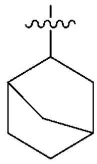, 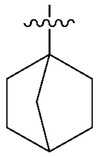.

In some embodiments of the compound of formula (I), Y is a $-(CH_2)_t$-3-15 membered carbocyclyl, specifically a $-(CH_2)_t$-6-15 membered spiro or fused saturated or partially unsaturated carbocycle, more specifically a $-(CH_2)_t$-7-11 membered bicyclic spirocyclic or fused saturated carbocycle, optionally substituted by $-C_{1-6}$ alkyl, -OH or $-OC_{1-6}$ alkyl, wherein t is preferably 0 or 1, wherein the $-C_{1-6}$ alkyl is optionally substituted by halogen or CN; the group may also be represented as

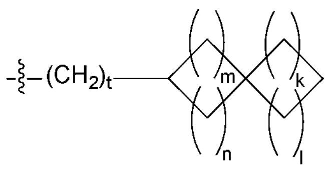 or 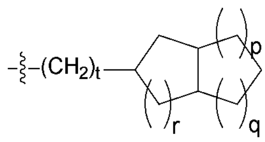,

wherein k is an integer from 0-2, m and n are each independently an integer from 1-3, l is an integer from 1-4, r and p are each independently an integer from 0-3, q is an integer from 1-4, optionally containing one or more double bond(s) (non-aromatic), and optionally substituted by $-C_{1-6}$ alkyl, -OH or $-OC_{1-6}$ alkyl; in this embodiment, the carbocycle of Y is more specifically -7-11 membered bicyclic spiro saturated carbocycle (wherein k is 0-2, m and n are each independently an integer from 1-2, and l is an integer from 1-3) or -7-11 membered bicyclic fused saturated carbocycle (wherein r is an integer from 0-1, p is an integer from 0-2, and q is an integer from 1 to 3); wherein the spiro and fused carbocycle are, for example but not limited to

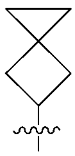, 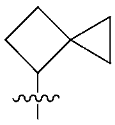, 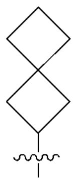, 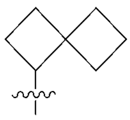, 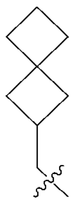, 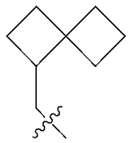, 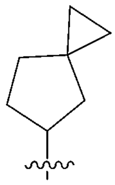, 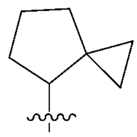, 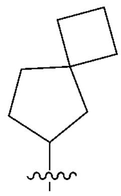,

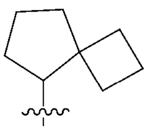, 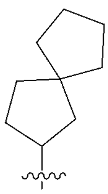, 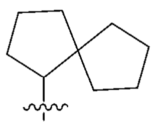, 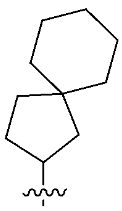, 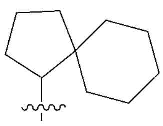, 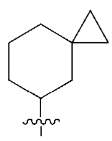, 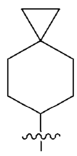, 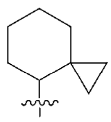, 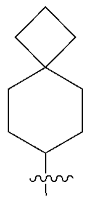,

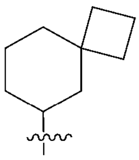, 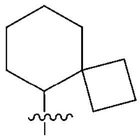, 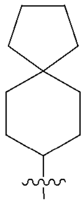, 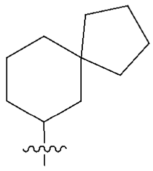, 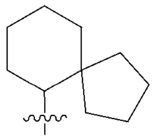, 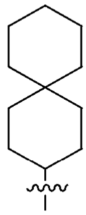, 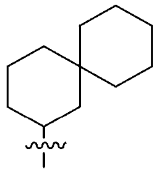, 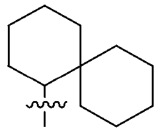,

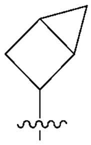, 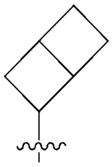, 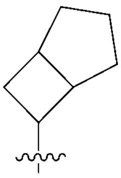, 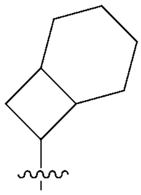, 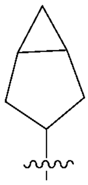, 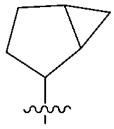, 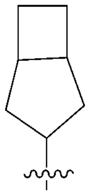, 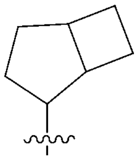, 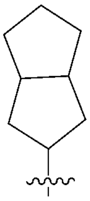,

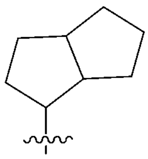, 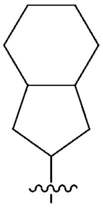, 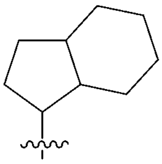, 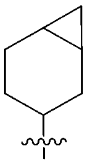, 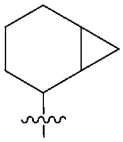, 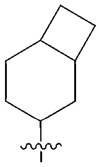, 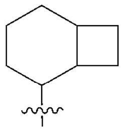, 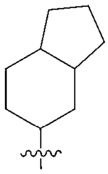, 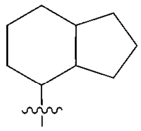,

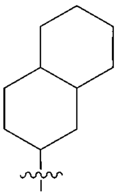, 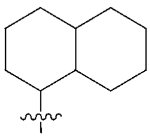, 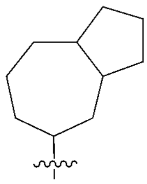,

or the exemplary groups having one or two carbon-carbon double bond(s).

In some embodiments of the compound of formula (I), Y is $-(CH_2)_t-C_{6-10}$ aryl, specifically - $(CH_2)_t$-phenyl, optionally substituted by $-C_{1-6}$ alkyl, -OH or $-OC_{1-6}$ alkyl, wherein t is preferably 0 or 1, wherein $-C_{1-6}$ alkyl is optionally substituted by halogen or CN; for example, but not limited to

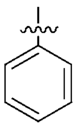, 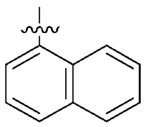, 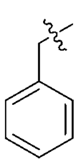, 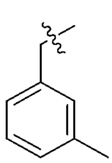, 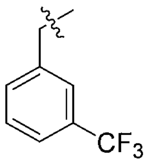, 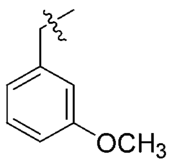,

preferably

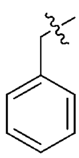.

In some embodiments of the compound of formula (I), Y is a $-(CH_2)_t$-5-12 membered heteroaryl having one or more heteroatom(s) independently selected from N, O, and S, specifically a $-(CH_2)_t$-5-12 membered monocyclic or bicyclic heteroaryl having 1-4 heteroatoms independently selected from N, O, and S, more specifically a $-(CH_2)_t$-5-6 membered monocyclic or bicyclic heteroaryl having 1-4 heteroatoms independently selected from N, O, and S, optionally substituted by $-C_{1-6}$ alkyl, -OH, or - $OC_{1-6}$ alkyl, wherein t is preferably 0 or 1, wherein the $-C_{1-6}$ alkyl is optionally substituted by halogen or CN; wherein the heteroaryl includes, but is not limited to, furanyl, pyridinyl, pyridazinyl, pyrimidinyl, pyrazinyl, thienyl,

isoxazolyl, oxazolyl, oxadiazolyl, imidazolyl, pyrroleyl, pyrazolyl, triazolyl, tetrazolyl, thiazolyl, isothiazolyl, 1,2,3-thiadiazolyl, benzodioxolyl, benzothienyl, benzimidazolyl, indolyl, isoindolyl, 1,3-dioxo-isoindolyl, quinolinyl, indazolyl, benzisothiazolyl, benzoxazolyl, benzisoxazolyl,

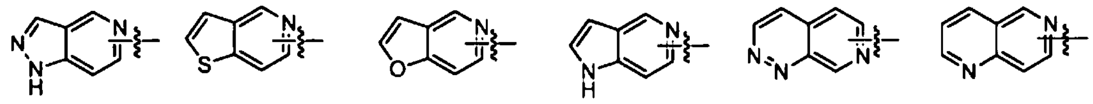

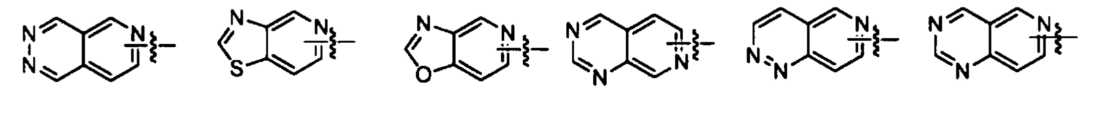

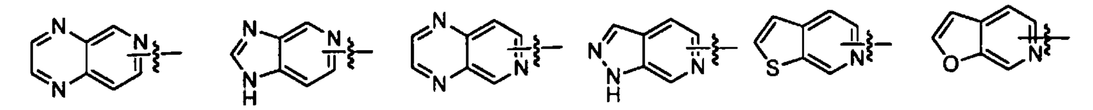

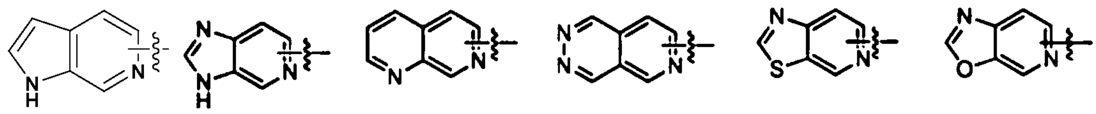

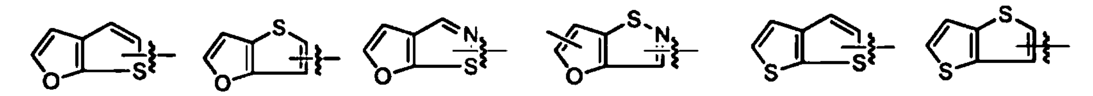

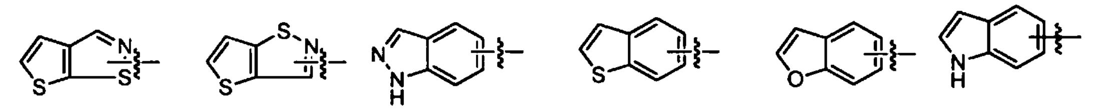

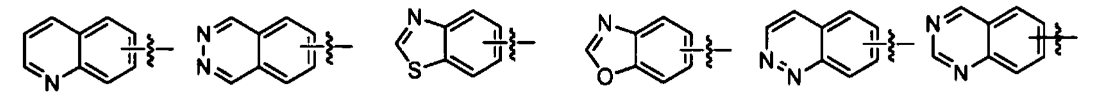

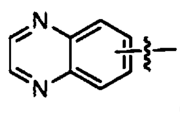 or 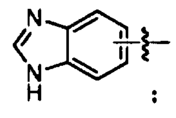 ;

and wherein the heteroaryl can be attached to the rest of the molecule at a heteroatom or a carbon atom of the heteroaryl.

In some embodiments of the compound of formula (I), Y is a 3-15 membered heterocyclyl having one or more heteroatom(s) independently selected from N, O, and S, specifically a 3-8 membered monocyclic saturated or partially unsaturated heterocyclyl having1-3 heteroatom(s) independently selected from N, O, and S, more specifically a 4-7 membered monocyclic saturated or partially unsaturated heterocyclyl having 1-2 heteroatom(s) independently selected from N, O, and S, optionally substituted by -$C_{1-6}$ alkyl, -OH, or -$OC_{1-6}$ alkyl, wherein t is preferably 0 or 1, wherein - $C_{1-6}$ alkyl is optionally substituted by halogen or CN; wherein the heterocyclyl is, for example, but not limited to, azetidinyl, oxetanyl, thietanyl, pyrrolidinyl (e.g., 1-pyrrolidinyl, 2-pyrrolidinyl, and 3-pyrrolidinyl), tetrahydrofuryl (e.g., 1-tetrahydrofuryl, 2-tetrahydrofuryl, and 3-tetrahydrofuryl), tetrahydrothienyl (e.g., 1-tetrahydrothienyl, 2-tetrahydrothienyl and 3-tetrahydrothienyl), piperidinyl (e.g., 1-piperidinyl, 2-piperidinyl, 3-piperidinyl and 4-piperidinyl), tetrahydropyranyl (e.g., 4-tetrahydropyranyl), tetrahydrothianyl (e.g., 4-tetrahydrothianyl), morpholinyl (e.g., morpholino), thiomorpholinyl, dioxanyl, piperazinyl or azepanyl, diazepanyl e.g., 1,4-diazepanyl, and pyrrololinyl (e.g., 1-pyrrololinyl, 2-pyrrolylyl, 3-pyrrololinyl, 4-pyrrololinyl, or 5-pyrrololinyl), dihydrofuranyl (e.g., 1-dihydrofuranyl, 2-dihydrofuranyl, 3-dihydrofuranyl, 4-dihydrofuranyl or 5-dihydrofuranyl), dihydrothienyl (e.g., 1-dihydrothienyl, 2-dihydrothienyl, 3-dihydrothienyl or 4-dihydrothienyl), tetrahydropyridyl (e.g., 1-, 2-, 3-, 4-, 5-or 6-tetrahydropyridyl), oxacyclohexenyl, dihydropyranyl (e.g., 4-dihydropyranyl) or dihydrothiopyranyl (e.g., 4-dihydrothiopyranyl), wherein the heterocyclyl can be attached to the rest of the molecule at a heteroatom or a carbon atom of the heterocycle, wherein the substituent can substitute on a ring carbon atom or a ring

heteroatom, as long as it is chemically feasible.

In some embodiments of the compound of formula (I), Y is a $-(CH_2)_t$-3-15 membered heterocyclyl having one or more heteroatom(s) independently selected from N, O, and S, specifically a $-(CH_2)_t$-5-10 membered bridged saturated or partially unsaturated heterocycle having 1-3 heteroatom(s) independently selected from N, O, and S, more specifically a $-(CH_2)_t$-5-8 membered bridged saturated or partially unsaturated heterocycle having 1-2 heteroatom(s) independently selected from N, O, and S, optionally substituted by $-C_{1-6}$ alkyl, -OH, or $-OC_{1-6}$ alkyl, wherein t is preferably 0 or 1, wherein the $-C_{1-6}$ alkyl is optionally substituted by halogen or CN; wherein the bridged heterocycle is, for example, but not limited to

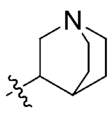, 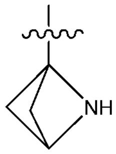, 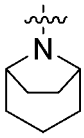, 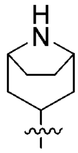, 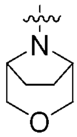,

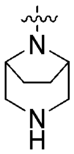, 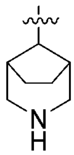, 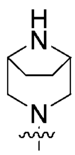, 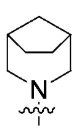, 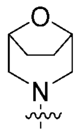, 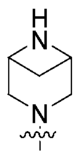, 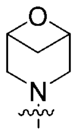, 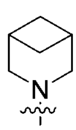, 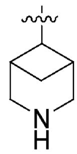, 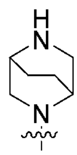, 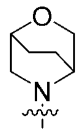, 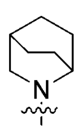, 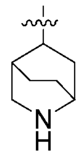, 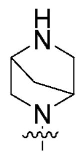,

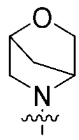, 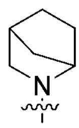, 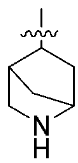, 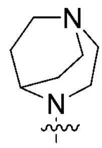,

wherein the heterocyclyl can be attached to the rest of the molecule at a heteroatom or a carbon atom of the heterocycle, and the substituents on it can substitute a ring carbon atom or a ring heteroatom, as long as it is chemically feasible.

In some embodiments of the compound of formula (I), Y is a $-(CH_2)_t$-3-15 membered heterocyclyl having one or more heteroatom(s) independently selected from N, O, and S, specifically a $-(CH_2)_t$-6-15 membered spiro or fused saturated or partially unsaturated heterocycle having 1-4 heteroatoms independently selected from N, O, and S, more specifically a $-(CH_2)_t$-7-11 membered bicyclic spiro or fused saturated heterocycle having 1-3 heteroatom(s) independently selected from N, O, and S, optionally substituted by $-C_{1-6}$ alkyl, -OH, or $-OC_{1-6}$ alkyl, wherein t is preferably 0 or 1, wherein the $-C_{1-6}$ alkyl is optionally substituted by halogen or CN; the group may also be represented as

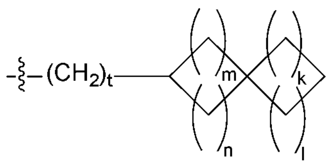 or 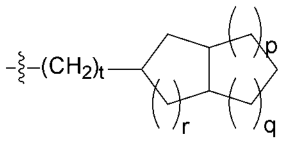,

wherein 1-4 ring atoms, preferably 1-3, more preferably 1-2 ring atoms, can be replaced by heteroatoms independently selected from N, O, and S, k is an integer from 0-2, m, n is an integer from 1-3, 1 is an integer from 1-4, r and p are each independently an integer from 0-3, q is an integer from 1-4, optionally having one or more double bond(s) (non-aromatic), and optionally substituted by $-C_{1-6}$ alkyl, -OH or $-OC_{1-6}$ alkyl, wherein the-$C_{1-6}$ alkyl is optionally substituted by halogen or CN; the heterocycle in Y in this embodiment is more specifically -7-11 membered bicyclic spiro saturated heterocycle (where k is 0-2, m and n are each an integer from 1-2, and 1 is an integer from 1-3) or -7-11 membered bicyclic fused saturated heterocycle (where r is an integer from 0-1, p is an integer from 0-2 and q is an integer from 1-3), wherein t is preferably 0 or 1; wherein the spiro heterocycle and fused heterocycle are, for example but not limited to

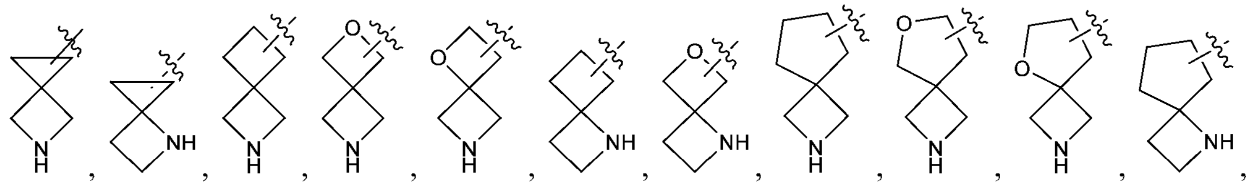

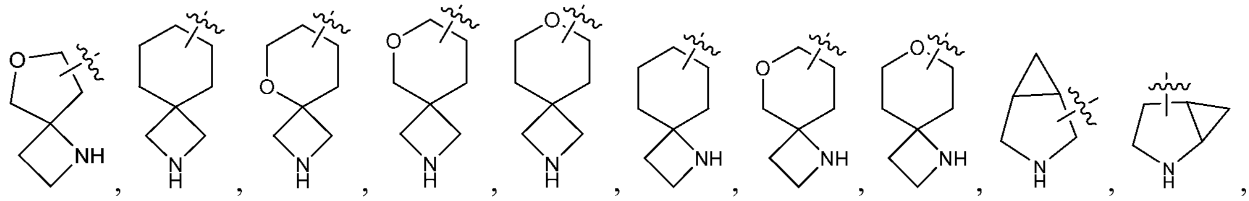

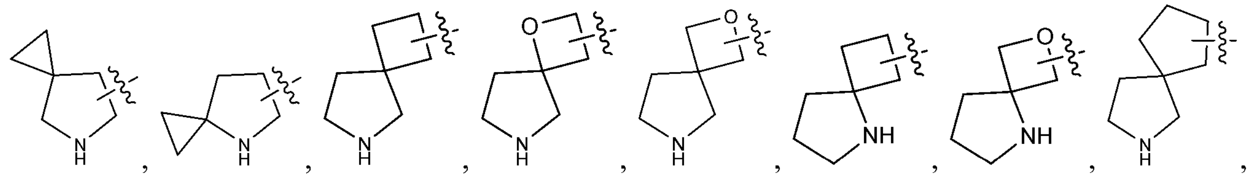

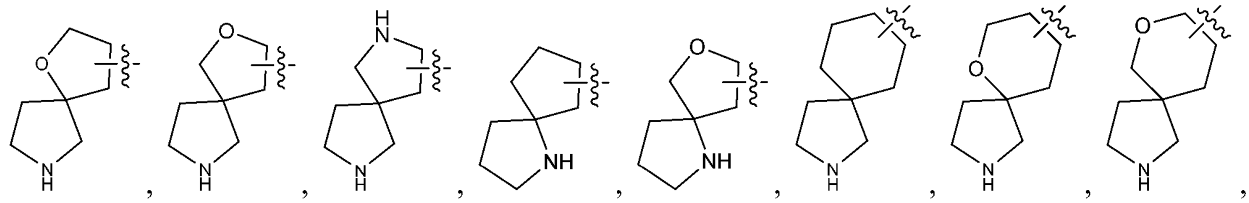

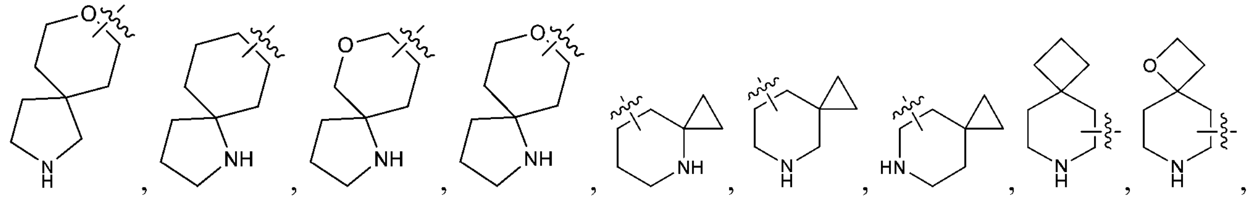

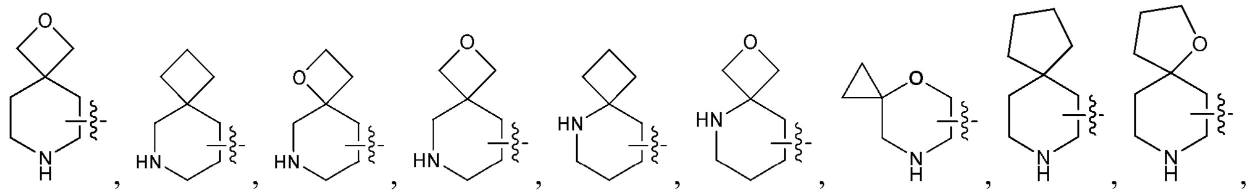

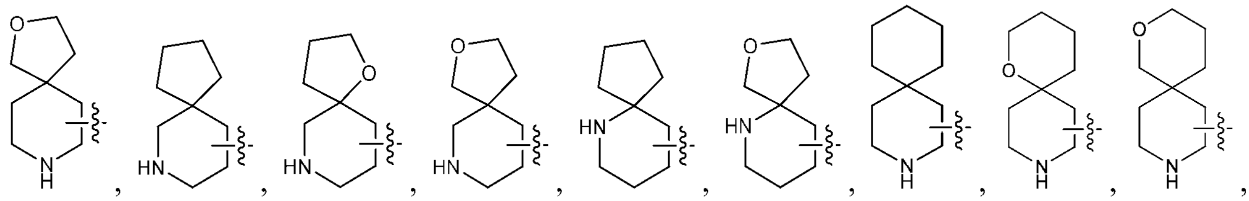

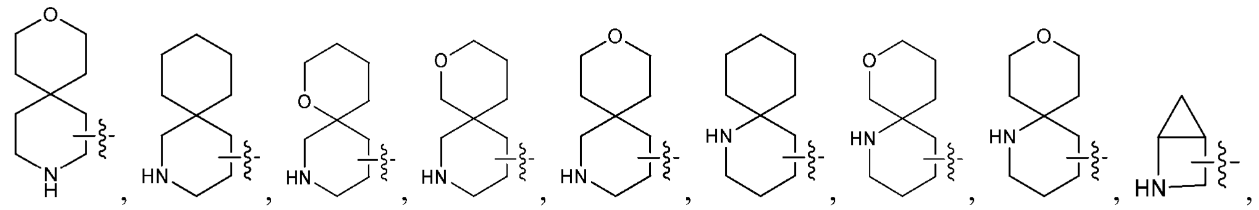

, , , , , , , , ,

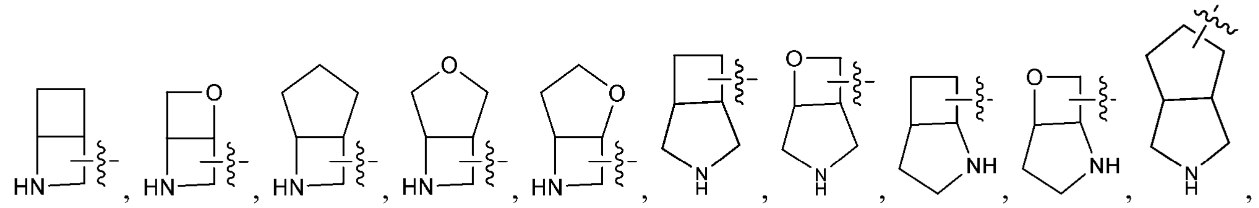

, , , , , , , , ,

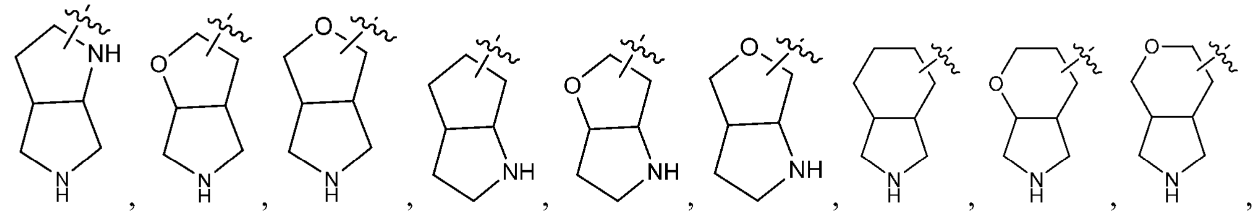

, , , , , , , , ,

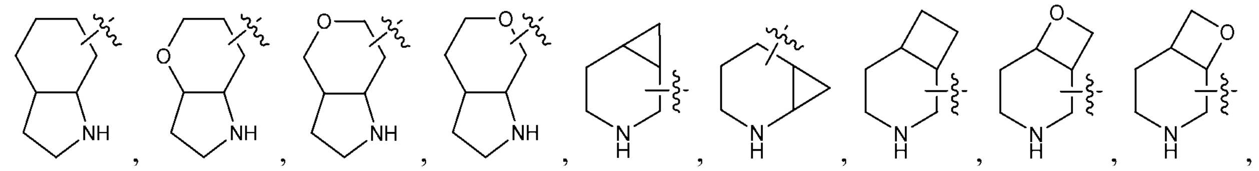

, , , , , , , , ,

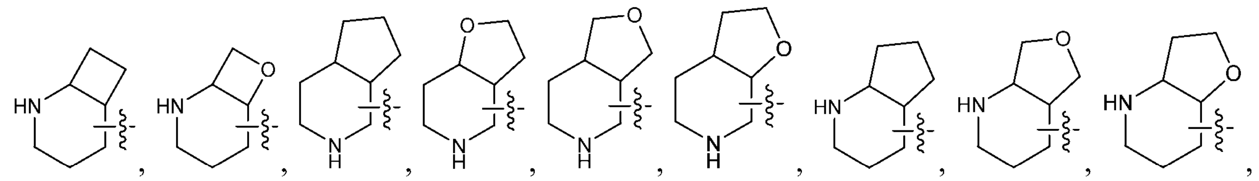

, , , , , , , , ,

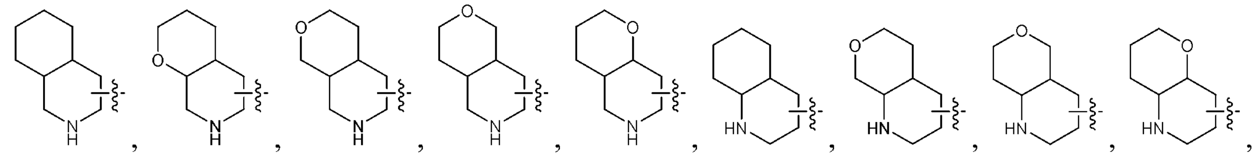

, , , , , , , , ,

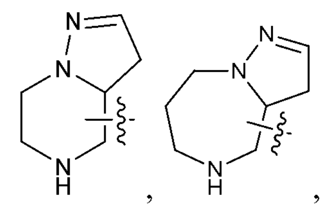

, ,

and wherein the heterocyclyl can be attached to the rest of the molecule at any feasible heteroatom or carbon atom of the entire heterocycle, and the substituents on it can substitute a ring carbon atom or a ring heteroatom, as long as it is chemically feasible.

In embodiments of the compound of formula (I), Y is an optionally substituted group selected from: $-C_{1-6}$ alkyl, $-(CH_2)_t$-3-15 membered carbocycle and $-(CH_2)_t-C_{6-10}$ aryl; more preferably Y is an optionally substituted group selected from: $-C_{1-6}$ alkyl, $-(CH_2)_t$-3-8 membered monocyclic saturated or partially unsaturated carbocycle, $-(CH_2)_t$-5-10 mem-

bered bridged saturated or partially unsaturated carbocycle and $-(CH_2)_t-C_{6-10}$ aryl; further more preferably Y is an optionally substituted group selected from: $-C_{1-6}$ alkyl, $-(CH_2)_t$-3-6 membered monocyclic saturated carbocycle, $-(CH_2)_t$-5-8 membered bicyclic bridged saturated carbocycle and $-(CH_2)_t$-phenyl.

In embodiments of the compound of formula (I), t in Y is preferably 0-2, more preferably 0-1.

In embodiments of the compound of formula (I), each cyclic group of Y is optionally substituted by one or more group(s) independently selected from $-C_{1-6}$ alkyl, -OH or $-OC_{1-6}$ alkyl, wherein the - $C_{1-6}$ alkyl or $-(CH_2)_t-$ appearing in Y and its substituents is optionally substituted by halogen or CN and wherein any carbon chain atom is optionally substituted by NR', O or S, wherein R' is selected from H or $-C_{1-6}$ alkyl; preferably the $-C_{1-6}$ alkyl of Y is optionally substituted by halogen or CN, and the cyclic group of Y is optionally substituted by 1-3, preferably 1 or 2, more preferably 1 group selected from $-C_{1-6}$ alkyl and $-OC_{1-6}$ alkyl, wherein non-limiting examples of $-C_{1-6}$ alkyl and $-OC_{1-6}$ alkyl are as defined above correspondingly for the substituent X. In embodiments where Y is a-$(CH_2)_t$-3-15 membered carbocyclyl, when t is 0 and the 3-15 membered carbocycle is a 3-6 membered monocyclic saturated carbocycle, the carbocycle optionally carries a substituent $C_{1-6}$ alkyl, preferably when present, the $C_{1-6}$ alkyl is attached to the ring carbon atom of the carbocycle that is attached to the rest of the molecule.

**In some embodiments of the compound of formula (I),** Z is $-C_{6-10}$ aryl-, optionally substituted by halogen, $-C_{1-6}$ alkyl or $-OC_{1-6}$ alkyl, wherein the $-C_{1-6}$ alkyl is optionally substituted by halogen or CN; for example, but not limited to

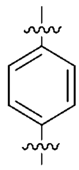 , 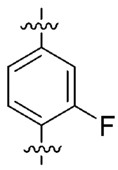 , 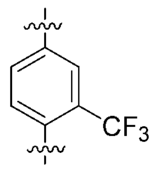 , 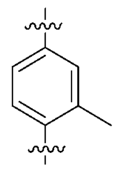 , 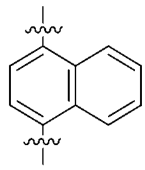 ,

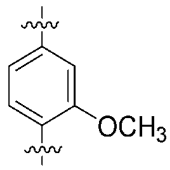 ,

preferably

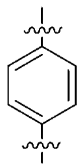 .

In some embodiments of the compound of formula (I), Z is a -3-10 membered carbocyclyl, specifically a -3-8 membered monocyclic saturated or partially unsaturated carbocycle-, more specifically a -3-6 membered monocyclic saturated or partially unsaturated carbocycle, optionally substituted by halogen, $-C_{1-6}$ alkyl or an-$OC_{1-6}$ alkyl, wherein the $-C_{1-6}$ alkyl is optionally substituted by halogen or CN; for example, but not limited to,

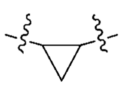 , 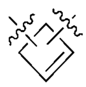 , 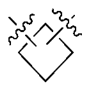 , 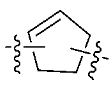 , 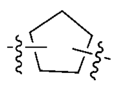 ,

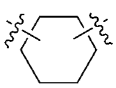 , 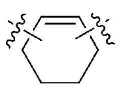 , 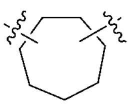 , 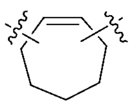 , 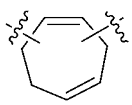 .

It should be noted that the divalent attachment points in these carbocycle to the rest of the molecule are merely exemplary and the carbocycle may be attached to the rest of the molecule at any chemically feasible group.

In some embodiments of the compound of formula (I), Z is a -3-10 membered carbocyclyl, specifically a -5-10 membered bridged saturated or partially unsaturated carbocycle-, more specifically a -5-8 membered bicyclic bridged

saturated carbocycle, optionally substituted by halogen, $-C_{1-6}$ alkyl or $-OC_{1-6}$ alkyl, wherein the $-C_{1-6}$ alkyl is optionally substituted by halogen or CN; wherein the bridged carbocycle is, for example, but not limited to

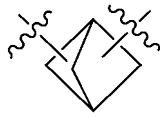 , 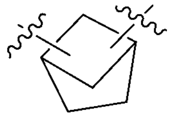 , 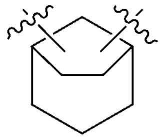 ,

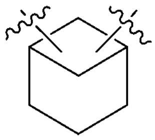 , 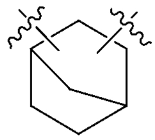 .

In some embodiments of compounds of formula (I), Z is a 5-12 membered heteroaryl having one or more heteroatom(s) independently selected from N, O, and S-, specifically a 5-12 membered monocyclic or bicyclic heteroaryl having 1-4 heteroatoms independently selected from N, O, and S, more specifically a 5-6 membered monocyclic or bicyclic heteroaryl having 1-4 heteroatoms independently selected from N, O, and S, optionally substituted by halogen, $-C_{1-6}$ alkyl, or $-OC_{1-6}$ alkyl, wherein the $-C_{1-6}$ alkyl is optionally substituted by halogen or CN; examples of the heteroaryl are shown above for Y as "a 5-6 membered monocyclic or 8-10 membered bicyclic heteroaryl having 1-4 heteroatoms independently selected from N, O, and S". The difference is that it is a divalent group, and wherein the heteroaryl and the rest of the molecule can be attached at a heteroatom or a carbon atom of the heteroaryl, and the substituent on it can be substituted at a ring carbon atom or a ring heteroatom, as long as it is chemically feasible.

In some embodiments of compounds of formula (I), Z is a divalent 3-15 membered heterocyclyl having one or more heteroatom(s) independently selected from N, O, and S, specifically a -3-8 membered monocyclic saturated or partially unsaturated heterocycle having 1-3 heteroatoms independently selected from N, O, and S-, more specifically a -4-7 membered monocyclic saturated or partially unsaturated heterocycle having 1-2 heteroatoms independently selected from N, O, and S-, optionally substituted by halogen, $-C_{1-6}$ alkyl, or $-OC_{1-6}$ alkyl, wherein the $-C_{1-6}$ alkyl is optionally substituted by halogen or CN; wherein examples of the heterocycle are shown above for Y as "a 3-8 membered monocyclic saturated or partially unsaturated heterocycle having 1-3 heteroatoms independently selected from N, O, and S". The difference is that it is a divalent group, and wherein the heterocyclyl and the rest of the molecule can be attached at a heteroatom or a carbon atom of the heterocycle, and on it can be substituted at a ring carbon atom or a ring heteroatom, as long as it is chemically feasible.

In some embodiments of compounds of formula (I), Z is a 3-15 membered heterocyclyl having one or more heteroatom(s) independently selected from N, O, and S, specifically a 5-10 membered bridged saturated or partially unsaturated heterocycle having 1-3 heteroatoms independently selected from N, O, and S, more specifically a 5-8 membered bridged saturated or partially unsaturated heterocycle having 1-2 heteroatoms independently selected from N, O, and S, optionally substituted by halogen, $-C_{1-6}$ alkyl, or $-OC_{1-6}$ alkyl, wherein the $-C_{1-6}$ alkyl is optionally substituted by halogen or CN; wherein examples of the bridged heterocycle are shown above for Y as "a 5-10 membered bridged saturated or partially unsaturated heterocycle having 1-3 heteroatoms independently selected from N, O and S. The difference is that it is a divalent group, and wherein the heterocyclyl and the rest of the molecule can be attached at a heteroatom or a carbon atom of the heterocycle, and the substituent on it can be substituted at a ring carbon atom or a ring heteroatom, as long as it is chemically feasible.

In some embodiments of compounds of formula (I), Z is a 3-15 membered heterocyclyl having one or more heteroatom(s) independently selected from N, O, and S, specifically a 6-15 membered spiro or fused saturated or partially unsaturated heterocycle having 1-4 heteroatoms independently selected from N, O, and S, more specifically a 7-11 membered bicyclic spiro or fused saturated heterocycle having 1-3 heteroatoms independently selected from N, O, and S, optionally substituted by halogen, $-C_{1-6}$ alkyl, or $-OC_{1-6}$ alkyl, wherein the $-C_{1-6}$ alkyl is optionally substituted by halogen or CN; wherein examples of spiro and fused heterocycle are shown above for Y as "a 6-15 membered spiro or fused heterocycle having 1-4 heteroatoms independently selected from N, O, and S". The difference is that it is a divalent group, and wherein the heterocyclyl and the rest of the molecule can be attached at a heteroatom or a carbon atom of the heterocycle, and the substituent on it can be substituted at a ring carbon atom or a ring heteroatom, as long as it is chemically feasible.

In some embodiments of the compound of formula (I), Z is preferably selected from $-C_{6-10}$ aryl-, -3-10 membered carbocyclyl-, and -3-15 membered heterocyclyl having one or more heteroatom(s) independently selected from N, O, and S, which is optionally substituted; more preferably, Z is selected from $-C_{6-10}$ aryl-, -3-8 membered monocyclic saturated or partially unsaturated carbocyclyl-, -5-10 membered bridged saturated or partially unsaturated carbocyclyl-, and -3-8

membered monocyclic saturated or partially unsaturated heterocycle having 1-3 heteroatoms independently selected from N, O, and S, which is optionally substituted; further more preferably, Z is selected from optionally substituted phenyl, -3-6 membered monocyclic saturated or partially unsaturated carbocyclyl-, -5-8 membered bridged saturated carbocycle-, and -4-7 membered monocyclic saturated or partially unsaturated heterocycle having 1-2 heteroatoms independently selected from N, O, and S; most preferably, Z is phenyl.

In an embodiment of the compound of formula (I), Z is optionally substituted by one or more group(s) independently selected from halogen, $-C_{1-6}$ alkyl or $-OC_{1-6}$ alkyl, wherein the $-C_{1-6}$ alkyl is optionally substituted by halogen or CN; for example, Z is optionally substituted by 1-2, preferably 1, groups selected from halogen, $-C_{1-6}$ alkyl and $-OC_{1-6}$ alkyl; in one embodiment, Z is not substituted.

**In some embodiments of the compound of formula (I),** R is an optionally substituted $-C_{6-10}$ aryl, preferably

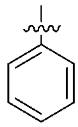 or 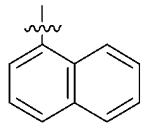.

In some embodiments of the compound of formula (I), R is an optionally substituted -3-15 membered carbocyclyl, specifically a -3-8 membered monocyclic saturated or partially unsaturated carbocycle, more specifically a $-(CH_2)_t$-3-6 membered monocyclic saturated carbocycle; the 3-8 membered monocyclic saturated or partially unsaturated carbocycle is, for example, but not limited to

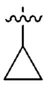, 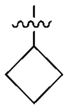, 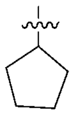, 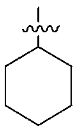, 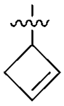, 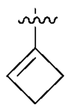, 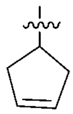, 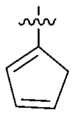, 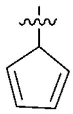, 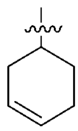, 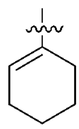.

In some embodiments of the compound of formula (I), R is an optionally substituted -3-15 membered carbocycle, specifically a -5-10 membered bridged saturated or partially unsaturated carbocycle, more specifically a -5-8 membered bicyclic bridged saturated carbocycle; the -5-10 membered bridged saturated or partially unsaturated carbocycle is, for example, but not limited to

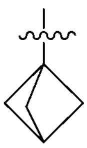, 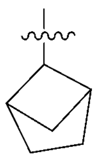,, 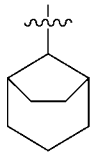, 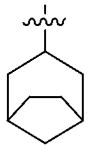, 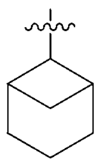, 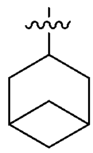, 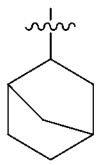, 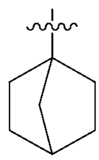.

In some embodiments of the compound of formula (I), R is an optionally substituted -3-15 membered carbocyclyl, specifically a -6-15 membered spiro or fused saturated or partially unsaturated carbocycle, more specifically a -7-11 membered bicyclic spiro or fused saturated carbocycle; the 6-15 membered spiro or fused saturated or partially unsaturated carbocycle may also be represented as

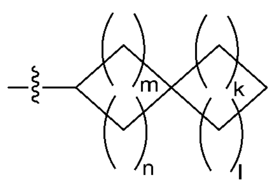 or 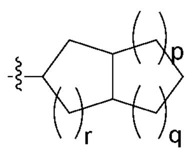,

where k is an integer from 0-2; m and n are each independently an integer from 1-3, 1 is an integer from 1-4, r and p are each independently an integer from 0-3, q is an integer from 1-4, optionally containing one or more double bond(s) (non-aromatic), in this embodiment R is more specifically an optionally substituted -7-11 membered bicyclic spiro saturated carbocycle (wherein k is 0-2, m and n are each independently an integer from 1-2, 1 is an integer from 1-3) or a -7-11 membered bicyclic fused saturated carbocycle (wherein r is an integer from 0-1, p is an integer from 0 to 2 and q is an integer from 1 to 3; wherein the spirocarbocycle and fused carbocycle are, for example but not limited to, the groups exemplified above for "a 6-15 spiro or fused saturated or partially unsaturated carbocycle" of Y.

In some embodiments of the compound of formula (I), R is an optionally substituted 5-12 membered heteroaryl having one or more heteroatom(s) independently selected from N, O and S, specifically a 5-6 membered monocyclic heteroaryl or an 8-10 membered bicyclic heteroaryl having 1-4 heteroatoms independently selected from N, O and S, more specifically a 5-6 membered monocyclic heteroaryl having 1-4 heteroatoms independently selected from N, O and S; wherein the heteroaryl can be attached to the rest of the molecule by a ring carbon atom or a ring heteroatom, specific examples are as exemplified above for the heteroaryl of Y, such as thienyl, pyrazolyl, etc.

In some embodiments of the compound of formula (I), R is an optionally substituted 3-15 membered heterocyclyl having one or more heteroatom(s) independently selected from N, O and S, specifically a 3-8 membered monocyclic saturated or partially unsaturated heterocycle having 1-3 heteroatoms independently selected from N, O and S, more specifically a 4-7 membered monocyclic saturated or partially unsaturated heterocycle having 1-2 heteroatoms independently selected from N, O and S; wherein the heterocycle is, for example but not limited to the groups exemplified above for Y as "a 3-8 membered monocyclic saturated or partially unsaturated heterocycle having 1-3 heteroatoms independently selected from N, O and S", such as oxetanyl, tetrahydropyranyl, morpholinyl, oxocyclopentenyl, oxocyclohexenyl, piperidinyl, pyrrolidinyl, azetidinyl, etc.

In some embodiments of compounds of formula (I), R is an optionally substituted 3-15 membered heterocyclyl having one or more heteroatom(s) independently selected from N, O and S, specifically a 5-10 membered bridged saturated or partially unsaturated heterocycle having 1-3 heteroatoms independently selected from N, O and S, more specifically a 5-8 membered bicyclic bridged saturated heterocycle having 1-2 heteroatoms independently selected from N, O and S; examples of the bridged heterocycle are shown above for Y as "a 5-10 membered bridged saturated or partially unsaturated heterocycle having 1-3 heteroatoms independently selected from N, O and S ", for example

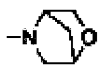

.

In a further embodiment, the bridged heterocycle is attached to the rest of the molecule by a nitrogen heteroatom. In a further embodiment, the bridged heterocycle is unsubstituted. In a further embodiment, the bridged heterocycle is substituted by 1-3 substituents independently selected from: halogen, CN, -$C_{1-6}$ alkyl, -4-7 membered monocyclic saturated heterocycle having 1-3 heteroatoms independently selected from N, O, and S, wherein the $C_{1-6}$ alkyl in the substituent is optionally further substituted by halogen, -OH, or -$OC_{1-6}$ alkyl.

In some embodiments of the compound of formula (I), R is an optionally substituted 3-15 membered heterocyclyl having one or more heteroatom(s) independently selected from N, O and S, specifically a 6-15 membered spiro or fused saturated or partially unsaturated heterocyclyl having 1-4 heteroatoms independently selected from N, O and S, more specifically a 7-11 membered bicyclic spiro or fused saturated heterocyclyl having 1-3 heteroatoms independently selected from N, O and S; wherein the spiro and fused heterocyclyl are, for example but not limited to, the "6-15 membered spiro or fused saturated or partially unsaturated heterocyclyl having 1-4 heteroatoms independently selected from N, O and S" as shown above for Y.

In some embodiments of the compound of formula (I), wherein R is a 3-15 membered heterocyclyl having one or more heteroatom(s) independently selected from N, O and S, including the various embodiments described above, attached to the rest of the molecule by a ring heteroatom.

In embodiments of the compound of formula (I), preferably R is an optionally substituted group selected from: -5-12 membered heteroaryl having one or more heteroatom(s) independently selected from N, O, and S and -3-15 membered heterocyclyl having one or more heteroatom(s) independently selected from N, O, and S; more preferably R is an optionally substituted group selected from: -5-6 membered monocyclic heteroaryl or 8-10 membered bicyclic heteroaryl having 1-4 heteroatoms independently selected from N, O, and S, -3-8 membered monocyclic saturated or partially unsaturated heterocycle having 1-3 heteroatoms independently selected from N, O, and S, -5-10 membered bridged saturated or partially unsaturated heterocycle having 1-3 heteroatoms independently selected from N, O, and S, -6-15 membered spiro or fused saturated or partially unsaturated heterocycle having 1-4 heteroatoms independently selected from N, O, and S; further more preferably R is an optionally substituted group selected from: -5-6 membered monocyclic heteroaryl having 1-4 heteroatoms independently selected from N, O, and S; -4-7 membered monocyclic saturated or unsaturated heterocycle having 1-2 heteroatoms independently selected from N, O, and S; 5-8 membered bicyclic bridged saturated heterocycle having 1-2 heteroatoms independently selected from N, O, and S; and 7-11 membered spiro or fused saturated heterocycle with 1-3 heteroatoms independently selected from N, O, and S.

In embodiments of compounds of formula (I), each type of R group carries 0 to 4, preferably 0 to 3, more preferably 0-2, optional substituents independently selected from: halogen, CN, -OH, - $NH_2$, -$C_{1-6}$ alkyl, -$C_{2-6}$ alkenyl, -$C_{2-6}$ alkynyl, -$OC_{1-6}$ alkyl, -$NHC_{1-6}$ alkyl, -$N(C_{1-6}$ alkyl$)_2$, -$(CH_2)_t$-3-8 membered monocyclic saturated or partially unsaturated carbocycle, -$(CH_2)_t$-5-8 membered bridged saturated or partially unsaturated carbocycle, -$(CH_2)_t$-3-8 membered monocyclic saturated or partially unsaturated heterocycle having 1-3 heteroatoms independently selected from N, O, and S,

$-(CH_2)_t$-5-8 membered bridged saturated or partially unsaturated heterocycle having 1-3 heteroatoms independently selected from N, O, and S, wherein two substituents attached to the same ring carbon atom together with the carbon atom to which they are attached may form a -3-8 membered saturated spirocarbocycle, and the $-C_{1-6}$ alkyl, $-C_{2-6}$ alkenyl, $-C_{2-6}$ alkynyl or cyclic group in the substituents are each independently and optionally substituted by halogen, CN, -OH or $-OC_{1-6}$ alkyl; preferably is selected from: halogen, CN, -OH, $-C_{1-6}$ alkyl, $-OC_{1-6}$ alkyl, $-(CH_2)_t$-4-7 membered monocyclic saturated heterocycle having 1-2 heteroatoms independently selected from N, O and S, wherein two substituents attached to the same carbon atom together with the carbon atom to which they are attached may form a -3-6 membered saturated spirocarbocycle, and the $C_{1-6}$ alkyl is optionally substituted by halogen, CN, -OH or $-OC_{1-6}$ alkyl; the substituents are, for example but not limited to -OH, -CN, F, Cl, $-CH_3$, $-CF_3$, $-CH_2CH_3$, $-CH=CH_2$, $-CH=CHCH_3$, -CH=CHF, -C=CH, $-CH_2CF_3$, $-CH_2CH_2CH_3$, $-CH_2OH$, $-CH_2CH_2OH$, $-CH(OH)CH_3$, $-CH(OH)(CF_3)$-, $-C(CH_3)(OH)(CH_3)$, $-CH_2CH(OH)CH_3$, $-CH(OH)CH_2CH_3$, $-CH_2C(CH_3)(OH)CH_3$, $-CH_2CH_2C(CH_3)(OH)CH_3$, $-CH_2CN$, $-CH_2-OCH_3$, $-CH_2-OCH_2CH_3$, $-CH_2CH_2-OCH_3$, $-NH_2$, $-NHCH_3$, $-NCH_3CH_3$,

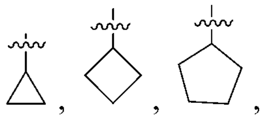

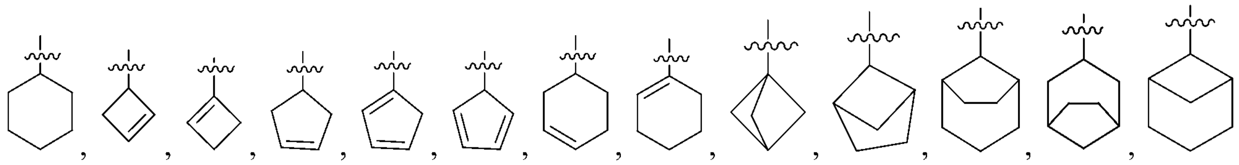

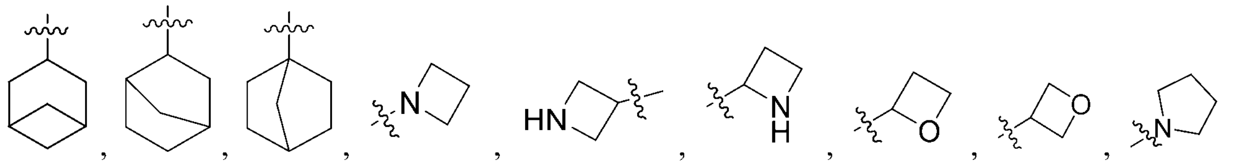

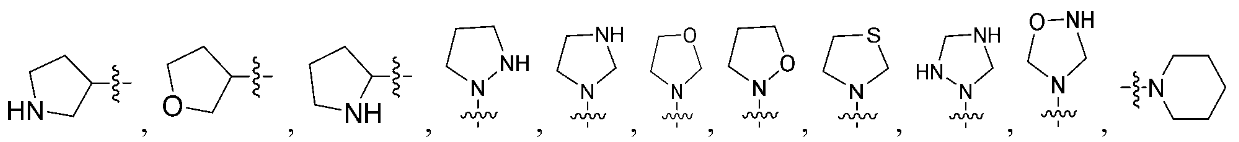

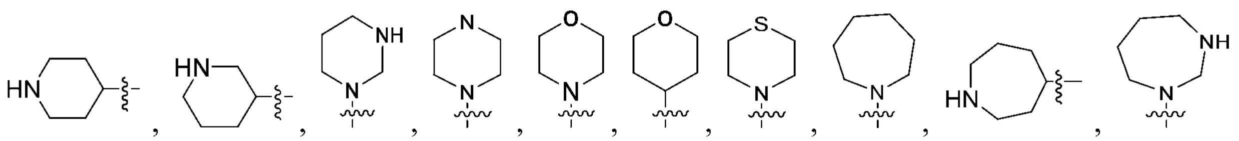

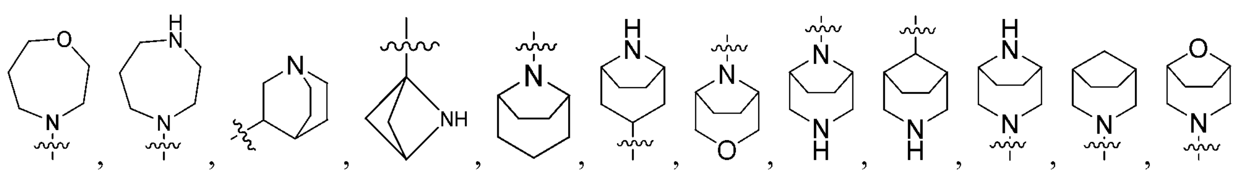

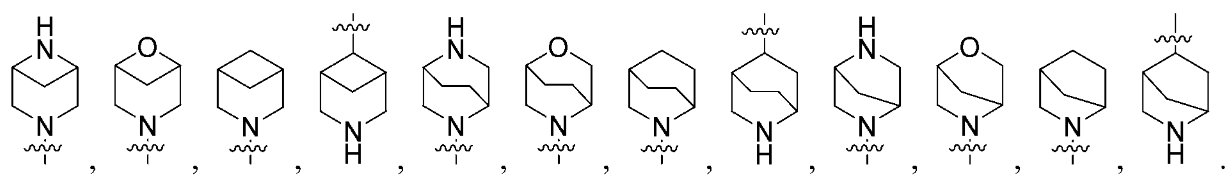

The substituents of R can substitute on a ring carbon atom or a ring heteroatom, as long as it is chemically feasible.

In embodiments of the compound of formula (I), it is preferred that t in the R substituent is 0-2, more preferably 0-1.

**In** some embodiments of the compound of formula (I), R has formula (A)

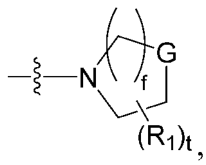

wherein,

f is selected from an integer from 0 to 3, preferably an integer from 0 to 2;

t is selected from an integer from 0 to 3, preferably an integer from 0 to 1;

G is selected from O, N-$R_2$ and $CR_3R_4$; preferably O and $CR_3R_4$, and most preferably $CR_3R_4$.

$R_1$ is selected from H and -$C_{1-6}$ alkyl optionally substituted by halogen, CN, -OH or -$OC_{1-6}$ alkyl, wherein two $R_1$ attached to the same carbon atom in the ring together with the carbon atom to which they are attached may form a -3-8 membered saturated spirocarbocycle; preferably $R_1$ is selected from H and -$C_{1-6}$ alkyl optionally substituted by -OH or -$OC_{1-6}$ alkyl; most preferably $R_1$ is selected from H and -$C_{1-6}$ alkyl.

$R_3$ and $R_4$ are each independently selected from H, halogen, CN, -OH, -$NH_2$, -$C_{1-6}$ alkyl, -$C_{2-6}$ alkenyl, -$C_{2-6}$ alkynyl, -$OC_{1-6}$ alkyl, -$NHC_{1-6}$ alkyl, -N($C_{1-6}$ alkyl)$_2$, -$(CH_2)_t$-3-8 membered monocyclic saturated or partially unsaturated carbocycle, -$(CH_2)_t$-5-8 membered bridged saturated or partially unsaturated carbocycle, -$(CH_2)_t$-3-8 membered monocyclic saturated or partially unsaturated heterocycle having 1-3 heteroatoms independently selected from N, O, and S, -$(CH_2)_t$-5-8 membered bridged saturated or partially unsaturated heterocycle having 1-3 heteroatoms independently selected from N, O, and S, or $R_3$ and $R_4$ together with the ring carbon atom to which they are attached form a -3-8 membered saturated spirocarbocycle, and the -$C_{1-6}$ alkyl, -$C_{2-6}$ alkenyl, -$C_{2-6}$ alkynyl or cyclic group in the substituents is each independently and optionally substituted by halogen, CN, -OH or - $OC_{1-6}$ alkyl; preferably $R_3$ and $R_4$ are selected from: H, halogen, CN, -OH, -$C_{1-6}$ alkyl, -$OC_{1-6}$ alkyl, -$(CH_2)_t$-4-7 membered monocyclic saturated heterocycle having 1-2 heteroatoms independently selected from N, O and S, or $R_3$ and $R_4$ together with the ring carbon atom to which they are attached form a -3-6 membered saturated spirocarbocycle, and the $C_{1-6}$ alkyl in the substituents is optionally substituted by halogen, CN, -OH or -$OC_{1-6}$ alkyl; more preferably $R_3$ and $R_4$ are selected from H, halogen, CN, -$C_{1-6}$ alkyl, -4-7 membered monocyclic saturated heterocycle having 1-2 heteroatoms independently selected from N, O, and S, wherein the $C_{1-6}$ alkyl in the substituents is optionally further substituted by halogen, -OH or -$OC_{1-6}$ alkyl;

$R_2$ is selected from H, -$C_{1-6}$ alkyl, -$(CH_2)_t$-3-8 membered monocyclic saturated or partially unsaturated carbocycle, -$(CH_2)_t$-5-8 membered bridged saturated or partially unsaturated carbocycle, -$(CH_2)_t$-3-8 membered monocyclic saturated or partially unsaturated heterocycle having 1-3 heteroatoms independently selected from N, O, and S, and -$(CH_2)_t$-5-8 membered bridged saturated or partially unsaturated heterocycle having 1-3 heteroatoms independently selected from N, O, and S, wherein the $C_{1-6}$ alkyl or cyclic group in the substituent is optionally substituted by halogen, CN, - OH, or -$OC_{1-6}$ alkyl; preferably $R_2$ is selected from: H, -$C_{1-6}$ alkyl, -$(CH_2)_t$-4-7 membered monocyclic saturated heterocycle having 1-2 heteroatoms independently selected from N, O, and S, wherein the $C_{1-6}$ alkyl or cyclic group in the substituent is optionally substituted by halogen, CN, -OH, or -$OC_{1-6}$ alkyl; more preferably $R_2$ is selected from: H, -$C_{1-6}$ alkyl, -4-7 membered monocyclic saturated heterocycle having 1-2 heteroatoms independently selected from N, O and S, wherein the $C_{1-6}$ alkyl in the substituent is optionally substituted by halogen, -OH or -$OC_{1-6}$ alkyl;

When f is 0, G is -$CH_2$-.

In some embodiments of formula (A), when G is $CR_3R_4$, one of $R_3$ and $R_4$ is selected from H, halogen and -$C_{1-6}$ alkyl, and the other is selected from -$C_{1-6}$ alkyl substituted by OH or further substituted by halogen; in some embodiments of formula (A), one of $R_3$ and $R_4$ is selected from H and -$C_{1-6}$ alkyl, and the other is CN; in some embodiments of formula (A), one of $R_3$ and $R_4$ is H, and the other is a 4-7 membered monocyclic saturated heterocycle having 1-2 heteroatoms independently selected from N, O and S.

In a further such embodiment, R is selected from

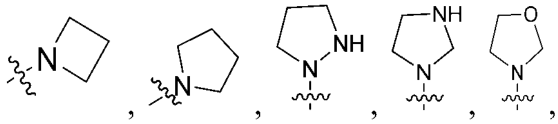

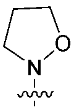 , 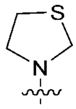 , 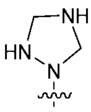 , 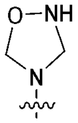 , 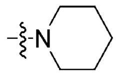 , 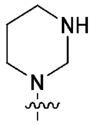 , 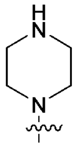 , 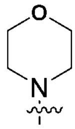 , 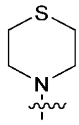 , 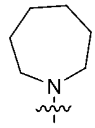 , 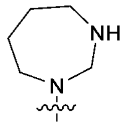 ,

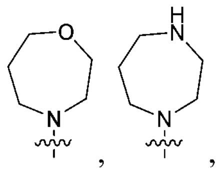

optionally substituted by $R_1$~$R_4$ as defined above, preferably substituted by 1 to 3 of the above preferred $R_1$~$R_4$; exemplary groups of $R_1$~$R_4$ are as shown above for the substituents carried on R, and preferably exemplary groups of $R_1$~$R_4$ are -OH, -CN, F, Cl, $-CH_3$, $-CF_3$, $-CH_2CH_3$, $-CH_2CF_3$, $-CH_2CH_2CH_3$, $-CH_2OH$, $-CH_2CH_2OH$, $-CH(OH)CH_3$, $-CH(OH)(CF_3)$-, - $C(CH_3)(OH)(CH_3)$, $-CH_2CH(OH)CH_3$, $-CH(OH)CH_2CH_3$, $-CH_2C(CH_3)(OH)CH_3$, - $CH_2CH_2C(CH_3)(OH)CH_3$, $-CH_2CN$, $-CH_2-OCH_3$, $-CH_2-OCH_2CH_3$, $-CH_2CH_2-OCH_3$,

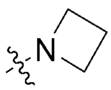 ,

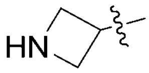 , 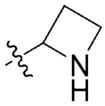 , 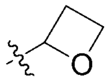 , 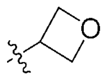 , 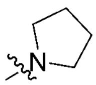 , 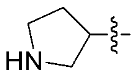 , 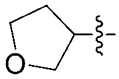 , 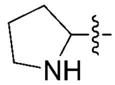 , 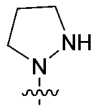 ,

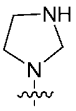 , 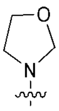 , 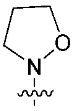 , 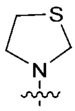 , 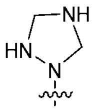 , 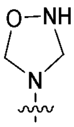 , 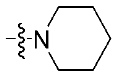 , 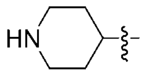 , 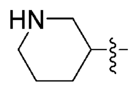 , 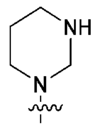 , 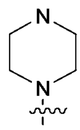 ,

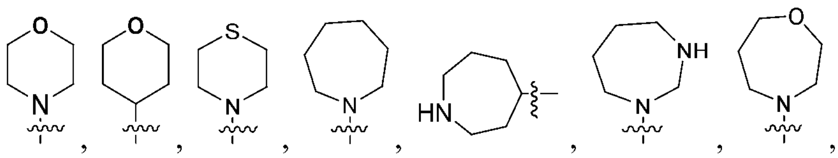

more preferably -CN, F, $-CH_3$, - $CH_2CH_3$, $-CH_2OH$, $-CH_2CH_2OH$, $-CH(OH)CH_3$,

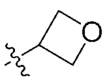 ,

$-CH_2C(CH_3)(OH)CH_3$, - $CH_2CH_2C(CH_3)(OH)CH_3$, $-CH(OH)CH_2CH_3$, $-CH(OH)(CF_3)$-, $-C(CH_3)(OH)(CH_3)$, $-CH_2-OCH_3$.

In a further such embodiment, exemplary groups of R include, but are not limited to:

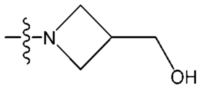, 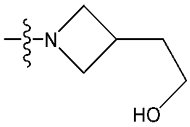, 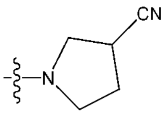, 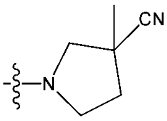, 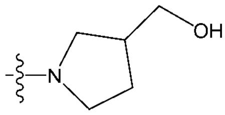, 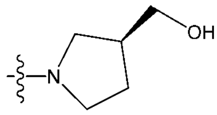,

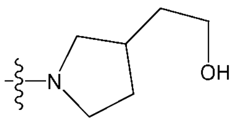, 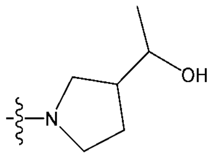, 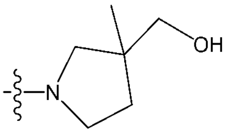, 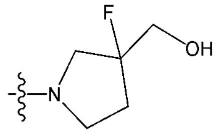, 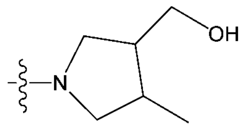,

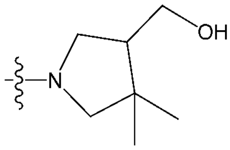, 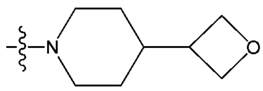, 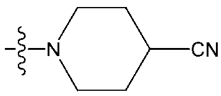, 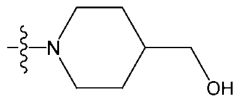, 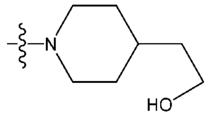,

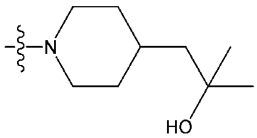, 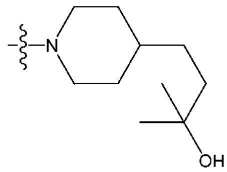, 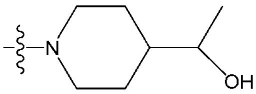, 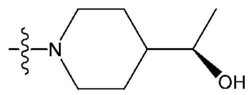,

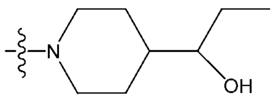, 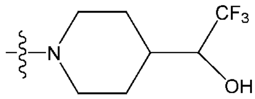, 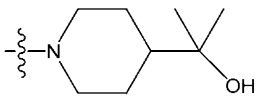, 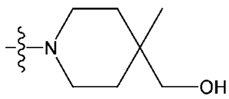,

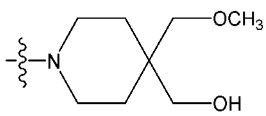, 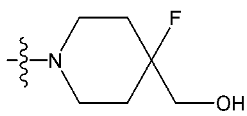, 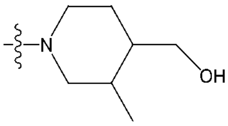, 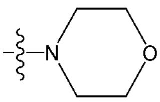, 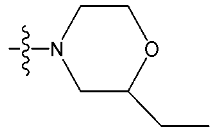.

**In some embodiments of the compound of formula (I),** R has the following formula:

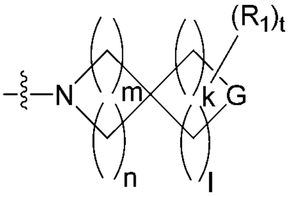 (B) or 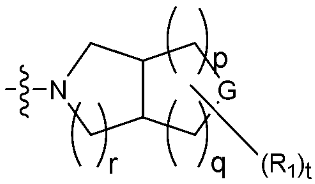 (C)

wherein:

k is an integer from 0-2, m and n are each independently an integer from 1-3, 1 is an integer from 1-4, preferably k is an integer from 0-2, m and n are each independently an integer from 1-2, and 1 is an integer from 1-3;

r and p are each independently an integer from 0-3, and q is an integer from 1-4, preferably r is an integer from 0-1, p is an integer from 0-2, and q is an integer from 1-3;

t is selected from an integer from 0 to 3, preferably from an integer from 0-1;

G is selected from O, N-$R_2$ and $CR_3R_4$, preferably O and $CR_3R_4$;

$R_1$ is selected from H and $-C_{1-6}$ alkyl optionally substituted by halogen, CN, -OH or $-OC_{1-6}$ alkyl, wherein two $R_1$ attached to the same ring carbon atom together with the carbon atom to which they are attached may form a -3-8 membered saturated spirocarbocycle; preferably $R_1$ is selected from H and $-C_{1-6}$ alkyl optionally substituted by -OH or $-OC_{1-6}$ alkyl; more preferably $R_1$ is H;

$R_3$ and $R_4$ are each independently selected from H, halogen, CN, -OH, $-NH_2$, $-C_{1-6}$ alkyl, $-C_{2-6}$ alkenyl, $-C_{2-6}$ alkynyl, $-OC_{1-6}$ alkyl, $-NHC_{1-6}$ alkyl, $-N(C_{1-6}$ alkyl$)_2$, $-(CH_2)_t$-3-8 membered monocyclic saturated or partially unsaturated carbocycle, $-(CH_2)_t$-5-8 membered bridged saturated or partially unsaturated carbocycle, $-(CH_2)_t$-3-8 membered monocyclic saturated or partially unsaturated heterocycle having 1-3 heteroatoms independently selected from N, O, and S, $-(CH_2)_t$-5-8 membered bridged saturated or partially unsaturated heterocycle having 1-3 heteroatoms independently selected from N, O, and S, or $R_3$ and $R_4$ together with the ring carbon atom to which they are attached form a -3-8 membered saturated spirocarbocycle, and the $-C_{1-6}$ alkyl, $-C_{2-6}$ alkenyl, $-C_{2-6}$ alkynyl or cyclic group in the substituents is each independently and optionally substituted by halogen, CN, -OH or $-OC_{1-6}$ alkyl; preferably $R_3$ and $R_4$ are selected from: H, halogen, CN, -OH, $-C_{1-6}$ alkyl, $-OC_{1-6}$ alkyl, $-(CH_2)_t$-4-7 membered monocyclic saturated heterocycle having 1-2 heteroatoms independently selected from N, O and S, or $R_3$ and $R_4$ together with the ring carbon atom to which they are attached form a -3-6 membered saturated spirocarbocycle, and the $C_{1-6}$ alkyl in the substituents is optionally substituted by halogen, CN, -OH or $-OC_{1-6}$ alkyl; more preferably $R_3$ and $R_4$ are selected from H, - OH, $-C_{1-6}$ alkyl, wherein the $C_{1-6}$ alkyl in the substituents is optionally further substituted by halogen, -OH or $-OC_{1-6}$ alkyl;

$R_2$ is selected from H, $-C_{1-6}$ alkyl, $-(CH_2)_t$-3-8 membered monocyclic saturated or partially unsaturated carbocycle, $-(CH_2)_t$-5-8 membered bridged saturated or partially unsaturated carbocycle, $-(CH_2)_t$-3-8 membered monocyclic saturated or partially unsaturated heterocycle having 1-3 heteroatoms independently selected from N, O, and S, and $-(CH_2)_t$-5-8 membered bridged saturated or partially unsaturated heterocycle having 1-3 heteroatoms independently selected from N, O, and S, wherein the $C_{1-6}$ alkyl or cyclic group in the substituent is optionally substituted by halogen, CN, - OH, or $-OC_{1-6}$ alkyl; preferably $R_2$ is selected from: H, $-C_{1-6}$ alkyl, $-(CH_2)_t$-4-7 membered monocyclic saturated heterocycle having 1-2 heteroatoms independently selected from N, O, and S, wherein the $C_{1-6}$ alkyl or cyclic group in the substituent is optionally substituted by halogen, CN, -OH, or $-OC_{1-6}$ alkyl; more preferably $R_2$ is selected from H, $-C_{1-6}$ alkyl, wherein the $C_{1-6}$ alkyl is optionally further substituted by halogen, -OH or $-OC_{1-6}$ alkyl.

In some embodiments of formula (B) and formula (C), when G is O, $R_1$ is H.

In some embodiments of formula (B) and formula (C), when G is $CR_3R_4$, one of $R_3$ and $R_4$ is selected from -OH and $-C_{1-6}$ alkyl substituted by -OH, and the other is selected from H and $-C_{1-6}$ alkyl optionally substituted by halogen.

In a further such embodiment, R is selected from

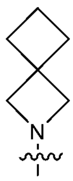 , 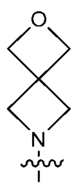 , 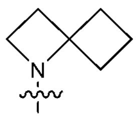 , 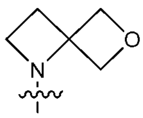 , 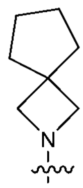 ,

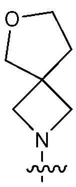 , 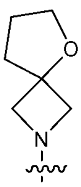 , 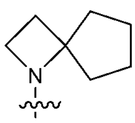 , 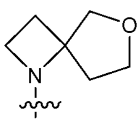 , 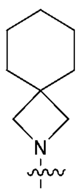 , 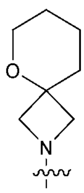 , 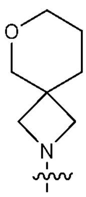 , 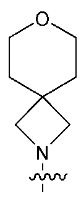 , 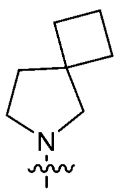 , 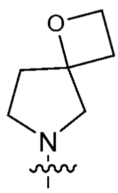 , 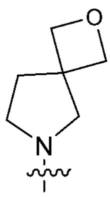 ,

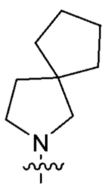 , 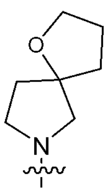 , 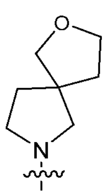 , 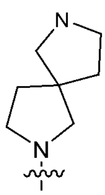 , 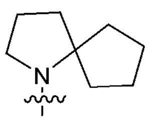 , 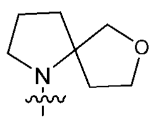 , 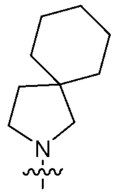 , 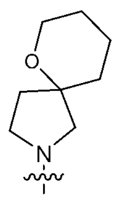 , 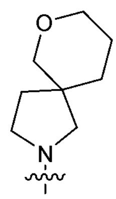 ,

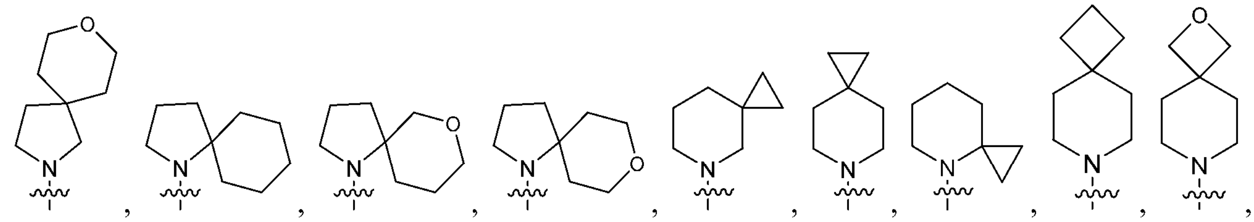
, , , , , , , , ,

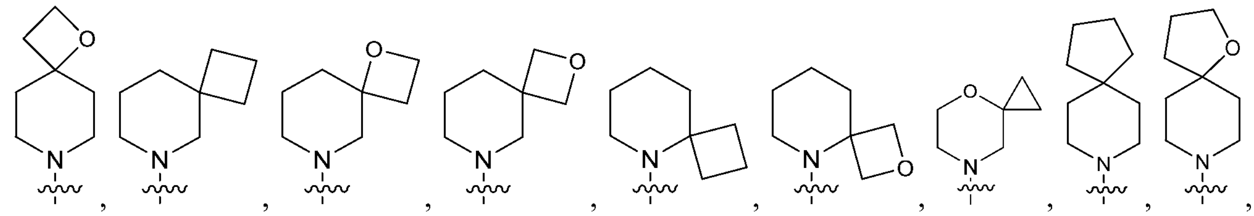
, , , , , , , , ,

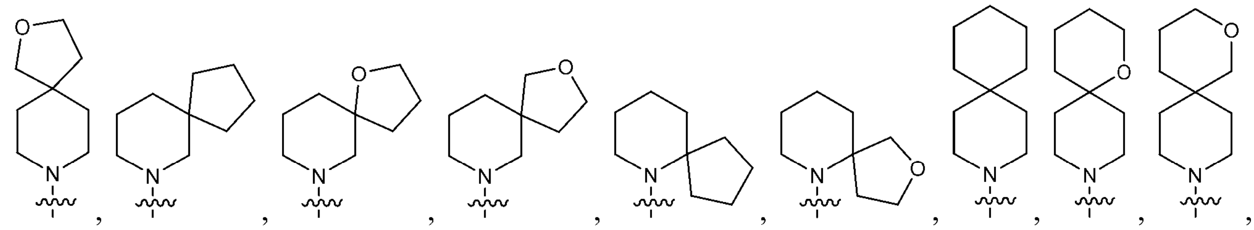
, , , , , , , , ,

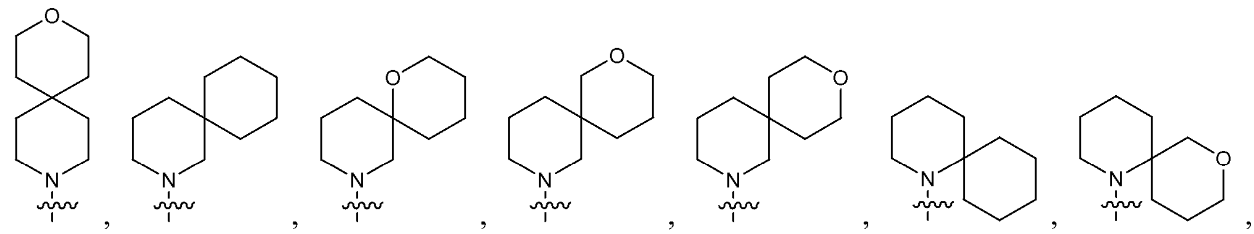
, , , , , , ,

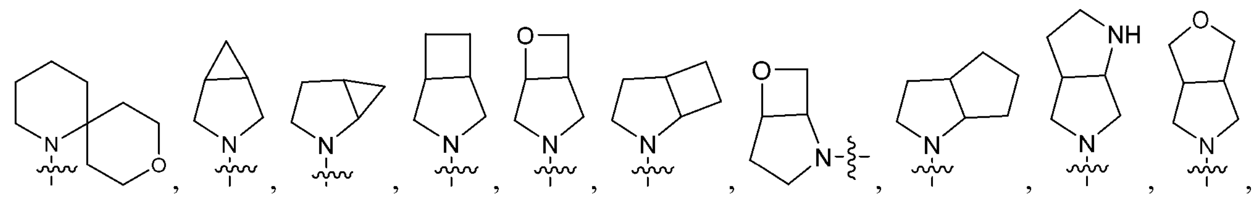
, , , , , , , , , , ,

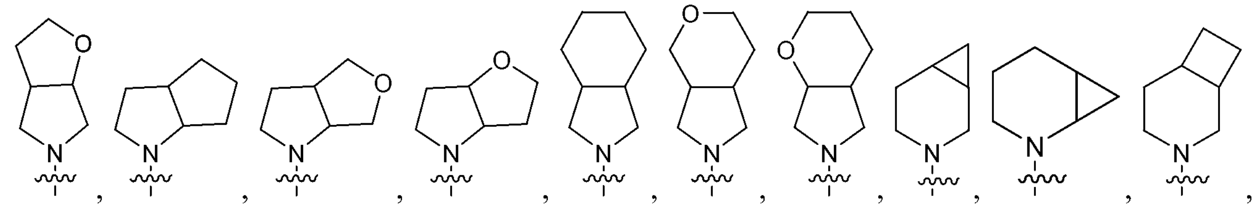
, , , , , , , , , , ,

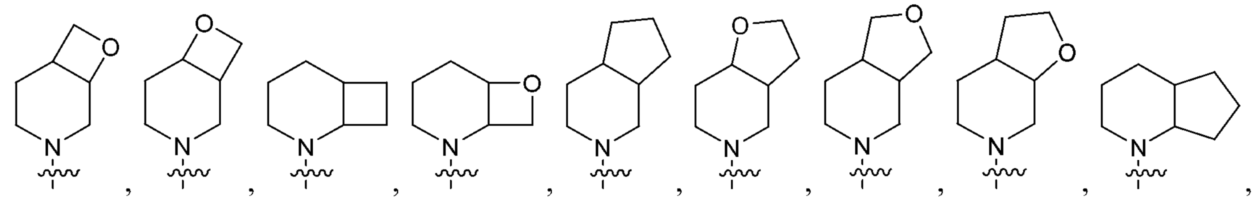
, , , , , , , , ,

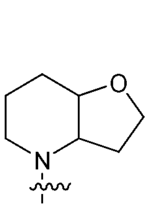, 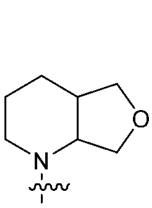, 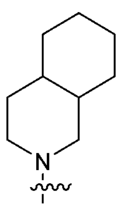, 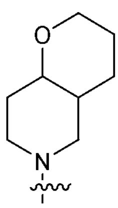, 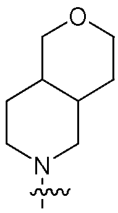, 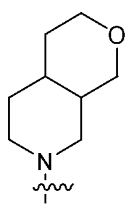, 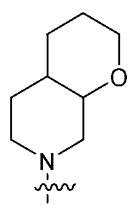, 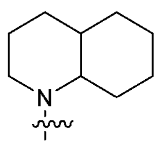,

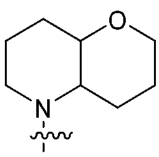, 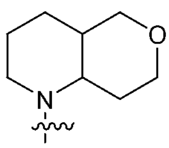, 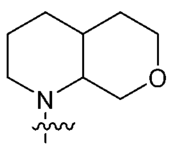, 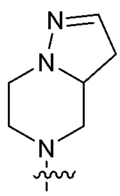, 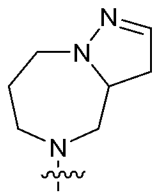;

preferably

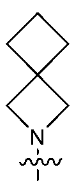, 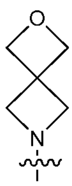, 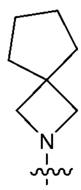,

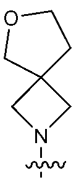, 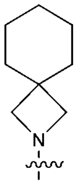, 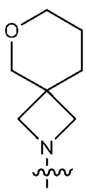, 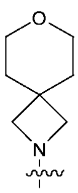, 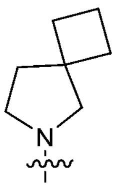, 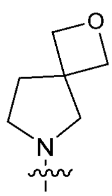, 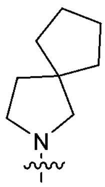, 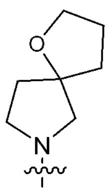, 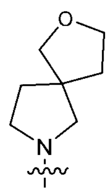, 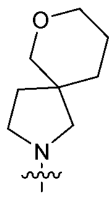, 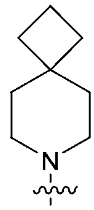,

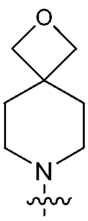, 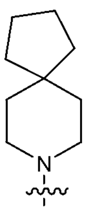, 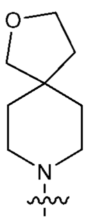, 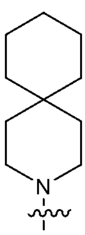, 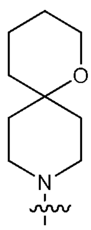, 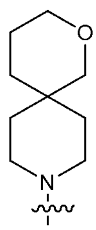, 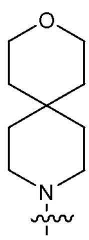, 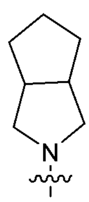, 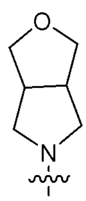;

optionally substituted by $R_1$~$R_4$ as defined above, preferably substituted by 1 to 3 of the above preferred $R_1$~$R_4$; exemplary groups of $R_1$~$R_4$ are as shown above for the substituents carried on R, and preferably exemplary groups of $R_1$~$R_4$ are include, but are not limited to, H, -OH, $-CH_3$, $-CF_3$, $-CH_2OH$, -C $(CH_3)(OH)(CH_3)$.

In a further such embodiment, exemplary groups of R include, but are not limited to:

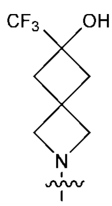,

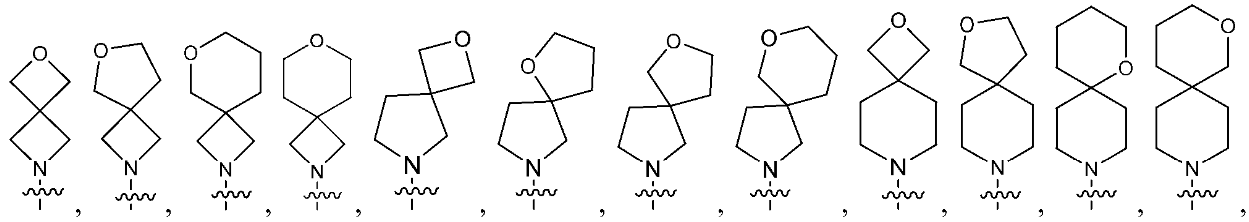

, , , , , , , , , , , ,

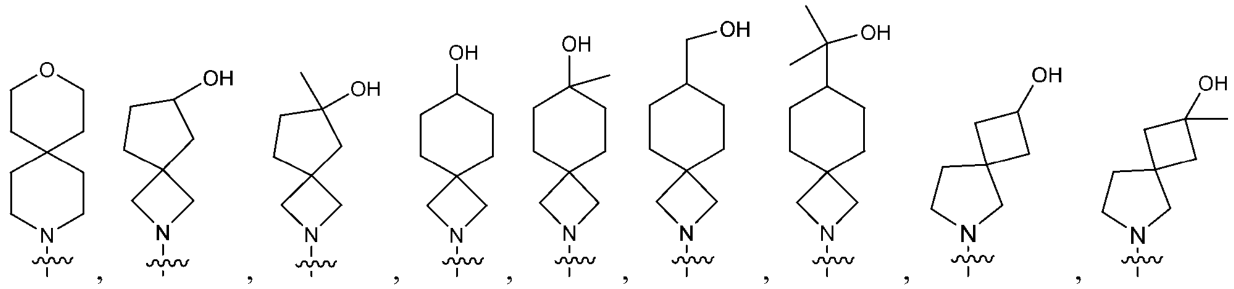

, , , , , , , , ,

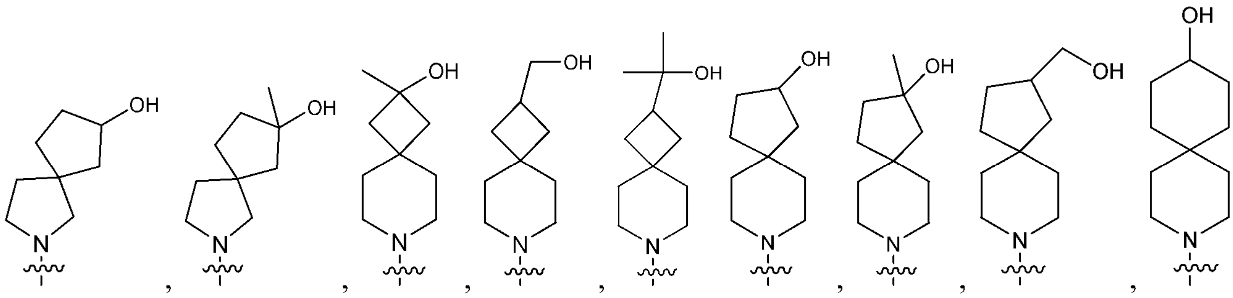

, , , , , , , , ,

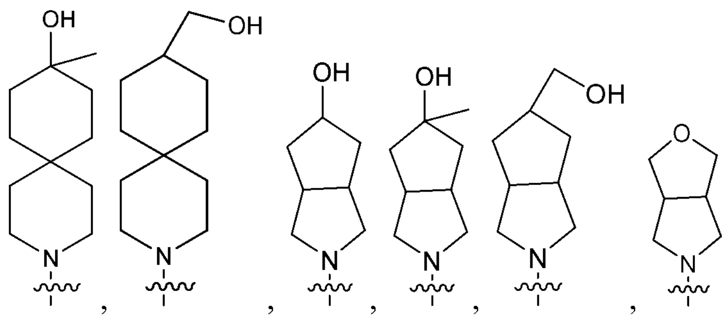

, , , , , .

**In a further embodiment of the compound of formula** (I), the compound of formula (I), or a pharmaceutically acceptable salt, isomer, solvate, hydrate, or stable isotopic variant thereof, is provided, wherein:

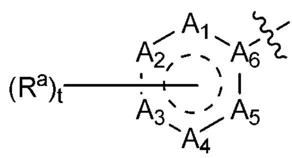

,

optionally substituted by 0-1 $R^a$, having 3-4 cyclic heteroatoms selected from N, O and S; preferably a five-membered aromatic group, optionally substituted by 0-1 $R^a$, having 3-4 nitrogen heterocyclic atoms; most preferably a five-membered aromatic group, optionally substituted by 0-1 $R^a$, having 4 nitrogen heterocyclic atoms.

$R^a$ is selected from -H, -CN, halogen, =O and -$C_{1-6}$ alkyl substituted by halogen, preferably H;

$A_7$ and $A_9$ is a heteroatom selected from N, O and S, and the other is C, and $A_8$ is a carbon atom; preferably $A_7$ is selected from C-Y and N-Y, $A_9$ is selected from C, O and S, and only one of $A_7$ and $A_9$ is a heteroatom, and $A_8$ is a

carbon atom;

$A_{10}$ is selected from C-$R^b$ and N, and $A_{11}$ is selected from C-X and N; preferably, $A_{10}$ is C-$R^b$ and $A_{11}$ is C-X.

$R^b$ is selected from H, halogen, CN and -$C_{1-6}$ alkyl substituted by halogen, preferably H;

X is selected from H, halogen, and -OH, preferably halogen;

Y is an optionally substituted group selected from:

- $C_{1-6}$ alkyl,
- $(CH_2)_t$-3-8 membered monocyclic saturated or partially unsaturated carbocycle,
- $(CH_2)_t$-5-10 membered bridged saturated or partially unsaturated carbocycle-,
- $(CH_2)_t$-$C_{6-10}$ aryl,

Z is an optionally substituted divalent group selected from:

- $C_{6-10}$ aryl-
- 3-8 membered monocyclic saturated or partially unsaturated carbocycle-,
- *5-10* membered bridged saturated or partially unsaturated carbocycle-,
- 3-8 membered monocyclic saturated or partially unsaturated heterocycle having 1-3 heteroatoms independently selected from N, O and S;

R is an optionally substituted group selected from:

- 5-12 membered heteroaryl having 1-4 heteroatoms independently selected from N, O, and S,
- 3-8 membered monocyclic saturated or partially unsaturated heterocycle having 1-3 heteroatoms independently selected from N, O and S;
- 5-10 membered bridged saturated or partially unsaturated heterocycle having 1 to 3 heteroatoms independently selected from N, O and S;
- 6-15 membered spiro or fused saturated or partially unsaturated heterocycle having 1-4 heteroatoms independently selected from N, O, and S; and

t is an integer from 0 to 3.

In these further embodiments, each cyclic group of Y is independently and optionally substituted by 1-3 -$C_{1-6}$ alkyl, OH, or -$OC_{1-6}$ alkyl, wherein the -$C_{1-6}$ alkyl or -$(CH_2)_t$- appearing in Y and its substituents are optionally substituted by halogen or CN and wherein any carbon chain atom is optionally substituted by NR', O, or S, R' is selected from H or -$C_{1-6}$ alkyl, as generally or specifically exemplified above regarding the substituents of Y; preferably, each cyclic group of Y is independently and optionally substituted by 1 or 2, more preferably 1 -$C_{1-6}$ alkyl or -$OC_{1-6}$ alkyl, wherein the -$C_{1-6}$ alkyl or -$(CH_2)_t$- appearing in Y and its substituents are optionally substituted by halogen. In embodiments wherein Y is -$(CH_2)_t$-carbocyclyl, preferably t is 0 and the carbocycle is a 3-6 membered monocyclic saturated carbocycle, optionally carrying a $C_{1-6}$ alkyl substituent, more preferably when present, the $C_{1-6}$ alkyl is substituted on the ring carbon atom of the carbocycle that is attached to the rest of the molecule.

In these further embodiments, each cyclic group of Z is optionally and independently substituted by one or more group(s) independently selected from halogen, -$C_{1-6}$ alkyl, or -$OC_{1-6}$ alkyl, wherein the -$C_{1-6}$ alkyl is optionally substituted by halogen or CN, as generally or specifically exemplified above regarding the substituents of Z; preferably, Z is optionally substituted by 1-2, more preferably 1, groups selected from halogen, -$C_{1-6}$ alkyl, and -$OC_{1-6}$ alkyl. In one embodiment, Z is not substituted.

In these further embodiments, the R group carries 0 to 4, preferably 0 to 3, more preferably 0-2, optional substituents independently selected from: halogen, CN, -OH, $-NH_2$, $-C_{1-6}$ alkyl, $-C_{2-6}$ alkenyl, $-C_{2-6}$ alkynyl, $-OC_{1-6}$ alkyl, $-NHC_{1-6}$ alkyl, $-N(C_{1-6}$ alkyl$)_2$, $-(CH_2)_t$-3-8 membered monocyclic saturated or partially unsaturated carbocycle, $-(CH_2)_t$-5-8 membered bridged saturated or partially unsaturated carbocycle, $-(CH_2)_t$-3-8 membered monocyclic saturated or partially unsaturated heterocycle having 1-3 heteroatoms independently selected from N, O, and S, $-(CH_2)_t$-5-8 membered bridged saturated or partially unsaturated heterocycle having 1-3 heteroatoms independently selected from N, O, and S, wherein two substituents attached to the same ring carbon atom together with the carbon atom to which they are attached may form a -3-8 membered saturated spirocarbocycle, and the $-C_{1-6}$ alkyl, $-C_{2-6}$ alkenyl, $-C_{2-6}$ alkynyl or cyclic group in the substituents are each independently and optionally substituted by halogen, CN , -OH or $-OC_{1-6}$ alkyl, as generally or specifically exemplified above for the substituents of R; preferably is selected from: halogen, CN, -OH, $-C_{1-6}$ alkyl, $-OC_{1-6}$ alkyl, $-(CH_2)_t$-4-7 membered monocyclic saturated heterocycle having 1-2 heteroatoms independently selected from N, O and S, wherein two substituents attached to the same carbon atom together with the carbon atom to which they are attached may form a -3-6 membered saturated spirocarbocycle, and the $C_{1-6}$ alkyl in the substituents is optionally substituted by halogen, CN, -OH or $-OC_{1-6}$ alkyl; examples of the substituents for example, but are not limited to, the general and specific definitions of R-carrying substituents above, with preferred exemplary groups including -OH, -CN, F, Cl, $-CH_3$, $-CF_3$, $-CH_2CH_3$, $-CH_2CF_3$, $-CH_2CH_2CH_3$, $-CH_2OH$, $-CH_2CH_2OH$, $-CH(OH)CH_3$, $-CH(OH)(CF_3)$-, $-C(CH_3)(OH)(CH_3)$, $-CH_2CH(OH)CH_3$, $-CH(OH)CH_2CH_3$, $-CH_2C(CH_3)(OH)CH_3$, $-CH_2CH_2C(CH_3)(OH)CH_3$, $-CH_2CN$, $-CH_2-OCH_3$, $-CH_2-OCH_2CH_3$, $-CH_2CH_2-OCH_3$,

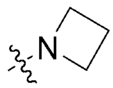
,

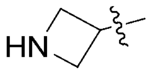, 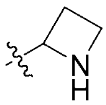, 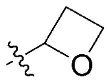, 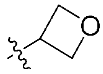, 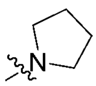, 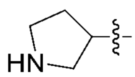, 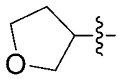, 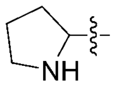, 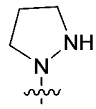,

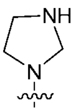, 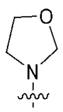, 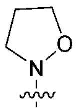, 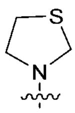, 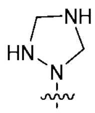, 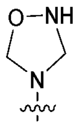, 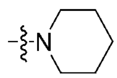, 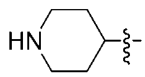, 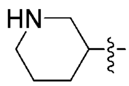, 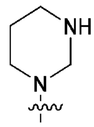, 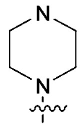,

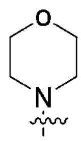, 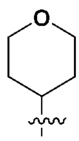, 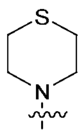, 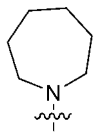, 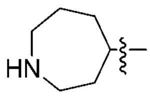, 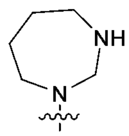, 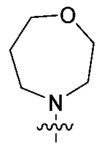;

more preferably -CN, F, -OH, $-CH_3$, $-CF_3$, $-CH_2CH_3$, $-CH_2CF_3$, $-CH_2OH$, $-CH_2CH_2OH$, $-CH(OH)CH_3$,

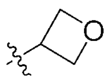, 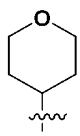, $-CH_2C(CH_3)(OH)CH_3$, $-CH_2CH_2C(CH_3)(OH)CH_3$, $-CH(OH)CH_2CH_3$, $-CH(OH)(CF_3)$-, $-C(CH_3)(OH)(CH_3)$, $-CH_2-OCH_3$.

The present disclosure further provides a compound of general formula (I-1), or a pharmaceutically acceptable salt, isomer, solvate, hydrate, or stable isotopic variant thereof.

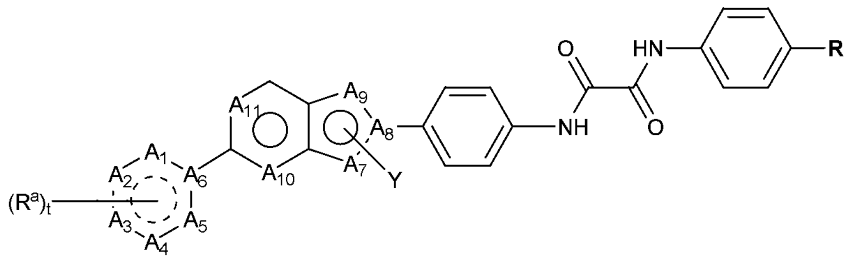

(I-1),

wherein $A_1$~$A_{11}$, $R_a$, Y, R, and t are each as defined above correspondingly for the general and specific embodiments of the compound of formula (I); wherein

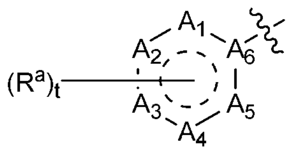

preferably is a five-membered unsaturated or aromatic group having 3-4 cyclic heteroatoms selected from N, O, and S, optionally substituted by 0-1 $R^a$; more preferably is a five-membered aromatic group having 3-4 nitrogen heterocyclic atoms, optionally substituted by 0-1 $R^a$; most preferably is five-membered aromatic group having 4 nitrogen heterocyclic atoms; $A_{11}$ is preferably C-X, $A_{10}$ is selected from N and C-$R^b$, $A_8$ is C, $R^b$ is preferably H, $A_7$ is selected from C-Y or N-Y, $A_9$ is selected from C, O, and S, and only one of $A_7$ and $A_9$ is a heteroatom. Specifically, formula (I-1) can be a compound of the following formula:

| |
|---|
| 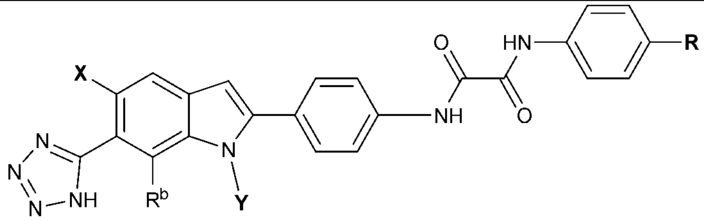 (I-1-1) |
| 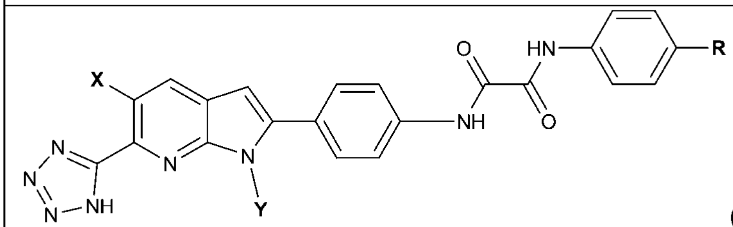 (I-1-2) |
| 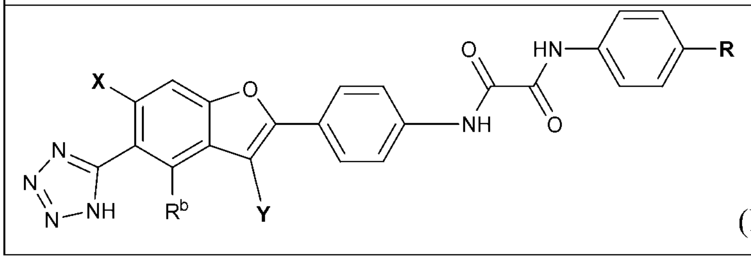 (I-1-3) |

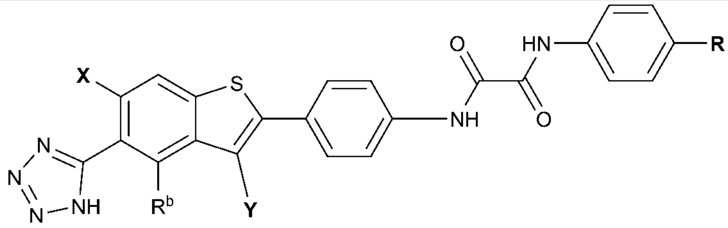

(I-1-4)

wherein X, Y, $R^b$ and R are each as defined above correspondingly for the general and specific embodiments of the compound of formula (I).

The present invention further provides a compound of general formula (I-2), or a pharmaceutically acceptable salt, isomer, solvate, hydrate, or stable isotopic variant thereof.

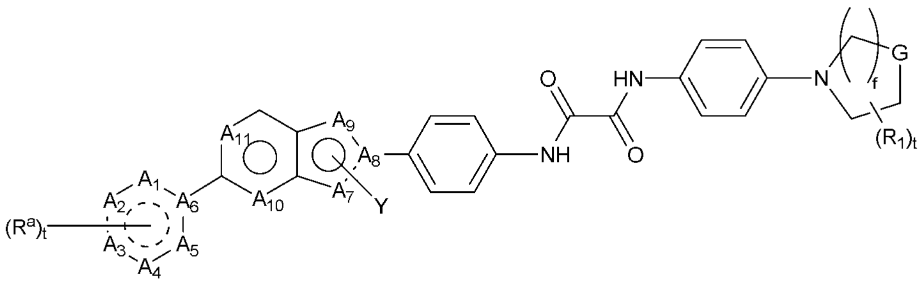

(I-2)

wherein $A_1$~$A_{11}$, $R^a$, Y, G, $R_1$, t, and f are each as defined above correspondingly for the general and specific embodiments of the compound of formula (I); wherein

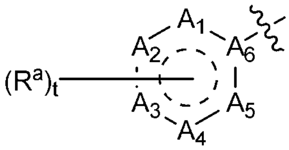

preferably is a five-membered unsaturated or aromatic group having 3-4 cyclic heteroatoms selected from N, O, and S, optionally substituted by 0-1 $R^a$; more preferably is a five-membered aromatic group having 3-4 nitrogen heterocyclic atoms, optionally substituted by 0-1 $R^a$; most preferably is a five-membered aromatic group having 4 nitrogen heterocyclic atoms; $A_{11}$ is preferably C-X, $A_{10}$ is selected from N and C-$R^b$, $A_8$ is C, $R^b$ is preferably H, $A_7$ is selected from C-Y or N-Y, $A_9$ is selected from C, O, and S, and only one of $A_7$ and $A_9$ is a heteroatom. Specifically, formula (I-2) can be a compound of the following formula:

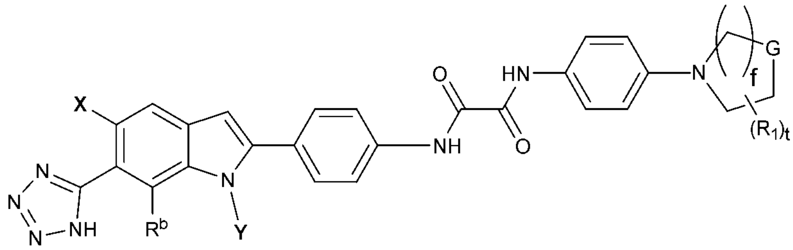

(I-2-1)

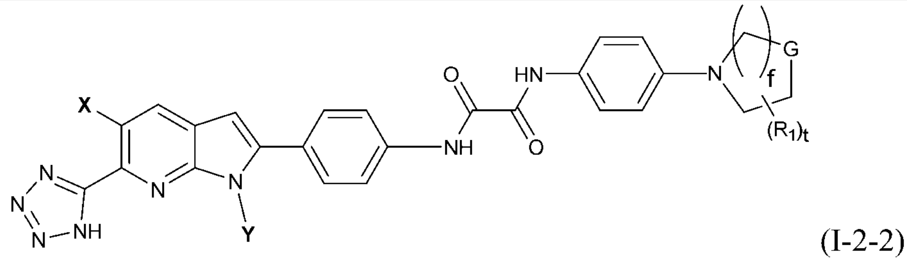

(I-2-2)

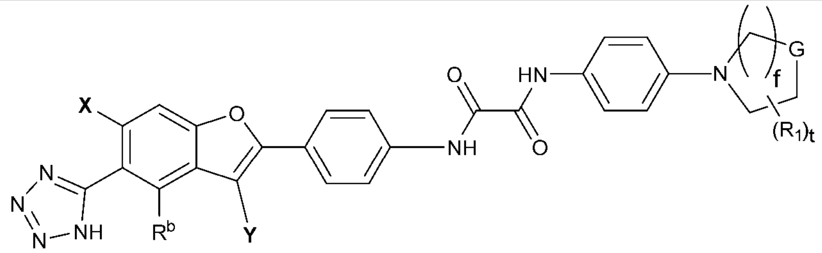

(I-2-3)

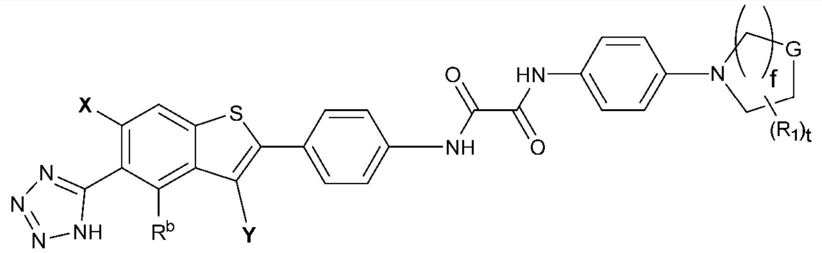

(I-2-4)

wherein X, Y, and $R^b$ are each as defined above correspondingly for the general and specific embodiments of the compound of formula (I), wherein

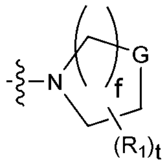

is as defined above correspondingly for the general and specific embodiments of the structural fragment of formula (A).

The present invention further provides a compound of general formula (I-3), or a pharmaceutically acceptable salt, isomer, solvate, hydrate, or stable isotopic variant thereof.

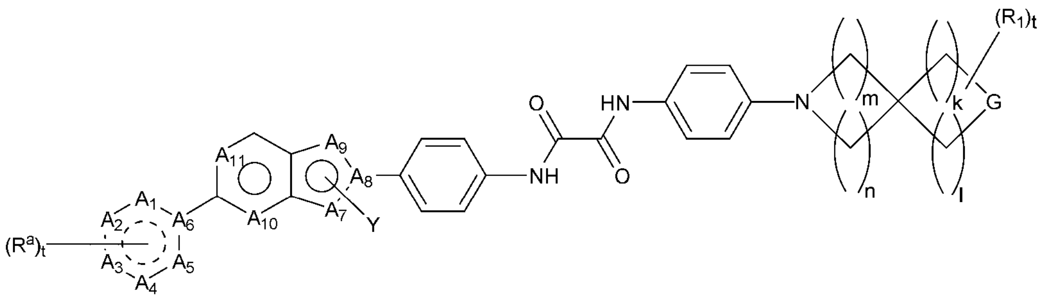

(I-3)

wherein $A_1$~$A_{11}$, $R^a$, Y, G, $R_1$, t, m, k, n, and l are each as defined above correspondingly for the general and specific embodiments of the compound of formula (I); wherein

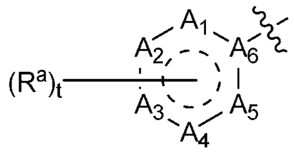

preferably is a five-membered unsaturated or aromatic group having 3-4 cyclic heteroatoms selected from N, O, and S, optionally substituted by 0-1 $R^a$; more preferably is a five-membered aromatic group having 3-4 nitrogen heterocyclic atoms, optionally substituted by 0-1 $R^a$; most preferably is a five-membered aromatic group having 4 nitrogen heterocyclic atoms; $A_{11}$ is preferably C-X, $A_{10}$ is selected from N and C-$R^b$, $A_8$ is C, $R^b$ is preferably H, $A_7$ is selected from C-Y or N-Y, $A_9$ is selected from C, O, and S, and only one of $A_7$ and $A_9$ is a heteroatom. Specifically, formula (I-3) can be a compound of the following formula:

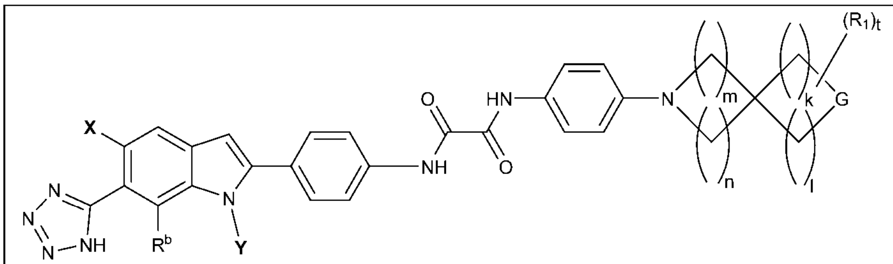

(I-3-1)

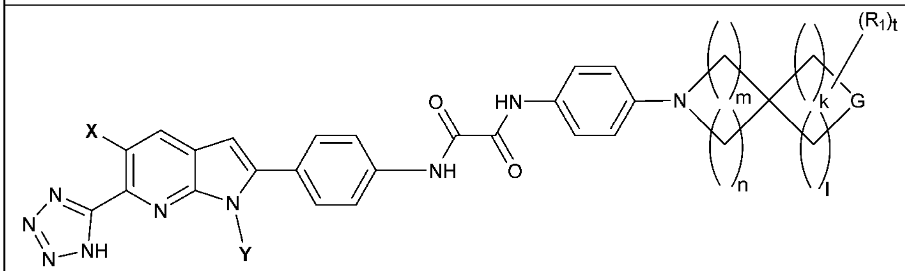

(I-3-2)

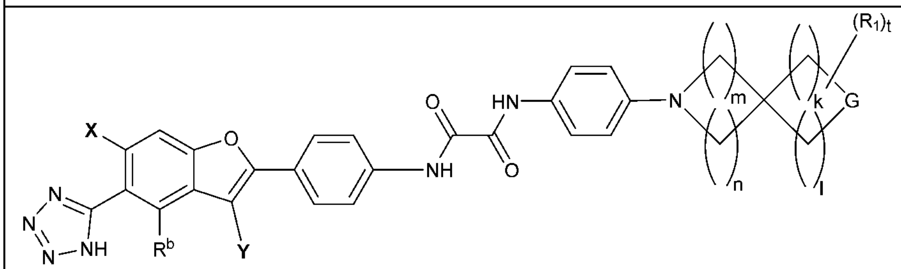

(I-3-3)

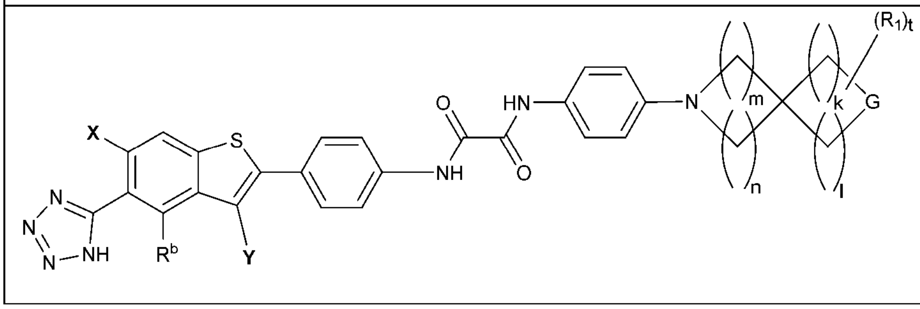

(I-3-4)

wherein X, Y, and $R^b$ are each as defined above correspondingly for the general and specific embodiments of the compound of formula (I), wherein

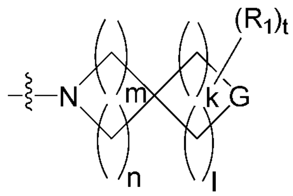

is as defined above correspondingly for the general and specific embodiments of the structural fragment of formula (B).

The present invention further provides a compound of general formula (I-4), or a pharmaceutically acceptable salt, isomer, solvate, hydrate, or stable isotopic variant thereof.

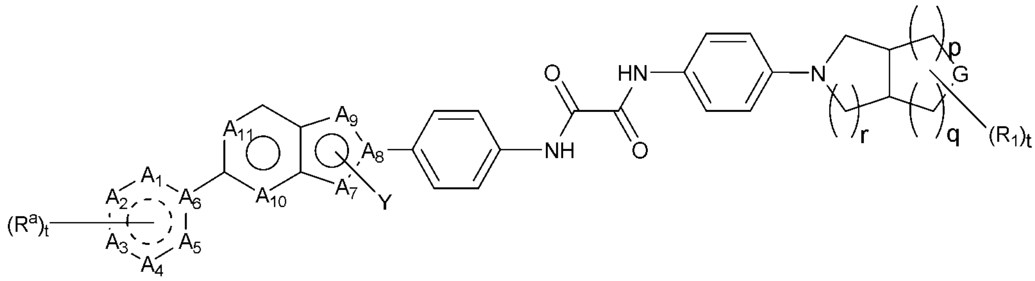

(I-4)

wherein $A_1$~$A_{11}$, $R^a$, Y, G, $R_1$, t, r, p, and q are each as defined above correspondingly for the general and specific embodiments of the compound of formula (I); wherein

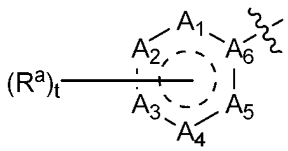

preferably is a five-membered unsaturated or aromatic group having 3-4 cyclic heteroatoms selected from N, O, and S, optionally substituted by 0-1 $R^a$; more preferably is a five-membered aromatic group having 3-4 nitrogen heterocyclic atoms, optionally substituted by 0-1 $R^a$; most preferably is a five-membered aromatic group having 4 nitrogen heterocyclic atoms; $A_{11}$ is preferably C-X, $A_{10}$ is selected from N and C-$R^b$, $A_8$ is C, $A_7$ is selected from C-Y or N-Y, $A_9$ is selected from C, O, and S, and only one of $A_7$ and $A_9$ is a heteroatom. Specifically, formula (I-4) can be a compound of the following formula:

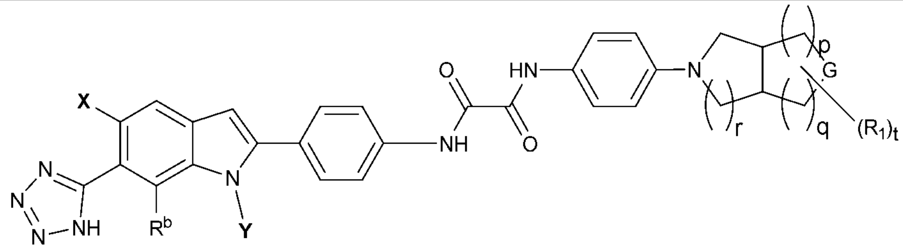

(I-4-1)

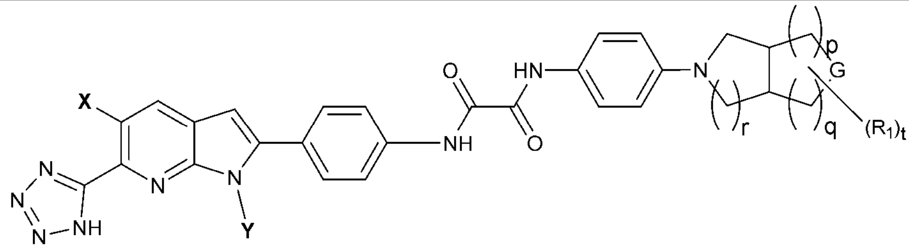

(I-4-2)

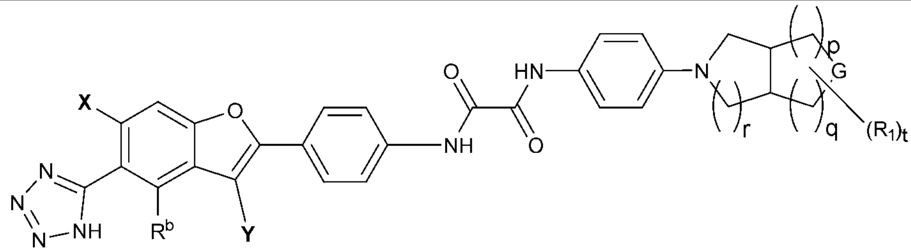

(I-4-3)

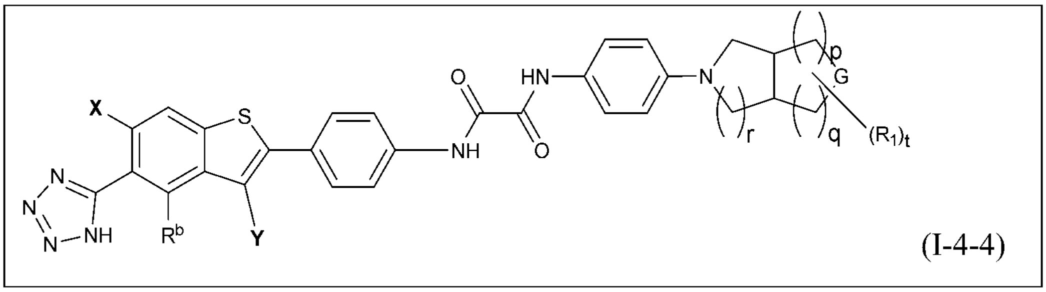

(I-4-4)

wherein X, Y, and $R^b$ are each as defined above correspondingly for the general and specific embodiments of the compound of formula (I), wherein

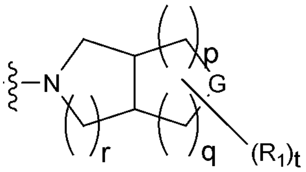

is as defined above correspondingly for the general and specific embodiments of the structural fragment of formula (C).

In specific embodiments, the present disclosure provides the compounds of Examples 1-134 below, or a pharmaceutically acceptable salt, isomer, solvate, hydrate, or stable isotopic variant thereof.

It should be noted that the compounds disclosed herein encompass each of the above independent embodiments or specific embodiments, as well as embodiments consisting of any combination or sub-combination of the above embodiments or specific embodiments, and embodiments consisting of any combination of any of the above preferred or exemplary embodiments.

## Beneficial effects of the invention

As previously described, SHP2 is known to play a crucial role in tumorigenesis and a variety of other diseases. Inventors have surprisingly discovered that by introducing a low-ionization "conditional" acidic group into the molecular structure to avoid highly ionized strong acidic groups, the compounds of this disclosure not only overcome the permeability/transportation problems of existing active site inhibitors but also exhibit potent inhibitory effects by binding strongly to the catalytic domain of the active site of SHP2 enzymes. Furthermore, they have demonstrated highly selective inhibition of SHP2, thus possessing potential value as antiproliferative and / or anti-invasive agents in the prevention, suppression, and / or treatment of related diseases, particularly cancers. In particular, the compounds of this disclosure are anticipated for the prevention or treatment of SHP2 - mediated diseases or diseases benefiting from SHP2- inhibition, such as those defined below, especially cancers or tumors.

Specifically, research has found that the compounds disclosed herein can achieve one or more of the following technical effects:

- Highly potent SHP2 inhibitory activity: The compounds disclosed herein, especially those specifically exemplified in the context, exhibited generally enhanced inhibitory activity against prior art inhibitor 11a-1 in SHP2 enzyme inhibition assays, with IC50 values for the SHP2 catalytic domain in the range of 0.001-1 μM, for example 0.001-0.5 μM, 0.001-0.1 μM, 0.001-0.05 μM, 0.01-1 μM, 0.01-0.5 μM, 0.01-0.1 μM, preferably 0.01-0.5 μM, more preferably 0.01-0.2 μM, generally as verified in Activity Example 1; and/or
- Potent tumor cell proliferation inhibitory activity: The compounds disclosed herein, especially those specifically exemplified in the context, exhibited significantly enhanced proliferation inhibitory activity relative to the prior art inhibitor 11a-1 in various SHP2-dependent tumor cell lines, with IC50 values generally in the range of 0.001 to 1 μM, for example 0.001 to 0.5 μM, 0.001 to 0.2 μM, 0.001 to 0.1 μM, 0.01 to 0.5 μM, 0.01 to 0.2 μM, 0.01 to 0.1 μM, 0.05 to 0.1 μM, preferably 0.01 to 0.5 μM, more preferably 0.01 to 0.1 μM, as verified in Activity Example 2; and/or
- Broad-spectrum tumor cell inhibitory activity: The compounds disclosed herein, especially those specifically exemplified in the context, exhibited cell proliferation inhibitory activity across a variety of tumor cell lines spanning solid tumors and hematologic malignancies, with IC50 values generally in the range of 0.001 to 1 μM, for example 0.001 to 0.5 μM, 0.001 to 0.2 μM, 0.01 to 0.2 μM, 0.01 to 0.1 μM, preferably 0.01 to 0.5 μM, more preferably 0.01 to 0.1 μM, as verified in Activity Example 3; and/or
- Favorable pharmacokinetic properties, such as a longer $t_{1/2}$ compared to prior art inhibitors 11a-1, which could allow for longer dosing intervals and better patient compliance; a significantly increased oral $AUC_{0-t}$, potentially leading to higher bioavailability, as demonstrated in Activity Example 4; and/or
- Excellent selective inhibitory activity against SHP2 in the tyrosine phosphatase group, as verified in Activity Example 5; and/or
- Satisfactory cardiac safety, with no inhibitory activity against hERG at a concentration of 10 *μM;* and a low risk of drug interactions, with no significant inhibitory effect on key CYP subtypes of drug metabolism, as verified in Activity Examples 6-7.

Based on the beneficial effects of the compounds of the present invention, the present invention also provides technical solutions in the following aspects.

## The compounds disclosed herein are for therapeutic or medicinal purposes.

In one aspect, this disclosure provides the compounds described above, preferably pharmaceutically acceptable salts or solvates thereof, for use as pharmaceuticals, specifically as SHP2 inhibitors.

In another aspect, this disclosure provides the compounds disclosed herein, preferably pharmaceutically acceptable salts or solvates thereof, for the treatment and/or prevention of SHP2 - mediated disease or diseases benefiting from SHP2- inhibition.

In specific embodiments, this disclosure provides compounds for treating and/or preventing diseases in which abnormal SHP2 activity promotes the occurrence and development of the disease or where inhibiting SHP2 activity reduces the incidence of the disease, reduces or eliminates disease symptoms. The diseases are selected from, but are not limited to, cancer or tumors, cardiovascular diseases, immune disorders, fibrosis, eye disorders, systemic lupus erythematosus, diabetes, neutropenia, or combinations thereof. Preferably, the diseases are selected from Noonan

syndrome (NS), leopard syndrome (LS), juvenile myelomonocytic leukemia (JMML), myelodysplastic syndrome (MDS), neuroblastoma, melanoma, squamous cell carcinoma of the head and neck, acute myeloid leukemia (AML), B-cell acute lymphoblastic leukemia (B-ALL), breast cancer, esophageal cancer, lung cancer, colon cancer, head cancer, gastric cancer, lymphoma, glioblastoma, stomach cancer, pancreatic cancer, and combinations thereof.

## Pharmaceutical Compositions and Their Administration

In one aspect, this disclosure provides pharmaceutical compositions comprising a compound of formula (I) as defined above, preferably a pharmaceutically acceptable salt or solvate thereof, and a pharmaceutically acceptable carrier or excipient. The pharmaceutical compositions of this disclosure may be used to treat or prevent SHP2 -mediated diseases, such as tumors or cancers.

The pharmaceutical compositions disclosed herein can be formulated using techniques known to those skilled in the art, such as those disclosed in Remington's Pharmaceutical Sciences, 20th edition. For example, they can be formulated as tablets, powders, capsules, lozenges, granules, solutions, dispersants, suspensions, syrups, sprays, suppositories, gels, emulsions, patches, etc. The compositions may contain conventional components found in pharmaceutical preparations, such as diluents (e.g., glucose, lactose, or mannitol), carriers, pH adjusters, buffers, sweeteners, fillers, stabilizers, surfactants, wetting agents, lubricants, emulsifiers, suspending agents, preservatives, antioxidants, opacifiers, flow aids, processing aids, colorants, flavorings, tasters, other known additives, and other active agents. Suitable carriers and excipients are well known to those skilled in the art and detailed in, for example, Ansel, Howard C., et al., Ansel's Pharmaceutical Dosage Forms and Drug Delivery Systems. Philadelphia: Lippincott, Williams & Wilkins, 2004.

The administration and delivery of the pharmaceutical compositions disclosed herein conform to good medical practice. Factors to be considered in this context include the specific disorder being treated, the specific mammal being treated, the individual patient's clinical condition, the cause of the disorder, the location of drug delivery, the method of administration, the administration schedule, and other factors familiar to physician practitioners. The optimal dosage level and frequency of administration of the compounds or pharmaceutical compositions disclosed herein can be determined by those skilled in the art through standard experiments in the field of pharmaceutical research.

The compositions disclosed herein can be administered in any suitable manner, including oral, topical (including buccal and sublingual), rectal, vaginal, transdermal, parenteral, subcutaneous, intraperitoneal, intrapulmonary, intradermal, intrathecal, inhalation, epidural, and intranasal administration, and, if local treatment is required, intralesional administration. Parenteral infusion includes intramuscular, intravenous, intraarterial, intraperitoneal, or subcutaneous administration. In a preferred embodiment, the pharmaceutical compositions of this disclosure are administered orally.

When the compounds disclosed herein are administered to human subjects, the daily dose is typically determined by the prescribing physician based on the individual patient's age, weight, sex, and disease severity. Generally, a dose level of about 0.1 mg/kg to about 150 mg/kg body weight is used to treat the aforementioned diseases; for a human subject weighing 70 kg, the appropriate dose range of the compounds disclosed herein can be routinely determined by those skilled in the art, for example, it can be 1-1000 mg/ day.

When this document describes a dosage of a drug or its pharmaceutically acceptable salt, it should be understood that the dosage is based on the weight of the free base, excluding any of its hydrates or solvates, unless the package insert indicates that the dosage is based on the weight of the salt, hydrate, or solvate.

## Treatment methods and uses

As demonstrated above, and as verified in the examples below, the compounds of this disclosure and compounds of various specific embodiments thereof, especially the compounds specifically prepared and characterized in the examples, exhibit potent and selective inhibition of SHP2. Inhibition of SHP2 activity further leads to ERK dephosphorylation and inhibition of the oncogenic function of the RAS-RAF-ERK pathway.

Therefore, in another aspect, this disclosure provides for the medical use of the compounds of this disclosure, preferably pharmaceutically acceptable salts or solvates thereof, or pharmaceutical compositions comprising them, or pharmaceutical combinations of this disclosure described below, for inhibiting abnormal activity of SHP2 in cells, particularly for inhibiting abnormal cell proliferation in mammals, or for treating and/or preventing SHP2-mediated diseases, particularly those that benefit from SHP2 inhibition.

In another aspect, this disclosure also provides a method for inhibiting abnormal cell proliferation in mammals, comprising administering to the mammal a therapeutically effective amount of a compound of the disclosure, preferably a pharmaceutically acceptable salt or solvate thereof, or a pharmaceutical composition comprising thereof.

In another aspect, this disclosure provides a method for treating and/or preventing SHP2-mediated diseases, particularly those that benefit from SHP2 inhibition, including administering to a subject in need a therapeutically effective amount of a compound of this disclosure, preferably a pharmaceutically acceptable salt or solvate thereof, or a pharmaceutical composition comprising thereof, or a pharmaceutical combination of this disclosure as described below.

In another aspect, the present disclosure provides the use of a compoundsdisclosed herein, preferably pharmaceutically acceptable salts or solvates thereof, or a pharmaceutical composition comprising thereof, in the preparation of medicaments for treating and/or preventing SHP2-mediated diseases, particularly those that benefit from SHP2 inhibition.

Based on the same properties, this disclosure also provides a method for inhibiting SHP2 activity, particularly an in vitro method, which includes contacting or applying a compound of this disclosure, preferably pharmaceutically acceptable salts or solvates thereof, to a sample (e.g., a biological sample), specifically including using cells, cellular or subcellular extracts, and/or biomolecules in an artificial environment. Specifically, this disclosure also provides the in vitro use of a compound of this disclosure, preferably pharmaceutically acceptable salts or solvates thereof, as a SHP2 inhibitor in studies, particularly as research tool compounds for inhibiting SHP2.

With respect to the various methods and uses provided in this disclosure, the SHP2-mediated diseases are selected from: cancer or tumors, cardiovascular diseases, immune disorders, fibrosis, ocular disorders, systemic lupus erythematosus, diabetes, neutropenia, or combinations thereof. Preferably, the diseases are selected from Noonan syndrome (NS), leopard syndrome (LS), juvenile myelomonocytic leukemia (JMML), myelodysplastic syndrome (MDS), neuroblastoma, melanoma, squamous cell carcinoma of the head and neck, acute myeloid leukemia (AML), B - cell acute lymphoblastic leukemia (B-ALL), breast cancer, esophageal cancer, lung cancer, colon cancer, head cancer, gastric cancer, lymphoma, glioblastoma, stomach cancer, pancreatic cancer, or combinations thereof. Preferably, any of the cancers is primary cancer or metastatic cancer.

SHP2-mediated diseases specifically refer to diseases caused by abnormal cell proliferation, whether malignant or benign, and all precancerous cells and cancer cells and tissues, particularly cancer or tumors. The cancer or tumor can be a solid tumor or a hematologic malignancy, and may be hormone-dependent or hormone-resistant; in some embodiments, the cancer is a drug-resistant phenotype of cancer disclosed herein or known in the art, and may be primary or metastatic. Therefore, the compounds, compositions, combinations, and methods disclosed herein can be used to treat precancerous tumor formation, tumor growth, tumor invasion, tumor metastasis, and angiogenesis.

Cancers that can be treated with the compounds disclosed herein are selected from, but are not limited to, the following: juvenile myelomonocytic leukemia (JMML), myelodysplastic syndrome (MDS), acute myeloid leukemia (AML), B-cell acute lymphoblastic leukemia (B-ALL), neuroblastoma, esophageal cancer, breast cancer (including triple-negative breast cancer), lung cancer (including small cell lung cancer, non-small cell lung cancer, bronchioloalveolar carcinoma), lung adenocarcinoma, colon cancer, rectal adenocarcinoma, adenoid cystic carcinoma, gastric cancer, gastrointestinal stromal tumor, head and neck cancer (e.g., squamous cell carcinoma of the head and neck), ovarian cancer, prostate cancer, melanoma, cutaneous or intraocular melanoma, soft tissue sarcoma; oral cavity and pharynx (lips, tongue, mouth, larynx, nasopharynx). Cancers of the stomach, small intestine, large intestine, colon, rectum, anal region, liver and biliary tract, pancreas, bones, connective tissue, skin (including epithelial cell carcinoma), vagina, vulva, cervix, uterus, endometrium, fallopian tubes, urethra, penis, testes, bladder, ureters, kidneys, and other urinary tissues, including renal cell carcinoma, renal pelvis carcinoma, and hepatocellular carcinoma; gastroesophageal cancer, and cancers of the eye, brain, spinal cord, and central and peripheral nervous systems and related structures, such as meningeal carcinoma, primary CNS lymphoma, and oligodendroglioma, neuroblastoma, spinal tumors, brainstem gliomas, or pituitary adenomas; cancers of the thyroid and other endocrine glands, Hodgkin's disease, non-Hodgkin's lymphoma, parathyroid carcinoma, adrenal carcinoma, multiple myeloma, medulloblastoma, and hematopoietic malignancies, including chronic or acute lymphocytic leukemia, chronic or acute myeloid leukemia, chronic myelomonocytic leukemia, and lymphomas, including lymphocytic, granulocytic, and monocyte lymphomas, mantle cell lymphoma, and histiocytic lymphoma.

Therefore, in a preferred embodiment of this aspect, this disclosure provides methods and uses for treating or preventing cancer or tumors by inhibiting SHP2 activity. In a further preferred embodiment, this disclosure provides methods and uses for treating or preventing Noonan syndrome (NS), leopard syndrome (LS), juvenile myelomonocytic leukemia (JMML), myelodysplastic syndrome (MDS), neuroblastoma, melanoma, squamous cell carcinoma of the head and neck, acute myeloid leukemia (AML), B-cell acute lymphoblastic leukemia (B-ALL), breast cancer, esophageal cancer, lung cancer, colon cancer, head cancer, gastric cancer, lymphoma, glioblastoma, stamach cancer, pancreatic cancer, and combinations thereof by inhibiting SHP2 activity.

## Drug combination

The compounds disclosed herein can be administered as the sole active ingredient or in combination with other drugs or therapies.

Therefore, in another aspect, this disclosure provides pharmaceutical combinations comprising a compound disclosed herein, preferably pharmaceutically acceptable salts or solvates thereof, and other active agents, or both. These pharmaceutical combinations are used to treat and/or prevent SHP2 -mediated diseases.

The other active agents may be one or more other compounds disclosed herein, or may be a second or other (e.g., a third) compound that is compatible with the compounds disclosed herein, i.e., does not adversely affect each other,

or has complementary activity. For example, these active agents may be compounds known to regulate other biological active pathways, or may be compounds that regulate different components in the biological active pathways involved by the compounds disclosed herein.

In some embodiments of this disclosure, other active agents that can be used in combination with the compounds of this disclosure include, but are not limited to, alkylating agents, antimetabolites, antimitotic agents, checkpoint inhibitors, topoisomerase inhibitors, cytotoxic antibiotics, aromatase inhibitors, angiogenesis inhibitors, antisteroids or antiandrogens, mTOR inhibitors, tyrosine kinase inhibitors, etc.

The compounds disclosed herein can also be combined with antitumor therapies, including but not limited to surgery, radiation therapy, transplantation (e.g., stem cell transplantation, bone marrow transplantation), tumor immunotherapy, and chemotherapy.

Other active agents used in combination with the compounds of this disclosure may be administered simultaneously, separately, or sequentially with the compounds of this disclosure via the same or different routes of administration. These other active agents may be co-administered with the compounds of this disclosure in a single pharmaceutical composition or separately in different discrete units, such as a combination product, preferably in the form of a kit, and when administered separately, may be simultaneous or sequential, with the sequential administration occurring close together or spaced apart in time. They may be prepared and/or formulated by the same or different manufacturers. Furthermore, the compounds of this disclosure and other active agents may be combined into a combination (i) before being delivered to a physician (e.g., in the case of a kit containing the compounds of this disclosure and other drugs); (ii) by the physician themselves (or under the guidance of a physician) prior to administration; or (iii) by the patient themselves, for example, during the sequential administration of the compounds of this disclosure and other active agents, in combination therapy.

Therefore, in another aspect, this disclosure also provides a kit containing two or more separate pharmaceutical compositions, at least one of which contains a compound of this disclosure or a pharmaceutically acceptable salt, isomer, solvate, hydrate, or stable isotopic variant thereof, and means for containing said compositions, such as containers, dispensing vials, or separate foil packages, and includes instructions for use. The kits of this disclosure are particularly suitable for administering different dosage forms, such as oral and parenteral dosage forms, or for administering different compositions at different dose intervals.

With respect to the technical solutions of the pharmaceutical compositions, pharmaceutical combinations or kits disclosed above, the diseases mediated by SHP2 involved are as defined above for the methods and uses of this disclosure.

With respect to the compounds, pharmaceutical compositions, methods, uses, pharmaceutical combinations and kits disclosed herein, the compounds of the embodiments herein are preferred.

## Preparation method of the compound of the present invention

In another aspect, this disclosure also provides methods for preparing the defined compounds.

Compounds disclosed herein can be prepared by a variety of methods, including the general methods given below, the methods disclosed in the examples, or similar methods.

Standard synthetic methods and procedures for the preparation of organic compounds and the transformation and manipulation of functional groups are known in the art and can be found in standard textbooks, such as Smith MB, "March's Advanced Organic Chemistry: Reactions, Mechanisms, and Structure", 7th edition, Wiley, 2013. For each reaction step of each general synthetic scheme, appropriate reaction conditions are known to those skilled in the art or can be routinely determined. The method steps for synthesizing the disclosed compounds may be carried out under reaction conditions known per se (including those specifically mentioned), in the absence or generally in the presence of solvents or diluents (including solvents or diluents that are inert to the reagents used and soluble in the reagents used), in the absence or in the presence of catalysts, condensing agents or neutralizing agents (e.g. ion exchangers, such as cation exchangers, e.g., in the $H^+$ form), depending on the nature of the reaction and/or the reactants, at reduced, normal or elevated temperatures (e.g., from about -100°C to about 190°C, including, for example, from about -78°C to about 150°C, e.g., from about 0 °C to about 125 °C, room temperature, -20 to 40°C or reflux temperature), at atmospheric pressure or in a closed container, under pressure when appropriate, and/or in an inert atmosphere such as argon or nitrogen.

Unless otherwise specified, the starting materials and reagents used in the preparation of the compounds are commercially available, or are known in the literature, or can be prepared by those skilled in the art using the methods described below, similar methods, or standard methods known in the art. Unless otherwise stated in the method description, applicable solvents are those conventional solvents well-known to those skilled in the art suitable for the specific reaction type involved, such as water, esters, ethers, liquid aromatic hydrocarbons, alcohols, nitriles, halogenated hydrocarbons, amides, bases, carboxylic anhydrides, cyclic, straight-chain or branched hydrocarbons, or mixtures of these solvents. Such solvent mixtures can also be used for post-processing, such as post-processing by chromatography or partitioning.

If necessary, the starting materials and intermediates in the synthetic reaction scheme can be separated and purified using conventional techniques, including but not limited to filtration, distillation, crystallization, and chromatography. If the intermediates and final products are obtained in solid form, purification can also be performed by recrystallization or aging. The materials can be characterized using conventional methods, including physical constants and spectral data. The reaction mixture is post-treated in a conventional manner, for example by mixing with water, separating the phases, and purifying the crude product by chromatography where appropriate.

Those skilled in the art will recognize the presence of a stereocenter in the compounds disclosed herein. At all stages of the reaction, the mixture of isomers formed can be separated into individual isomers, such as diastereomers or enantiomers, or into any desired mixture of isomers, such as racemates or mixtures of diastereomers, see, for example, "Stereochemistry of Organic Compounds" (Wiley-Interscience, 1994) by E.L. Eliel, S.H. Wilen, and L.N. Mander.

In certain specific situations, it may be necessary to use appropriate protecting groups to protect specific reactive groups in order to avoid interfering with the reactions of other reactive groups. Suitable protecting groups and methods for protection and deprotection using such suitable protecting groups are well known to those skilled in the art, and examples can be found in T. Greene and P. Wuts, *Protective Groups in Organic Synthesis* (3rd ed.), John Wiley & Sons, NY (1999).

The following description only illustrates general synthetic schemes for synthesizing the compounds disclosed herein. Other routes and other reactants and intermediates known to those skilled in the art can also be used to obtain the compounds disclosed herein.

For clarity, in the exemplary synthetic schemes described below, unless otherwise specified, X, Y, Z, and R appearing in the structural formulas of the various intermediate compounds are as defined above for the compounds disclosed herein, wherein PG represents a suitable protecting group that can be determined by those skilled in the art based on their knowledge of organic chemistry.

**Synthesis of the compounds of disclosure**

**General Synthesis Methods**

In the following schemes, each variable in the general formula has the same meaning as the corresponding variable in formula (I) or its various specific embodiments given above, unless otherwise stated.

**Scheme 1**

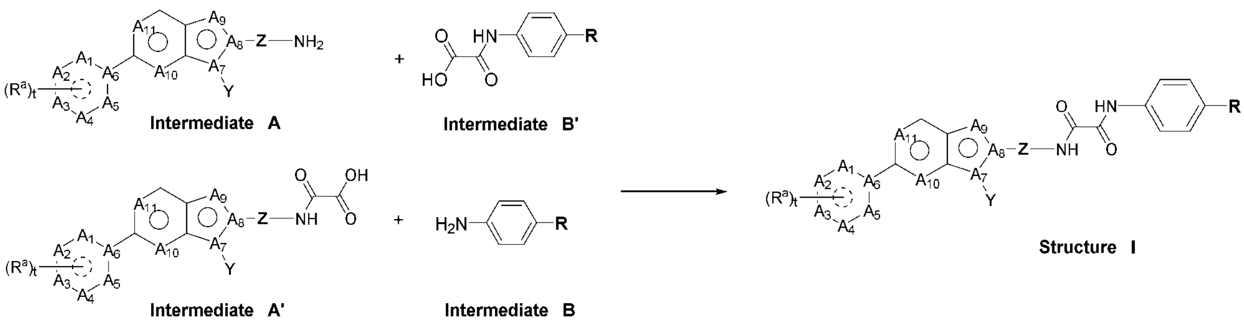

Intermediate A, Intermediate B', Intermediate A', Intermediate B, Structure I

**Scheme** 1 illustrates a general synthetic route that can be used to prepare the compounds of this disclosure and their various specific embodiments.

To take 6-tetrazol indoles as an example:

**Scheme 2**

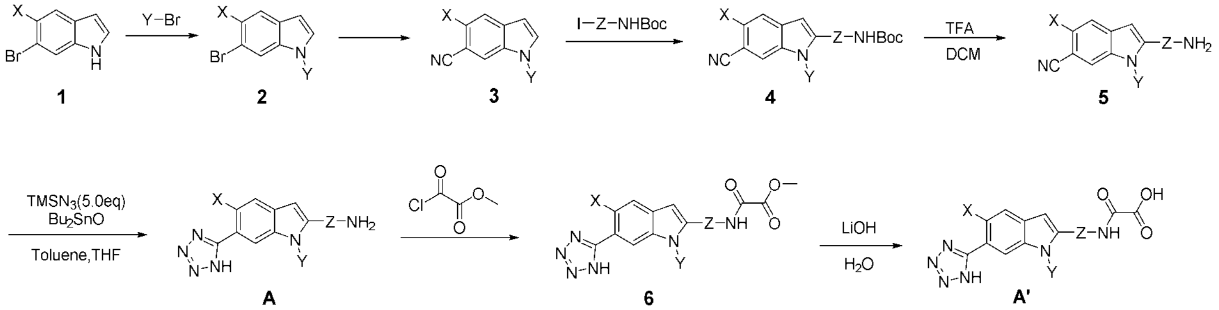


1, 2, 3, 4, 5, A, 6, A'

Scheme 2 illustrates a synthetic route for the compounds of formula A and A' in Scheme 1.

## Scheme 3

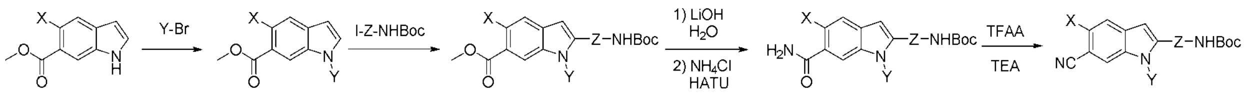


7 8 9 10 4

Scheme 3 illustrates a synthetic route for the intermediate of formula 4 in Scheme 2.

## Scheme 4

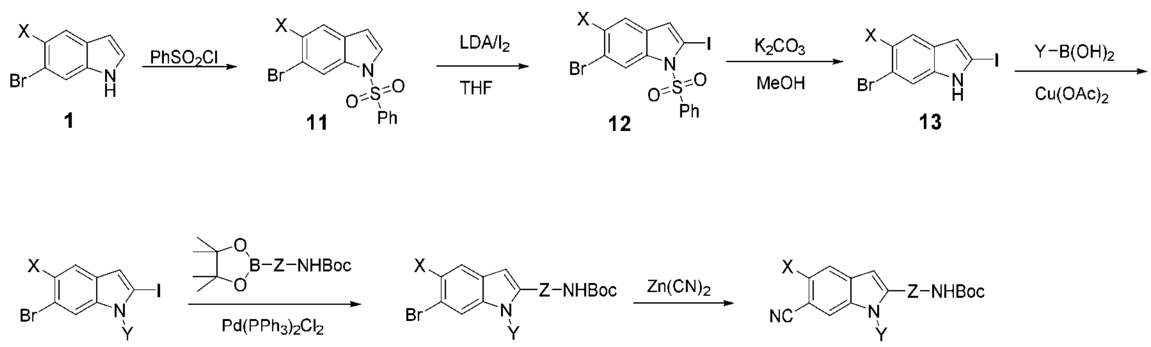


1 11 12 13

14 15 4

Scheme 4 illustrates a synthetic route for the intermediate of formula 4 in Scheme 2.

## Scheme 5

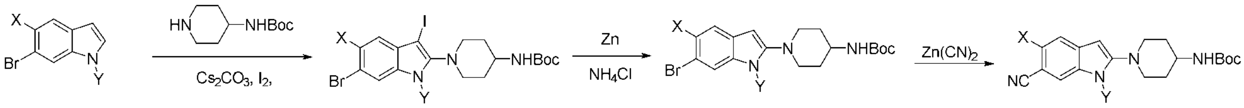


2 16 17 **18** (one of **4**)

Scheme 5 illustrates a synthetic route for the intermediate 4 in Scheme 2 (when Z is a cyclic amine (e.g., an alicyclic amine)).

## Scheme 6

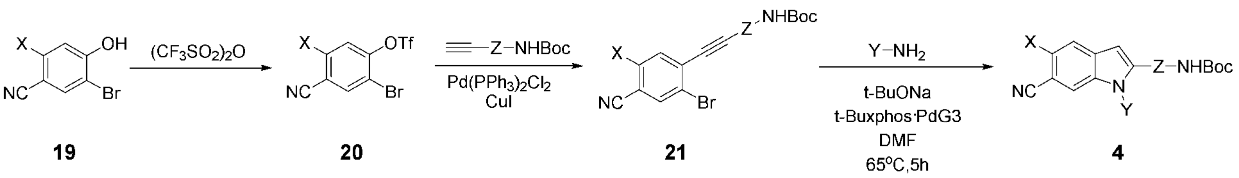


19 20 21 4

Scheme 6 illustrates a synthetic route for the intermediate of formula 4 in Scheme 2.

## Scheme 7

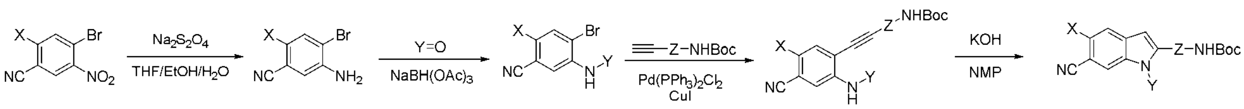


22 23 24 25 4

Scheme 7 illustrates a synthetic route for the intermediate of formula 4 in Scheme 2.

For a non-indole intermediate A

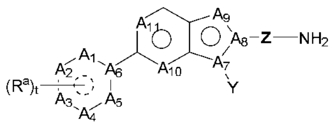
,

azaindole compounds can be synthesized from commercial azaindole

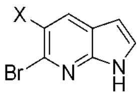

as a raw material, similar to Scheme 2.

For a non-indole intermediate A

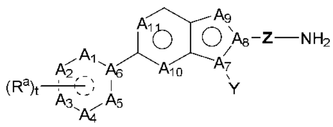
,

benzofuran compounds can be synthesized using a similar scheme as in Scheme 2. Scheme 8 illustrates one synthetic route for the intermediate 4.

## Scheme 8

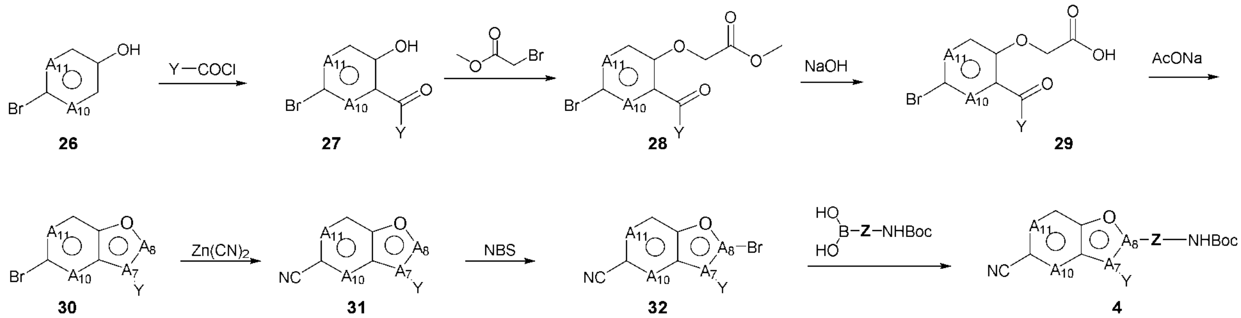


26 27 28 29

30 31 32 4

For a non-indole intermediate A

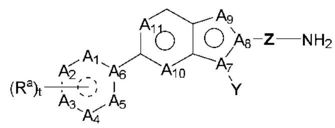
,

benzothiophene compounds can be synthesized using a scheme for benzofuran compounds. Scheme 9 illustrates one synthetic route for benzothiophene intermediate 27a (a substitute for the intermediate 27).

## Scheme 9

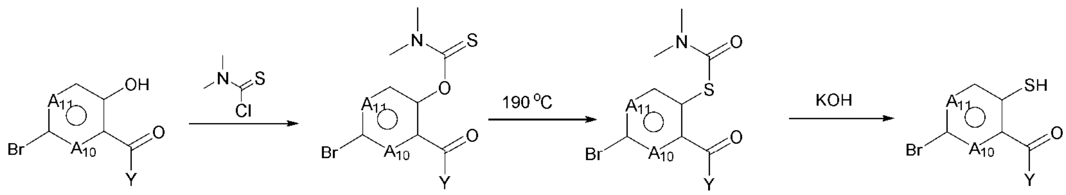


27 33 34 27a

For a non-indole intermediate A

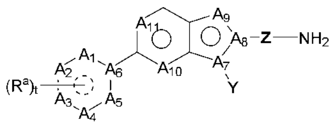

,

such as pyrimidine-containing fused-ring compounds, A can be synthesized using a similar scheme as in Scheme 2. Scheme 10 illustrates one synthetic route for the intermediate 4.

## Scheme 10

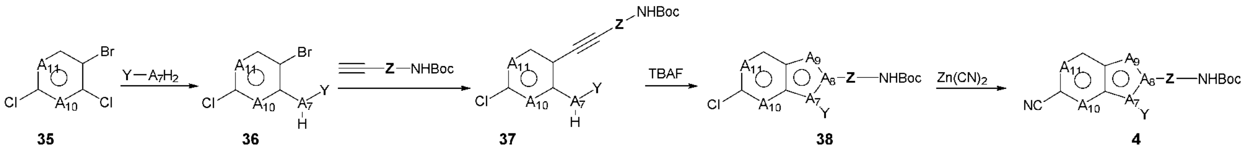


35 36 37 38 4

For a non-6-tetrazazole indole intermediate A

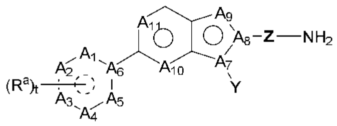

,

when

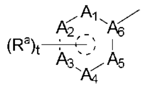

is

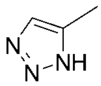

,

Scheme 11 illustrates a synthetic route for the intermediate A.

## Scheme 11

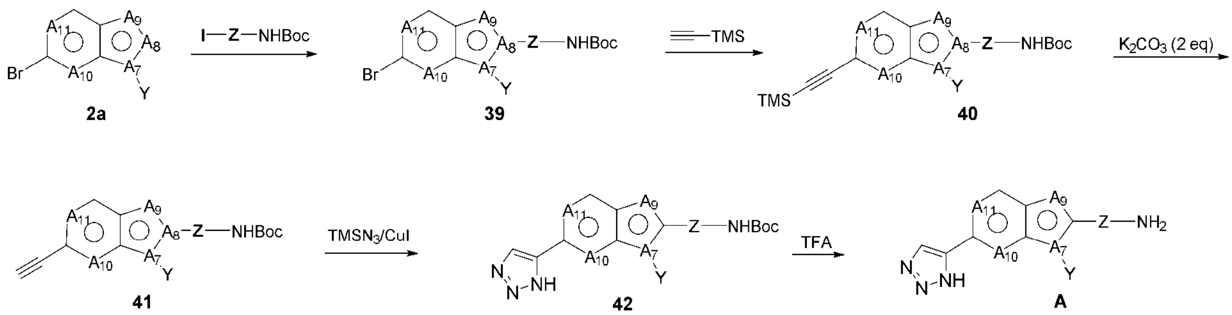


2a 39 40 41 42 A

When

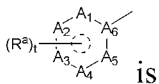 is 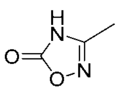, 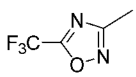, 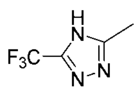,

Scheme 12 illustrates a synthetic route for the intermediate A.

## Scheme 12

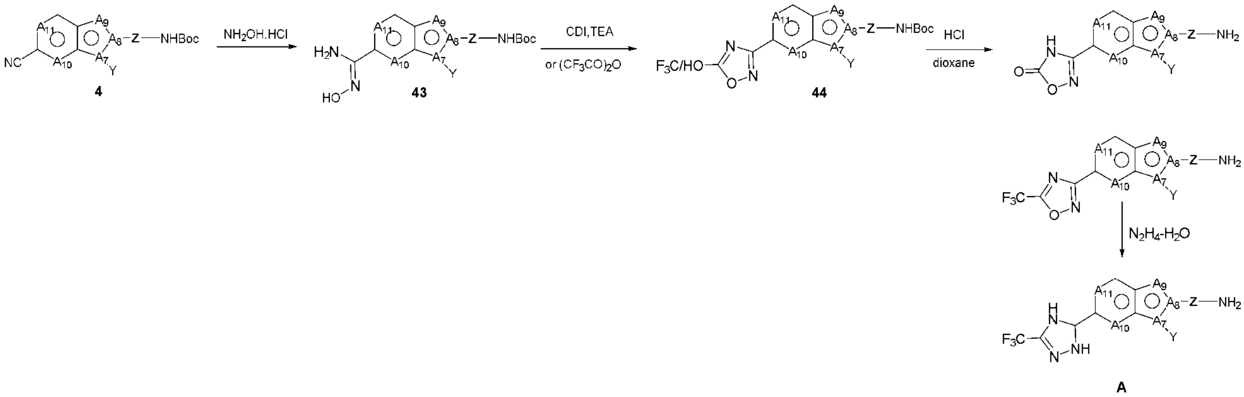


When

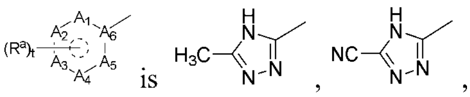
is , ,

Scheme 13 illustrates a synthetic route for the intermediate A.

## Scheme 13

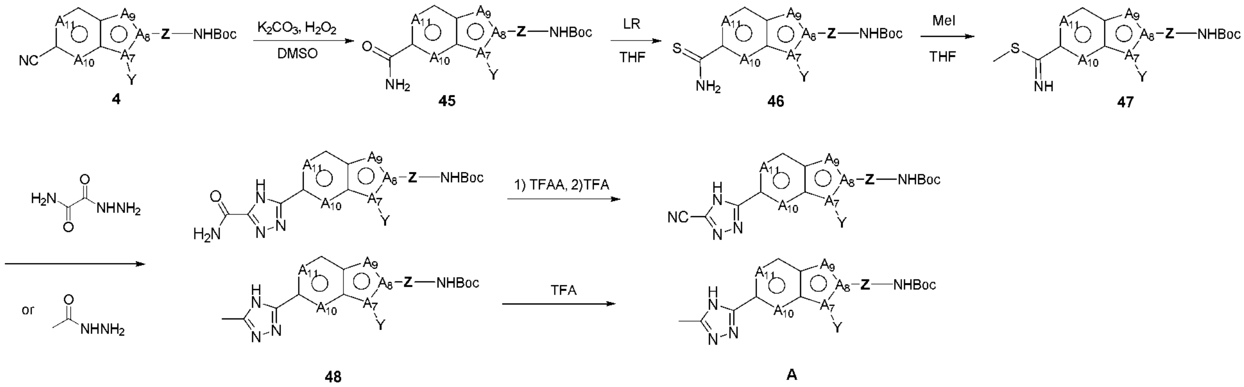


## Scheme 14

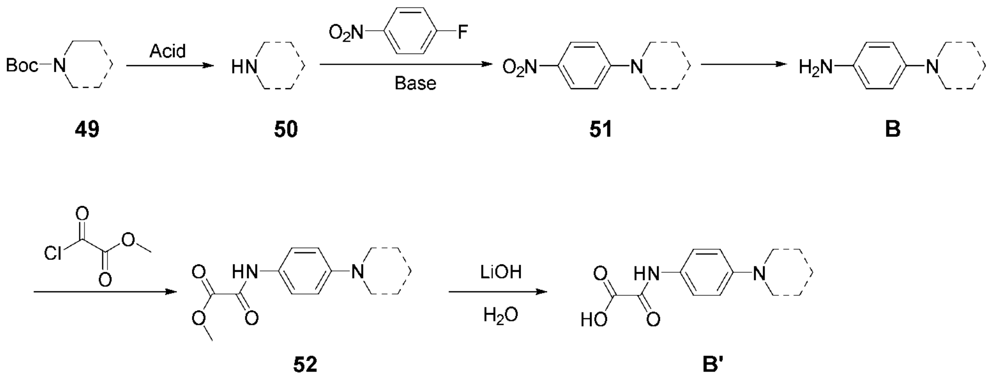


Scheme 14 illustrates a synthetic route for compounds of formula B and B' when R in Scheme 1 is a cyclic amine

(especially an alicyclic amine).

## Scheme 15

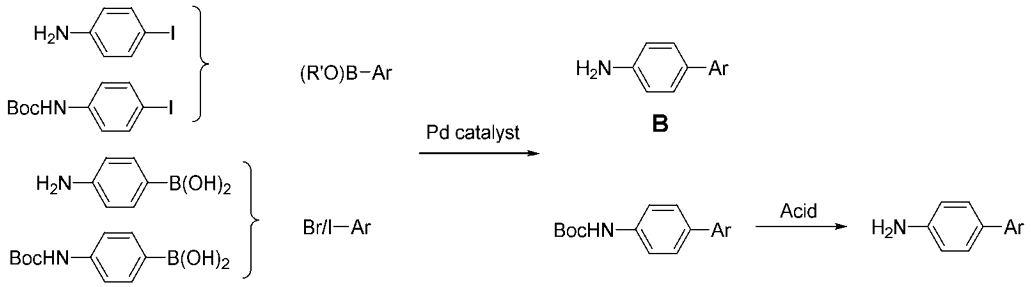


**53** **54** **55** **B**

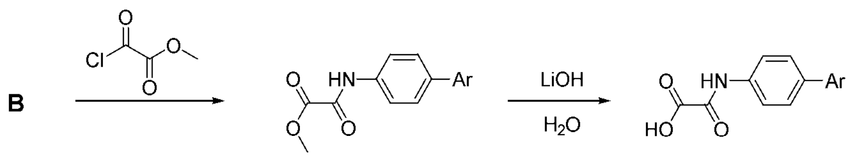


**52** **B'**

Scheme 15 illustrates that, when R in Scheme 1 is an aryl group (including heteroaryl groups), compounds B and B' can be obtained by any aryl-aryl coupling reaction.

In the above Schemes, the order of specific unit reactions can be flexibly adjusted according to conventional conditions known to those skilled in the art.

Specifically, the present invention provides methods for preparing the above-mentioned compounds, comprising:

**1. Preparation of intermediates A and A'**

Compound of formula 1

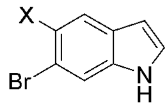

**1**

is reacted with a halogenated compound (such as Y-Br) in the presence of with a base (such as cesium carbonate) in a solvent such as DMF, to produce Compound 2, which can also be obtained by reacting a compound of formula 1 with the corresponding organohalide (such as an iodide) or organoboronic acid in the presence of a catalyst such as a copper salt (such as copper iodide, copper acetate, etc.).

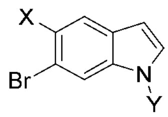

**2**

Compound 2 is reacted with a cyaniding agent (e.g., copper cyanide or zinc cyanide) in an organic solvent such as NMP to produce Compound 3.

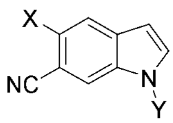

**3**

Compound of formula 3 and a halogenated compound (such as I-Z-NHBoc) are catalyzed in the presence of silver oxide with a catalyst such as palladium/2-nitrobenzoic acid to introduce a substituent Z, yielding Compound of Formula 4. Compound of gormula 4 can also be obtained by reacting Compound of formula 3 with an organoboronic acid under the co-catalysis of a catalyst such as a silver salt (such as silver trifluoroacetate) and a rhodium reagent (such as $(RhCpCl_2)_2$).

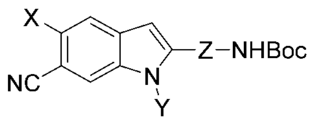

**4**

The cyano group in Compound of formula 4 can also be prepared by a series of transformations of carboxylic acid derivatives (such as Compound of formula 7) via an intermediate of formula 10.

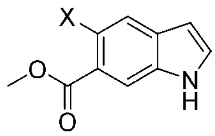

**7**

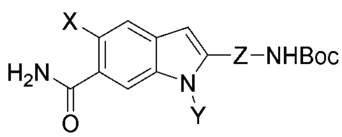

**10**

The introduction of substituent Z can also be achieved by reacting organoboronic acid with intermediate 14 under the catalysis of palladium reagent (such as $(Pd(PPh_3)_2Cl_2)$).

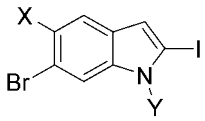

**14**

When Z is an alicyclic amine (e.g., piperidine), the Compound of formula 2 reacts with the amine in the presence of iodine to produce an intermediate of formula 16.

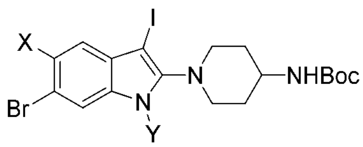

**16**

The iodine atom can be removed by reduction with zinc powder. The bromide is converted into a cyano homologue of formula 4 by using a cyaniding reagent, as shown above.

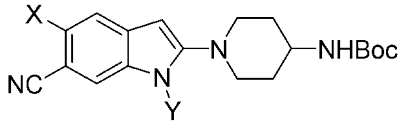

**18** (one of **4**)

Another method for preparing Compound 4 is to activate phenolic Compound 19 to trifluoromethanesulfonate 20.

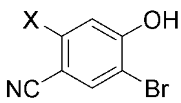
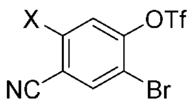

**19** **20**

Then, it reacts with terminal alkynes in the presence of a catalyst such as palladium (e.g., $Pd(PPh_3)\ _2Cl_2$) to produce the phenylacetylene 21.

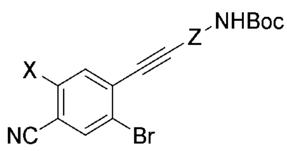

**21**

Then, under the action of a strong base (such as sodium tert-butoxide) and a palladium catalyst (such as *t*-Buxphos. $PdG_3$), it directly reacts with Y-$NH_2$ to form a cyclization compound, thus obtaining Compound of formula 4.

The preparation of Compound of formula 4 can also start from nitrobenzene derivative 22, which is reduced to the corresponding aniline 23 by a reducing agent (such as sodium dithionate), and then is reductively aminated with ketone Y=O to obtain an intermediate of formula 24.

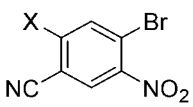
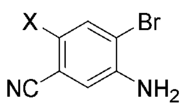
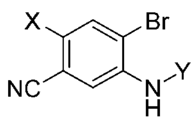

**22** **23** **24**

Using similar reaction conditions, the intermediate of formula 24 is reacted with a terminal alkyne in the presence of a catalyst such as palladium (e.g., $Pd(PPh_3)_2Cl_2$) to produce the phenylacetylene 25.

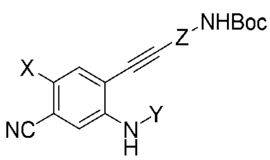

**25**

The phenylacetylene 25 is then treated with a strong inorganic base (such as potassium hydroxide) to obtain Compound formula 4. Then, under standard conditions, the amino protecting group Boc is removed to give the intermediate 5.

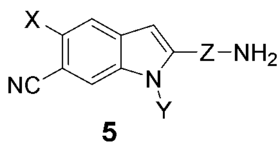

**5**

Further treatment with an azide reagent (sodium azide, trimethyl silane azide, etc.) yields the intermediate A-series of compounds.

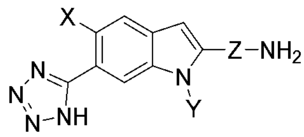

A

Further reaction with monomethyl oxalate chloride yields Compound of formula 6.

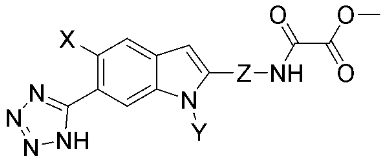

6

Compound of formula 6 is treated under standard ester hydrolysis conditions, the intermediate A' series of compounds can be prepared.

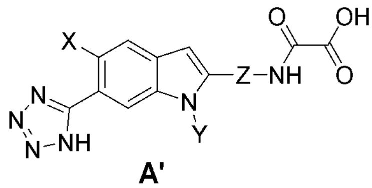

A'

Azaindole compounds can be synthesized from a commercial azaindole

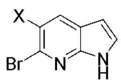

using procedures similar to those described above.

Benzofuran compounds can be synthesized using a scheme similar to Scheme 2. The preparation of its key intermediate 4 can begin with Compound 26, which reacts with an acyl chloride in the presence of anhydrous aluminum trichloride to yield Compound 27.

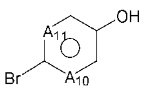

26

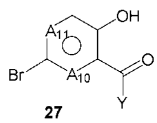

27

Then, under the action of a base (such as sodium hydride), it undergoes substitution with a haloacetic acid ester (such as methyl 2-bromoacetate) to obtain Compound of formula 28.

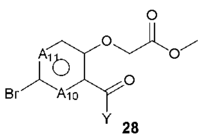

28

Compound of formula 28 is hydrolyzed again and treated with an alkali (such as sodium acetate) to cyclize and obtain Compound 30. The bromide Compound 30 is then converted to a cyano compound using a cyaniding reagent (such as zinc cyanide), and then brominated using a brominating reagent (such as *N*-bromosuccinimide) to obtain Compound 32, which is then processed by introducing a substituent Z with an organoboronic acid under the catalysis of a palladium reagent (such as ($Pd(PPh_3)_2Cl_2$)).

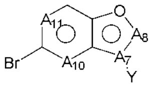
30

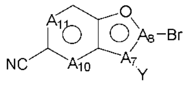
32

For benzothiophene compounds, A can be synthesized using the scheme for benzofuran compounds. The preparation of its key intermediate 27a can start with Compound 27, treated with Dimethylthiocarbamoyl chloride to obtain Compound 33 which is rearranged upon heating to obtain Compound 34, and then treated with an alkali (such as sodium hydroxide) to obtain 27a.

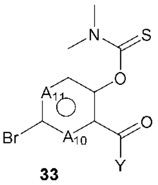
33

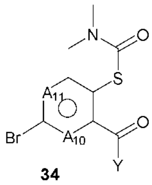
34

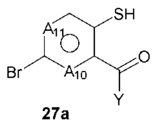
27a

For pyrimidine-containing fused-ring compounds, A can be synthesized using a scheme similar to Scheme 2. The preparation of its key intermediate 4 can begin with Compound **35,** which undergoes a substitution reaction in the presence of an organic base (such as *N,N*-diisopropylethylamine) to obtain Compound **36.**

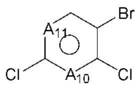
35

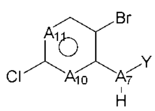
36

Then, it reacts with a terminal alkyne under the catalysis of catalysts such as palladium (e.g., $Pd(PPh_3)_2Cl_2$) and Cu (e.g., cuprous iodide) to generate the phenylacetylene **37.**

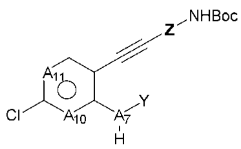
37

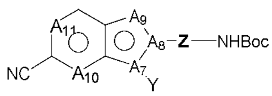
4

After treatment with tetrabutylammonium fluoride, the chlorinated compound is converted into Compound of formula 4 using a cyaniding agent (such as zinc cyanide).

Compound of formula **4** can also be prepared by starting from the commercial raw material **56,** which undergoes a substitution reaction in the presence of a base (such as cesium carbonate) to obtain Compound of formula **57.** Then, in the presence of silver oxide and under the catalysis of a catalyst such as palladium/2-nitrobenzoic acid, it reacts with a halogenated compound (such as I-Z-NHBoc) to introduce the substituent Z, thus obtaining Compound of formula **4.**

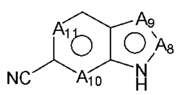
56

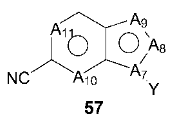
57

For the synthesis of non-tetrazole compounds, following the heterocycle formation, a similar scheme to Scheme 2 is used to synthesize A. The heterocyclic synthesis process is illustrated below. Triazole compounds can be synthesized from commercial starting material 2a, which is reacted with a halide (such as I-Z-NHBoc) in the presence of silver oxide and catalyzed by a catalyst such as palladium/2-nitrobenzoic acid to introduce a substituent Z, yielding Compound 39. This Compound 39 is then reacted with trimethylethynylsilane in the presence of a copper catalyst (such as cuprous iodide) and a palladium catalyst (such as tetrakis(triphenylphosphine)-palladium) to yield 40. Further deprotection, followed by treatment with azide-trimethylsilane and further deprotection, A is prepared.

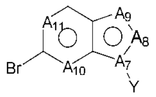
2a
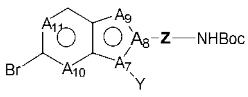
39
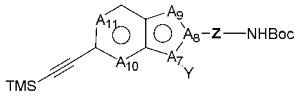
40
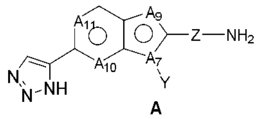
A

More non-tetrazole acidic heterocyclic intermediates A can be obtained by starting from intermediate 4, treated with hydroxylamine hydrochloride to yield Compound 43, then dehydrated with a dehydrating agent (such as CDI, trifluoroacetic anhydride, etc.) to yield Compound 44, and then deprotected to prepare A.

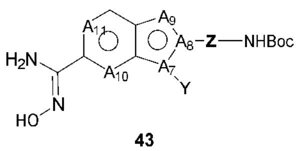
43
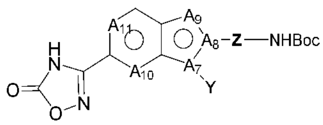
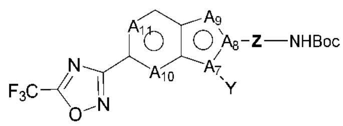
44

Intermediate 4, when reacted with an inorganic strong base (such as potassium hydroxide) and hydrogen peroxide in a solvent (such as DMSO), can yield the amide intermediate 45. This intermediate then reacts with Lawson's reagent to generate the corresponding thioamide 46. After treatment with a methylating agent (such as iodomethane), Compound 47 is obtained. This compound then reacts with 2-hydrazino-2-carbonylacetamide and is dehydrated in the presence of trifluoroacetic anhydride to form 48. Finally, the protecting group is removed to obtain the cyano-substituted intermediate A.

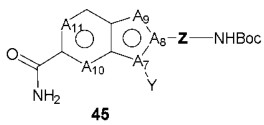
45
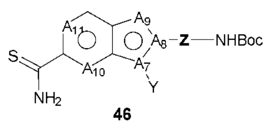
46
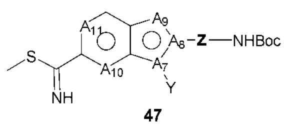
47
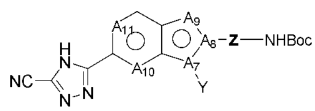
48

If Compound 47 is treated with acetylhydrazine, the protecting group can be removed to prepare the methyl-substituted intermediate A.

## 2. Preparation of intermediates B and B'

When R is aryl (including heterocyclic aryl), the intermediate of formula B can be obtained by various aryl-aryl coupling reactions, such as the reaction of Suzuki arylboronic acid derivatives with their corresponding haloaryles in the presence of a palladium catalyst. In this disclosure, both components of the coupling reaction can be arylboronic acid derivatives or their corresponding haloaryles, such as Compounds 53 and 54, coupling to form the intermediate of formula B.

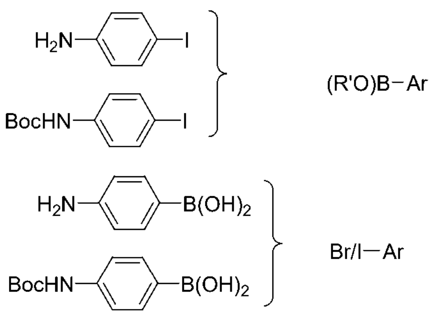
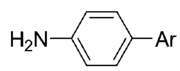
B

53 54

Intermediate B undergoes a transformation process similar to that described above from intermediate A to intermediate A', and is transformed into intermediate B'.

When R is an alicyclic amine, the free amine 50 (or deprotecting Compound 49 to obtain the corresponding free amine) and p-nitrobenzene

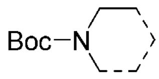
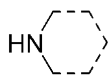

**49** **50**

undergoes aromatic ring substitution in the presence of a weak base (e.g., potassium carbonate) to yield Compound 51.

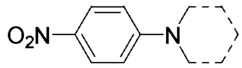

**51**

The nitro group can be converted by palladium-carbon or an inorganic reducing agent (such as sodium dithionate) to obtain an aniline derivatives of formula B.

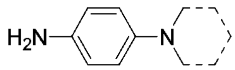

**B**

Intermediate B reacts with oxaloyl chloride monoester, followed by hydrolysis, to form Compound of formula B'.

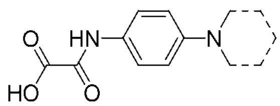

**B'**

## 3. Preparation of example compounds of this disclosure

Example compounds of the disclosure can be prepared by converting oxalic acid intermediate A' or B' into an active intermediate acyl chloride, and then reacting it with B or A respectively. Alternatively, they can be obtained by reacting free acid A' or B' with free amine B or A respectively in the presence of an amide condensing agent (such as $T_3P$, DCC, PyBOP, etc.). Generally, an organic base (such as triethylamine, diisopropylethylamine, etc.) is used simultaneously.

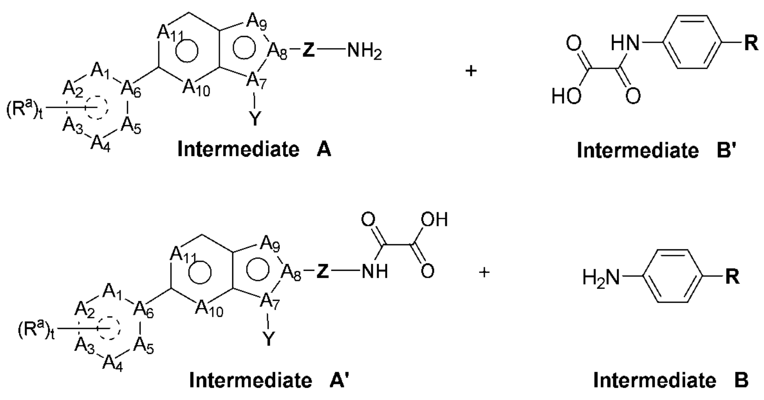

Intermediate A + Intermediate B'

Intermediate A' + Intermediate B

Unless otherwise specified, the reactions in the above general synthesis methods are usually carried out under the conventional conditions known to those skilled in the art, or under the conditions recommended by the manufacturers.

**Specific Embodiments**

The present invention will be further described below with reference to embodiments. It should be noted that the following embodiments are exemplary and should not be regarded as limiting the scope of protection of the present

invention.

In the description of the embodiments subsequent specific examples, the following abbreviations are used: ACN (acetonitrile); AcOH (acetic acid); AgO (silver oxide); b/d/t/q (peak in NMR, b (broad peak)/d(doublet)/t(triplet)/q (quadruplet); Boc (tert-butoxycarbonyl); BSA (bovine serum albumin); t-BuONa (sodium tert-butoxide); $Bu_2SnO$ (di-n-butyltin oxide); *t*-Buxphos-Pd$G_3$ (methanesulfonic acid (2-di-tert-butylphosphine-2',4',6'-triisopropyl-1,1'-biphenyl)(2'-amino-1,1'-biphenyl-2-yl) palladium (II)); $CDCl_3$ (deuterated chloroform); CDI (*N',N'*-carbonyldiimidazole); $CD_3OD$ (deuterated methanol); DiFMUP (6,8-difluoro-4-methyl-7-(phosphoryloxy)-2H-1-benzopyran-2-one); $(CF_3SO_2)_2O$ (trifluoromethanesulfonic anhydride); $CO_2$ (carbon dioxide); $Cs_2CO_3$ (cesium carbonate); CuI (cuprous iodide); $Cu(OAc)_2$ (copper acetate); CYP (cytochrome proteins, such as CYP1A2/CYP2B6/CYP2C8/CYP2C9/CYP2C19/2D6/CYP3A4); DCC (dicyclohexylcarbodiimide); DCM (dichloromethane); DIEA or DIPEA (N,N-diisopropylethylamine); DMEM (a cell culture medium); DMF (*N,N*-dimethylformamide); DMSO (dimethyl sulfoxide); DMSO-$d_6$ (hexadeuterated dimethyl sulfoxide); dppf (1,1'-bis(diphenylphosphine)ferrocene); DTT (dithiothreitol); *E* (double bond trans isomer); EA (ethyl acetate); EDTA (ethylenediaminetetraacetic acid); hERG (hERG gene-encoded Kv11.1 potassium ion channel conduction); Et (ethyl); EtOH (ethanol); FBC (fed-batch fermentation); FCC (fast column chromatography); g (gram); GLP (Good Laboratory Practice for Non-clinical Studies); h (hour); HATU (2-(7-azobenzotriazole)-N,*N,N',N'*-tetramethylurea hexafluorophosphate); HCl (hydrogen chloride); HCl-MeOH (hydrogen chloride in methanol); HEPES (4-hydroxyethylpiperazine ethanesulfonic acid); HFiPA (hexafluoroisopropanol); HLM (human liver microsomes); $H_2O$ (water); $H_3PO_4$ (phosphoric acid); $H_2SO_4$ (sulfuric acid); Hz (Hertz); $I_2$ (iodine); IC50 (inhibitor concentration at 50% inhibition); IV (intravenous administration); IMDM (a cell culture medium); *J* (nuclear magnetic resonance coupling constant); $K_2CO_3$ (potassium carbonate); Km (Michaelis constant for enzyme reaction); KOH (sodium hydroxide); L (volume liter); LCMS (Liquid chromatography-mass spectrometry); LC-MS/MS (Liquid-to-Mass Spectrometry-to-Mass Spectrometry); LDA (lithium diisopropyl amide); LiOH (lithium hydroxide); LR (Rosen reagent); Lum (luminescence intensity); MeI (methane iodide); MEM (a cell culture medium); MeMgBr (magnesium methyl bromide); MeOH (methanol); Methanol-*d4* (tetradeuterated methanol); mg (milligram); MHz (megahertz); min (minute); mL (milliliter); mmol (millimole); MOM (methoxymethyl ether); MS-ESI (electrospray ionization mass spectrometry); MTBE (methyl tert-butyl ether); m/z (mass-to-charge ratio); $N_2$ (nitrogen); $NaBH(OAc)_3$ (sodium borohydride triacetate); NaCl (sodium chloride); NADPH (nicotinamide adenine dinucleotide phosphate); NaH (sodium hydride); $NaHCO_3$ (sodium bicarbonate); $Na_2SO_2O_4$ (Sodium dithionite); $Na_2SO_3$ (sodium sulfite); $Na_2SO_4$ (sodium sulfate); NBS (bromosuccinimide); NCS (chlorosuccinimide); $NH_4Cl$ (ammonium chloride); $N_2H_4$-$H_2O$ (hydrazine hydrate); NMP (*N*-methylpyrrolidone); NMR (nuclear magnetic resonance, $^{1H}$ (proton)/$^{13c}$(carbon-13)/19F (fluorine-19)); pNPP (disodium p-nitrophenyl phosphate); $PdCl_2$(dtbpf) (1,1'-bis(di-tert-butylphosphine)ferrocene palladium dichloride); Pd(dppf)$Cl_2$ (1,1'-bis(diphenylphosphine)ferrocene palladium dichloride); Pd(OAc)(palladium acetate); $Pd(PPh_3)_4$ (tetraphenylphosphine palladium); $Pd(PPh_3)_2Cl_2$ (Tetraphenylphosphine palladium dichloride); PE (petroleum ether); $PhSO_2Cl$ (benzenesulfonyl chloride); PO (oral administration); $POCl_3$ (phosphorus oxychloride); PTP (protein tyrosine phosphatase, e.g., SHP1 (Src homology region 2 protein tyrosine phosphatase 1)/SHP2 (Src homology region 2 protein tyrosine phosphatase 2)/HePTP (hematopoietic protein tyrosine phosphatase)/Laforin (EPM2A dextran phosphatase)/LMWPTP (low molecular weight protein tyrosine phosphatase)/LYP (lymphoid tyrosine phosphatase)/PTP1B (protein tyrosine phosphatase 1B)/PRL (liver regeneration phosphatase)/SSU72 (SSU72 protein phosphatase)/VHR (bispecific protein phosphatase 3)/FAP1 (S.parasanguinis adhesin)/STEP (striatal-enriched protein tyrosine phosphatase)/CDC14A (cyclin 14A) / CD45 (C-type protein tyrosine phosphatase receptor) / PP5 (protein phosphatase 5)); PyBOP (benzotriazol-1-yl-oxytripyrrolidine phosphonium); RT (room temperature); QT (time required for ventricular depolarization and repolarization); $SiO_2$ (silica gel); RFU (fluorescence intensity); rpm (revolutions per minute); T3P (1-Propylphosphonic acid cyclic anhydride); TBAF (tetrabutylammonium fluoride); TEA (triethylamine); Tf (trifluoromethanesulfonyl); TFA (trifluoroacetic acid); TFAA (trifluoroacetic anhydride); THF (tetrahydrofuran); TIPS (triisopropylsilyl); TLC (thin-layer chromatography); TMS (trimethylsilane); $TMSN_3$ (Trimethylsilyl azide); TsOH (p-toluenesulfonic acid); TsOH-$H_2O$ (p-toluenesulfonic acid monohydrate); *Z* (Ci-isomer of double bond); Zn (zinc); $Zn(CN)_2$ (zinc cyano); $\delta$ (nuclear magnetic resonance chemical shift); $\mu$L (microliter); $\mu$M (micromolar concentration); $\mu$mol (micromolar).

In the following examples, the names and structures of the synthesized compounds are given. Any discrepancies between the names and structures are not intentional; in this case, the structure controls.

Experimental methods in the following examples that are not specified for conditions, are generally performed under standard conditions for such reactions or as recommended by the manufacturers. Unless otherwise specified, percentages and parts are by weight, and liquid ratios are by volume, and all temperatures mentioned are given in degrees Celsius.

Unless otherwise specified, all experimental materials and reagents used in the following examples are commercially available, prepared using prior art methods, or prepared using methods similar to those disclosed in this application. Unless otherwise stated, all reagents are used without further purification.

In the following examples, $^1$H-NMR spectra were recorded using a Bruker (400 MHz) instrument, and chemical shifts are expressed as $\delta$ (ppm) relative to the deuterated solvent peaks ($CDCl_3$: $\delta$ = 7.26 ppm; $CD_3OD$: $\delta$ = 3.31 ppm;

DMSO-$d_6$: δ = 2.50 ppm). Liquid chromatography-mass spectrometry (LC-MS) was performed using an Aglient 1260 LC-MS system with an Aglient G6125B mass spectrometer. Gas chromatography-mass spectrometry (GC-MS) was performed using a Shimadzu GCMS-QP2010SE.

## 1. Synthesis of intermediates

### Synthesis of intermediate AA1: 6-bromo-1-cyclopropyl-5-fluoro-*1H*-indole

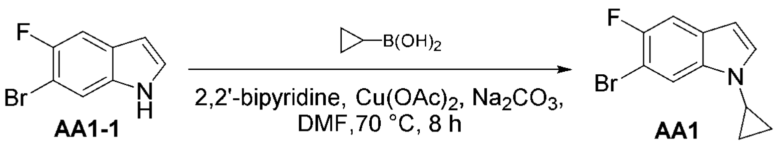


To a DMF solution (20 mL) of 6-bromo-5-fluoro-*1H*-indole **AA1-1** (1.00 g, 4.67 mmol) and cyclopropylboronic acid (1.20 g, 14.0 mmol), 2,2'-bipyridine (2.20 g, 14.0 mmol), copper acetate (1.70 g, 9.34 mmol), and sodium carbonate (0.99 g, 9.34 mmol) were added, and the mixture was reacted at 70 °C for 8 hours. The reaction mixture was cooled to room temperature, added with water (20 mL) and extracted three times with ethyl acetate (30 mL). The organic phase was washed with saturated brine, dried over anhydrous sodium sulfate, filtered, and concentrated to obtain the crude product. The crude product was subjected to silica gel column chromatography (PE/EA = 5/1) to give a yellow liquid AA1 (0.91 g, 77% yield). $^1$H NMR (400 MHz, $CDCl_3$) δ 7.72-7.71 (m, 1H), 7.31 (d, *J* = 9.2 Hz, 1H), 7.15 (d, *J* = 3.2 Hz, 1H), 6.37-6.36 (m, 1H), 3.32-3.29 (m, 1H), 1.11-1.00 (m, 2H), 0.99-0.98 (m, 2H). MS-ESI $[M+H]^+$: 253.9.

### Synthesis of intermediate AA2: 2-(4-aminophenyl)-1-cyclobutyl-6-(1H-tetrazol-5-yl)-1H-indole-5-ol

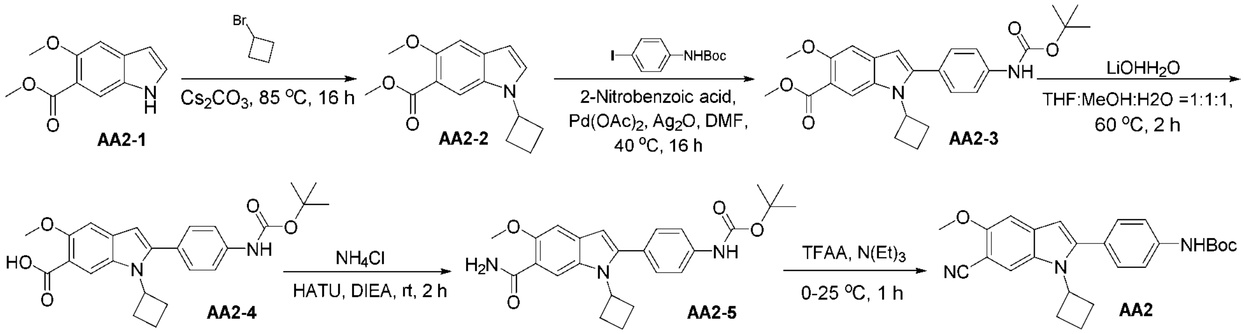


**Step** 1: Methyl 1-cyclobutyl-5-methoxy-*1H*-indole-6-carboxylic acid

Cyclobutyl bromide (5.05 g, 37.4 mmol) was added to a DMF solution (18 mL) of methyl 5-methoxy-*1H*-indole-6-carboxylate **AA2-1** (2.40 g, 11.7 mmol) and cesium carbonate (13.3 g, 40.9 mmol). The resulting mixture was heated to 85 °C under nitrogen atmosphere and stirred for 16 hours. After the reaction was complete, the mixture was cooled, filtered, and the filtrate was washed with lithium chloride aqueous solution and concentrated. The filtrate was purified by silica gel column chromatography (PE/EA = 4/1) to give a light green oily substance **AA2-2** (2.3 g). MS-ESI $[M+H]^+$ : 260.0.

**Step 2:** 2-(4-((tert-butoxycarbonyl)amino)phenyl)-1-cyclobutyl-5-methoxy-*1H*-indole-6-carboxylic acid methyl ester

Palladium acetate (115 mg, 0.511 mmol) was added to a DMF solution (25 mL) of methyl 1-cyclobutyl-5-methoxy-1H-indole-6-carboxylic acid **AA2-2** (2.65 g, 10.2 mmol), tert-butyl (4-iodophenyl)carbamate (4.89 g, 15.3 mmol), 2-nitrobenzoic acid (2.56 g, 15.3 mmol) and silver oxide (1.78 g, 7.66 mmol). The resulting mixture was heated to 40 °C under nitrogen atmosphere and stirred for 16 hours. After the reaction was completed, the mixture was cooled, filtered, and the filtrate was washed with lithium chloride aqueous solution and concentrated. The filtrate was purified by silica gel column chromatography (PE/EA = 65/35) to give a pale yellow solid **AA2-3** (1.5 g). MS-ESI $[M+H]^+$: 451.1.

**Step 3:** 2-(4-((tert-butoxycarbonyl)amino)phenyl)-1-cyclobutyl-5-methoxy-*1H*-indole-6-carboxylic acid

Lithium hydroxide (223.5 mg, 5.33 mmol) was added to a mixed solution of methyl 2-(4-((tert-butoxycarbonyl) amino)phenyl)-1-cyclobutyl-5-methoxy-*1H*-indole-6-carboxylic acid ester **AA2-3** (0.6 g, 1.33 mmol) in THF/MeOH/$H_2O$ = 1/1/1 (12 mL). The resulting mixture was heated to 60 °C and stirred for 2 hours. After the reaction was completed, the mixture was concentrated, the pH was adjusted to 7 with 1 N hydrochloric acid, extracted with ethyl acetate (20 mL), and concentrated to give a white solid **AA2-4** (580 mg). MS-ESI $[M+H]^+$: 437.1.

**Step** 4: (6-Carbamoyl-1-cyclobutyl-5-methoxy-*1H*-indole-2-yl)phenyl)tert-butyl carbamate

HATU (598.9 mg, 1.57mmol) was added to a DMF solution (5 mL) of 2-(4-((tert-butoxycarbonyl)amino)phenyl)-1-cyclobutyl-5-methoxy-1H-indole-6-carboxylic acid **AA2-4** (550 mg, 1.26 mmol), ammonium chloride (674.0 mg, 12.6 mmol), and *N*-ethyl-*N*-isopropyl-2-propane amine (650.2 mg, 1.57 mmol). The mixture was stirred at room temperature for 2 hours. After the reaction was complete, ethyl acetate (20 mL) was added, and the mixture was washed with lithium chloride aqueous solution, concentrated, and purified by silica gel column chromatography (PE/EA = 3/1) to give a pale yellow solid **AA2-5** (550 mg). MS-ESI $[M+H]^+$: 436.1.

**Step 5:** tert-Butyl (4-(6-cyano-1-cyclobutyl-5-methoxy-*1H*-indole-2-yl)phenyl) carbamate

To a DCM solution (6 mL) of (6-carbamoyl-1-cyclobutyl-5-methoxy-*1H*-indol-2-yl)phenyl)carbamate tert-butyl ester **AA2-5** (550 mg, 1.26 mmol) and triethylamine (817.9 mg, 8.08 mmol) was added trifluoroacetic anhydride (848.8 mg, 4.04 mmol) dropwise under an ice-water bath. The mixture was stirred for 1 hour under nitrogen protection. After the reaction was complete, the solution was concentrated, ethyl acetate (20 mL) was added, and the mixture was washed with sodium bicarbonate aqueous solution (10 mL), concentrated again, and purified by silica gel column chromatography (PE/EA = 7/3) to give a brown solid **AA2** (250 mg). MS-ESI $[M+H]^+$ : 418.1.

<u>Synthesis of intermediate AA3:</u> **tert-Butyl (4-(6-cyano-1-cyclopropyl-5-fluoro-*1H*-indol-2-yl)cyclohex-3-en-1-yl) carbamate**

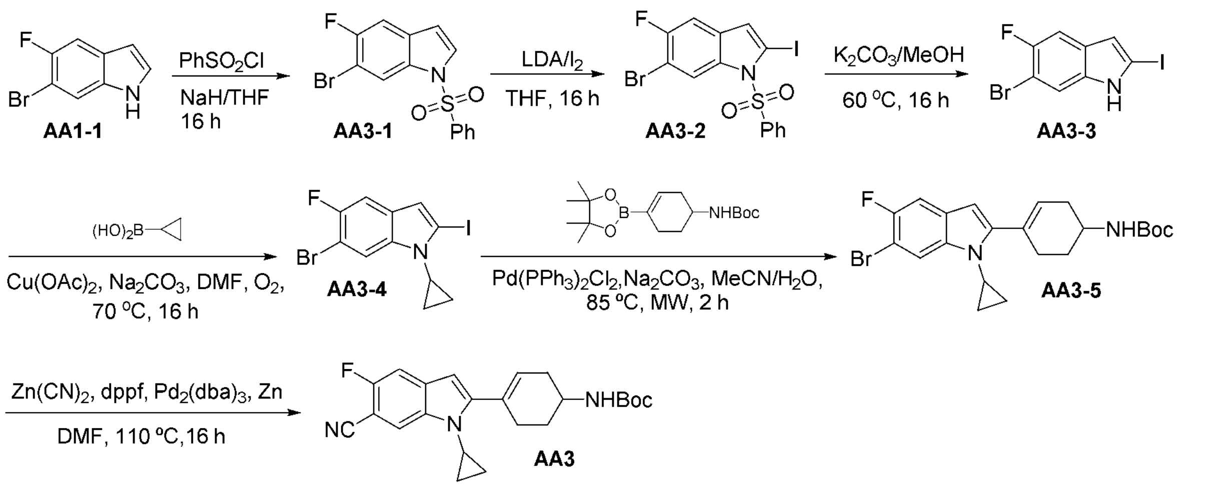


AA1-1 AA3-1 AA3-2 AA3-3 AA3-4 AA3-5 AA3

**Step 1:** 6-Bromo-5-fluoro-1-(benzenesulfonyl)-1H-indole

Sodium hydride (0.90 g, 22.4 mmol) was added to a THF (60 mL) solution of 6-bromo-5-fluoro-*1H*-indole **AA1-1** (4.00 g, 18.7 mmol) at 0 °C and stirred for 20 minutes. Then, benzenesulfonyl chloride (2.90 mL, 22.4 mmol) was added to the reaction solution. The reaction solution was slowly heated to room temperature and stirred for 16 hours. The reaction was quenched by adding saturated ammonium chloride aqueous solution (15 mL), and the mixture was extracted three times with ethyl acetate (80 mL). The organic phase was dried over anhydrous sodium sulfate, filtered, and concentrated to obtain the crude product. The crude product was subjected to silica gel column chromatography (PE/EA = 5/1) to give a white solid **AA3-1** (6.0 g, 90% yield). $^1H$ NMR (400 MHz, DMSO-d6) δ 8.16 (d, *J* = 8.0 Hz, 1H), 8.04-8.02 (m, 2H), 7.96 (d, *J* = 4.0 Hz, 1H), 7.75-7.71 (m, 1H), 7.65-7.60 (m, 3H), 6.87 (d, *J* = 4.0 Hz, 1H).

**Step 2:** 6-Bromo-5-fluoro-2-iodo-1-(benzenesulfonyl)-1H-indole

To a solution of 6-bromo-5-fluoro-1-(phenylsulfonyl)-1H-indole **AA3-1** (3.0 g, 8.5 mmol) in THF (80 mL) at -70 °C, LDA (6.0 mL, 12.7 mmol) was added, and the mixture was stirred for 1.5 hours. Then, a THF (15 mL) solution of elemental iodine (3.2 g, 12.7 mmol) was added to the reaction mixture. The reaction mixture was slowly heated to room temperature and stirred for 16 h. The reaction was quenched by adding a saturated ammonium chloride aqueous solution (15 mL), and the mixture was extracted three times with ethyl acetate (100 mL). The organic phase was dried over anhydrous sodium sulfate, filtered, and concentrated to obtain the crude product. The crude product was subjected to silica gel column chromatography (PE/EA = 5/1) to give a pale yellow solid **AA3-2** (2.0 g, yield 49%). 1H NMR (400 MHz, DMSO-*d6*) δ 8.39-8.38 (m, 1H), 7.90-7.87 (m, 2H), 7.75-7.71 (m, 1H), 7.67-7.63 (m, 2H), 7.54 (d, *J* = 8.0 Hz, 1H), 7.22(s, 1H). MS-ESI [M+H]+: 479.8.

**Step 3:** 6-Bromo-5-fluoro-2-iodo-*1H*-indole

To a MeOH/$H_2O$ solution (80mL, V/V = 5/3) of 6-bromo-5-fluoro-2-iodo-1-(benzenesulfonyl)-1H-indole **AA3-2** (1.4 g, 2.9 mmol), potassium carbonate (4.0 g, 29.2 mmol) was added. The resulting mixture was reacted overnight at 60 °C. The reaction solution was cooled to room temperature and concentrated under reduced pressure to remove the solvent. The residue was dissolved in ethyl acetate (100 mL), washed with water (20 mL), and the organic phase was dried over anhydrous sodium sulfate. The solution was filtered and concentrated to give the crude product. The crude product was subjected to silica gel column chromatography (PE/EA = 3/1) to give a pale yellow solid **AA3-3** (0.7 g, 70% yield). $^1$H NMR (400 MHz, DMSO-$d_6$) δ 11.90 (s, 1H), 7.57-7.55 (m, 1H), 7.44 (d, *J*= 12.0 Hz, 1H), 6.67-6.66 (m, 1H).

**Step 4:** 6-Bromo-1-cyclopropyl-5-fluoro-2-iodo-*1H*-indole

To a DMF (20 mL) solution of 6-bromo-5-fluoro-2-iodide-*1H*-indole **AA3-3** (700 mg, 2.1 mmol) and cyclopropylboronic acid (525 mg, 6.2 mmol), 2,2'-bipyridine (315 mg, 6.2 mmol), copper acetate (1.1 g, 6.2 mmol), and sodium carbonate (455 mg, 4.1 mmol) were added. The resulting mixture was reacted at 70 °C for 16 h under an oxygen atmosphere. The reaction solution was cooled to room temperature, added with water (20 mL) and extracted three times with ethyl acetate (60 mL). The organic phase was washed with saturated brine (10 mL), dried over anhydrous sodium sulfate, filtered, and concentrated to give the crude product. The crude product was subjected to silica gel column chromatography (PE/EA = 5/1) to give a white solid **AA3-4** (350 mg, 44% yield). $^1$H NMR (400 MHz, DMSO-$d_6$) δ 7.82-7.80 (m, 1H), 7.45 (d, *J* = 12.0 Hz, 1H), 6.79 (s, 1H), 3.31-3.24 (m, 1H), 1.30-1.23 (m, 2H), 1.04-1.00 (m, 2H). MS-ESI $[M+H]^+$: 379.9.

**Step 5:** tert-Butyl (4-(6-bromo-1-cyclopropyl-5-fluoro-*1H*-indol-2-yl)cyclohex-3-en-1-yl) carbamate

Potassium carbonate (637 mg, 4.6 mmol) and bis(triphenylphosphine)palladium(II) dichloride (129 mg, 0.18 mmol) were added to a mixed acetonitrile-water solution (40 mL, V/V = 3/1) of 6-bromo-1-cyclopropyl-5-fluoro-2-iodo-1H-indole **AA3-4** (700 mg, 1.8 mmol) and tert-butyl (4-(4,4,5,5-tetramethyl-1,3,2-1,3,2-dioxaborolane-2-yl)cyclohex-3-en-1-yl)carbamate (656 mg, 2.0 mmol). The resulting mixture was heated to 85 °C in a microwave reactor for 2 h. The reaction mixture was cooled to room temperature, added with water (10 mL) and extracted three times with ethyl acetate (60 mL). The organic phase was washed with saturated brine (10 mL), dried over anhydrous sodium sulfate, filtered, and concentrated to give the crude product. The crude product was subjected to silica gel column chromatography (PE/EA = 1/1) to obtain a brownish-yellow solid **AA3-5** (737 mg, yield 88%). $^1$H NMR (400 MHz, DMSO-$d_6$) δ 7.72 (d, *J* = 8.0 Hz, 1H), 7.42 (d, *J* = 8.0 Hz, 1H), 6.89 (d, *J* = 8.0 Hz, 1H), 6.31 (s, 1H), 6.05 (s, 1H), 3.58-3.57 (m, 1H), 3.41-3.38 (m, 1H), 2.49-2.41 (m, 3H), 2.15-2.12 (m, 1H), 1.99-1.90 (m, 1H), 1.62-1.59 (m, 1H), 1.41 (s, 9H), 1.19-1.13 (m, 2H), 0.77-0.73 (m, 2H). MS-ESI $[M+H]^+$: 449.1.

**Step 6:** tert-Butyl (4-(6-cyano-1-cyclopropyl-5-fluoro-*1H*-indol-2-yl)cyclohex-3-en-1-yl) carbamate

To a DMF (20 mL) solution of tert-butyl (4-(6-bromo-1-cyclopropyl-5-fluoro-1H-indol-2-yl)cyclohex-3-en-1-yl) carbamate **AA3-5** (700 mg, 1.54 mmol), zinc cyanide (238 mg, 2.03 mmol), 1,1'-bis(diphenylphosphine)ferrocene (113 mg, 0.20 mmol), zinc powder (160 mg, 2.46 mmol), and tris(dibenzylideneacetone)dipalladium (186 mg, 0.20 mmol) were added. The resulting mixture was reacted at 110 °C for 16 hours. The reaction solution was cooled to room temperature, added with water (20 mL), extracted three times with ethyl acetate (60 mL), washed with saturated brine (10 mL), dried over anhydrous sodium sulfate, filtered, and concentrated to obtain the crude product. The crude product was subjected to silica gel column chromatography (PE/EA = 1/1) to obtain a light yellow solid **AA3** (0.3 g, yield 49%). $^1$H NMR (400 MHz, DMSO-$d_6$) δ 8.01 (d, *J* = 4.0 Hz, 1H), 7.52 (d, *J* = 8.0 Hz, 1H), 6.91 (d, *J* = 8.0 Hz, 1H), 6.44 (s, 1H), 6.14 (s, 1H), 3.59-3.57 (m, 1H),

3.47-3.44 (m, 1H), 2.53-2.51 (m, 3H), 2.16-2.12 (m, 1H), 1.99-1.91 (m, 1H), 1.62-1.56 (m, 1H), 1.41 (s, 9H), 1.19-1.16 (m, 2H), 0.81-0.78 (m, 2H). MS-ESI [M+H] $^+$: 396.2.

**Synthesis of intermediate AA4: tert-Butyl (3-(6-cyano-1-cyclobutyl-5-fluoro-*1H*-indol-2-yl)bicyclo[1.1.1]pentan-1-yl)carbamate**

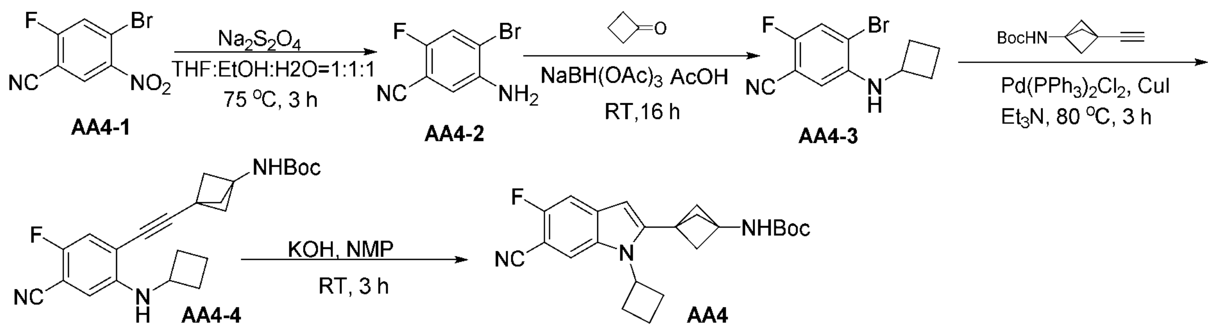


AA4-1 AA4-2 AA4-3 AA4-4 AA4

**Step 1:** 5-Amino-4-bromo-2-fluorobenzonitrile

Sodium dithionite (5.68 g, 32.6 mmol) was added to a mixed solution of 4-bromo-2-fluoro-5-nitrobenzene **AA4-1** (1 g, 4.1 mmol) in tetrahydrofuran (10 mL), ethanol (10 mL), and water (10 mL). The reaction was stirred at 75 °C for 3 hours. Then, ethyl acetate (50 mL) and water (10 mL) were added, the mixture was separated, the organic phase was dried, concentrated, and purified by silica gel column chromatography (PE/EA = 3/1) to give 5-amino-4-bromo-2-fluorobenzonitrile **AA4-2** (540 mg, yield 61%). MS-ESI [M+H] $^+$: 215.9.

**Step** 2: 4-Bromo-5-(cyclobutylamino)-2-fluorobenzonitrile

To a solution of 5-amino-4-bromo-2-fluorobenzonitrile **AA4-2** (500 mg, 2.32 mmol, 1 eq) and cyclobutanone (1.1 eq) in dichloromethane (15 mL), acetic acid (139.6 g, 2.32 mmol) and sodium borohydride acetate (986 mg, 4.65 mmol) were added, and the reaction was stirred at room temperature for 16 hours. Then, dichloromethane (20 mL) and water (10 mL) were added, the mixture was separated, the organic phase was dried, concentrated, and purified by column chromatography (PE/EA = 4/1) to give 4-bromo-5-(cyclobutylamino)-2-fluorobenzonitrile **AA4-3** (240 mg, 38% yield). MS-ESI [M+H] $^+$: 269.0.

**Step 3:** tert-Butyl (3-((4-cyano-2-(cyclobutylamino)-5-fluorophenyl)ethynyl)bicyclo[1.1.1]pentan-1-yl)carbamate

To a triethylamine (5 mL) solution of 4-bromo-5-(cyclobutylamino)-2-fluorobenzonitrile **AA4-**3 (240 mg, 0.89 mmol) and *N*-{3-ethynylbicyclo[1.1.1]pentan-1-yl}carbamate tert-butyl ester (203 mg, 0.98 mmol), cuprous iodide (139.6 g, 2.32 mmol) and bis(triphenylphosphine)palladium dichloride (62.6 mg, 0.09 mmol) were added, and the reaction was stirred at 80 °C for 3 h. The mixture was then concentrated and purified by silica gel column chromatography (PE/EA = 5/2) to give (3-((4-cyano-2-(cyclobutylamino)-5-fluorophenyl)ethynyl)bicyclo[1.1.1]pentan-1-yl)carbamate tert-butyl ester **AA4-4** (210 mg, 60% yield). MS-ESI [M+H] $^+$: 396.2.

**Step 4:** tert-Butyl (3-(6-cyano-1-cyclobutyl-5-fluoro-*1H*-indole-2-yl)bicyclo[1.1.1]pentan-1-yl) carbamate

Potassium hydroxide (179 mg, 31.9 mmol) was added to a solution of (3-((4-cyano-2-(cyclobutylamino)-5-fluorophenyl)ethynyl)bicyclo[1.1.1]pentan-1-yl)carbamate tert-butyl ester **AA4-4** (180 mg, 0.46 mmol) in N-methylpyrrolidone (6 mL), and the reaction was stirred at 80 °C for 3 hours. Then, ethyl acetate (20 mL) and water (10 mL) were added, the mixture was separated, the organic phase was dried, concentrated, and purified by silica gel column chromatography (PE/EA = 3/1) to give (3-(6-cyano-1-cyclobutyl-5-fluoro-*1H*-indol-2-yl)bicyclo[1.1.1]pentan-1-yl)carbamate tert-butyl ester **AA4** (140 mg, 78% yield). MS-ESI [M+H] $^+$: 396.2.

Synthesis of intermediate AA5: **tert-Butyl (1-(6-cyano-1-cyclobutyl-5-fluoro-*1H*-indol-2-yl)piperidin-4-yl)carbamate**

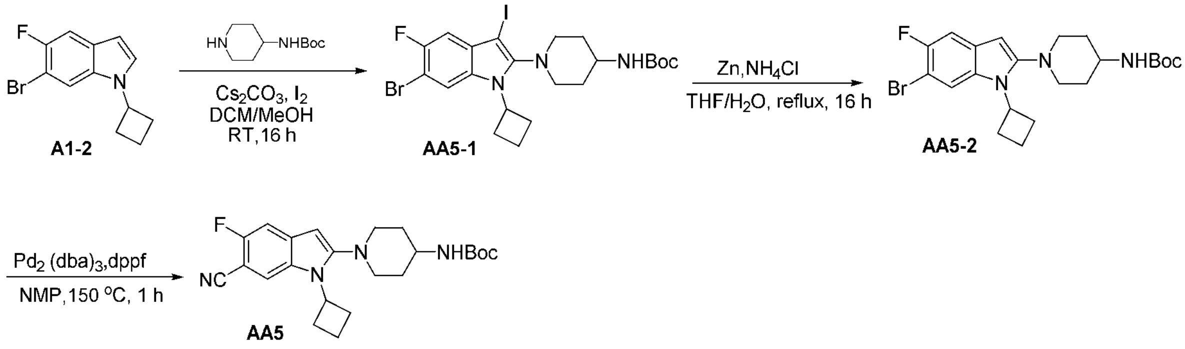


**Step** 1: tert-Butyl (1-(6-bromo-1-cyclobutyl-5-fluoro-3-iodo-1H-indol-2-yl)piperidin-4-yl) carbamate

Cesium carbonate (1.82 g, 5.6 mmol) and iodine (1.4 g, 5.6 mmol) were added to a mixed solution of 6-bromo-1-cyclobutyl-5-fluoro-*1H*-indole **A1-2** (750 mg, 2.8 mmol) and tert-butyl piperidin-4-ylcarbamate (1.12 g, 5.6 mmol) in methanol (10 mL) and dichloromethane (10 mL). The reaction was stirred at room temperature for 16 hours. Then dichloromethane (30 mL) and water (10 mL) were added, and the mixture was separated. The organic phase was washed with saturated sodium thiosulfate (10 mL) and saturated brine (10 mL), respectively, dried, concentrated, and purified by silica gel column chromatography (PE/EA = 3/1) to give (1-(6-bromo-1-cyclobutyl-5-fluoro-3-iodo-*1H*-indol-2-yl)piperidin-4-yl) carbamate tert-butyl ester **AA5-1** (400 mg, yield 24%). MS-ESI $[M+H]^+$: 592.1.

**Step 2:** tert-Butyl (1-(6-bromo-1-cyclobutyl-5-fluoro-*1H*-indol-2-yl)piperidin-4-yl)carbamate

To a mixed solution of tert-butyl 1-(6-bromo-1-cyclobutyl-5-fluoro-1H-indol-2-yl)piperidin-4-yl) carbamate **AA5-1** (400 mg, 0.67 mmol) and zinc powder (132 mg, 2.03 mmol) in tetrahydrofuran (10 mL) and water (1 mL), ammonium chloride (108 mg, 2.03 mmol) was added, and the reaction was heated under reflux and stirred for 16 hours. Then, ethyl acetate (20 mL) and water (10 mL) were added, the mixture was separated, the organic phase was washed with saturated brine, dried, concentrated, and purified by silica gel column chromatography (PE/EA = 3/1) to give 300 mg (96% yield) of tert-butyl 1-(6-bromo-1-cyclobutyl-5-fluoro-*1H*-indol-2-yl)piperidin-4-yl)carbamate **AA5-2.** MS-ESI $[M+H]^+$: 466.2.

**Step 3:** tert-Butyl (1-(6-cyano-1-cyclobutyl-5-fluoro-*1H*-indol-2-yl)piperidin-4-yl)carbamate

To a solution of tert-butyl 1-(6-bromo-1-cyclobutyl-5-fluoro-*1H*-indol-2-yl)piperidin-4-yl)carbamate **AA5-2** (300 mg, 0.64 mmol) and zinc cyanide (45 mg, 0.38 mmol) in *N*-methylpyrrolidone (10 mL), tris(dibenzylideneacetone) dipalladium (12 mg, 0.013 mmol) and 1,1'-bis(diphenylphosphine)ferrocene (14 mg, 0.026 mmol) were added, and the reaction was carried out under nitrogen protection and stirred in a microwave at 150 ° C for 16 hours. Then, ethyl acetate (20 mL) and water (10 mL) were added, the mixture was separated, the organic phase was washed with saturated brine (10 mL), dried, concentrated, and subjected to silica gel column chromatography (PE/EA = 3/2) to give (1-(6-cyano-1-cyclobutyl-5-fluoro-*1H*-indol-2-yl)piperidine-4-yl)carbamate tert-butyl ester **AA5** (150 mg, yield 57%). MS-ESI $[M+H]^+$: 413.2.

Synthesis of intermediate AA6: **tert-Butyl 2-bromo-4-cyano-5-fluorophenyl-4-(ethynylphenyl)carbamate**

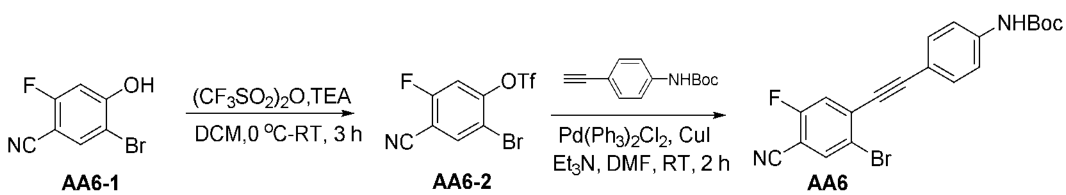

**Step 1:** 2-Bromo-4-cyano-5-fluorophenyltrifluoromethanesulfonic acid

To a solution of 5-bromo-2-fluoro-4-hydroxybenzonitrile **AA6-1** (5.4 g, 25 mmol) and trifluoromethanesulfonic anhydride (10.6 g, 37.5 mmol) in DCM (100 mL), TEA (5.05 g, 50 mmol) was slowly added dropwise under ice bath. The mixture was stirred at room temperature for 3 hours. The reaction mixture was then poured into water (200 mL) and extracted twice with DCM (200 mL each time). The organic phases were combined, washed twice with saturated brine (200 mL each time), and dried over anhydrous sodium sulfate. The crude product obtained by concentration was purified by silica gel column chromatography (PE/EA = 4/1) to give a yellow oily substance **AA6-2** (8.17 g).

**Step 2:** 2-Bromo-4-cyano-5-fluorophenyl-4-(ethynylbenzene)carbamate tert-butyl ester

To a solution of Compound **AA6-2** (8.17 g, 23.5 mmol) and tert-butyl 4-(ethynylphenyl)carbamate (5.35 g, 24.65 mmol)in DMF (100 mL), bis(triphenylphosphine)palladium dichloride (825 mg, 1.2 mmol), cuprous iodide (890 mg, 4.7 mmol), and triethylamine (9.5 g, 94 mmol) were added, and the reaction was stirred at room temperature for 2 hours under nitrogen protection. Then, ethyl acetate (200 mL) and water (50 mL) were added, and the mixture was separated. The organic phase was dried, concentrated, and purified by silica gel column chromatography (DCM/PE = 1/1), yielding tert-butyl 2-bromo-4-cyano-5-fluorophenyl-4-(ynynebenzene)carbamate **AA6** (8 g). MS-ESI [M-55] $^+$: 360.3.

**Synthesis of intermediate AA7: 2-(4-tert-butoxycarbonylaminophenyl)-5-fluoro-1-(1-methylcyclobutyl)-1H-indole-6-carbonitrile**

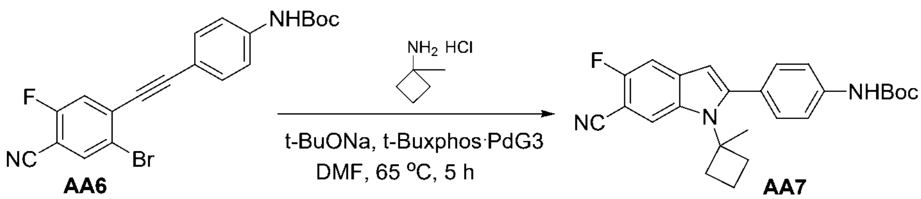


AA6 AA7

To a solution of tert-butyl 2-bromo-4-cyano-5-fluorophenyl-4-(ethynylbenzene)carbamate **AA6** (5 g, 12 mmol), 1-methylcyclobutane-1-amine hydrochloride (1.75 g, 14.45 mmol) in *N,N*-dimethylformamide (50 mL), methanesulfonic acid (2-di-tert-butylphosphino-2',4',6'-triisopropyl-1,1'-biphenyl)(2'-amino-1,1'-biphenyl-2-yl)palladium(II) (480 mg, 0.6 mmol) and sodium tert-butoxide (3.45 g, 36 mmol) were added, and the mixture was heated to 65 °C and stirred for 5 hours under nitrogen protection. The reaction mixture was filtered, and ethyl acetate (100 mL) and water (20 mL) were added. The mixture was separated, the organic phase was dried, concentrated, and purified by silica gel column chromatography (PE/EA = 10/1) to give 2-(4-tert-butoxycarbonylaminophenyl)-5-fluoro-1-(1-methylcyclobutyl)-1H-indole-6-carbonitrile **AA7** (2.2 g). MS-ESI [M-55] $^+$: 364.1.

Using the same method as intermediate **AA7,** the following intermediates **AA8** and **AA9** were prepared.

| Intermediate | Structure | Raw material | Characterization data |
|---|---|---|---|
| **AA8** | 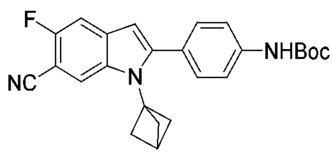 | **AA6** + 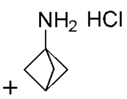 | MS-ESI [M+H] $^+$: 418.2 |
| **AA9** | 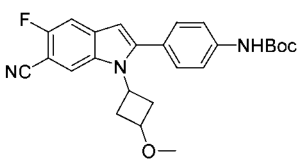 | **AA6** + 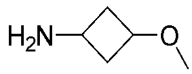 | MS-ESI [M+H] $^+$: 380.1 |

**Synthesis of intermediate AA10: tert-Butyl (4-(6-cyano-5-fluoro-1-(1-methylcyclopropyl)-1H-indol-2-yl)phenyl) carbamate**

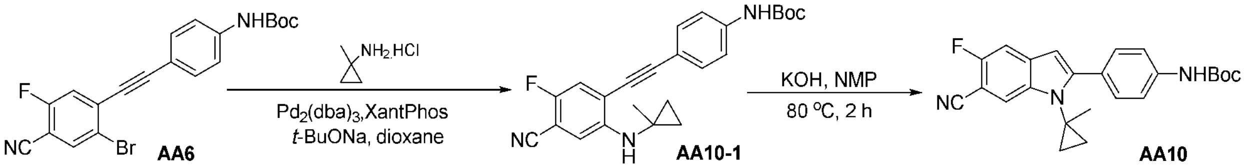


**Step 1:** tert-Butyl (4-((4-cyano-5-fluoro-2-((1-methylcyclopropyl)amino)phenyl)ethynyl)carbamate

To a solution of tert-butyl (4-((2-bromo-4-cyano-5-fluorophenyl)ethynyl)carbamate **AA9** (4 g, 9.6 mmol) and 1-methylcyclopropylamine hydrochloride (1.55 g, 14.4 mmol) in 1,4-dioxane (10 mL), tris(dibenzylideneacetone)dipalladium (176 mg, 0.20 mmol), 4,5-bis(diphenylphosphine-9,9-dimethyloxanthracene) (223 mg, 0.38 mmol), and sodium tert-butoxide (2.8 g, 28.9 mmol) were added. The reaction was heated and stirred under reflux for 16 hours under nitrogen protection. Then, ethyl acetate (20 mL) and water (10 mL) were added, the mixture was separated, the organic phase was washed with saturated brine (10 mL), dried, concentrated, and purified by silica gel column chromatography (PE/EA = 8/1) to give tert-butyl (4-((4-cyano-5-fluoro-2-((1-methylcyclopropyl)amino)phenyl)ethynyl)carbamate **AA10-1** (1.01 g, yield 26%). MS-ESI $[M+H]^+$: 406.2.

**Step 2:** tert-Butyl (4-(6-cyano-5-fluoro-1-(1-methylcyclopropyl)-*1H*-indol-2-yl)phenyl)carbamate

tert-Butyl (4-((4-cyano-5-fluoro-2-((1-methylcyclopropyl)amino)phenyl)ethynyl)carbamate **AA10-1** (1.01 g, 0.25 mmol) in N-methylpyrrolidone (10 mL), potassium hydroxide (700 mg, 12.5 mmol) was added, and the reaction was stirred at 80 °C for 2 hours. Then, ethyl acetate (20 mL) and water (10 mL) were added, and the mixture was separated. The organic phase was dried, concentrated, and purified by silica gel column chromatography (PE/EA = 10/1) to give tert-butyl (4-(6-cyano-5-fluoro-1-(1-methylcyclopropyl)-1H-indol-2-yl)phenyl)carbamate **AA10** (840 mg, 83% yield). *MS*-ESI $[M+H]^+$: 406.2.

**Synthesis of intermediate AA11: tert-Butyl (4-(2-cyano-7-cyclobutyl-7*H*-pyrrolo[2,3-*d*]pyrimidin-6-yl)phenyl) carbamate**

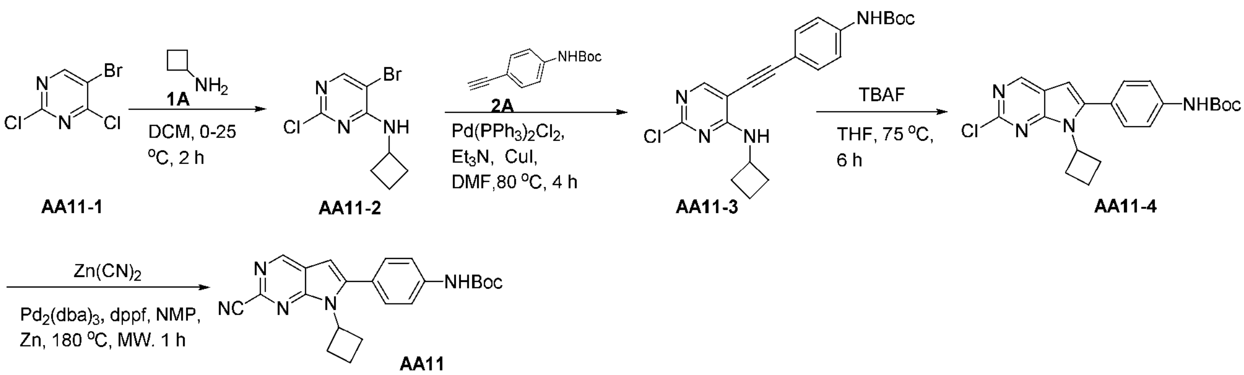


**Step** 1: 5-Bromo-2-chloro-*N*-cyclobutylpyrimidine-4-amine

A solution of cyclobutylamine (780 mg, 11.0 mmol) in dichloromethane (5 mL) was added dropwise to a dichloromethane solution (2.55 mL) of 5-bromo-2,4-dichloropyrimidine **AA11-1** (2.50 g, 11.0 mmol) and N,N-diisopropylethylamine (2.83 g, 22.0 mmol) at 0 °C, and the resulting mixture was stirred at room temperature for 2 hours. After the reaction was completed, the mixture was concentrated and purified by silica gel column chromatography (PE/EA = 85/15) to give a white solid **AA11-2** (2.5 g, yield 89 %). MS-ESI $[M+H]^+$: 263.1.

**Step 2:** tert-Butyl (4-((2-chloro-4-(cyclobutylamino)pyrimidin-5-yl)ethynyl)phenyl)carbamate

To a solution of 5-bromo-2-chloro-*N*-cyclobutylpyrimidin-4-amine **AA11-2** (511 mg, 1.95 mmol), (4-ethynylphenyl)carbamate tert-butyl ester (507 mg, 2.33 mmol), cuprous iodide (74 mg, 0.39 mmol) and triethylamine (788 mg, 7.78 mmol) in DMF (15 mL) were added bis(triphenylphosphine)palladium(II) dichloride (68.3 mg, 0.097 mmol). The resulting mixture was heated to 80 °C and stirred for 4 hours under nitrogen atmosphere. After the reaction was complete, 50 mL of ethyl acetate and 50 mL of water were added. The organic phase was washed with 10 mL of brine and concentrated, and purified by silica gel column chromatography (PE/THF = 5/1) to give a yellow solid **AA11-3** (650 mg, yield 83 %). MS-ESI $[M+H]^+$: 399.1.

**Step 3:** tert-Butyl (4-(2-chloro-7-cyclobutyl-*7H*-pyrrolo[2,3-*d*]pyrimidin-6-yl)phenyl)carbamate

1 M tetrabutylammonium fluoride in tetrahydrofuran (3.03 mL, 3.03 mmol) was added to a solution of tert-butyl 4-(2-chloro-4-(cyclobutylamino)pyrimidin-5-yl)ethynyl)phenyl)carbamate (550 mg, 1.38 mmol) in tetrahydrofuran (8 mL). The resulting mixture was heated to 75 ° C and stirred for 6 hours. After the reaction was completed, the mixture was cooled, and ethyl acetate (30 mL) and water (30 mL) were added. The organic phase was washed with brine (10 mL) and concentrated, and purified by silica gel column chromatography (PE/EA = 1/1) to give a yellow solid **AA11-4** (230 mg, yield 41%). MS-ESI $[M+H]^+$: 399.1.

**Step 4:** tert-Butyl (4-(2-cyano-7-cyclobutyl-*7H*-pyrrolo[2,3-*d]*pyrimidin-6-yl)phenyl)carbamate

To a solution of *tert*-butyl (4-(2-chloro-7-cyclobutyl-*7H*-pyrrolo[2,3-d]pyrimidin-6-yl)phenyl)carbamate **AA11-4** (0.40 g, 1.0 mmol), zinc cyanide (71 mg, 0.6 mmol), zinc powder (7.8 mg, 0.12 mmol), and 1,1'-bis(diphenylphosphine) ferrocene (22 mg, 0.04 mmol) in N-methylpyrrolidone **(10** mL), tris(dibenzylideneacetone)dipalladium (18 mg, 0.02 mmol) was added. The resulting mixture was heated to 180 ° C and stirred for 1 hour. After the reaction was complete, the mixture was cooled, and ethyl acetate (30 mL) and water (20 mL) were added. The organic phase was washed with brine (10 mL) and concentrated, and purified by silica gel column chromatography (PE/THF = 5). 0/5 0), yielding a yellow solid **AA11** (18 0 mg, yield 62%). MS-ESI $[M+H]^+$: 390.1.

**Synthesis of intermediate AA12: tert-Butyl (4-(6-cyano-1-cyclobutyl-5-fluoro-*1H*-pyrrolo[2,3-b]pyridin-2-yl)carbamate**

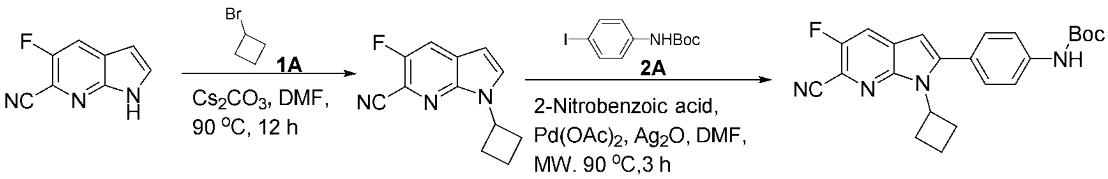


AA12-1 AA12-2 AA12

**Step** 1: 1-Cyclobutyl-5-fluoro-*1H*-pyrrolo[2,3-*b*]pyridine-6-carbonitrile

To a solution of 5-fluoro-1H-pyrrolo[2,3-b]pyridine-6-carbonitrile **AA12-1** (500 mg, 3.10 mmol) and cesium carbonate (3.03 g, 9.31 mmol)in DMF (6 mL) was added cyclobutyl bromide (1.26 g, 9.31 mmol). The resulting mixture was heated to 90 °C and stirred for 16 hours under nitrogen atmosphere. After the reaction was completed, the mixture was cooled, filtered, and the filtrate was washed three times with lithium chloride aqueous solution (10 mL) and concentrated, and purified by silica gel column chromatography (PE/EA = 85/15) to give a light green solid **AA12-2** (600 mg, yield 89%). MS-ESI $[M+H]^+$: 216.1.

**Step 2: tert**-Butyl (4-(6-cyano-1-cyclobutyl-5-fluoro-*1H*-pyrrolo[2,3-*b*]pyridin-2-yl)carbamate

Palladium acetate (23.4 mg, 0.104 mmol) was added to a DMF solution (8 mL) of 1-cyclobutyl-5-fluoro-*1H*-pyrrolo[2,3-*b*]pyridine-6-carbonitrile **AA12-2** (560 mg, 1.49 mmol), tert-butyl (4-iodophenyl)carbamate (711.7 mg, 2.23 mmol), 2-nitrobenzoic acid (372.7 mg, 2.23 mmol), and silver oxide (258.4 mg, 1.12 mmol). The resulting mixture was microwave-heated to 90 °C and stirred for 3 hours under nitrogen atmosphere. After the reaction was completed, the mixture was cooled, filtered, and the filtrate was washed three times with lithium chloride aqueous solution (10 mL),

concentrated, and purified by silica gel column chromatography (PE/DCM = 1/3) to obtain a light yellow solid **AA12** (380 mg, yield 62%). MS-ESI [M+H] $^+$: 407.2.

Using the synthetic method of intermediate AA12, the following intermediates AA13 to AA15 were prepared.

| Intermediate | Structure | Raw material | Characterization data |
|---|---|---|---|
| **AA13** | 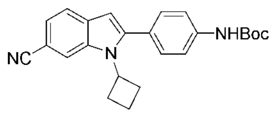 | 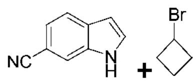 | MS-ESI [M+H] $^+$: 388.2 |
| **AA14** | 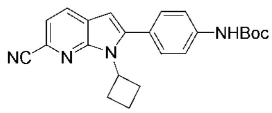 | 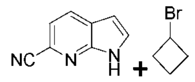 | MS-ESI [M+H] $^+$: 389.1 |
| **A A15** | 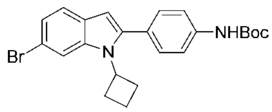 | 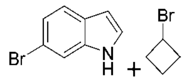 | MS-ESI [M+H] $^+$: 441.1 |

**Synthesis of intermediate AA16: tert-Butyl (4-(1-cyclobutyl-5-fluoro-6-(N'-hydroxyaminomethyliminoyl)-1H-indol-2-yl)phenyl)carbamate**

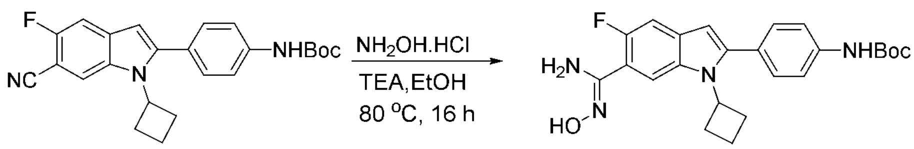


**AA16-1** **AA16**

Triethylamine (747 mg, 7.38 mmol) was added to an ethanol solution (20 mL) of 4-(6-cyano-1-cyclobutyl-5-fluoro-*1H*-indol-2-yl)phenyl)carbamate tert-butyl ester **AA16-1** (500 mg, 1.23 mmol) and hydroxylamine hydrochloride (514 mg, 7.4 mmol). The resulting mixture was heated to 80 °C and stirred for 16 hours under nitrogen. The reaction solution was cooled to room temperature and water (10 mL) was added. The mixture was extracted three times with ethyl acetate (60 mL). The organic phase was washed with saturated brine (10 mL), dried over anhydrous sodium sulfate, filtered, concentrated, and purified by silica gel column chromatography (PE/EA = 1/1) to give the product **AA16** (450 mg, 83% yield) as a yellow solid. MS-ESI [M+H] $^+$: 439.1.

**Synthesis of intermediate AA17: tert-Butyl (4-(5-cyano-3-cyclobutyl-6-fluorobenzofuran-2-yl)phenyl)carbamate**

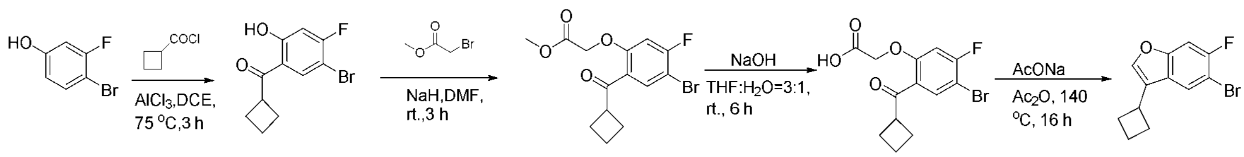


AA17-1 AA17-2 AA17-3 AA17-4 AA17-5

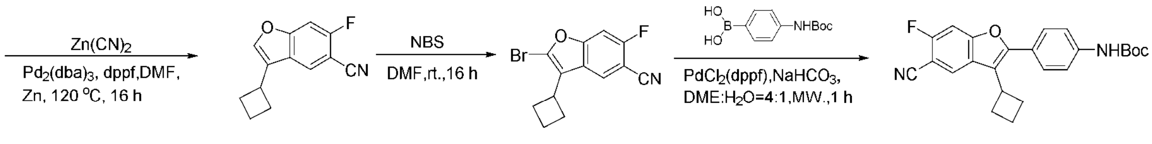


AA17-6 AA17-7 AA17

**Step** 1: (5-Bromo-4-fluoro-2-hydroxyphenyl)(cyclobutyl)methyl ketone

To a 1,2-dichloroethane solution (20 mL) of 4-bromo-3-fluorophenol **AA17-1** (1.0 g, 5.2 mmol) and anhydrous aluminum trichloride (1.05 g, 7.9 mmol), cyclobutane acyl chloride (0.68 g, 5.7 mmol) was added dropwise. The resulting mixture was heated to 75 °C and stirred for 3 hours under nitrogen atmosphere. After the reaction was complete, the mixture was cooled and poured into a dilute hydrochloric acid aqueous solution (15 mL) under ice-water bath. The mixture was filtered, and the filtrate was washed with brine (10 mL), concentrated, and purified by silica gel column chromatography (PE/EA = 95/5) to give a colorless oily substance **AA17-2** (1.2 g, yield 84%). MS-ESI $[M+H]^+$ : 274.1.

**Step 2:** Methyl 2-(4-bromo-2-(cyclobutoxycarbonyl)-5-fluorophenoxy)acetate

To a DMF solution (15 mL) of (5-bromo-4-fluoro-2-hydroxyphenyl)(cyclobutyl)ketone **AA17-2** (4.0 g, 14.6 mmol), 60% sodium hydride (0.878 g, 22 mmol) was added. The resulting mixture was stirred at room temperature for 0.5 h, followed by the addition of methyl 2-bromoacetate (2.91 g, 19.0 mmol). The mixture was stirred at room temperature for 3 h. After the reaction was complete, water (30 mL) was added, and the mixture was extracted twice with ethyl acetate (30 mL), concentrated, and purified by silica gel column chromatography (PE/EA = 85/15) to give a yellow solid **AA17-3** (3.5 g, 69% yield). MS-ESI $[M+H]^+$: 346.1.

**Step 3:** 2-(4-Bromo-2-(cyclobutylcarbonyl)-5-fluorophenoxy)acetic acid

Sodium hydroxide (573 mg, 17.7 mmol) was added to a solution of methyl 2-(4-bromo-2-(cyclobutylcarbonyl)-5-fluorophenoxy)acetate **AA17-3** (3.3 g, 9.56 mmol) in THF/$H_2O$ = 3/1. The mixture was stirred at room temperature for 6 hours. After the reaction was complete, ethyl acetate (30 mL) and water (30 mL) were added, and the pH was adjusted to 4.0 with 1 N hydrochloric acid (50 mL). The organic phase was concentrated to dryness to give a yellow solid **AA17-4** (3.0 g, 94% yield). MS-ESI $[M+H]^+$: 332.1.

**Step 4:** 5-Bromo-3-cyclobutyl-6-fluorobenzofuran

Sodium acetate (1.57 g, 19.1 mmol) was added to a solution of 2-(4-bromo-2-(cyclobutylcarbonyl)-5-fluorophenoxy)acetic acid **AA17-4** (3.16 g, 9.54 mmol) in acetic anhydride (25 mL). The resulting mixture was heated to 140 °C and stirred for 16 hours. After the reaction was complete, the mixture was concentrated, and ethyl acetate (30 mL) and water (30 mL) were added. The pH was adjusted to 9.0 with 1 N sodium hydroxide aqueous solution (50 mL). The organic phase was concentrated to dryness and purified by silica gel column chromatography (PE/EA = 50/50) to give **AA17-5** (2.0 g, 77% yield). MS-ESI $[M+H]^+$: 270.1.

**Step 5:** 3-Cyclobutyl-6-fluorobenzofuran-5-carbonitrile

To a solution of 5-bromo-3-cyclobutyl-6-fluorobenzofuran **AA17-5** (2.0 g, 7.43 mmol), zinc cyanide (0.524 g, 4.46 mmol), zinc powder (58 mg, 0.89 mmol), and 1,1'-bis (diphenylphosphine)ferrocene (165 mg, 0.297 mmol), tris(dibenzylideneacetone)dipalladium (136 mg, 0.148 mmol) was added. The resulting mixture was heated to 120 °C for 16 hours under nitrogen atmosphere. After the reaction was complete, the mixture was cooled, and ethyl acetate (40 mL) and water (40 mL) were added. The mixture was filtered, and the filtrate was washed with brine (15 mL), concentrated to dryness, and purified by silica gel column chromatography (PE/EA = 60/40) to give **AA17-6** (1.2 g, 75% yield). MS-ESI $[M+H]^+$: 216.1.

**Step 6:** 2-Bromo-3-cyclobutyl-6-fluorobenzofuran-5-carbonitrile

*N*-bromosuccinimide (1.27 g, 7.13 mmol) was added to a DMF (15 mL) solution of 3-cyclobutyl-6-fluorobenzofuran-5-carbonitrile **(AA17-6)** (1.28 g, 5.95 mmol). The resulting mixture was reacted at room temperature for 16 hours. After the reaction was complete, ethyl acetate (20 mL) and water (30 mL) were added, the organic phase was washed with brine (15 mL), concentrated to dryness, and purified by silica gel column chromatography (PE/EA = 85/15) to give **AA17-7** (1.31 g, 74% yield). MS-ESI $[M+H]^+$: 294.1.

**Step 7:** tert-Butyl (4-(5-cyano-3-cyclobutyl-6-fluorobenzofuran-2-yl)phenyl)carbamate

To a solution of 2-bromo-3-cyclobutyl-6-fluorobenzofuran-5-carbonitrile **AA17-7** (1.31 g, 4.45 mmol), (4-((tert-butoxycarbonyl)amino)phenyl)boronic acid (1.58 g, 6.68 mmol) and sodium bicarbonate (1.12 g, 13.36 mmol) in DME/$H_2O$ = 3/1 (20 mL), 1,1'-bis(diphenylphosphine)ferrocene)palladium dichloride (325 mg, 0.445 mmol) was added. The resulting mixture was heated to 100 °C for 3 hours under nitrogen atmosphere. After the reaction was complete, the mixture was cooled, and ethyl acetate (50 mL) and water (50 mL) were added. The organic phase was washed with saturated brine (15 mL), concentrated to dryness, and purified by silica gel column chromatography (PE/EA = 70/30) to give **AA17** (1.34 g, 74% yield). MS-ESI $[M+H]^+$: 407.1.

**<u>Synthesis of intermediate AA18:</u> 2-(4-((tert-butoxycarbonyl)amino)phenyl)-1-cyclobutyl-5-fluoro-*1H*-indole-6-carbazideimide thiomethyl ester**

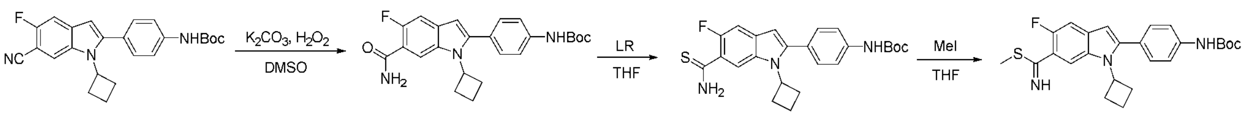


AA18-1 AA18-2 AA18-3 AA18

**Step** 1: tert-Butyl (4-(6-carbamoyl-1-cyclobutyl-5-fluoro-*1H*-indol-2-yl)phenyl) carbamate

Hydrogen peroxide (2 mL) was added to a solution of tert-butyl (4-(6-cyano-1-cyclobutyl-5-fluoro-*1H*-indol-2-yl) phenyl)carbamate **AA18-1** (200 mg, 0.5 mmol) and potassium carbonate (103 mg, 0.7 mmol) in dimethyl sulfoxide (10 mL). The resulting mixture was stirred under nitrogen atmosphere for 2 hours. After the reaction was complete, ice water (80 mL) was added to precipitate the solid, which was then filtered to give a white solid **AA18-2** (200 mg, 97% yield). MS-ESI $[M+H]^+$: 424.2.

**Step 2:** tert-Butyl (4-(6-thiocarbamoyl-1-cyclobutyl-5-fluoro-*1H*-indol-2-yl)phenyl)carbamate

Lawesson's reagent (400 mg, 1 mmol) was added to a toluene solution (5 mL) of tert-butyl (4-(6-carbamoyl-1-cyclobutyl-5-fluoro-*1H*-indol-2-yl)phenyl)carbamate **AA18-2** (200 mg, 0.47 mmol). The resulting mixture was heated to 100 °C and stirred for 16 hours under nitrogen. After the reaction was completed, the mixture was directly evaporated to dryness and purified by silica gel column chromatography (PE/EA = 3/1) to give the product **AA18-3** (200 mg, 96% yield) as a yellow solid. MS-ESI $[M+H]^+$: 440.1.

**Step 3:** Methyl 2-(4-((tert-butoxycarbonyl)amino)phenyl)-1-cyclobutyl-5-fluoro-*1H*-indole-6-carbamimidothioate

Methyl iodide (130 mg, 0.1 mmol) was added to a mixed solution of tert-butyl 4-(4-(6-carbamoyl-1-cyclobutyl-5-fluoro-1H-indol-2-yl)phenyl)carbamate **AA18-3** (200 mg, 0.4 mmol) in tetrahydrofuran (5 mL). The resulting mixture was reacted for 3 hours. The mixture was then directly concentrated and purified by silica gel column chromatography (PE/EA = 4/1) to give the product **AA18** as a yellow solid (100 mg, 55% yield). MS-ESI $[M+H]^+$: 454.2.

**<u>Synthesis of intermediate AA19:</u> tert-Butyl (4-(5-cyano-3-cyclobutyl-6-fluorobenzo[b]thiophen-2-yl)phenyl) carbamate**

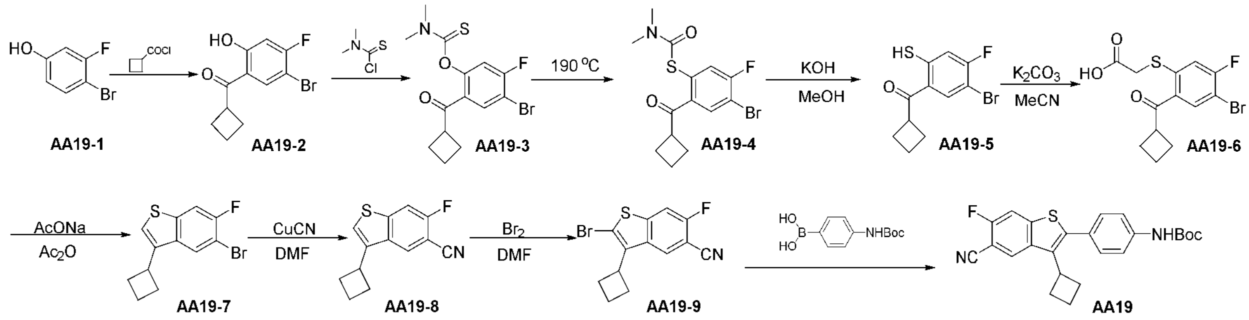

AA19-1 AA19-2 AA19-3 AA19-4 AA19-5 AA19-6
AA19-7 AA19-8 AA19-9 AA19

**Step 1:** (5-Bromo-4-fluoro-2-hydroxyphenyl)(cyclobutyl)ketone

To a 1,2-dichloroethane solution (40 mL) of 4-bromo-3-fluorophenol **AA19-1** (5 g, 26.18 mmol) and anhydrous aluminum trichloride (5.24 g, 39.27 mmol), cyclobutyl acyl chloride (3.42 g, 28.8 mmol) was added. The resulting mixture was heated to 85 °C and stirred for 4 hours under nitrogen. The reaction mixture was cooled to room temperature and added with water (100 mL), extracted three times with ethyl acetate (200 mL). The organic phase was washed with saturated brine (100 mL), dried over anhydrous sodium sulfate, filtered, concentrated, and purified by silica gel column chromatography (PE/EA = 20/1) to give product **AA19-2** (5 g, 70% yield). MS-ESI $[M+H]^+$: 273.2.

**Step Two:** *O*-(4-bromo-2-(cyclobutylcarbonyl)-5-fluorophenyl)dimethylamino thioformate

To a solution of (5-bromo-4-fluoro-2-hydroxyphenyl)(cyclobutyl)ketone **AA19-2** (5 g, 18 mmol) in DMF (40 mL), 1,4-diazabicyclo[2,2,2]octane (4.11 g, 37 mmol) and dimethylamino thiocarbamoyl chloride (4.53 g, 37 mmol) were added. The resulting mixture was stirred under nitrogen for 3 hours. The reaction solution was cooled to room temperature, and water (100 mL) was added. The mixture was extracted three times with ethyl acetate (200 mL). The organic phase was washed with saturated brine (100 mL), dried over anhydrous sodium sulfate, filtered, concentrated, and purified by silica gel column chromatography (PE/EA = 5/1) to give product **AA19-3** (6.2 g, 96% yield). MS-ESI $[M+H]^+$: 360.2.

**Step 3:** *S*-(4-bromo-2-(cyclobutylcarbonyl)-5-fluorophenyl)dimethylamino thioformate

*O*-(4-bromo-2-(cyclobutylcarbonyl)-5-fluorophenyl)dimethylamino thioformate **AA19-3** (100 mg, 0.28 mmol) was heated to 210 °C and reacted for 2 hours. The reaction solution was used directly for the next reaction.

**Step 4:** (5-Bromo-4-fluoro-2-mercaptophenyl)(cyclobutyl)ketone

To a methanol solution (3 mL) of *S*-(4-bromo-2-(cyclobutylcarbonyl)-5-fluorophenyl)dimethylamino thioformate **AA19-4** (100 mg, 0.28 mmol) was added potassium hydroxide (93 mg, 1.7 mmol). The resulting mixture was heated to 70 °C and stirred for 3 hours under nitrogen atmosphere. The reaction solution was cooled to room temperature and added with water (100 mL) and hydrochloric acid (1 N, 1.8 mL), extracted three times with ethyl acetate (200 mL). The organic phase was washed with saturated brine (100 mL), dried over anhydrous sodium sulfate, filtered, concentrated, and purified by silica gel column chromatography (PE/EA = 20/1) to give product **AA19-5** (65 mg, 96% yield). MS-ESI $[M+H]^+$: 289.2.

**Step 5:** 2-((4-Bromo-2-(cyclobutylcarbonyl)-5-fluorophenyl)thio)acetic acid

To a solution of (5-bromo-4-fluoro-2-mercaptophenyl)(cyclobutyl) ketone **AA19-5** (65 mg, 0.23 mmol) in acetonitrile (4 mL), 2-bromoacetic acid (38 mg, 0.28 mmol) and potassium carbonate (62 mg, 0.45 mmol) were added, and the mixture was stirred at room temperature for 2 hours. The reaction solution was added with water (15 mL), extracted three times with ethyl acetate (15 mL). The organic phase was washed with saturated brine (10 mL), dried over anhydrous sodium sulfate, filtered, concentrated, and purified by silica gel column chromatography (PE/EA = 5/1) to give product **AA19-6** (70 mg, 85% yield). MS-ESI $[M+H]^+$ : 346.2.

**Step 6:** 5-Bromo-3-cyclobutyl-6-fluorobenzo[b]thiophene

Sodium acetate (87 mg, 0.64 mmol) was added to a 3 mL acetic acid solution of 2-((4-bromo-2-(cyclobutylcarbonyl)-5-fluorophenyl)thio)acetic acid **AA19-6** (74 mg, 0.21 mmol). The resulting mixture was heated to 140 °C and stirred for 2 hours. The reaction mixture was cooled to room temperature and then added with water (20 mL) and sodium

hydroxide solution (1 N, 2 mL), extracted three times with ethyl acetate (20 mL). The organic phase was washed with saturated brine (10 mL), dried over anhydrous sodium sulfate, filtered, concentrated, and purified by silica gel column chromatography (PE/EA = 20/1) to give product **AA19-7** (34 mg, 57% yield). MS-ESI [M+H] $^+$: 285.2.

**Step 7:** 3-Cyclobutyl-6-fluorobenzo[*b*]thiophene-5-carbonitrile

To a DMF solution (1 mL) of 5-bromo-3-cyclobutyl-6-fluorobenzo[b]thiophene **AA19-7** (20 mg, 0.07 mmol), were added zinc cyanide (5 mg, 0.04 mmol), 1,1'-bis(diphenylphosphine)ferrocene (1.5 mg, 0.002 mmol), zinc (0.6 mg, 0.008 mmol), and tris(dibenzylideneacetone)dipalladium (1.3 mg, 0.02 mmol). The resulting mixture was heated to 120 °C and stirred for 16 hours. The reaction mixture was cooled to room temperature and added with water (20 mL), extracted three times with ethyl acetate (20 mL). The organic phase was washed with saturated brine (10 mL), dried over anhydrous sodium sulfate, filtered, concentrated, and purified by silica gel column chromatography (PE/EA = 20/1) to give product **AA19-8** (15 mg, 80% yield). MS-ESI [M+H] $^+$: 232.2.

**Step 8:** 2-Bromo-3-cyclobutyl-6-fluorobenzo[b]thiophene-5-carbonitrile

To an acetic acid solution (6 mL) of 3-cyclobutyl-6-fluorobenzo[b]thiophene-5-carbonitrile **AA19-8** (300 mg, 1.3 mmol) was added brine (829 mg, 5.2 mmol). The resulting mixture was stirred at room temperature for 16 hours. The reaction mixture was cooled to room temperature and then added with water (20 mL) and sodium thiosulfate (15 mL), extracted three times with ethyl acetate (20 mL). The organic phase was washed with saturated brine (10 mL), dried over anhydrous sodium sulfate, filtered, concentrated, and purified by silica gel column chromatography (PE/EA = 6/1) to give product **AA19-9** (320 mg, 80% yield). MS-ESI [M+H] $^+$: 310.2.

**Step 9:** 3-Cyclobutyl-6-fluorobenzo[b]thiophene-5-carbonitrile

To a mixed solution of 5-bromo-3-cyclobutyl-6-fluorobenzo[b]thiophene **AA19-9** (320 mg, 1.03 mmol) in DME (8 mL) and water (2 mL), were added (4-((tert-butoxycarbonyl)amino)phenyl)boronic acid (366 mg, 1.55 mmol), 1,1'-bis(diphenylphosphine)ferrocene palladium dichloride (75 mg, 0.1 mmol), and sodium bicarbonate (260 mg, 3.1 mmol). The resulting mixture was heated to 100 °C and stirred for 3 hours. The reaction mixture was cooled to room temperature and added with water (20 mL), extracted three times with ethyl acetate (20 mL). The organic phase was washed with saturated brine (10 mL), dried over anhydrous sodium sulfate, filtered, concentrated, and purified by silica gel column chromatography (PE/EA = 20/1) to give product AA19 (260 mg, 61% yield). MS-ESI [M+H] $^+$: 423.2.

**Synthesis of intermediate A1: 4-(1-Cyclobutyl-5-fluoro-6-(1H-tetrazol-5-yl)-1H-indol-2-yl)aniline**

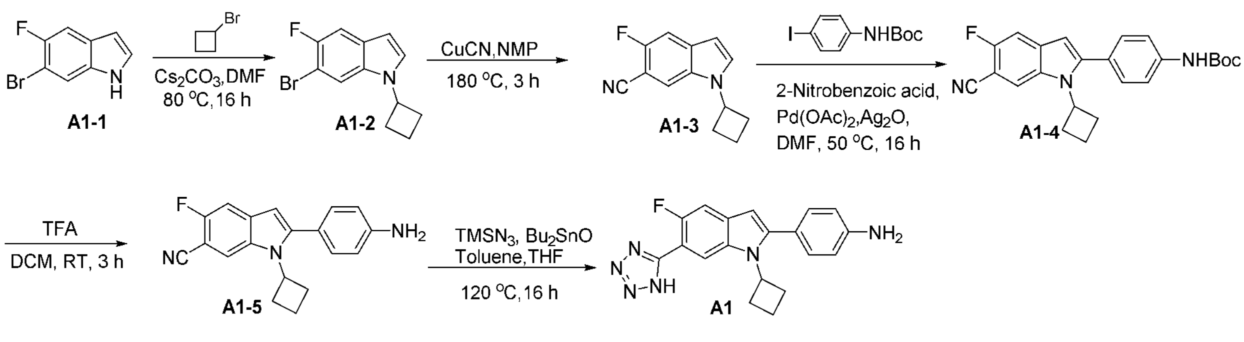


**Step** 1: 6-Bromo-1-cyclobutyl-5-fluoro-*1H*-indole

Cesium carbonate (6.85 g, 21.0 mmol) was added to a solution of 6-bromo-5-fluoro-1H-indole **A1-1** (1.5 g, 7.0 mmol) and bromocyclobutane (2.17 g, 16.0 mmol) in N,N-dimethylformamide (35 mL), and the mixture was heated to 80 °C and stirred for 16 hours. The reaction mixture was filtered, and ethyl acetate (50 mL) and water (10 mL) were added. The mixture was separated. The organic phase was dried, concentrated, and purified by silica gel column chromatography (PE/ EA = 10/1) to give 6-bromo-1-cyclobutyl-5-fluoro-*1H*-indole **A1-2** (1.7 g, 91% yield). MS-ESI [M+H] $^+$: 267.9.

**Step 2:** 1-Cyclobutyl-5-fluoro-*1H*-indole-6-carbonitrile

To a solution of 6-bromo-1-cyclobutyl-5-fluoro-*1H*-indole **A1-2** (1.7 g, 6.3 mmol) in *N*-methylpyrrolidone (15 mL) was added cuprous cyanide (1.13 g, 12.7 mmol). The mixture was heated to 180 °C and stirred for 3 hours under nitrogen protection and microwave irradiation. The reaction mixture was filtered, and ethyl acetate (30 mL) and water (10 mL) were added. The mixture was separated. The organic phase was dried, concentrated, and purified by silica gel column chromatography (PE/ EA = 8/1) to give 1-cyclobutyl-5-fluoro-*1H*-indole-6-carbonitrile A1-3 (1.1 g, 81% yield). MS-ESI $[M+H]^+$: 215.0.

**Step 3:** tert-Butyl (4-(6-cyano-1-cyclobutyl-5-fluoro-*1H*-indole-2-yl)phenyl)carbamate

To a solution of 1-cyclobutyl-5-fluoro-*1H*-indole-6-carbonitrile **A1-3** (1.1 g, 5.1 mmol), o-nitrobenzoic acid (1.29 g, 7.7 mmol), and tert-butyl (4-iodophenyl)carbamate (2.46 g, 7.7 mmol) in *N,N*-dimethylformamide (40 mL), were added palladium acetate (57.6 mg, 0.26 mmol) and silver oxide (892 mg, 3.85 mmol) were added, and the mixture was heated to 50 °C and stirred for 16 hours under nitrogen protection. The reaction mixture was filtered, and ethyl acetate (60 mL) and water (10 mL) were added. The mixture was separated, the organic phase was dried, concentrated, and purified by silica gel column chromatography (PE/ EA = 3/1) to give (4-(6-cyano-1-cyclobutyl-5-fluoro-*1H*-indol-2-yl)phenyl)carbamate tert-butyl ester **A1-4** (950 mg, yield 46%). MS-ESI $[M+H]^+$: 406.1.

**Step 4:** 2-(4-Aminophenyl)-1-cyclobutyl-5-fluoro-*1H*-indole-6-carbonitrile

To a solution of (4-(6-cyano-1-cyclobutyl-5-fluoro-*1H*-indol-2-yl)phenyl)carbamate tert-butyl ester **A1-4** (950 mg, 2.34 mmol) in dichloromethane (10 mL), was added trifluoroacetic acid (2 mL), and the mixture was stirred at room temperature for 3 hours. The reaction mixture was concentrated, and ethyl acetate (30 mL) and water (10 mL) were added. The trifluoroacetic acid was then neutralized with saturated sodium bicarbonate. The mixture was separated from the organic phase; the organic phase was washed with saturated brine (10 mL), dried over anhydrous sodium sulfate, and concentrated to give 2-(4-aminophenyl)-1-cyclobutyl-5-fluoro-*1H*-indole-6-carbonitrile **A1-5** (700 mg, 98% yield). MS-ESI $[M+H]^+$: 306.0.

**Step 5:** 4-(1-Cyclobutyl-5-fluoro-6-(*1H*-tetrazol-5-yl)-1H-indole-2-yl)aniline

To a mixed solution of 2-(4-aminophenyl)-1-cyclobutyl-5-fluoro-*1H*-indole-6-carbonitrile **A1-5** (200 mg, 0.65 mmol) in toluene (6 mL) and tetrahydrofuran (1.5 mL), azidotrimethylsilane (226 mg, 1.96 mmol) and dibutyltin oxide (16.2 mg, 0.065 mmol) were added. The tube was sealed, heated to 120 °C, and stirred for 16 hours. The reaction was concentrated and purified by silica gel column chromatography (MeOH / DCM = 1/15) to give 4-(1-cyclobutyl-5-fluoro-6-(1H-tetrazol-5-yl)-1H-indole-2-yl)aniline **A1** (100 mg, 44% yield). $^1$H NMR (400 MHz, DMSO-$d_6$) δ 8.31 (d, *J* = 5.8 Hz, 1H), 7.55 (d, *J* = 11.4 Hz, 1H), 7.16 (d, *J*= 8.4 Hz, 2H), 6.69 (d, *J* = 8.5 Hz, 2H), 6.41 (d, *J* = 0.7 Hz, 1H), 5.12-5.03 (m, 1H), 3.34 (s, 2H), 2.79-2.71 (m, 2H), 2.41-2.34 (m, 2H), 1.92-1.75 (m, 2H). MS-ESI $[M+H]^+$: 349.1.

Using the synthetic method of intermediate **A1,** the following intermediates **A2** to **A8** were prepared.

| intermediate | structural | raw material | Characterization data |
|---|---|---|---|
| **V** | 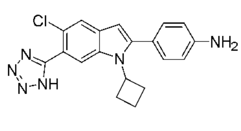 | 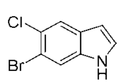 | $^1$H NMR (400 MHz, DMSO-d $_6$) δ 8.10 (s, 1H), 7.81 (s, 1H), 7.17-7.15 (m, 2H), 6.70-6.68 (m, 2H), 6.41 (s, 1H), 5.08-5.55 (m, 1H), 2.79-2.68 (m, 2H), 2.37-2.30 (m, 2H), 1.97-1.70 (m, 2H). MS-ESI [M+ H] $^+$ : 365.1 |
| **A3** | 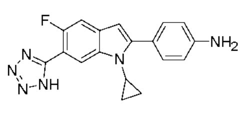 | 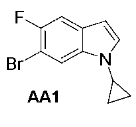 **AA1** | $^1$ H NMR (400 MHz, DMSO-d $_6$) δ 8.20 (d, *J* = 8.5 Hz, 1H), 7.51 (d, *J* = 11.6 Hz, 1H), 7.46-7.43 (m, 2H), 6.69-6.66 (m, 2H), 6.46 (s, 1H), 3.71-3.67 (m, 1H), 1.11-1.06 (m, 2H), 0.62-0.58 (m, 5H). MS-ESI [M+ H] $^+$: 335.1 |

(continued)

| intermediate | structural | raw material | Characterization data |
|---|---|---|---|
| **A4** | 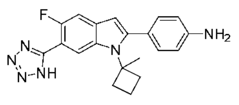 | 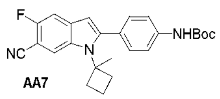 **AA7** | $^{1}$H NMR (400 MHz, DMSO-$d_6$) δ 7.92 (d, *J* = 6.0 Hz, 1H), 7.52 (d, *J* = 7.6 Hz, 1H), 7.13 (d, *J* = 8.4 Hz, 2H), 6.62 (d, *J* = 8.4 Hz, 2H), 6.34 (s, 1H), 2.29-2.20 (m, 2H), 2.19-2.05 (m, 2H), 1.94 (s, 3H), 1.91-1.85 (m, 1H), 1.66-1.60 (m,1H). MS-ESI [M+ H] $^{+}$: 363.1 |
| **A5** | 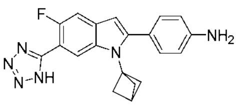 | 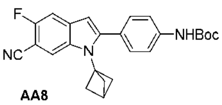 **AA8** | MS-ESI [M+ H] $^{+}$: 361.3 |
| **A6** | 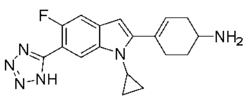 | 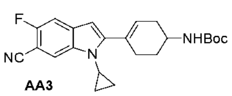 **AA3** | $^{1}$H NMR (400 MHz, DMSO-$d_6$) δ 8.00 (d, *J* = 4.0 Hz, 1H), 7.83 (s, 2H), 7.24 (d, *J* = 8.0 Hz, 1H), 6.29 (s, 1H), 6.09-6.08 (m, 1H), MS-ESI [M+ H] $^{+}$: 339.2 |
| **A7** | 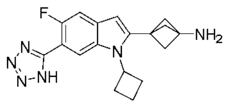 | 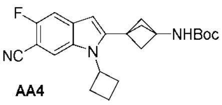 **AA4** | MS-ESI [M+ H] $^{+}$: 339.2 |
| **A8** | 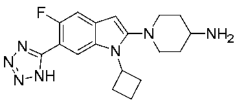 | 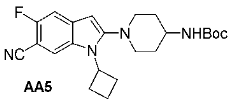 **AA5** | MS-ESI [M+ H] $^{+}$: 356.2 |

<u>**Synthesis of intermediate A9:**</u> **2-(4-Aminophenyl)-1-cyclobutyl-6-(1H-tetrazol-5-yl)-1H-indole-5-ol**

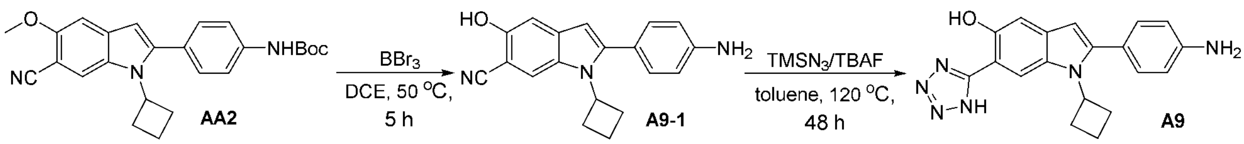


**AA2** **A9-1** **A9**

**Step 1:** 2-(4-Aminophenyl)-1-cyclobutyl-5-hydroxy-*1H*-indole-6-carbonitrile

To a solution of (4-(6-cyano-1-cyclobutyl-5-methoxy-*1H*-indol-2-yl)phenyl)carbamate tert-butyl ester **AA2** (160 mg, 0.504 mmol) in 1,2-dichloroethane (4 mL) was added dropwise boron tribromide (624 mg, 2.49 mmol) in an ice-water bath. The mixture was heated to 50 °C and stirred for 5 hours under nitrogen protection. After the reaction was complete, the temperature was lowered to 0 °C in an ice-water bath, the mixture was quenched by adding methanol dropwise, concentrated, and then ethyl acetate (15 mL) was added. The mixture was washed with sodium bicarbonate aqueous solution (5 mL) and concentrated. The solution was purified by silica gel column chromatography (PE/ EA = 1/1) to give a pale yellow solid **A9-1** (60 mg). MS-ESI [M+H] $^{+}$: 304.0.

**Step 2:** 2-(4-Aminophenyl)-1-cyclobutyl-6-(1H-tetrazol-5-yl)-1H-indole-5-ol

To a toluene solution (1.5 mL) of 2-(4-aminophenyl)-1-cyclobutyl-5-hydroxy-*1H*-indole-6-carbonitrile **A9-1** (60 mg, 197.8 μmol) and tetrabutylammonium fluoride in tetrahydrofuran (0.79 mL, 791 μmol), azidotrimethylsilane (91.2 mg, 791 μmol) was added. The mixture was heated to 120 °C and stirred for 48 hours. After the reaction was complete, the mixture was concentrated and purified by silica gel column chromatography (DCM/MeOH = 92/8) to give a pale yellow solid **A9** (65 mg). MS-ESI [M+H] $^{+}$: 347.0.

**<u>Synthesis of intermediate A10:</u> 2-((4-(1-Cyclobutyl-5-fluoro-6-(1H-tetrazol-5-yl)-1H-indol-2-yl)phenyl)amino)-2-carbonylacetic acid**

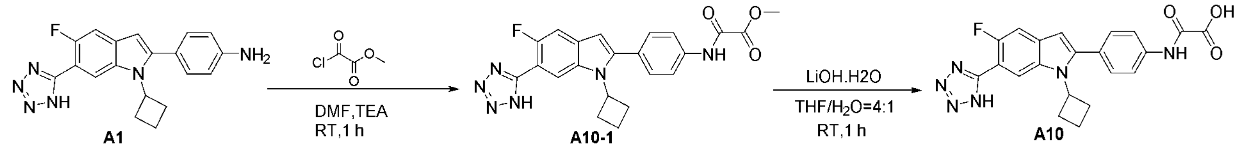

A1 A10-1 A10

**Step** 1: 2-((4-(1-Cyclobutyl-5-fluoro-6-(1H-tetrazol-5-yl)-1H-indol-2-yl)phenyl)amino)-2-carbonylacetic acid methyl ester

To a solution of 4-(1-cyclobutyl-5-fluoro-6-(1H-tetrazol-5-yl)-1H-indol-2-yl)aniline **A1** (100 mg, 0.29 mmol) and triethylamine (58 mg, 0.57 mmol) in tetrahydrofuran (5 mL), monomethyl oxaloyl chloride (37 mg, 0.30 mmol) was added dropwise under ice bath condition. The mixture was stirred at room temperature for 1 hour, quenched with saturated ammonium chloride (10 mL), and extracted with ethyl acetate (20 mL). The organic phase was dried, filtered, and concentrated to give 2-((4-(1-cyclobutyl-5-fluoro-6-(1H-tetrazol-5-yl)-1H-indol-2-yl)phenyl)amino)-2-carbonylacetic acid methyl ester **A10-1** (110 mg, 88% yield). MS-ESI [M+H] $^+$: 435.1.

**Step 2:** 2-((4-(1-Cyclobutyl-5-fluoro-6-(*1H*-tetrazol-5-yl)-1H-indole-2-yl)phenyl)amino)-2-carbonylacetic acid

To a mixed solution of 2-((4-(1-cyclobutyl-5-fluoro-6-(1H-tetrazol-5-yl)-1H-indol-2-yl)phenyl)amino)-2-carbonylacetic acid methyl ester **A10-1** (99 mg, 0.23 mmol) in tetrahydrofuran (2 mL) and water (0.5 mL), lithium hydroxide monohydrate (11.5 mg, 0.27 mmol) was added, and the reaction was stirred at room temperature for 1 hour. Then, 1 N hydrochloric acid was added to adjust the pH of the reaction solution to 5-6, ethyl acetate (15 mL) was added, and the mixture was separated. The organic phase was dried and concentrated to give 2-((4-(1-cyclobutyl-5-fluoro-6-(1H-tetrazol-5-yl)-1H-indol-2-yl)phenyl)amino)-2-carbonylacetic acid **A10** (80 mg, 82% yield). $^1$H NMR (400 MHz, DMSO-$d_6$) δ 10.94 (s, 1H), 8.34 (d, *J* = 5.8 Hz, 1H), 7.95 (d, *J* = 8.7 Hz, 2H), 7.63 (d, *J* = 11.3 Hz, 1H), 7.53 (d, *J* = 8.6 Hz, 2H), 6.58 (s, 1H), 5.13-5.04 (m, 1H), 2.75-2.64 (m, 2H), 2.43-2.36 (m, 2H), 1.87-1.73 (m, 2H). MS-ESI [M+H] $^+$: 421.1.

Using the synthetic method of intermediate **A10,** the following intermediates **A11** to **A13** were prepared.

| intermediate | structural | raw material | Characterization data |
|---|---|---|---|
| **A11** | 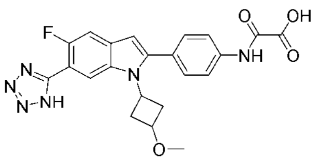 | 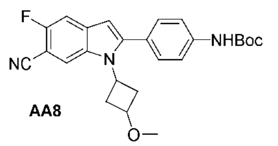 AA8 | MS-ESI [M+H]$^+$: 451.3 |
| **A12** | 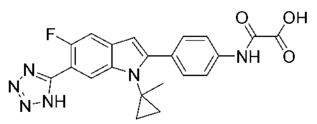 | 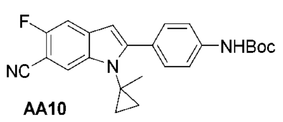 AA10 | MS-ESI [M+H]$^+$: 421.2 |
| **A13** | 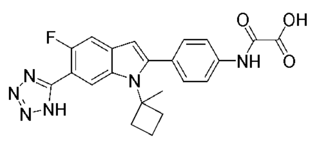 | 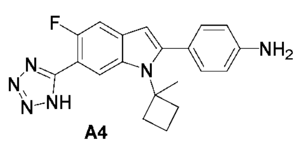 A4 | $^1$H NMR (400 MHz, DMSO-$d_6$) δ 10.91 (s, 1H), 7.97 (d, J=6.0 Hz, 1H), 7.89 (d, *J* = 8.8 Hz, 2H), 7.60 (d, *J* = 11.2 Hz, 1H), 7.50 (d, *J* = 8.8 Hz, 2H), 6.49 (s, 1H), 2.26-2.15 (m, 2H), 2.19-2.05 (m, 2H), 1.97 (s, 3H), 1.92-1.83(m, 1H), 1.67-1.60 (m,1H). MS-ESI [M+H]$^+$: 435.3 |

**Synthesis of intermediate A14: 2-((4-(1-(Cyclopropylmethyl)-5-fluoro-6-(1H-tetrazol-5-yl)-1H-indol-2-yl)phenyl) amino)-2-carbonylacetic acid**

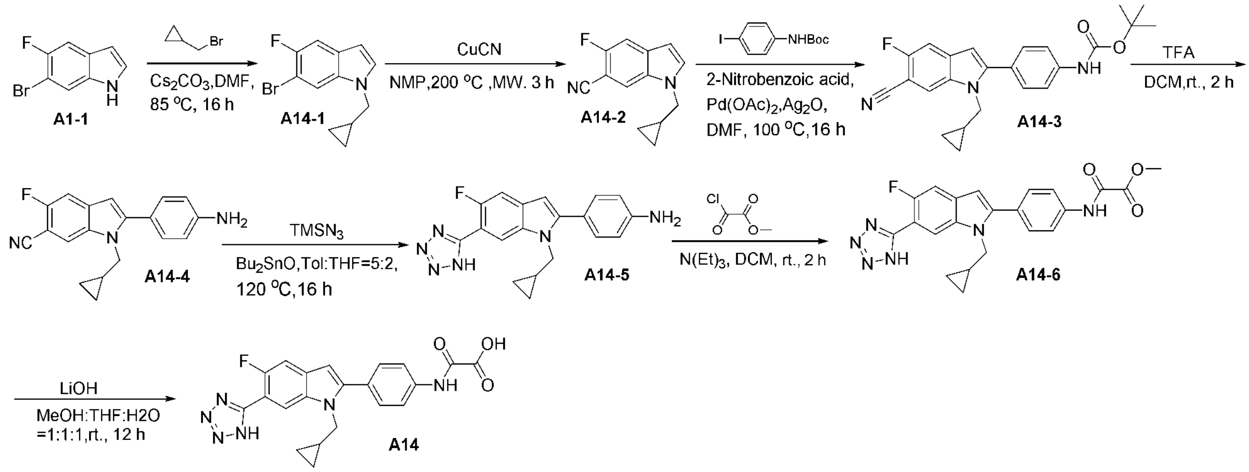


**Step 1:** 6-Bromo-1-(cyclopropylmethyl)-5-fluoro-*1H*-indole

To a DMF (70 mL) solution of 6-bromo-5-fluoro-*1H*-indole **A1-1** (3.0 g, 14.0 mmol) and cesium carbonate (13.7 g, 42.0 mmol), bromomethylcyclopropane (3.78 g, 28.0 mmol) was added. The mixture was heated to 85 °C and stirred for 16 hours. After the reaction was complete, the mixture was cooled, filtered, and the filtrate was washed with lithium chloride aqueous solution (10 mL) and concentrated. The filtrate was purified by silica gel column chromatography (PE/ EA = 4/1) to give a colorless oily substance **A14-1** (3.1 g). MS-ESI $[M+H]^+$: 268.0.

**Step 2:** 1-(Cyclopropylmethyl)-5-fluoro-*1H*-indole-6-carbonitrile

Cuprous cyanide (3.62 g, 40.5 mmol) was added to an NMP solution (60 mL) of 1-cyclobutyl-5-methoxy-1H-indole-6-carboxylic acid methyl ester **A14-1** (3.1 g, 11.56 mmol). The mixture was heated to 200 °C and stirred for 3 hours. After the reaction was complete, the mixture was cooled, filtered, and the filtrate was washed with an aqueous lithium chloride solution (10 mL) and concentrated. The filtrate was purified by silica gel column chromatography (PE/ EA = 1/1) to give a light green solid **A14-2** (2.0 g). MS-ESI $[M+H]^+$: 215.1.

**Step 3:** tert-Butyl (4-(6-cyano-1-(cyclopropylmethyl)-5-fluoro-*1H*-indol-2-yl)phenyl)carbamate

Palladium acetate (104.8 mg, 0.467 mmol) was added to a DMF (70 mL) solution of 1-(cyclopropylmethyl)-5-fluoro-*1H*-indole-6-carbonitrile **A14-2** (2.0 g, 9.34 mmol), (4-iodophenyl)carbamate tert-butyl ester (4.47 g, 14.0 mmol), 2-nitrobenzoic acid (2.34 g, 14.0 mmol), and silver oxide (1.62 g, 7.0 mmol). The mixture was heated to 100 °C and stirred for 16 hours under nitrogen. After the reaction was complete, the mixture was cooled, filtered, and the filtrate was washed with lithium chloride aqueous solution (10 mL) and concentrated. The filtrate was purified by silica gel column chromatography (PE/DCM = 1/1) to give a pale yellow solid **A14-3** (1.0 g). MS-ESI $[M+H]^+$: 406.2.

**Step 4:** 2-(4-Aminophenyl)-1-(cyclopropylmethyl)-5-fluoro-*1H*-indole-6-carbonitrile

Trifluoroacetic acid (2 mL) was added to a solution of tert-butyl (4-(6-cyano-1-(cyclopropylmethyl)-5-fluoro-*1H*-indol-2-yl)phenyl)carbamate **A14-3** (900 mg, 2.22 mmol) in dichloromethane (10 mL). The mixture was stirred at room temperature for 2 hours. After the reaction was complete, the solution was concentrated to dryness, and dichloromethane (50 mL) was added. The solution was washed twice with sodium bicarbonate aqueous solution (10 mL), dried, and concentrated to obtain crude product **A14-4.** MS-ESI $[M+H]^+$: 306.1.

**Step 5:** 4-(1-(Cyclopropylmethyl)-5-fluoro-6-(1H-tetrazol-5-yl)-1H-indol-2-yl)aniline

To a 25 mL mixed solution of 2-(4-aminophenyl)-1-(cyclopropylmethyl)-5-fluoro-*1H*-indole-6-carbonitrile **A14-4**

(720 mg, 2.36 mmol) and dibutyltin oxide (88.1 mg, 0.354 mmol) in toluene/ tetrahydrofuran (5/2), azidotrimethylsilane (1.63 g, 14.2 mmol) was added. The mixture was heated to 120 °C and stirred for 16 hours. After the reaction was complete, the mixture was concentrated and purified by silica gel column chromatography (DCM/MeOH = 95/5) to give a pale yellow solid **A14-5** (630 mg). MS-ESI [M+H] $^+$: 349.1.

**Step 6:** 2-((4-(1-(Cyclopropylmethyl)-5-fluoro-6-(1H-tetrazol-5-yl)-1H-indole-2-yl)phenyl)amino)-2-carbonylacetic acid methyl ester

Oxaloyl chloride monomethyl ester (158.2 mg, 1.29 mmol) was added to a solution of 4-(1-(cyclopropylmethyl)-5-fluoro-6-(1H-*tetrazol*-5-yl)-1H-indol-2-yl)aniline **A14-5** (300 mg, 0.861 mmol) and triethylamine (261.4 mg, 2.58 mmol) in dichloromethane (25 mL). The mixture was stirred at room temperature for 2 hours. After the reaction was complete, the mixture was washed with brine (10 mL), dried, and concentrated to obtain crude **A14-6** (300 mg). MS-ESI [M+H] $^+$: 435.1.

**Step 7:** 2-((4-(1-(Cyclopropylmethyl)-5-fluoro-6-(1H-tetrazol-5-yl)-1H-indole-2-yl)phenyl)amino)-2-carbonylacetic acid

Lithium hydroxide (58.0 mg, 1.38 mmol) was added to a THF/MeOH/$H_2O$=1/1/1 mixed solution (12 mL) of 2-((4-(1-(cyclopropylmethyl)-5-fluoro-6-(1H-tetrazol-5-yl)-1H-indol-2-yl)phenyl)amino)-2-carbonylacetic acid methyl ester **A14-6** (0.3 g, 0.691 mmol). The mixture was stirred at room temperature for 12 hours. After the reaction was complete, the mixture was concentrated, the pH was adjusted to 7 with 1 N hydrochloric acid, extracted with a DCM/MeOH=10/1 mixture (50 mL), then concentrated to give a white solid **A14** (230 mg). $^1$H NMR (400 MHz, DMSO-*d6*) δ 10.60 (s, 1H), 8.28 (d, *J* = 5.6 Hz, 1H), 7.96 (d, *J* = 8.4 Hz, 2H), 7.58-7.51 (m, 3H), 6.60 (s, 1H), 4.22 (d, *J* = 6.8 Hz, 2H), 0.98-0.89 (m, 1H), 0.33-0.27 (m, 2H), 0.10-0.04 (m, 2H). MS-ESI [M+H] $^+$: 421.1.

Using the synthetic method of intermediate **A14,** the following intermediates **A15 to A17** were prepared.

| Intermediate | Structural | Raw material | Characterization data |
|---|---|---|---|
| **A15** | 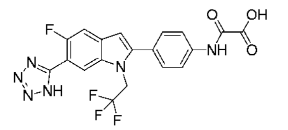 | 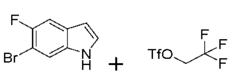 | MS-ESI [M+H]$^+$: 449.1 |
| **A16** | 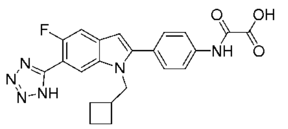 | 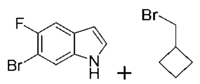 | $^1$H NMR (400 MHz, DMSO-$d_6$) δ 10.80 (s, 1H), 8.25 (d, *J* = 5.6 Hz, 1H), 7.97 (d, *J* = 8.4 Hz, 2H), 7.61-7.53 (m, 3H), 6.60 (s, 1H), 4.39 (d, *J* = 7.2 Hz, 2H), 2.49-2.43 (m, 1H)., 1.71-1.55 (m, 4H), 1.50-1.43 (m, 2H). MS-ESI [M+H]$^+$: 435.1 |
| **A17** | 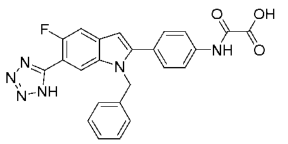 | 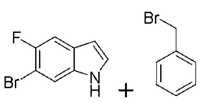 | MS-ESI [M+H]$^+$: 457.0 |

**Synthesis of intermediate A18:** **4-(1-Cyclobutyl-6-(*1H*-1,2,3-triazol-5-yl)-1H-indol-2-yl)aniline**

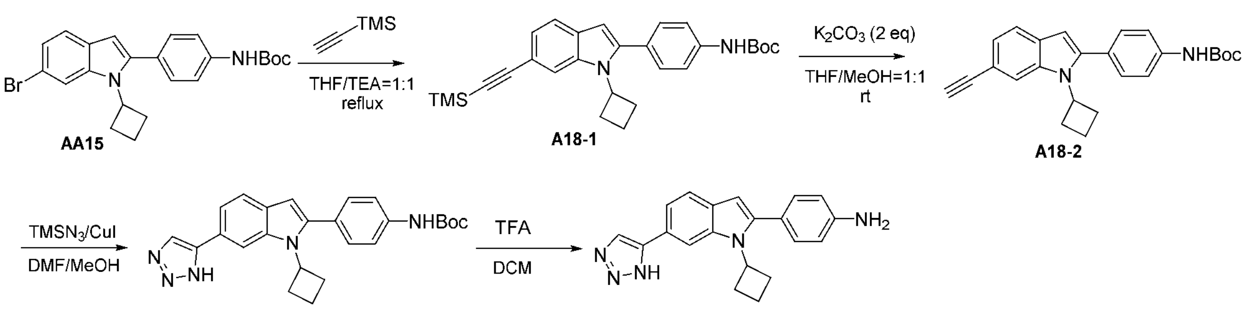


A18-3 A18

**Step 1:** tert-Butyl (4-(1-cyclobutyl-6-((trimethylsilyl)ethynyl)-1H-indol-2-yl)phenyl)carbamate

To a solution of tert-butyl (4-(6-bromo-1-cyclobutyl-1H-indol-2yl)phenyl)carbamate **AA15** (441 mg, 1 mmol) in a mixture of THF (30 mL) and TEA (20 mL), cuprous iodide (19 mg, 0.1 mmol), tetrakis(triphenylphosphine)-palladium (116 mg, 0.1 mmol), and trimethylethynylsilane (295 mg, 0.45 mmol) were added. The resulting mixture was heated to 100 °C for 24 h. The mixture was concentrated and purified by silica gel column chromatography (PE/EA = 10/1) to give a yellow solid product **A18-1** (445 mg, 81% yield). MS-ESI $[M+H]^+$: 459.1.

**Step Two:** tert-Butyl (4-(1-cyclobutyl-5-fluoro-6-(5-(trifluoromethyl)-1H-1,2,4-triazol-3-yl)-1H-indol-2-yl)phenyl)carbamate

Potassium carbonate (265 mg, 1.92 mmol) was added to a THF solution (60 mL) of tert-butyl (4-(1-cyclobutyl-6-((trimethylsilyl)ethynyl)-1H-indol-2-yl)phenyl)carbamate **A18-1** (440 mg, 0.96 mmol) and methanol (60 mL). The mixture was stirred at room temperature for 2 hours under nitrogen atmosphere. The reaction solution was concentrated and purified by silica gel column chromatography (PE/EA = 10/1) to give a yellow oily liquid product **A18-2** (245 mg, 61% yield). MS-ESI $[M+H]^+$: 387.1.

**Step 3:** tert-Butyl (4-(1-cyclobutyl-6-(1H-1,2,3-triazol-5-yl)-1H-indole-2-yl)phenyl)carbamate

To a solution of tert-butyl (4-(1-cyclobutyl-5-fluoro-6-(5-(trifluoromethyl)-1H-1,2,4-triazol-3-yl)-1H-indol-2-yl) phenyl)carbamate **A18-2** (240 mg, 0.62 mmol) in a mixture of DMF (0.9 mL) and methanol (0.1 mL), cuprous iodide (6 mg, 0.03 mmol) and azidotrimethylsilane (107 mg, 0.9 mmol) were added. The resulting mixture was heated to 100 °C for 16 h. The mixture was concentrated and purified by silica gel column chromatography (PE/EA = 3/1) to give a yellow solid product **A18-3** (150 mg, 62% yield). MS-ESI $[M+H]^+$: 430.1.

**Step 4:** 4-(1-Cyclobutyl-6-(*1H*-1,2,3-triazol-5-yl)-1H-indole-2-yl)aniline

To a solution of (4-(1-cyclobutyl-6-(*1H*-1,2,3-triazol-5-yl)-1H-indol-2-yl)phenyl)carbamate tert-butyl ester (150 mg, 0.35 mmol) in DCM (5 mL) was added trifluoroacetic acid (1 mL). The resulting mixture was reacted at room temperature for 3 hours. The reaction solution was cooled to room temperature and added with an aqueous sodium bicarbonate solution (50 mL), extracted three times with ethyl acetate (20 mL). The organic phase was washed with saturated brine (10 mL), dried over anhydrous sodium sulfate, filtered, concentrated, and purified by silica gel column chromatography (PE/EA = 1/1) to give a yellow solid product A18 (93 mg, 80% yield). MS-ESI $[M+H]^+$: 330.1.

**<u>Synthesis of intermediate A19:</u> 3-(2-(4-Aminophenyl)-1-cyclobutyl-5-fluoro-*1H*-indol-6-yl)-1,2,4-oxadiazole-5 (*4H*)-one hydrochloride**

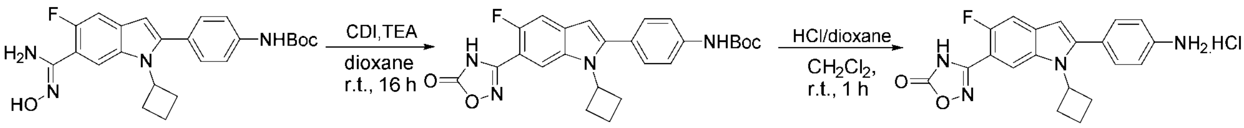


AA16 A19-1 A19

**Step** 1: tert-Butyl (4-(1-cyclobutyl-5-fluoro-6-(5-oxo-4,5-dihydro-1,2,4-oxadiazol-3-yl)-1H-indol-2-yl)phenyl)carbamate

CDI (148 mg, 0.96 mmol) and TEA (138 mg, 1.37 mmol) were added to a 1,4-dioxane solution (10 mL) of tert-butyl (4-(1-cyclobutyl-5-fluoro-6-(N'-hydroxyaminoformimidoyl)-1H-indol-2-yl)phenyl)carbamate **AA16** (200 mg, 0.46 mmol). The resulting mixture was stirred at room temperature under nitrogen for 16 hours. After the reaction was complete, the mixture was concentrated under reduced pressure and purified by silica gel column chromatography (PE/EA = 2/1) to give a yellow solid product **A19-1** (200 mg, yield 93%). MS-ESI $[M+H]^+$: 465.2.

**Step 2:** 3-(2-(4-Aminophenyl)-1-cyclobutyl-5-fluoro-*1H*- indol-6-yl)-1,2,4-oxadiazol-5(*4H*)-one hydrochloride

A solution of hydrochloride solution (4 M, 2 mL, 8 mmol) was added to a solution of (4-(1-cyclobutyl-5-fluoro-6-(5-oxo-4,5-dihydro-1,2,4-oxadiazol-3-yl)-1H-indol-2-yl)phenyl)carbamate tert-butyl ester **A19-1** (200 mg, 0.43 mmol) in

dichloromethane (10 mL), and the mixture was stirred at room temperature for 1 hour. After the reaction was complete, the mixture was concentrated under reduced pressure to give a yellow solid crude product **A19** (100 mg). MS-ESI [M+H] +: 365.1.

**Synthesis of intermediate A20: 4-(1-Cyclobutyl-5-fluoro-6-(5-(trifluoromethyl)-1H-1,2,4-triazol-3-yl)-1H-indol-2-yl)aniline**

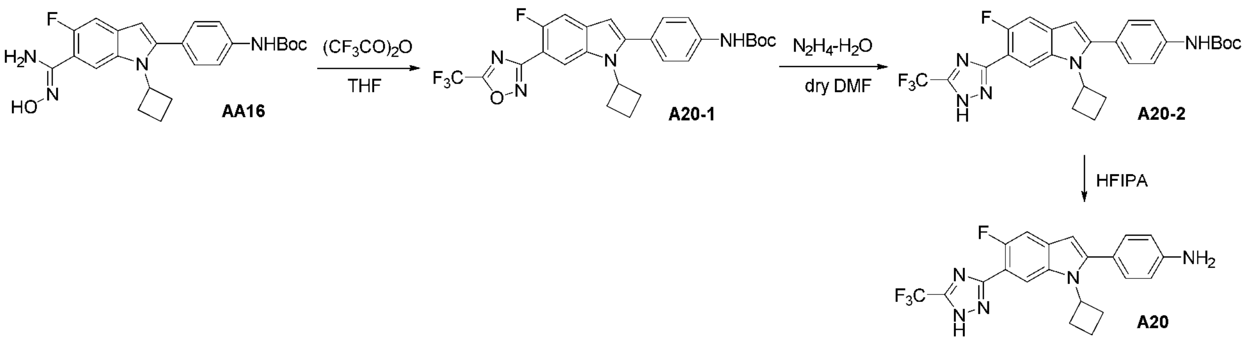


**Step 1:** tert-Butyl (4-(1-cyclobutyl-5-fluoro-6-(5-(trifluoromethyl)-1,2,4-oxadiazol-3-yl)-1H-indol-2-yl)phenyl)carbamate

Trifluoroacetic anhydride (115 mg, 0.55 mmol) was added to a mixture solution of (Z)-(4-(1-cyclobutyl-5-fluoro-6-(N'-hydroxyaminoformimidoyl)-*1H*-indol-2-yl)phenyl)carbamate tert-butyl ester **AA16** (200 mg, 0.45 mmol) and tetrahydrofuran (5 mL). The resulting mixture was reacted at room temperature for 16 hours. The mixture was concentrated and purified by silica gel column chromatography (PE/EA = 4/1) to give a yellow solid product **A20-1** (120 mg, 51% yield). MS-ESI [M+H] +: 517.2.

**Step 2:** tert-Butyl (4-(1-cyclobutyl-5-fluoro-6-(5-(trifluoromethyl)-1H-1,2,4-triazol-3-yl)-1H-indole-2-yl)phenyl)carbamate

Hydrazine hydrate (2 mL) was added to a DMF solution (3 mL) of tert-butyl (4-(1-cyclobutyl-5-fluoro-6-(5-(trifluoromethyl)-1,2,4-triazol-3-yl)-1H-indole-2-yl)phenyl)carbamate **A20-1** (120 mg, 0.23 mmol). The mixture was stirred at room temperature for 2 hours under nitrogen atmosphere. The reaction solution was added with water (10 mL), extracted three times with ethyl acetate (20 mL). The organic phase was washed with saturated brine (10 mL), dried over anhydrous sodium sulfate, filtered, concentrated, and purified by silica gel column chromatography (PE/EA = 4/1) to give a yellow solid product **A20-2** (100 mg, 83% yield). MS-ESI [M+H] +: 516.2.

**Step 3:** 4-(1-Cyclobutyl-5-fluoro-6-(5-methyl-*4H*-1,2,4-triazol-3-yl)-1H-indole-2-yl)aniline

To a solution of hexafluoroisopropanol (5 mL) was added tert-butyl (4-(1-cyclobutyl-5-fluoro-6-(5-(trifluoromethyl)-1H-1,2,4-triazol-3-yl)-1H-indol-2-yl)phenyl)carbamate (100 mg, 0.19 mmol). The resulting mixture was heated in a microwave tube to 120 °C for 12 h. Direct concentration gave a yellow solid crude product **A20** (80 mg). MS-ESI [M+H] +: 416.1.

**Synthesis of intermediate A21: 5-(2-(4-Aminophenyl)-1-cyclobutyl-5-fluoro-*1H*-indol-6-yl)-4H-1,2,4-triazol-3-carbonitrile**

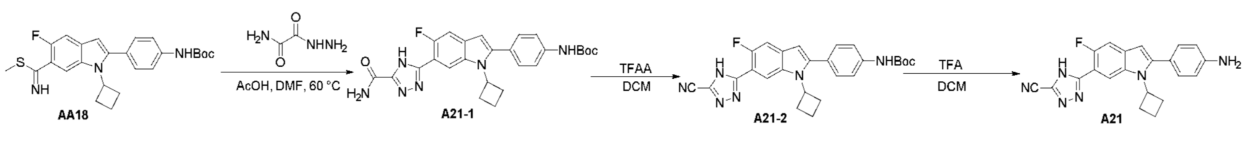

**Step 1:** tert-Butyl (4-(6-(5-carbamoyl-*4H*-1,2,4-triazol-3-yl)-1-cyclobutyl-5-fluoro-*1H*-indole-2-yl)phenyl)carbamate

To a mixture of methyl 2-(4-((tert-butoxycarbonyl)amino)phenyl)-1-cyclobutyl-5-fluoro-*1H*-indole-6-carbamimidothioate **AA18** (320 mg, 0.7 mmol) and DMF (5 mL), 2-hydrazino-2-carbonylacetamide (95 mg, 0.92 mmol) and acetic acid (1 drop) were added. The resulting mixture was heated to 65 °C for 3 hours. The reaction mixture was cooled to room temperature, and water (10 mL) was added. The mixture was extracted three times with ethyl acetate (20 mL), the organic phase was washed with saturated brine (10 mL), dried over anhydrous sodium sulfate, filtered, concentrated, and purified by silica gel column chromatography (DCM/EA = 2/1) to give a white solid product **A21-1** (220 mg, 89% yield). MS-ESI [M+H] $^+$: 491.2.

**Step 2:** tert-Butyl (4-(1-cyclobutyl-5-fluoro-6-(5-(trifluoromethyl)-1,2,4-oxadiazol-3-yl)-1H-indol-2-yl)phenyl)carbamate

To a mixture solution of tert-butyl (4-(6-(5-carbamoyl-*4H*-1,2,4-triazol-3-yl)-1-cyclobutyl-5-fluoro-*1H*-indole-2-yl) phenyl)carbamate **A21-1 (220** *mg,* 0.45 mmol) in dichloromethane (5 mL), trifluoroacetic anhydride (141 mg, 0.67 mmol) and triethylamine (91 mg, 0.89 mmol) were added. The mixture was reacted at room temperature for 2 hours. The product was concentrated and purified by silica gel column chromatography (DCM/EA = 3/1) to give a yellow solid product **A20-2** (110 mg, 50% yield). MS-ESI [M+H] $^+$: 472.2.

**Step 3:** 5-(2-(4-Aminophenyl)-1-cyclobutyl-5-fluoro-*1H*-indole-6-yl)-4H-1,2,4-triazol-3-carbonitrile

Trifluoroacetic acid (0.5 mL) was added to a solution of tert-butyl (4-(1-cyclobutyl-5-fluoro-6-(5-methyl-*4H*-1,2,4-triazol-3-yl)-1H-indol-2-yl)phenyl)carbamate (90 mg, 0.2 mmol) in dichloromethane (2 mL). The resulting mixture was reacted at room temperature for 3 hours. Direct concentration gave a yellow solid crude product **A21** (60 mg). MS-ESI [M+H] $^+$: 373.1.

<u>**Synthesis of intermediate A22:**</u> **4-(1-Cyclobutyl-5-fluoro-6-(5-methyl-*4H*-1,2,4-triazol-3-yl)-1H-indol-2-yl)aniline**

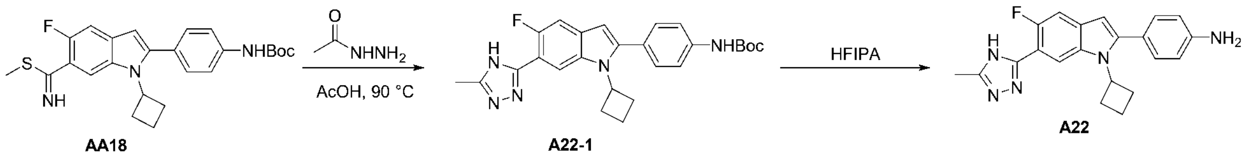


AA18 A22-1 A22

**Step 1:** tert-Butyl (4-(1-cyclobutyl-5-fluoro-6-(5-methyl-*4H*-1,2,4-triazol-3-yl)-1H-indol-2-yl)phenyl)carbamate

To a solution of methyl 2-(4-((tert-butoxycarbonyl)amino)phenyl)-1-cyclobutyl-5-fluoro-*1H*-indole-6-carbamimidothioate **AA18** (100 mg, 0.2 mmol) in DMF (5 mL), acetylhydrazine (25 mg, 0.3 mmol) and acetic acid (2 drops) were added. The resulting mixture was heated to 90 °C and reacted for 36 hours. The reaction mixture was cooled to room temperature and added with water (10 mL), extracted three times with ethyl acetate (20 mL). The organic phase was washed with saturated brine (10 mL), dried over anhydrous sodium sulfate, filtered, concentrated, and subjected to silica gel column chromatography (PE/EA = 4/1) to give a yellow solid product **A22-1** (90 mg, 89% yield). MS-ESI [M+H] $^+$: 462.2.

**Step 2:** 4-(1-Cyclobutyl-5-fluoro-6-(5-methyl-*4H*-1,2,4-triazol-3-yl)-1H-indol-2-yl)aniline

To a solution of hexafluoroisopropanol (5 mL) was added tert-butyl 4-(1-cyclobutyl-5-fluoro-6-(5-methyl-*4H*-1,2,4-triazol-3-yl)-1H-indol-2-yl)phenyl)carbamate (90 mg, 0.2 mmol). The resulting mixture was heated in a microwave tube to 120 °C for 12 hours. Direct concentration gave a yellow solid crude product **A22** (60 mg). MS-ESI [M+H] $^+$: 362.1.

<u>**Synthesis of intermediate A23:**</u> **4-(1-Cyclobutyl-5,7-difluoro-6-(*1H*-tetrazol-5-yl)-1H-indol-2-yl)aniline**

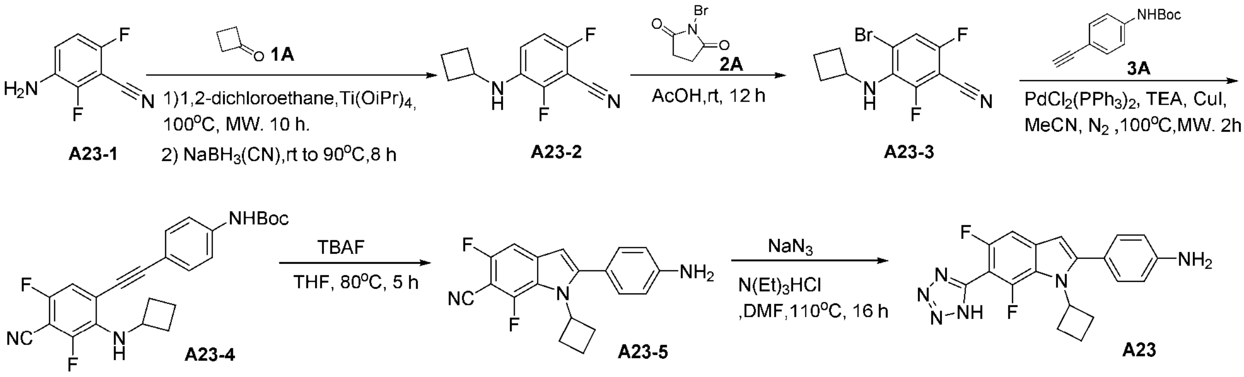


**Step 1:** 3-(Cyclobutylamino)-2,6-difluorobenzonitrile

To a solution of 3-amino-2,6-difluorobenzonitrile **A23-1** (2.0 g, 7.14 mmol) and cyclobutanone (4.51 g, 64.28 mmol) in 1,2-dichloroethane (20 mL) was added tetraisopropyl titanate (13.2 g, 37.4 mmol). The resulting mixture was heated to 100 °C and stirred for 10 hours under microwave conditions. After the reaction was complete, the mixture was cooled to room temperature, and sodium cyanoborohydride (12.1 g, 57.1 mmol) was added in portions with stirring. The mixture was heated to 90 °C and stirred for 8 hours. After the reaction was complete, the mixture was cooled, and the reaction mixture was poured into a saturated sodium bicarbonate aqueous solution (50 mL). The mixture was filtered, and the filtrate was washed with a saturated sodium bicarbonate aqueous solution and concentrated. The filtrate was purified by silica gel column chromatography (PE/EA = 80/20) to give a colorless oily substance **A23-2** (1.2 g, 80% yield). MS-ESI $[M+H]^+$: 209.1.

**Step 2:** 4-Bromo-3-(cyclobutylamino)-2,6-difluorobenzonitrile

N-bromosuccinimide (2.05 g, 11.5 mmol) was added to AcOH solution (15 mL) of 3-(cyclobutylamino)-2,6-difluorobenzonitrile **A23-2** (1.5 g, 7.20 mmol). The resulting mixture was stirred at room temperature for 12 hours. After the reaction was complete, the reactants were poured into 50 mL of saturated sodium bicarbonate aqueous solution under ice-water bath condition. The mixture was extracted three times with ethyl acetate (50 mL), concentrated, then purified by silica gel column chromatography (PE/EtOAc = 85/15) to give a colorless oily substance **A23-3** (1.2 g, yield 58%). MS-ESI $[M+H]^+$: 289.0.

**Step 3:** tert-Butyl (4-(4-cyano-2-(cyclobutylamino)-3,5-difluorophenyl)ethynyl)carbamate

To a solution of 4-bromo-3-(cyclobutylamino)-2,6-difluorobenzonitrile **A23-3** (0.6 g, 2.09 mmol), tert-butyl (4-ethynylphenyl)carbamate (0.454 g, 2.09 mmol), triethylamine (0.423 g, 4.18 mmol), and copper(I) iodide (27.9 mg, 146 μmol) in acetonitrile (10 mL) was added bis(triphenylphosphine)palladium(II) dichloride (103 mg, 146 μmol). The resulting mixture was microwave-heated to 100 °C and stirred for 2 hours under nitrogen atmosphere. After the reaction was complete, the mixture was cooled, filtered, concentrated to dryness, and purified by silica gel column chromatography (PE/DCM = 70/30) to give a colorless oil **A23-4** (280 mg, 41% yield). MS-ESI $[M+H]^+$: 424.1.

**Step 4:** 2-(4-Aminophenyl)-1-cyclobutyl-5,7-difluoro-1H-indole-6-carbonitrile

To a solution of tert-butyl (4-(4-cyano-2-(cyclobutylamino)-3,5-difluorophenyl)ethynyl)carbamate **A23-4** (0.36 g, 0.85 mmol) in tetrahydrofuran (8 mL) was added a 1 M solution of tetrabutylammonium fluoride in tetrahydrofuran (4.25 mL, 4.25 mmol). The resulting mixture was heated to 80 °C and stirred for 12 hours. After the reaction was complete, the mixture was cooled, and ethyl acetate (50 mL) was added. The organic phase was washed three times with 1 N hydrochloric acid aqueous solution (10 mL) and concentrated, and purified by silica gel column chromatography (PE/EA = 70/30) to give a colorless oily substance **A23-5** (230 mg, yield 83%). MS-ESI $[M+H]^+$: 324.1.

**Step 5:** 4-(1-Cyclobutyl-5,7-difluoro-6-(*1H*-tetrazol-5-yl)-1H-indole-2-yl)aniline

Sodium azide (402 mg, 6.19 mmol) was added to a DMF (15 mL) solution of 2-(4-aminophenyl)-1-cyclobutyl-5,7-difluoro-1H-indole-6-carbonitrile **A23-5** (200 mg, 0.619 mmol) and triethylamine hydrochloride (1.28 g, 9.28 mmol). The resulting mixture was heated to 110 °C and reacted for 16 hours. After the reaction was complete, the mixture was cooled,

filtered, and the filtrate was separated by alkaline method to obtain an off-white solid product **A23** (18 mg, yield 7%). $^1$H NMR (400 MHz, DMSO-$d_6$) δ 7.24 (d, *J* = 9.6 Hz, 1H), 7.16-7.12 (m, 2H), 6.69 (d, *J* = 8.4 Hz, 2H), 6.41 (d, *J* = 2.4 Hz, 1H), 5.00 (q, *J* = 9.2 Hz, 1H), 2.69-2.64 (m, 1H), 2.34-2.32 (m, 2H), 2.02-1.95 (m, 2H), 1.77-1.66 (m, 2H). MS-ESI [M+H] $^+$: 367.1.

**<u>Synthesis of intermediate A24:</u> 4-(1-Cyclobutyl-6-(*1H*-tetrazol-5-yl)-1H-indol-2-yl)aniline**

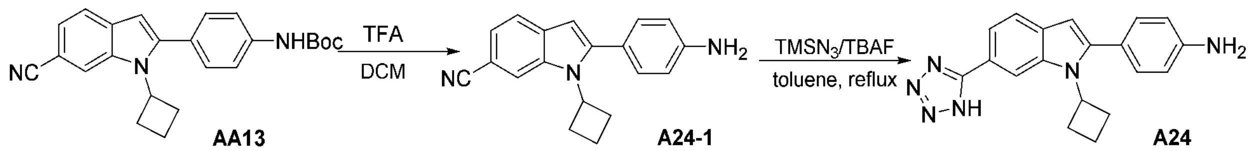


**AA13** **A24-1** **A24**

**Step** 1: 2-(4-Aminophenyl)-1-cyclobutyl-*1H*-indole-6-carbonitrile

Trifluoroacetic acid (2 mL) was added to a solution of tert-butyl 4-(6-cyano-1-cyclobutyl-1H-indol-2-yl)phenyl) carbamate **AA13** (700 mg, 1.8 mmol) in dichloromethane (10 mL). The mixture was stirred at room temperature for 2 hours. After the reaction was complete, the mixture was concentrated to dryness, and dichloromethane (30 mL) was added. The pH was adjusted to 9.0 with saturated sodium bicarbonate aqueous solution (5 mL). The organic phase was concentrated to dryness to give an off-white solid **A24-1** (310 mg, yield 59%). MS-ESI [M+H] $^+$: 288.0.

**Step 2:** 4-(1-Cyclobutyl-6-(*1H*-tetrazol-5-yl)-1H-indole-2-yl)aniline

A solution of 1 N tetrabutylammonium fluoride in tetrahydrofuran (0.5 mL, 0.5 mmol) was added to a toluene solution (8 mL) of 2-(4-aminophenyl)-1-cyclobutyl-*1H*-indole-6-carbonitrile **A24-2** (287 mg, 1.0 mmol) and trimethylcyanosilane (230 mg, 2.0 mmol). The resulting mixture was heated to 110 °C and stirred for 17 hours. After the reaction was complete, the mixture was concentrated to dryness and purified by silica gel column chromatography (DCM/MeOH = 10/1) to give an off-white solid **A24** (130 mg, yield 39%). MS-ESI [M+H] $^+$: 331.1.

**A25** to **A29** were prepared using the same method as intermediate **A24.**

| Intermediate | Structure | Raw material | Characterization data |
|---|---|---|---|
| **A25** | 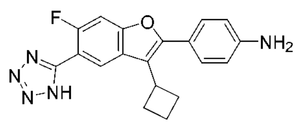 | 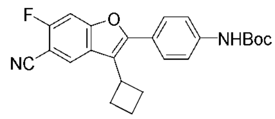 **AA17** | MS-ESI [M+H]$^+$: 350.1 |
| **A26** | 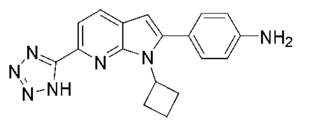 | 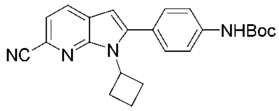 **AA14** | MS-ESI [M+H]$^+$: 332.1 |
| **A27** | 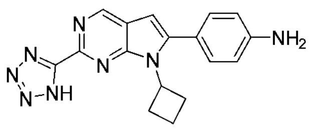 | 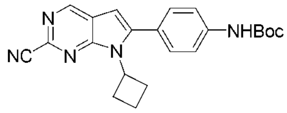 **AA11** | MS-ESI [M+H]$^+$: 333.1 |
| **A28** | 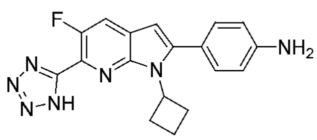 | 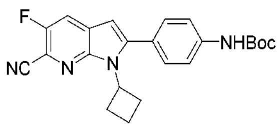 **AA12** | MS-ESI [M+H]$^+$: 350.1 |

(continued)

| Intermediate | Structure | Raw material | Characterization data |
|---|---|---|---|
| **A29** | 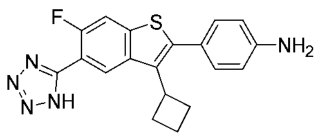 | 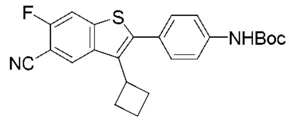 **AA19** | MS-ESI $[M+H]^+$: 366.1 |

**Synthesis of intermediate BB1: 2-(Pyrrolidine-3-yl)ethane-1-ol hydrochloride**

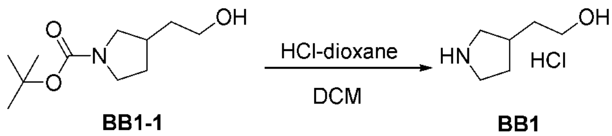


**BB1-1** **BB1**

To a solution of 3-(2-hydroxyethyl)pyrrolidine-1-carboxylic acid tert-butyl ester **BB1-1** (200 mg, 0.93 mmol) in dichloromethane (10 mL), dioxane hydrochloride solution (4 M, 2 mL, 8 mmol) was added, and the mixture was stirred at room temperature for 3 hours. The reaction was quenched with saturated sodium bicarbonate aqueous solution (10 mL), and the mixture was extracted three times with dichloromethane (20 mL). The organic phases were combined and evaporated to dryness to give crude product **BB1** (100 mg), which was used directly in the next step. MS-ESI $[M+H]^+$: 116.1.

The following intermediates **BB2 to BB15** were obtained using the synthesis method of intermediate **BB1.**

| Intermediate | Structure | Raw material | Characterization data |
|---|---|---|---|
| **BB2** | 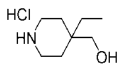 | 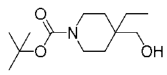 | MS-ESI $[M+H]^+$: 144.1 |
| **BB3** | 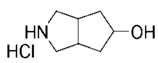 | 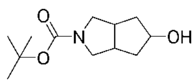 | MS-ESI $[M+H]^+$: 128.1 |
| **BB4** | 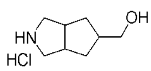 | 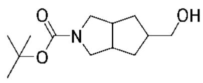 | MS-ESI $[M+H]^+$: 142.1 |
| **BBS** | 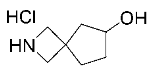 | 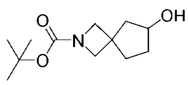 | MS-ESI $[M+H]^+$: 128.1 |
| **BB6** | 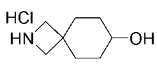 | 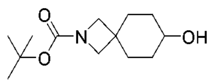 | MS-ESI $[M+H]^+$: 142.1 |
| **BB7** | 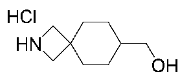 | 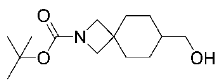 | MS-ESI $[M+H]^+$: 156.2 |
| **BB8** | 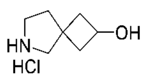 | 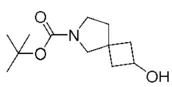 | MS-ESI $[M+H]^+$: 128.2 |
| **BB9** | 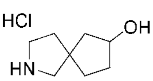 | 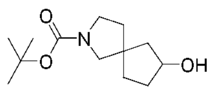 | MS-ESI $[M+H]^+$:142.2 |
| **BB10** | 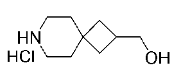 | 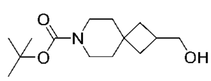 | MS-ESI $[M+H]^+$:156.1 |

(continued)

| Intermediate | Structure | Raw material | Characterization data |
|---|---|---|---|
| **BB11** | 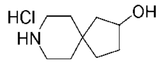 | 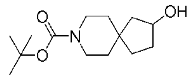 | MS-ESI [M+H]$^+$:247.3 |
| **BB12** | 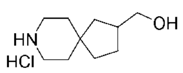 | 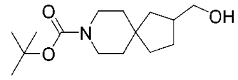 | MS-ESI [M+H]$^+$: 170.2 |
| **BB13** | 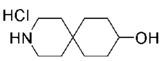 | 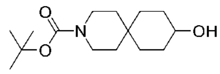 | MS-ESI [M+H]$^+$: 170.1 |
| **BB14** | 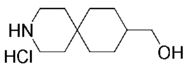 | 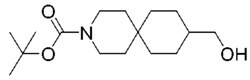 | MS-ESI [M+H]$^+$: 184.1 |
| **BB15** | 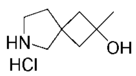 | 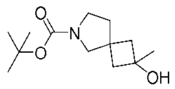 | MS-ESI [M+H]$^+$: 142.2 |

<u>Synthesis of intermediate BB16:</u> **(3-methylpiperidin-4-yl)methanol hydrochloride**

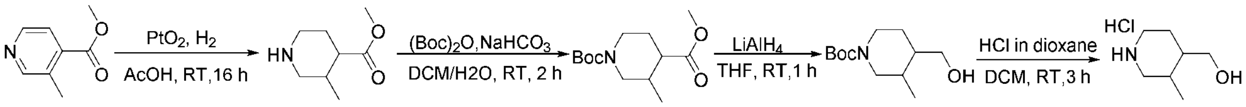


**BB16-1** **BB16-2** **BB16-3** **BB16-4** **BB16**

**Step** 1: Methyl-3-methylpiperidine-4-carboxylate

Platinum dioxide (150 mg, 12.5 mmol) was added to a 4 mL acetic acid solution of methyl-3-methylisonicotinate **BB16-1** (400 mg, 2.64 mmol). The reaction was stirred at room temperature for 16 hours under a hydrogen balloon. The solution was concentrated to give crude methyl-3-methylpiperidine-4-carboxylate **BB16-2.** MS-ESI [M+H] $^+$: 158.1.

**Step 2:** 1-(Tert-butyl)-4-methyl-3-methylpiperidine-1,4-dicarboxylate

To a mixture of crude methyl 3-methylpiperidine-4-carboxylate **BB16-2** in DCM (15 mL) and water (10 mL), sodium bicarbonate (444 mg, 5.29 mmol) was added. The reaction mixture was stirred at room temperature for 2 hours. The layers were then separated, and the organic phase was washed with saturated brine (10 mL), dried, concentrated, and purified by silica gel column chromatography (PE/EA = 3/1) to afford 1-(tert-butyl)-4-methyl-3-methylpiperidine-1,4-dicarboxylate **BB16-3** (600 mg, 88% yield). MS-ESI [M+H] $^+$: 258.1.

**Step 3:** 4-(Hydroxymethyl)-3-methylpiperidine-1-carboxylic acid tert-butyl ester

To a solution of 1-(tert-butyl)-4-methyl-3-methylpiperidine-1,4-dicarboxylate **BB16-3** (600 mg, 2.33 mmol) in tetrahydrofuran (10 mL), lithium aluminum hydride (124 mg, 3.27 mmol) was added under ice bath condition, and the reaction was stirred at room temperature for 1 hour. The reaction was then quenched with saturated ammonium chloride (5 mL), followed by the addition of ethyl acetate (30 mL) and water (10 mL). The mixture was separated, the organic phase was dried, concentrated, and purified by silica gel column chromatography (PE/ EA = 2/1) to give 4-(hydroxymethyl)-3-methylpiperidine-1-carboxylic acid tert-butyl ester **BB16-4** (500 mg, 94% yield). MS-ESI [M+H] $^+$: 230.1.

**Step 4:** (3-Methylpiperidin-4-yl)methanol hydrochloride

To a solution of tert-butyl 4-(hydroxymethyl)-3-methylpiperidine-1-carboxylate **BB16-4** (500 mg, 2.33 mmol) in dichloromethane (6 mL), a solution of hydrogen chloride in dioxane (4 M, 2.2 mL, 8.8 mmol) was added under ice-bath cooling. The reaction mixture was stirred at room temperature for 3 hours, and then concentrated to give (3-methylpiperidin-4-yl)methanol hydrochloride **BB16** (384 mg). MS-ESI [M+H] +: 130.1.

**Synthesis of intermediate BB17: (4-(Methoxymethyl)piperidin-4-yl)methanol hydrochloride**

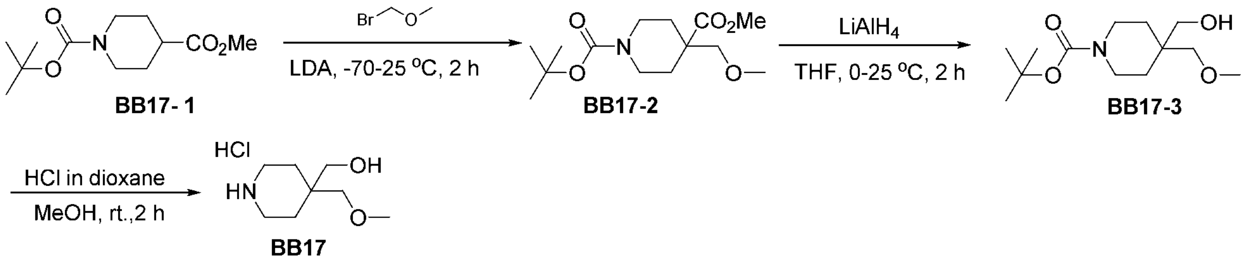


BB17-1 BB17-2 BB17-3 BB17

**Step** 1: 1-(tert-butyl)-4-methyl-4-(methoxymethyl)piperidine-1,4-dicarboxylate

Under a nitrogen atmosphere, to a solution of 1-(tert-butyl)-4-methylpiperidine-1,4-dicarboxylate **BB17-1** (1.0 g, 4.11 mmol) in tetrahydrofuran (15 mL), a solution of lithium diisopropylamide in tetrahydrofuran (1 M, 2.98 mL, 5.96 mmol) was added. Then, bromomethyl methyl ether (770.4 mg, 6.17 mmol) was added dropwise at -70 °C (dry ice-ethanol bath). The mixture was stirred at room temperature for 2 hours. After the reaction was complete, water (5 mL) was added to quench the reaction. The mixture was concentrated, extracted with ethyl acetate (30 mL), washed with saturated brine (10 mL), and concentrated again, then purified by silica gel column chromatography (PE/EA = 7/3) to give a light brown oil **BB17-2** (920 mg). MS-ESI [M-Boc]+: 188.1

**Step 2:** 4-(Hydroxymethyl)-4-(methoxymethyl)piperidine-1-carboxylic acid tert-butyl ester

Under a nitrogen atmosphere, to a solution of 1-(tert-butyl)-4-methyl-4-(methoxymethyl)piperidine-1,4-dicarboxylate **BB17-2** (0.50 g, 1.74 mmol) in tetrahydrofuran (10 mL), lithium aluminum hydride (59.4 mg, 1.57 mmol) was added portionwise at 0 °C (ice bath). The mixture was stirred at room temperature for 2 hours. After the reaction was complete, water (5 mL) was added dropwise at 0 °C to quench the reaction. The mixture was extracted with ethyl acetate (20 mL), dried over anhydrous sodium sulfate, and the filter cake was washed with ethyl acetate (10 mL). The combined filtrates were concentrated to dryness to give crude product **BB17-3** (500 mg), MS-ESI [M-55]+: 204.1.

**Step 3:** (4-(Methoxymethyl)piperidin-4-yl)methanol hydrochloride

To a solution of 1-(tert-butyl)-4-methyl-4-(methoxymethyl)piperidine-1,4-dicarboxylate **BB17**-3 (0.40 g, 1.54 mmol) in methanol (5 mL) was added a solution of hydrogen chloride in dioxane (4 M, 2 mL, 8 mmol). The mixture was stirred at room temperature for 2 hours. After the reaction was complete, the solution was concentrated to dryness to obtain crude product **BB17** (350 mg).

**Synthesis of intermediate BB18: 5-Methyloctahydrocyclopenta[*c*]pyrrole-5-phenol hydrochloride**

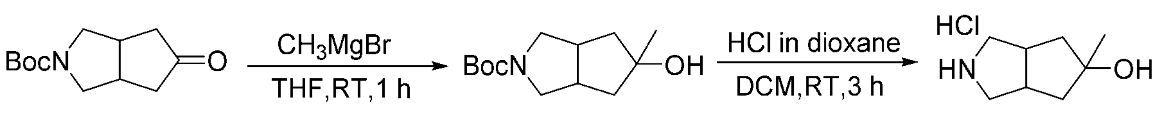


BB18-1 BB18-2 BB18

**Step 1:** 5-Hydroxy-5-methylhexahydrocyclopenta[c]pyrrole-2(IH)-carboxylic acid tert-butyl ester

To a solution of 5-carbonylhexahydrocyclopenta[c]pyrrole-2(1H)-carboxylic acid tert-butyl ester **BB18-1** (400 mg, 2.33 mmol) in tetrahydrofuran (6 mL) was added a tetrahydrofuran solution of methyl magnesium bromide (1 M, 2.3 mL, 2.3 mmol) under ice bath, and the reaction was stirred at room temperature for 1 hour. The reaction was then quenched with a saturated aqueous solution of ammonium chloride (5 mL), followed by the addition of ethyl acetate (30 mL) and water

(10 mL), the mixture was separated. The organic phase was dried, concentrated, and purified by silica gel column chromatography (PE/EA = 2/1) to give 5-hydroxy-5-methylhexahydrocyclopenta[c]pyrrole-2(*1H)*-carboxylic acid tert-butyl ester **BB18-2** (360 mg, 64% yield). MS-ESI [M+H] $^+$: 242.1.

**Step 2: 5-Methyloctahydrocyclopenta[c]pyrrole-5-phenol** hydrochloride

**5-Methyloctahydrocyclopenta[*c*]pyrrole-5-phenol** hydrochloride **BB18** was prepared from 5-hydroxy-5-methylhexahydrocyclopenta[c]pyrrole-2(1H)-carboxylic acid tert-butyl ester **BB18-2** using the synthesis method of Step 4 of intermediate **BB15.** MS-ESI [M+H] $^+$: 142.1.

The following intermediates **BB19** to **BB23** were prepared using the same method as intermediate **BB18.**

| Intermediate | Structural | Raw material | Characterization data |
|---|---|---|---|
| **BB19** | 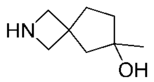 | 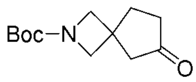 | MS-ESI [M+H]$^+$: 142.1 |
| **BB20** | 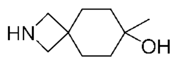 | 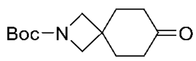 | MS-ESI [M+H]$^+$: 156.1 |
| **BB21** | 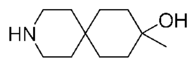 | 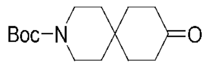 | MS-ESI [M+H]$^+$: 275.4 |
| **BB22** | 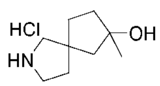 | 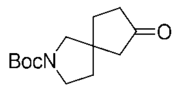 | MS-ESI [M+H]$^+$: 156.2 |
| **BB23** | 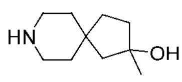 | 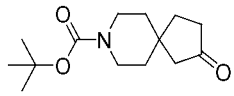 | MS-ESI [M+H]$^+$: 170.1 |

**Synthesis of intermediate BB24: 2-(tert-Butyl)-7-methyl-2-azaspiro[3.5]nonane-2,7-dicarboxylate**

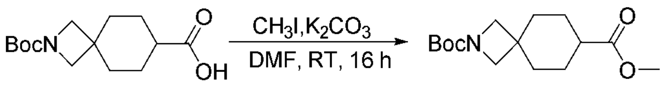


**BB24-1** **BB24**

Potassium carbonate (256 mg, 1.85 mmol) was added to a solution of 2-(tert-butoxycarbonyl)-2-azaspiro[3.5] nonane-7-carboxylic acid **BB24-1** (250 mg, 0.93 mmol) and iodomethane (171 mg, 1.2 mmol) in N,N-dimethylformamide (6 mL), and the reaction was stirred at room temperature for 16 hours. Ethyl acetate (20 mL) and water (10 mL) were added, the mixture was separated, the organic phase was dried, concentrated, and purified by silica gel column chromatography (PE/EA = 3/1) to give 2-(tert-butyl)-7-methyl-2-azaspiro[3.5]nonane-2,7-dicarboxylate **BB24** (260 mg, 99% yield). MS-ESI [M+H-56] $^+$ : 228.1.

Using the same method as intermediate **BB24,** the following intermediate **BB25** was prepared.

| Intermediate | Structure | Raw material | Characterization data |
|---|---|---|---|
| **BB25** | 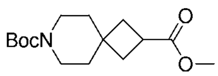 | 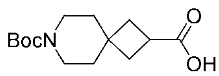 | MS-ESI [M+H] $^+$: 184.1 |

**Synthesis of intermediate BB26: 2-Methyl-1-(piperidin-4-yl)propane-2-ol hydrochloride**

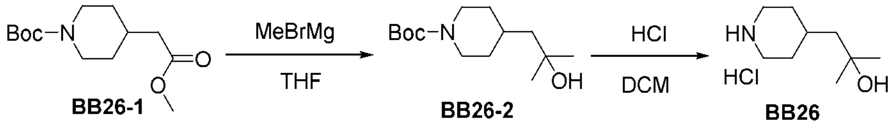


BB26-1 BB26-2 BB26

**Step 1:** 4-(2-Hydroxy-2-methylpropyl)piperidine-1-carboxylic acid tert-butyl ester

To a solution of 4-(2-methoxy-2-carbonylethyl)piperidine-1-carboxylic acid tert-butyl ester **BB26-1** (1000 mg, 3.89 mmol) in tetrahydrofuran (10 mL), methyl magnesium bromide (1 M, 12 mL, 12 mmol) was added under ice bath. The resulting mixture was stirred overnight at room temperature under nitrogen. The reaction was quenched with water (5 mL), and the mixture was extracted three times with dichloromethane (20 mL). The combined organic phases were evaporated to dryness and purified by silica gel column chromatography (PE/EA = 3/1) to give product **BB26-2** (920 mg, 92% yield). MS-ESI [M-55] $^+$: 202.1.

**Step 2:** 2-Methyl-1-(piperidin-4-yl)propane-2-ol hydrochloride

To a solution of tert-butyl 4-(2-hydroxy-2-methylpropyl)piperidine-1-carboxylate **BB26-2** (500 mg, 1.93 mmol) in dichloromethane (10 mL), a solution of dioxane hydrochloride (4 M, 2 mL, 8 mmol) was added, and the mixture was stirred at room temperature for 3 hours. The reaction was quenched with 10 mL of saturated sodium bicarbonate aqueous solution and extracted three times with dichloromethane (30 mL). The organic phases were combined and evaporated to dryness to give crude product **BB26** (300 mg), which was used directly in the next step. MS-ESI [M+H] $^+$: 158.1.

Using the same method as intermediate **BB26,** the following intermediates **BB27** to **B29** were prepared.

| Intermediate | Structure | Raw material | Characterization data |
|---|---|---|---|
| **BB27** | 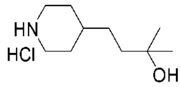 | 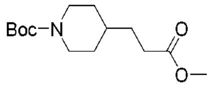 | MS-ESI [M+H] $^+$: 172.1 |
| **BB28** | 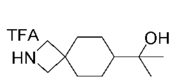 | 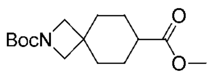 **BB24** | MS-ESI [M+H]$^+$: 184.1 |
| **BB29** | 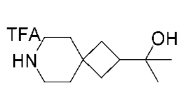 | 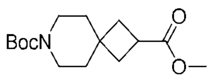 **BB25** | MS-ESI [M+H]$^+$: 184.1 |

**<u>Synthesis of intermediate B1:</u> 4-(Thien-3-yl)aniline**

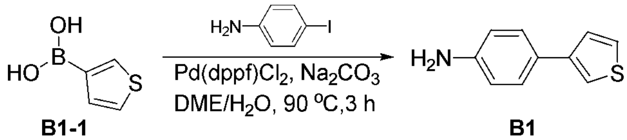


B1-1 B1

To a mixed solution of 3-thiopheneboronic acid **B1-1** (1.75 g, 13.7 mmol) and 4-iodoaniline (2 g, 9.1 mmol) in ethylene glycol dimethyl ether (30 mL) and water (3 mL), [1,1'-bis(diphenylphosphine)ferrocene]palladium dichloride (67 mg, 0.09 mmol) and sodium carbonate (1.93 g, 18.2 mmol) were added. Under nitrogen protection, the reaction was heated to 90 °C and stirred for 3 hours. Then, ethyl acetate (20 mL) and water (10 mL) were added, and the mixture was separated. The organic phase was washed with saturated saline (10 mL) water, dried, concentrated, and purified by silica gel column chromatography (PE/EA = 4/1) to give 4-(thiophene-3-yl)aniline **B1** (1.4 g, 88% yield). MS-ESI [M+H] $^+$: 176.2.

Using the same method as intermediate **B1,** the following intermediates **B2** to **B5** were prepared.

| Intermediate | Structure | Raw material | Characterization data |
|---|---|---|---|
| **B2** | 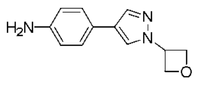 | 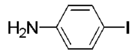 + 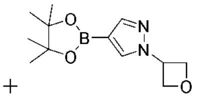 | MS-ESI $[M+H]^+$: 216.2 |
| **B3** | 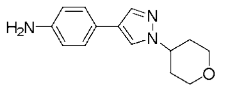 | 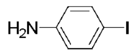 + 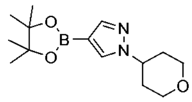 | MS-ESI $[M+H]^+$: 244.3 |
| **B4** | 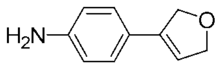 | 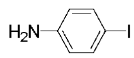 + 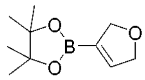 | MS-ESI $[M+H]^+$: 162.2 |
| **B5** | 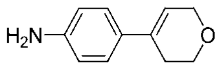 | 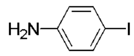 + 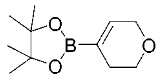 | MS-ESI $[M+H]^+$: 176.2 |

**Synthesis of intermediate B6: (4-(4-Aminophenyl)thiophen-2-yl)methanol**

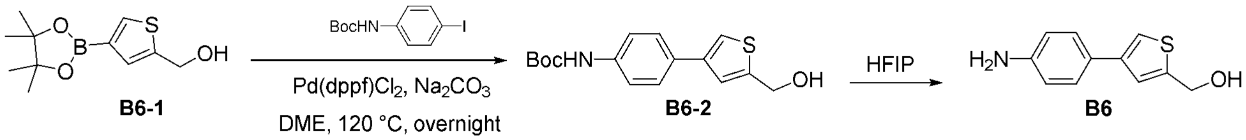

B6-1 B6-2 B6

**Step 1:** tert-Butyl (4-(5-(hydroxymethyl)thiophen-3-yl)phenyl)carbamate

To a solution of (4-(4,4,5,5-tetramethyl-1,3,2-dioxaborolane-2-yl)thiophen-2-yl)methanol **B6-1** (200 mg, 0.83 mmol) and (4-iodophenyl)carbamate tert-butyl ester (319 mg, 1 mmol) in ethylene glycol dimethyl ether/water (2 mL/0.5 mL), 1,1'-bis(diphenylphosphine)ferrocene palladium dichloride (61 mg, 0.083 mmol) and sodium carbonate (265 mg, 2.5 mmol) were added. The resulting mixture was stirred overnight at 120 °C under nitrogen protection. The mixture was evaporated to dryness and purified by silica gel column chromatography (PE/EA = 2/1) to give product **B6-2** (120 mg, yield 47%).

**Step 2:** (4-(4-Aminophenyl)thiophen-2-yl)methanol

(4-(5-(Hydroxymethyl)thiophene-3-yl)phenyl)carbamate tert-butyl ester **B6-2** (100 mg, 0.33 mmol) was added to hexafluoroisopropanol (4 mL), and the resulting mixture was stirred in a microwave oven at 120 °C for 8 hours. The mixture was rotary evaporated to dryness and purified by silica gel column chromatography (PE/EA = 1/2) to give (4-(4-aminophenyl)thiophene-2-yl)methanol **B6** (40 mg, yield 67%). MS-ESI [M+H]: 206.0.

**Synthesis of intermediate B7: 2-(4-(4-Aminophenyl)thiophen-2-yl)acetonitrile**

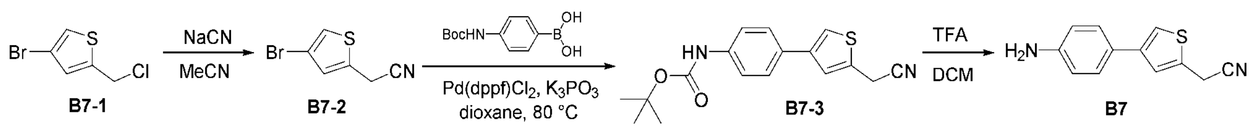

B7-1 B7-2 B7-3 B7

**Step 1:** 2-(4-Bromothiophen-2-yl)acetonitrile

To a solution of 4-bromo-2-(chloromethyl)thiophene **B7-1** (500 mg, 2.38 mmol) in acetonitrile (10 mL), sodium cyanide (210 mg, 4.3 mmol) was added. The resulting mixture was stirred at room temperature for 30 minutes and then stirred overnight at 85 °C. The mixture was rotary evaporated to dryness and purified by silica gel column chromatography

(PE/EA = 4/1) to afford 2-(4-(4-aminophenyl)thiophen-2-yl)acetonitrile **B7-2** (300 mg, 63% yield). MS-ESI [M+H]+: 201.9.

**Step 2:** tert-Butyl (4-(5-(cyanomethyl)thiophen-3-yl)phenyl)carbamate

To a solution of 2-(4-bromothiophene-2-yl)acetonitrile **B7-2** (100 mg, 0.49 mmol) in dioxane/water (2 mL/0.5 mL), 4-(tert-butoxycarbonyl)aminophenylboronic acid (236 mg, 1 mmol), 1,1-bis(diphenylphosphine)ferrocene palladium dichloride (36 mg, 0.05 mmol), and potassium phosphate (317 mg, 1.5 mmol) were added. The resulting mixture was stirred overnight at 90 °C. After rotary evaporation to dryness, the mixture was purified by silica gel column chromatography (PE/EA = 4/1) to give product **B7-3** (107 mg, yield 69%). MS-ESI [M+H] $^+$: 315.

**Step 3:** 2-(4-(4-Aminophenyl)thiophen-2-yl)acetonitrile

To a solution of tert-butyl (4-(5-(cyanomethyl)thiophen-3-yl)phenyl)carbamate **B7-3** (100 mg, 0.32 mmol) in dichloromethane (10 mL), trifluoroacetic acid (2 mL) was added, and the resulting mixture was stirred at room temperature for 3 hours. The reaction was quenched with saturated aqueous sodium bicarbonate solution, and extracted three times with dichloromethane (10 mL). The combined organic phases were rotary evaporated to dryness to give crude product **B7** (107 mg), which was used directly in the next step. MS-ESI [M+H]+: 215.1.

<u>Synthesis of intermediate B8:</u> **4-(2-Ethylmorpholino)aniline**

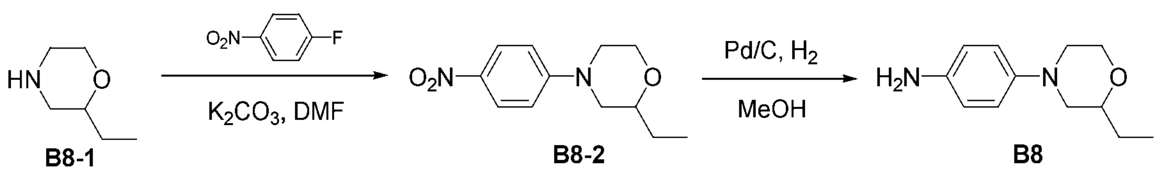


B8-1 B8-2 B8

**Step 1:** 2-Ethyl-4-(4-nitrophenyl) morpholine

To a solution of 2-ethylmorpholine **B8-1** (500 mg, 2.2 mmol) in DMF (5 mL), p-fluoronitrobenzene (613 mg, 4.3 mmol) and potassium carbonate (901 mg, 6.5 mmol) were added. The resulting mixture was stirred at 80 °C for 3 hours. The mixture was then rotary evaporated to dryness and purified by silica gel column chromatography (PE/EA = 5/1) to afford a yellow solid product **B8-2** (500 mg, 49% yield). MS-ESI [M+H]+: 237.1.1.

**Step 2:** 4-(2-Ethylmorpholino)aniline

Palladium on carbon (10%, 50 mg) was added to a solution of 2-ethyl-4-(4-nitrophenyl)morpholine **B8-2** (500 mg, 2.1 mmol) in methanol (5 mL), and the resulting mixture was stirred at room temperature for 3 hours under hydrogen atmosphere. The reaction solution was filtered through diatomaceous earth, and the filtrate was evaporated to dryness to give crude product **B8** (300 mg), which was used directly in the next reaction. MS-ESI [M+H] $^+$: 207.1.

Using the same method as intermediate **B8,** the following intermediates **B9** to **B74** were prepared.

| Intermediate | Structure | Raw material | Characterization data |
|---|---|---|---|
| **B9** | 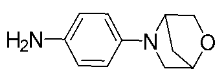 | 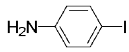 + 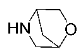 | MS-ESI [M+H] $^+$: 191.1 |
| **B10** | 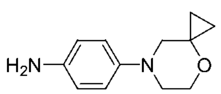 | 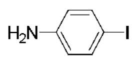 + 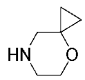 | MS-ESI [M+H] $^+$: 205.1 |
| **B11** | 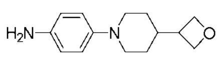 | 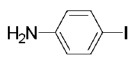 + 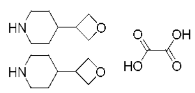 | MS-ESI [M+H] $^+$: 233.3 |
| **B12** | 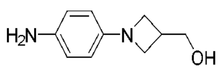 | 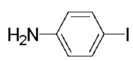 + 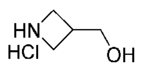 | MS-ESI [M+H] $^+$: 179.1 |

(continued)

| Intermediate | Structure | Raw material | Characterization data |
|---|---|---|---|
| **B13** | 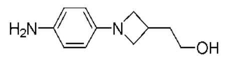 | 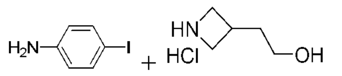 | MS-ESI $[M+H]^+$: 193.1 |
| **B14** | 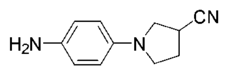 | 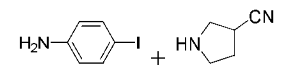 | MS-ESI $[M+H]^+$: 188.2 |
| **B15** | 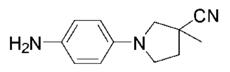 | 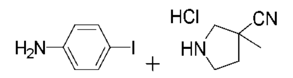 | MS-ESI $[M+H]^+$: 202.1 |
| **B16** | 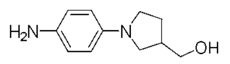 | 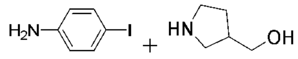 | $^1H$ NMR (400 MHz, DMSO-$d_6$)δ 6.55-6.43 (m, 2H), 6.33 (s, 21H), 4.65 (d, $J$ = 5.2 Hz, 1H), 4.27 (s, 2H), 3.41-3.35 (m, 2H), 3.20-3.04 (m, 4H), 2.42-2.31 (m, 1H), 2.01-1.92 (m, 1H), 1.70-1.60 (m, 1H). MS-ESI $[M+H]^+$: 193.0 |
| **B17** | 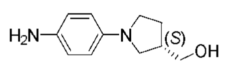 | 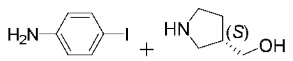 | MS-ESI $[M+H]^+$: 193.0 |
| **B18** | 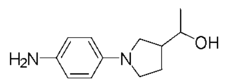 | 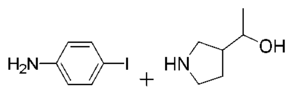 | MS-ESI $[M+H]^+$: 207.1 |
| **B19** | 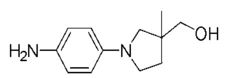 | 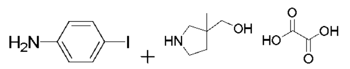 | MS-ESI $[M+H]^+$: 207.1 |
| **B20** | 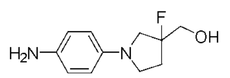 | 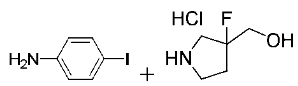 | MS-ESI $[M+H]^+$: 211.1 |
| **B21** | 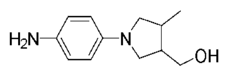 | 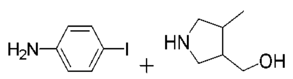 | MS-ESI $[M+H]^+$: 207.1 |
| **B22** | 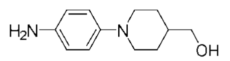 | 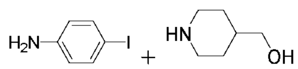 | MS-ESI $[M+H]^+$: 207.2 |
| **B23** | 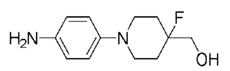 | 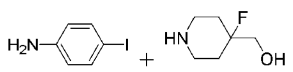 | MS-ESI $[M+H]^+$: 225.1 |
| **B24** | 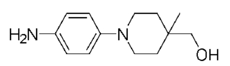 | 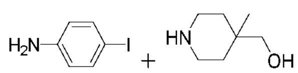 | MS-ESI $[M+H]^+$: 221.1 |
| **B25** | 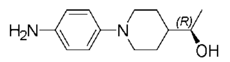 | 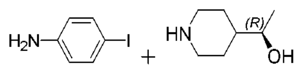 | MS-ESI $[M+H]^+$: 221.1 |
| **B26** | 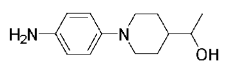 | 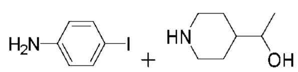 | MS-ESI $[M+H]^+$: 221.2 |
| **B27** | 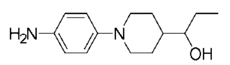 | 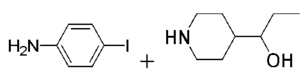 | MS-ESI$[M+H]^+$: 235.1 |
| **B28** | 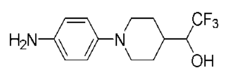 | 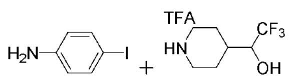 | MS-ESI $[M+H]^+$: 275.1 |
| **B29** | 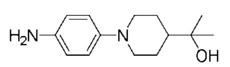 | 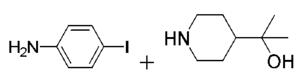 | MS-ESI $[M+H]^+$: 235.1 |

(continued)

| Intermediate | Structure | Raw material | Characterization data |
|---|---|---|---|
| **B30** | 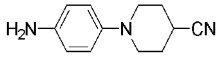 | 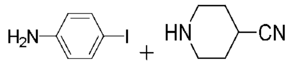 + | MS-ESI [M+H]$^+$: 202.1 |
| **B31** | 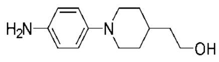 | 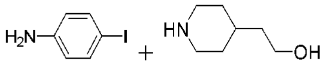 + | MS-ESI [M+H]$^+$: 221.1 |
| **B32** | 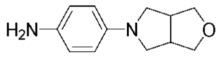 | 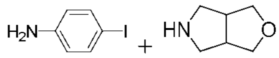 + | MS-ESI [M+H]$^+$: 205.2 |
| **B33** | 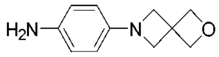 | 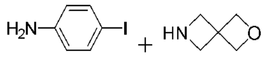 + | MS-ESI [M+H]$^+$: 191.1 |
| **B34** | 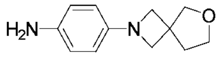 | 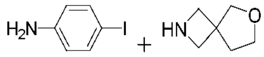 + | MS-ESI [M+H]$^+$: 205.2 |
| **B35** | 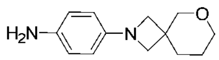 | 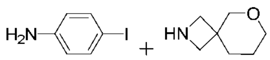 + | MS-ESI [M+H]$^+$: 219.3 |
| **B36** | 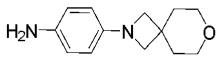 | 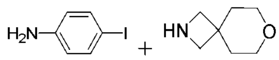 + | MS-ESI [M+H]$^+$: 219.1 |
| **B37** | 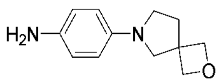 | 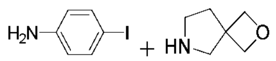 + | MS-ESI [M+H]$^+$: 205.2 |
| **B38** | 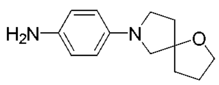 | 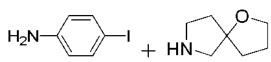 + | MS-ESI [M+H]$^+$: 219.2 |
| **B39** | 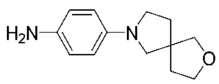 | 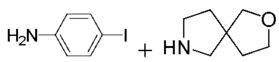 + | MS-ESI [M+H]$^+$: 219.2 |
| **B40** | 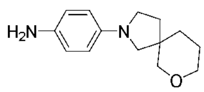 | 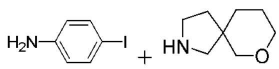 + | MS-ESI [M+H]$^+$: 233.1 |
| **B41** | 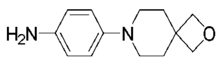 | 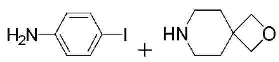 + | MS-ESI [M+H]$^+$: 219.1 |
| **B42** | 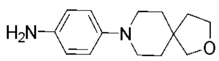 | 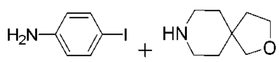 + | MS-ESI [M+H]$^+$: 233.2 |
| **B43** | 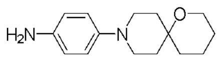 | 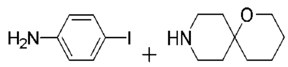 + | MS-ESI [M+H]$^+$: 247.3 |
| **B44** | 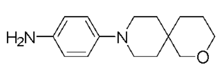 | 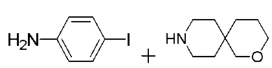 + | MS-ESI [M+H]$^+$: 247.2 |
| **B45** | 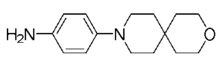 | 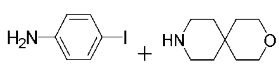 + | MS-ESI [M+H]$^+$: 247.1 |
| **B46** | 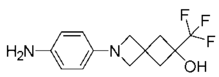 | 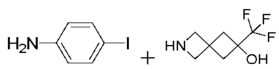 + | MS-ESI [M+H]$^+$: 273.1 |
| **B47** | 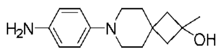 | 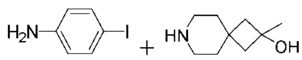 + | MS-ESI [M+H]$^+$: 247.2 |
| **B48** | 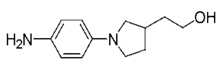 | 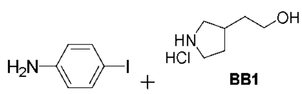 + BB1 | MS-ESI [M+H]$^+$: 207.1 |

(continued)

| Intermediate | Structure | Raw material | Characterization data |
|---|---|---|---|
| **B49** | 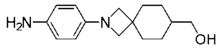 | 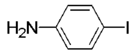 + 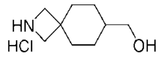 **BB7** | MS-ESI $[M+H]^+$: 247.1 |
| **B50** | 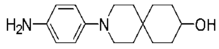 | 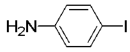 + 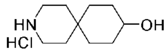 **BB13** | $^1$H NMR (400 MHz, DMSO-$d_6$) δ 6.68 (d, $J$ = 8.0 Hz, 2H), 6.47 (d, $J$ = 8.0 Hz, 2H), 4.65 (s, 2H), 4.42 (d, $J$ = 4.4 Hz, 1H), 3.47-3.37 (m, 1H), 2.93-2.75 (m, 4H), 1.62-1.50 (m, 6H), 1.45-1.38 (m, 2H), 1.35-1.25 (m, 2H), 1.15-1.05 (m, 2H). MS-ESI $[M+H]^+$: 261.2 |
| **B51** | 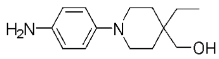 | 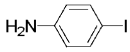 + 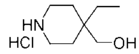 **BB2** | $^1$H NMR (400 MHz, DMSO-$d_6$) δ 6.67 (d, $J$ = 8.4 Hz, 2H), 6.47 (d, $J$ = 8.4 Hz, 2H), 4.55 (s, 2H), 4.37 (d, $J$ = 5.2 Hz, 1H), 3.25 (d, $J$ = 5.2 Hz, 2H), 2.93-2.77 (m, 4H), 1.54-1.45 (m, 2H), 1.42-1.31 (m, 4H), 0.77 (d, $J$ = 7.6 Hz, 3H). MS-ESI $[M+H]^+$: 235.2 |
| **B52** | 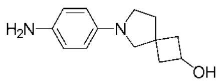 | 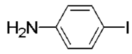 + 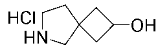 **BB8** | MS-ESI $[M+H]^+$: 219.1 |
| **B53** | 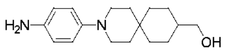 | 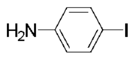 + 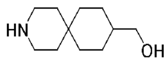 **BB14** | MS-ESI $[M+H]^+$: 275.1 |
| **B54** | 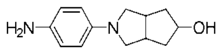 | 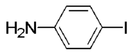 + 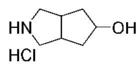 **BB3** | MS-ESI $[M+H]^+$: 219.1 |
| **B55** | 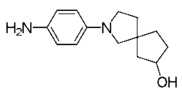 | 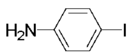 + 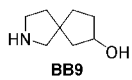 **BB9** | MS-ESI $[M+H]^+$: 233.1 |
| **B56** | 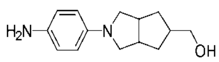 | 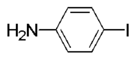 + 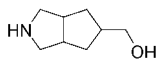 **BB4** | MS-ESI $[M+H]^+$: 233.1 |
| **B57** | 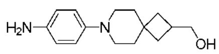 | 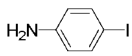 + 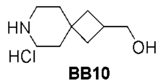 **BB10** | MS-ESI $[M+H]^+$: 247.1 |
| **B58** | 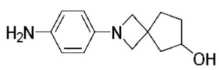 | 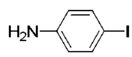 + 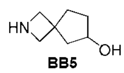 **BB5** | MS-ESI $[M+H]^+$: 219.1 |
| **B59** | 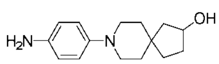 | 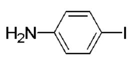 + 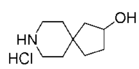 **BB11** | MS-ESI $[M+H]^+$: 247.3 |
| **B60** | 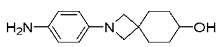 | 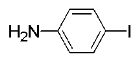 + 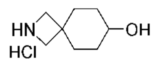 **BB6** | $^1$H NMR (400 MHz, DMSO-$d_6$) δ 6.46 (d, $J$ = 8.4 Hz, 2H), 6.19 (d, $J$ = 8.4 Hz, 2H), 4.47 (d, $J$ = 4.0 Hz, 1H), 4.34 (s, 2H), 3.47-3.38 (m, 1H), 3.35 (s, 2H), 3.31 (s, 2H), 1.86-1.77 (m, 2H), 1.70-1.61 (m, 2H), 1.50-1.40 (m, 2H), 1.28-1.17 (m, 2H). MS-ESI $[M+H]^+$: 233.1 |

(continued)

| Intermediate | Structure | Raw material | Characterization data |
|---|---|---|---|
| **B61** | 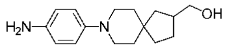 | 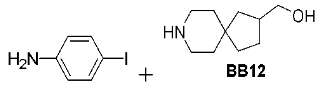 + BB12 | MS-ESI [M+H]$^+$: 261.2 |
| **B62** | 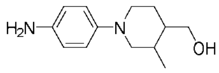 | 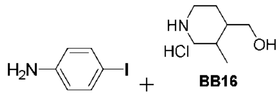 + BB16 | MS-ESI [M+H]$^+$: 221.1 |
| **B63** | 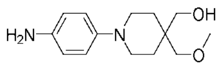 | 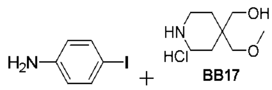 + BB17 | MS-ESI [M+H]$^+$: 251.1 |
| **B64** | 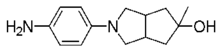 | 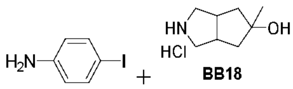 + BB18 | MS-ESI [M+H]$^+$: 233.2 |
| **B65** | 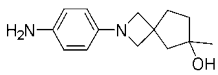 | 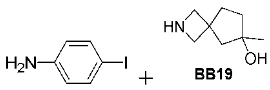 + BB19 | MS-ESI [M+H]$^+$: 233.2 |
| **B66** | 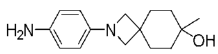 | 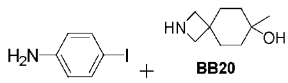 + BB20 | MS-ESI [M+H]$^+$: 247.2 |
| **B67** | 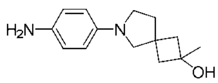 | 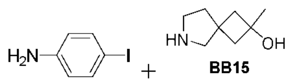 + BB15 | MS-ESI [M+H]$^+$: 233.1 |
| **B68** | 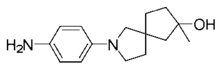 | 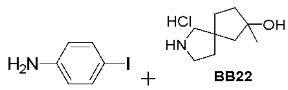 + BB22 | MS-ESI [M+H]$^+$: 247.2 |
| **B69** | 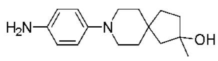 | 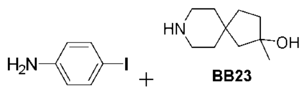 + BB23 | MS-ESI [M+H]$^+$: 261.2 |
| **B70** | 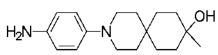 | 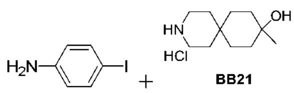 + BB21 | MS-ESI [M+H]$^+$: 275.4 |
| **B71** | 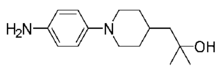 | 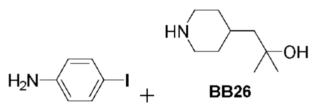 + BB26 | MS-ESI [M+H]$^+$: 249.2 |
| **B72** | 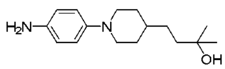 | 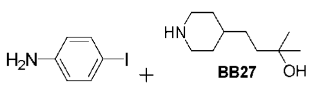 + BB27 | MS-ESI [M+H]$^+$: 263.2 |
| **B73** | 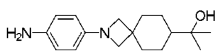 | 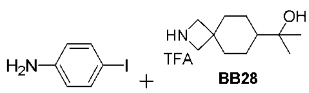 + BB28 | MS-ESI [M+H]$^+$: 275.1 |
| **B74** | 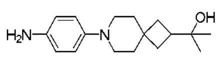 | 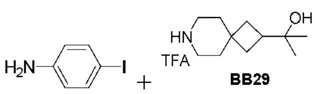 + BB29 | MS-ESI [M+H]$^+$: 275.1 |

**<u>Synthesis of intermediate B75:</u> (1-(4-Aminophenyl)-4,4-dimethylpyrrolidine-3-yl)methanol**

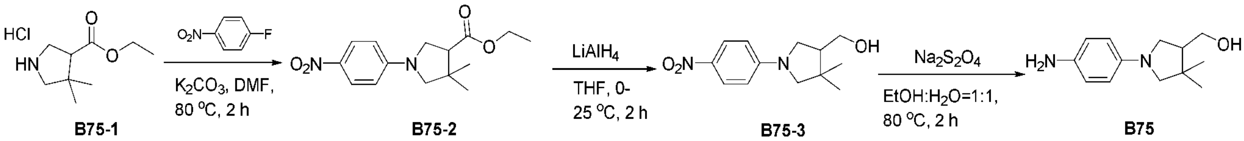


B75-1 B75-2 B75-3 B75

**Step 1:** 4,4-Dimethyl-1-(4-nitrophenyl)pyrrolidine-3-carboxylic acid ethyl ester

To a solution of 4,4-dimethylpyrrolidine-3-carboxylic acid ethyl ester hydrochloride **B75-1** (250 mg, 1.20 mmol) and potassium carbonate (582.2 g, 4.21 mmol) in DMF (10 mL), 1-fluoro-4-nitrobenzene (424.6 mg, 3.01 mmol) was added. The resulting mixture was heated to 80 °C and stirred for 2 hours, then cooled, filtered, and ethyl acetate (50 mL) was added to the filtrate. The mixture was washed with lithium chloride aqueous solution (10 mL), concentrated, and purified by silica gel column chromatography (PE/EA = 3/2) to give a pale yellow solid **B75-2** (290 mg, yield 82%). MS-ESI [M+H] $^+$: 293.2.

**Step 2:** (4,4-Dimethyl-1-(4-nitrophenyl)pyrrolidine-3-yl)methanol

To a solution of 4,4-dimethyl-1-(4-nitrophenyl)pyrrolidine-3-carboxylic acid ethyl ester **B75-2** (290 mg, 0.992 mmol) in THF (20 mL), lithium aluminum hydride (49.0 mg, 1.29 mmol) was added in portions under ice-water bath condition. The mixture was then allowed to war to room temperature under nitrogen protection and stirred for 2 hours. After the reaction was complete, the temperature was lowered to 0 °C, and the reaction was quenched by slow dropwise addition of water (10 mL), extracted with ethyl acetate (30 mL), dried over anhydrous sodium sulfate, filtered, concentrated to dryness, and purified by silica gel column chromatography (DCM/MeOH = 10/1) to give a brown oily substance **B75-3** (190 mg, 77% yield). MS-ESI [M+H] $^+$: 251.1.

**Step 3:** (1-(4-Aminophenyl)-4,4-dimethylpyrrolidine-3-yl)methanol

To a solution of (4,4-dimethyl-1-(4-nitrophenyl)pyrrolidine-3-yl)methanol **B75-3** (100 mg, 0.454 mmol) in EtOH/$H_2O$ = 1/1 (15 mL), sodium dithionite (632.6 mg, 3.63 mmol) was added. The mixture was heated to 80 °C and stirred for 2 hours. After the reaction was complete, the mixture was cooled to room temperature and extracted with a mixed solvent of DCM/MeOH = 10/1 (30 mL), washed with brine (5 mL), dried, concentrated, and purified by silica gel column chromatography (DCM/MeOH = 10/1) to give a pale yellow solid B75 (45 mg, yield 88%). MS-ESI [M+H] : 191.1.

<u>Synthesis of intermediate B76:</u> **2-((4-(2-oxa-8-azaspiro[4.5]decane-8-yl)phenyl)amino)-2-carbonyl acetic acid**

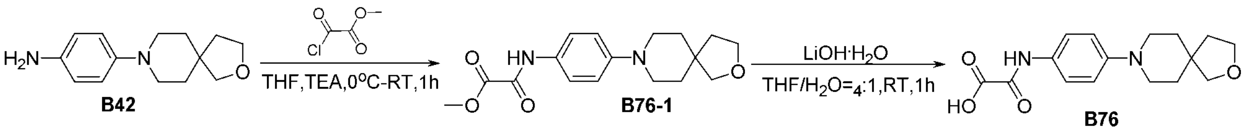


B42 B76-1 B76

**Step 1:** 2-((4-(2-Oxa-8-azaspiro[4.5]decane-8-yl)phenyl)amino)-2-carbonylacetic acid methyl ester

To a solution of 4-(2-oxa-8-azaspiro[4.5]decane-8-yl)aniline **B42** (3 g, 12.9 mmol) in tetrahydrofuran (50 mL), triethylamine (2.6 g, 25.8 mmol) was added, followed by dropwise addition of monomethyl oxaloyl chloride (2.37 g, 19.35 mmol) under ice bath condition. The mixture was stirred at room temperature for 1 hour, quenched with saturated ammonium chloride aqueous solution (10 mL), and extracted with ethyl acetate (60 mL). The organic phase was dried, filtered, and concentrated to give product **B76-1** (3.9 g). MS-ESI [M+H] $^+$: 319.3.

**Step 2:** 2-((4-(2-Oxa-8-azaspiro[4.5]decane-8-yl)phenyl)amino)-2-carbonylacetic acid

To a solution of 2-((4-(2-oxa-8-azaspiro[4.5]decane-8-yl)phenyl)amino)-2-carbonylacetic acid methyl ester **B76-1** (3.9 g, 12.2 mmol) in tetrahydrofuran (50 mL), an aqueous solution of lithium hydroxide monohydrate (12.5 mL, 1.02 g, 24.4 mmol) was added, and the reaction was stirred at room temperature for 1 hour. Then, 1 N hydrochloric acid was added to adjust the pH of the reaction solution to 3-4. The solid precipitated and was extracted twice with ethyl acetate (150 mL each time). The organic phases were combined, dried over anhydrous sodium sulfate, and concentrated to give a pale yellow solid **B76** (3.7 g, 99% yield). MS-ESI [M+H] $^+$: 305.3.

Using the same method as intermediate **B76,** the following intermediates **B77 and B78 were** prepared.

| Intermediate | Structure | Raw material | Characterization data |
|---|---|---|---|
| **B77** | 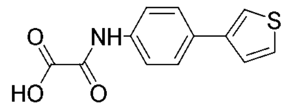 | 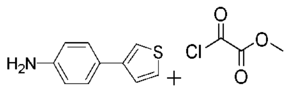 | MS-ESI [M+ H] $^+$: 248.3 |
| **B 78** | 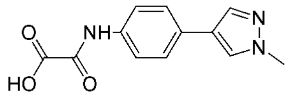 | 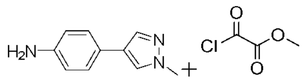 | MS-ESI [M+ H] $^+$: 246.3 |

## 2. Synthesis of the Examples

Method **C:**

To a solution of intermediate **A** (0.05 mmol, 1 eq) and intermediate **B** (1.5 eq) in *N,N*-dimethylformamide (2 mL), a solution of 1-propylphosphonic anhydride in *N,N*-dimethylformamide (50%, 2.0 eq) and *N,N*-diisopropylethylamine (3.0 eq) were added. The mixture was stirred at room temperature for 3 hours, and the target product was obtained by preparative HPLC separation and purification.

Method **D:**

To a solution of intermediate **B** (0.05 mmol, 1 eq) in dichloromethane (3 mL), oxalyl chloride (4.0 eq) was added dropwise under ice bath condition, followed by 1 drop of DMF. The reaction was stirred at room temperature for 0.5 hours, and concentrated to obtain the crude product. The crude product was then dissolved in *N,N*-dimethylformamide (3 mL), followed by the addition of intermediate **A1** (1.0 eq) and N,N-diisopropylethylamine (2.0 eq). The reaction was stirred at room temperature for 0.5 hours. The target product was obtained by preparative HPLC separation and purification.

The compounds described in the examples were prepared using the above synthetic method and corresponding intermediates:

| Ex. | Structure | Syn. method | Raw material 1 | Characterization data |
|---|---|---|---|---|
| 1 | 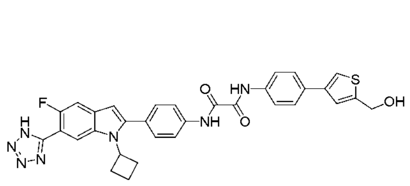 | C | 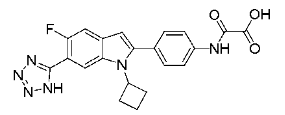 A10 + 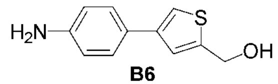 B6 | 1H NMR (400 MHz, DMSO-d6) δ 11.09 (s, 1H), 10.98 (s, 1H), 8.31 (d, J = 5.8 Hz, 1H), 8.06 (d, J = 8.7 Hz, 2H), 7.94 (d, J = 8.8 Hz, 2H), 7.76-7.68 (m, 3H), 7.55 (dd, J = 9.8, 6.5 Hz, 3H), 7.40 (s, 1H), 6.55 (s, 1H), 5.53 (s, 1H), 5.15-5.02 (m, 1H), 4.67 (s, 2H), 2.78-2.68 (m, 2H), 2.44-2.34 (m, 2H), 1.93-1.74 (m, 2H). 19F NMR (376 MHz, DMSO-d6) δ -123.90. MS-ESI [M+H]+: 608.2 |
| 2 | 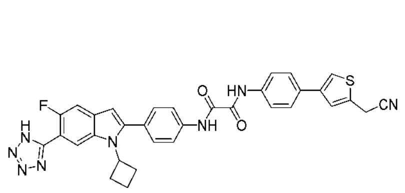 | C | 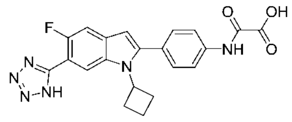 A10 + 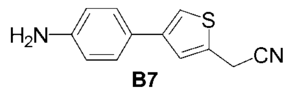 B7 | 1H NMR (400 MHz, DMSO-d6) δ 11.09 (s, 1H), 10.99 (s, 1H), 8.32 (d, J = 5.8 Hz, 1H), 8.06 (d, J = 8.7 Hz, 2H), 7.95 (d, J = 8.8 Hz, 2H), 7.82 (d, J = 1.6 Hz, 1H), 7.73 (d, J = 8.7 Hz, 2H), 7.61-7.54 (m, 3H), 7.51 (s, 1H), 6.56 (s, 1H), 5.14-5.05 (m, 1H), 4.33 (s, 2H), 2.77-2.66 (m, 2H), 2.44-2.36 (m, 2H), 1.93-1.76 (m, 2H). 19F NMR (376 MHz, DMSO-d6) δ -124.10. MS-ESI [M+H]+: 617.2 |
| 3 | 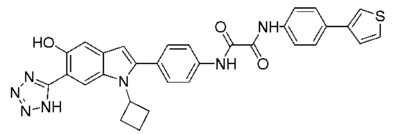 | D | 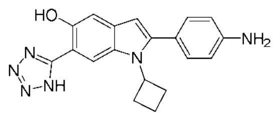 A9 + 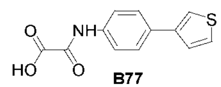 B77 | 1H NMR (400 MHz, DMSO-d6) δ 11.08 (s, 1H), 10.98 (s, 1H), 8.54-8.37 (m, 1H), 8.05 (d, J = 8.4 Hz, 2H), 7.95 (d, J = 8.0 Hz, 2H), 7.87 (d, J = 2.8 Hz, 1H), 7.77 (d, J = 8.4 Hz, 2H), 7.65 (d, J = 4.0 Hz, 1H), 7.59 (d, J = 5.2 Hz, 1H), 7.53 (d, J = 8.4 Hz, 2H), 6.80 (s, 1H), 6.42 (s, 1H), 5.43 (s, 1H), 5.09-4.99 (m, 1H), 2.76-2.72 (m, 2H), 2.41-2.32 (m, 2H), 1.96-1.74 (m, 2H). MS-ESI [M+H]+: 576.0 |
| 4 | 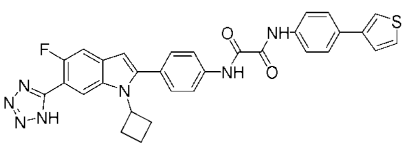 | D | 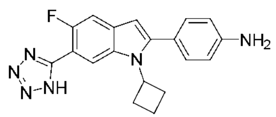 A1 + 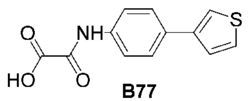 B77 | 1H NMR (400 MHz, DMSO-d6) δ 11.11 (s, 1H), 10.99 (s, 1H), 8.33 (d, J = 5.8 Hz, 1H), 8.06 (d, J = 8.6 Hz, 2H), 7.96 (d, J = 8.7 Hz, 2H), 7.88 (d, J = 2.9 Hz, 1H), 7.77 (d, J = 8.6 Hz, 2H), 7.65 (dd, J = 5.0, 2.9 Hz, 1H), 7.58 (dd, J = 11.9, 6.9 Hz, 4H), 6.58 (s, 1H), 5.40 (s, 1H), 5.15-5.06 (m, 1H), 2.76-2.67 (m, 2H), 2.45-2.38 (m, 2H), 1.92-1.77 (m, 2H). MS-ESI [M+H]+: 578.0 |

(continued)

| Ex. | Structure | Syn. method | Raw material 1 | Characterization data |
|---|---|---|---|---|
| 5 | 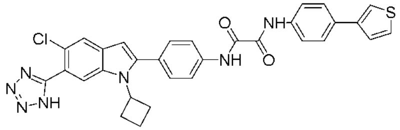 | D | 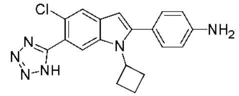 A2 + 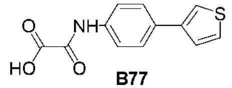 B77 | 1H NMR (400 MHz, DMSO-d6) δ 11.10 (s, 1H), 10.98 (s, 1H), 8.15-8.00 (m, 3H), 7.99-7.91 (m, 2H), 7.90-7.83 (m, 2H), 7.76 (d, J = 8.4 Hz, 2H), 7.65 (dd, J = 5.1, 2.9 Hz, 1H), 7.61-7.52 (m, 3H), 6.58 (s, 1H), 5.11-5.02 m, 1H), 2.76-2.65 (m, 2H), 2.39-2.30 (m, 2H), 1.91-1.72 (m, 2H). MS-ESI [M+H]+: 594.7 |
| 6 | 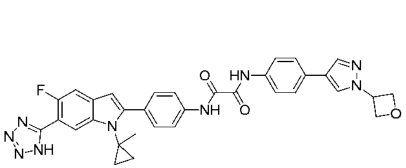 | C | 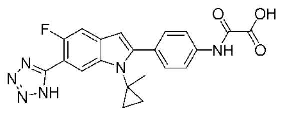 A12 + 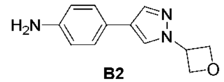 B2 | 1H NMR (400 MHz, DMSO-d6) δ 11.07 (s, 1H), 10.91 (s, 1H), 8.36 (s, 1H), 8.24 (d, J = 5.9 Hz, 1H), 8.07-8.04 (m, 3H), 7.90 (d, J = 8.7 Hz, 2H), 7.69 (d, J = 8.7 Hz, 2H), 7.64 (d, J = 8.7 Hz, 2H), 7.55 (d, J = 11.2 Hz, 1H), 6.59 (s, 1H), 5.63-5.56 (m, 1H), 4.97-4.91 (m, 4H), 1.83 (s, 3H), 1.19-1.11 (m, 1H), 0.95-0.82 (m, 2H), 0.73-0.63 (m, 1H). MS-ESI [M+H]+: 618.7 |
| 7 | 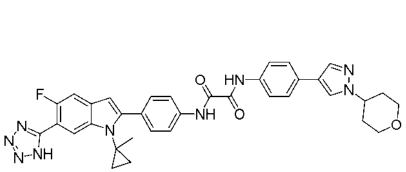 | C | 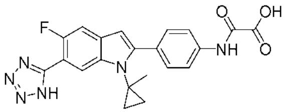 A12 + 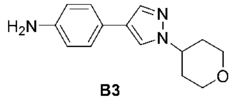 B3 | 1H NMR (400 MHz, DMSO-d6) δ 11.07 (s, 1H), 10.89 (s, 1H), 8.26-8.24 (m, 2H), 8.06 (d, J = 8.8 Hz, 2H), 7.89 (d, J = 8.5 Hz, 3H), 7.69 (d, J = 8.7 Hz, 2H), 7.63-7.56 (m, 3H), 6.60 (s, 1H), 4.45-4.37 (m, 1H), 4.0-3.96 (m, 2H), 3.52-3.45 (m, 2H), 2.02-1.95 (m, 4H), 1.83 (s, 3H), 1.19-1.12 (m, 1H), 0.96-0.83 (m, 2H), 0.72-0.63 (m, 1H). MS-ESI [M+H]+: 646.7 |
| 8 | 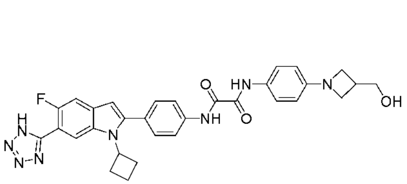 | C | 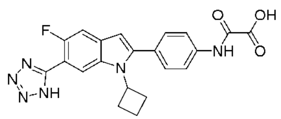 A10 + 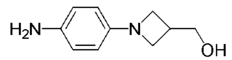 B12 | 1H NMR (400 MHz, DMSO-d6) δ 11.01 (s, 1H), 10.63 (s, 1H), 8.34 (d, J = 5.8 Hz, 1H), 8.05 (d, J = 8.7 Hz, 2H), 7.68 (d, J = 8.9 Hz, 2H), 7.61 (d, J = 11.3 Hz, 1H), 7.55 (d, J = 8.7 Hz, 2H), 6.58 (s, 1H), 6.42 (d, J = 9.0 Hz, 2H), 5.15-5.04 (m, 1H), 4.76 (s, 1H), 3.82 (t, J = 7.5 Hz, 2H), 3.60-3.52 (m, 4H), 2.80-2.67 (m, 3H), 2.45-2.36 (m, 2H), 1.94-1.75 (m, 2H). 19F NMR (376 MHz, DMSO-d6) δ -124.35. MS-ESI [M+H]+: 581.3 |

(continued)

| Ex. | Structure | Syn. method | Raw material 1 | Characterization data |
|---|---|---|---|---|
| 9 | 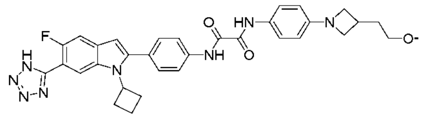 | C | 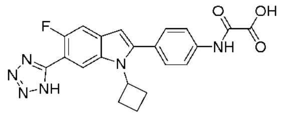 A10 + 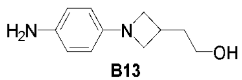 B13 | 1H NMR (400 MHz, DMSO-d6) δ 10.99 (s, 1H), 10.64 (s, 1H), 8.28 (d, J = 5.9 Hz, 1H), 8.03 (d, J = 8.7 Hz, 2H), 7.68 (d, J = 8.9 Hz, 2H), 7.53 (d, J = 8.7 Hz, 2H), 7.44 (d, J = 11.1 Hz, 1H), 6.49 (s, 1H), 6.42 (d, J = 9.0 Hz, 2H), 5.12-5.00 (m, 1H), 4.47 (s, 1H), 3.92 (t, J = 7.4 Hz, 2H), 3.44 (dt, J = 6.9, 3.5 Hz, 4H), 2.82-2.70 (m, 3H), 2.44-2.34 (m, 2H), 1.91-1.72 (m, 4H). 19F NMR (376 MHz, DMSO-d6) δ -123.39. MS-ESI [M+H]+: 595.3 |
| 10 | 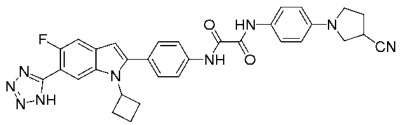 | C | 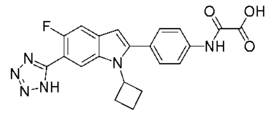 A10 + 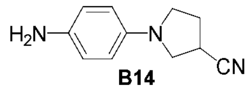 B14 | 1H NMR (400 MHz, DMSO-d6) δ 11.00 (s, 1H), 10.65 (s, 1H), 8.31 (d, J = 5.6 Hz, 1H), 8.04 (d, J = 8.4 Hz, 2H), 7.73 (d, J = 8.8 Hz, 2H), 7.55-7.51 (m, 3H), 6.32 (d, J = 8.8 Hz, 2H), 6.54 (s, 1H), 5.10-5.04 (m, 1H), 3.60-3.55 (m, 2H), 3.48-3.52 (m, 2H), 3.29-3.28 (m, 1H), 2.78-2.66 (m, 2H), 2.43-3.32 (m, 3H), 2.28-2.22 (m, 1H), 1.95-1.75 (m, 2H). MS-ESI [M+H]+: 690.3 |
| 11 | 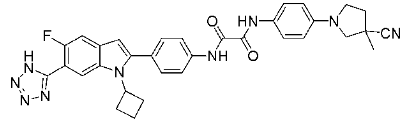 | C | 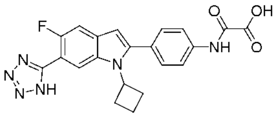 A10 + 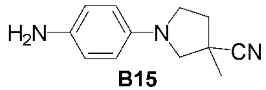 B15 | 1H NMR (400 MHz, DMSO-d6) δ 11.01 (s, 1H), 10.66 (s, 1H), 8.34 (d, J = 5.8 Hz, 1H), 8.05 (d, J = 8.8 Hz, 2H), 7.73 (d, J = 9.0 Hz, 2H), 7.62 (d, J = 11.2 Hz, 1H), 7.55 (d, J = 8.6 Hz, 2H), 6.65-6.54 (m, 3H), 5.14-5.05 (m, 1H), 3.74 (d, J = 9.9 Hz, 1H), 3.48-3.40 (m, 2H), 3.25 (d, J = 9.9 Hz, 1H), 2.77-2.66 (m, 2H), 2.47-2.36 (m, 3H), 2.17-2.08 (m, 1H), 1.94-1.76 (m, 2H), 1.52 (s, 3H). 19F NMR (376 MHz, DMSO-d6) δ -124.42. MS-ESI [M+H]+: 604.3 |
| 12 | 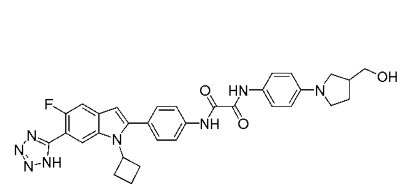 | C | 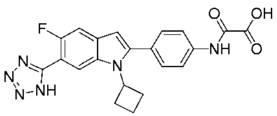 A10 + 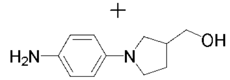 B16 | 1H NMR (400 MHz, DMSO-d6) δ 10.99 (s, 1H), 10.60 (s, 1H), 8.32 (d, J = 6.0 Hz, 1H), 8.03 (d, J = 8.4 Hz, 2H), 7.66 (d, J = 8.8 Hz, 2H), 7.61-7.47 (m, 3H), 6.61-6.54 (m, 1H), 6.51 (d, J = 8.8 Hz, 2H), 5.45 (s, 1H), 5.14-5.00 (m, 1H), 3.46-3.44 (m, 2H), 3.18-3.16 (m, 2H), 3.01 (s, 1H), 2.75-2.66 (m, 2H), 2.44-2.33 (m, 2H), 1.92-1.74 (m, 2H), 1.74-1.66 (m, 2H). MS-ESI [M+H]+: 595.3 |

(continued)

| Ex. | Structure | Syn. method | Raw material 1 | Characterization data |
|---|---|---|---|---|
| 13 | 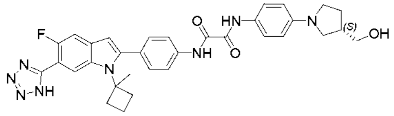 | C | 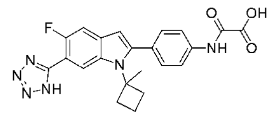 A13 + 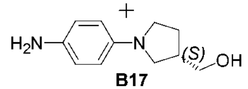 B17 | 1H NMR (400 MHz, DMSO-d6) δ 10.96 (s, 1H), 10.57 (s, 1H), 7.99 (d, J = 8.8 Hz, 2H), 7.96 (d, J = 6.0 Hz, 1H), 7.68 (d, J = 9.2 Hz, 2H), 7.58 (d, J = 11.2 Hz, 1H), 7.52 (d, J = 8.8 Hz, 2H), 6.53 (d, J = 9.2 Hz, 2H), 6.48 (s, 1H), 4.71 (s, 1H), 3.47-3.38 (m, 3H), 3.25-3.18 (m, 2H), 3.04-2.99 (m, 1H), 2.45-2.39 (m, 1H), 2.28-2.19 (m, 2H), 2.17-2.08 (m, 2H), 2.07-2.00 (m, 1H), 1.98 (s, 3H), 1.91-1.83 (m, 1H), 1.79-1.71 (m, 1H), 1.68-1.60 (m, 1H), MS-ESI [M+H]+: 609.7 |
| 14 | 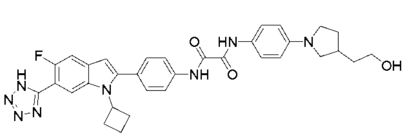 | C | 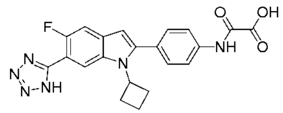 A10 + 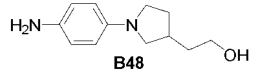 B48 | 1H NMR (400 MHz, DMSO-d6) δ 10.98 (s, 1H), 10.59 (s, 1H), 8.31 (d, J = 5.8 Hz, 1H), 8.04 (d, J = 8.6 Hz, 2H), 7.69 (d, J = 8.9 Hz, 2H), 7.63-7.39 (m, 3H), 6.65-6.44 (m, 3H), 5.13-5.03 (m, 1H), 3.49 (t, J = 6.6 Hz, 2H), 3.45-3.36 (m, 2H), 3.32-3.27 (m, 1H), 3.24-3.19 (m, 1H), 2.89-2.83 (m, 1H), 2.78-2.68 (m, 2H), 2.41-2.33 (m, 2H), 2.17-2.07 (m, 1H), 1.95-1.75 (m, 2H), 1.70-1.49 (m, 3H). 19F NMR (376 MHz, DMSO-d6) δ -123.80. MS-ESI [M+H]+: 609.3 |
| 15 | 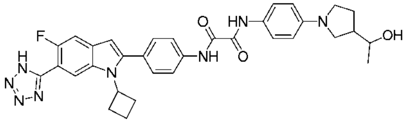 | C | 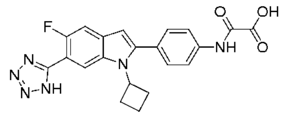 A10 + 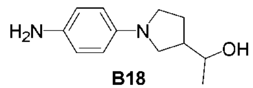 B18 | 1H NMR (400 MHz, DMSO-d6) δ 10.99 (s, 1H), 10.58 (s, 1H), 8.33 (d, J = 5.8 Hz, 1H), 8.05 (d, J = 8.8 Hz, 2H), 7.68 (d, J = 9.0 Hz, 2H), 7.56 (dd, J = 13.7, 9.9 Hz, 3H), 6.61-6.45 (m, 3H), 5.14-5.04 (m, 1H), 4.65 (s, 1H), 3.59-3.52 (m, 1H), 3.33-3.31 (m, 2H), 3.24-3.20 (m, 1H), 3.15-3.08 (m, 1H), 2.78-2.66 (m, 2H), 2.44-2.36 (m, 2H), 2.25-2.17 (m, 1H), 1.98-1.67 (m, 4H), 1.13 (d, J = 6.2 Hz, 3H). 19F NMR (376 MHz, DMSO-d6) δ -124.18. MS-ESI [M+H]+: 609.3 |
| 16 | 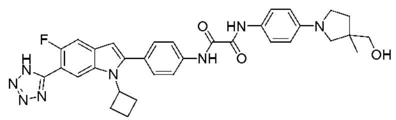 | C | 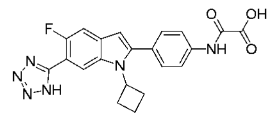 A10 + 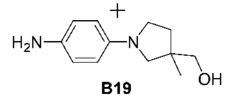 B19 | 1H NMR (400 MHz, DMSO-d6) δ 10.99 (s, 1H), 10.58 (s, 1H), 8.32 (d, J = 5.8 Hz, 1H), 8.04 (d, J = 8.7 Hz, 2H), 7.68 (d, J = 8.8 Hz, 2H), 7.60-7.50 (m, 3H), 6.58-6.46 (m, 3H), 5.15-5.02 (m, 1H), 4.80 (s, 1H), 3.32-3.25 (m, 6H), 3.20 (d, J = 9.4 Hz, 2H), 2.88 (d, J = 9.3 Hz, 1H), 2.80-2.67 (m, 2H), 2.45-2.34 (m, 2H), 1.98-1.74 (m, 3H), 1.69-1.58 (m, 1H), 1.08 (s, 3H). 19F NMR (376 MHz, DMSO-d6) δ -124.10. MS-ESI [M+H]+: 609.3 |

(continued)

| Ex. | Structure | Syn. method | Raw material 1 | Characterization data |
|---|---|---|---|---|
| 17 | 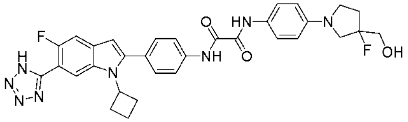 | C | 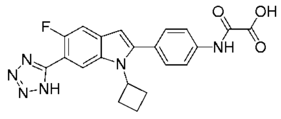 A10 + 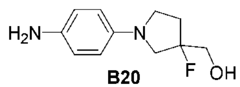 B20 | 1H NMR (400 MHz, DMSO-d6) δ 11.00 (s, 1H), 10.63 (s, 1H), 8.32 (d, J = 5.8 Hz, 1H), 8.05 (d, J = 8.7 Hz, 2H), 7.72 (d, J = 9.0 Hz, 2H), 7.59-7.50 (m, 3H), 6.66-6.45 (m, 3H), 5.28 (s, 1H), 5.13-5.02 (m, 1H), 3.73-3.65 (m, 2H), 3.57-3.46 (m, 2H), 3.43-3.39 (m, 2H), 2.78-2.67 (m, 2H), 2.44-2.35 (m, 2H), 2.29-2.12 (m, 2H), 1.93-1.76 (m, 2H). 19F NMR (376 MHz, DMSO-d6) δ - 123.95, -151.88. MS-ESI [M+H]+: 613.3 |
| 18 | 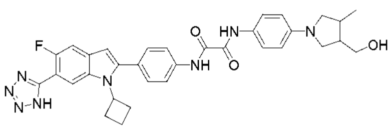 | C | 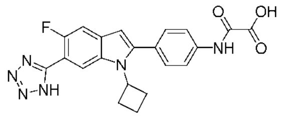 A10 + 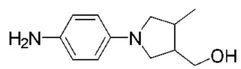 B21 | 1H NMR (400 MHz, DMSO-d6) δ 10.97 (s, 1H), 10.58 (s, 1H), 8.29 (d, J = 6.0 Hz, 1H), 8.03 (d, J = 8.8 Hz, 2H), 7.68 (d, J = 8.8 Hz, 2H), 7.53 (d, J = 8.8 Hz, 2H), 7.48 (d, J = 11.2 Hz, 1H), 6.51 (s, 2H), 6.49 (s, 1H), 5.12-5.02 (m, 1H), 4.67 (s, 1H), 3.59-3.53 (m, 1H), 3.50-3.46 (m, 1H), 3.45-3.40 (m, 2H), 3.12-3.06 (m, 1H), 2.88-2.81 (m, 1H), 2.80-2.68 (m, 2H), 2.43-2.34 (m, 2H), 2.16-2.07 (m, 1H), 2.03-1.94 (m, 1H), 1.94-1.75 (m, 2H), 1.08 (d, J = 6.4 Hz, 3H), MS-ESI [M+H]+: 609.3 |
| 19 | 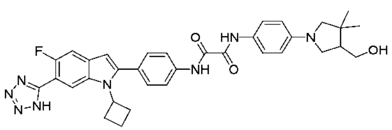 | C | 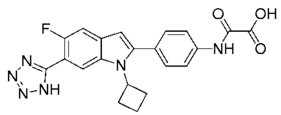 A10 + 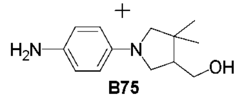 B75 | 1H NMR (400 MHz, DMSO-d6) δ 10.99 (s, 1H), 10.58 (s, 1H), 8.33 (d, J = 6.0 Hz, 1H), 8.04 (d, J = 8.8 Hz, 2H), 7.67 (d, J = 9.2 Hz, 2H), 7.58 (d, J = 11.2 Hz, 1H), 7.54 (d, J = 8.8 Hz, 2H), 6.56 (s, 1H), 6.47 (d, J = 9.2 Hz, 2H), 5.14-5.04 (m, 1H), 4.57 (s, 1H), 3.63-3.58 (m, 1H), 3.46-3.40 (m, 2H), 3.17-3.12 (m, 1H), 3.11-3.01 (m, 2H), 2.76-2.66 (m, 2H), 2.43-2.36 (m, 2H), 2.11-2.03 (m, 1H), 1.92-1.75 (m, 2H), 1.14 (s, 3H), 0.95 (s, 3H). MS-ESI [M+H]+: 623.7 |
| 20 | 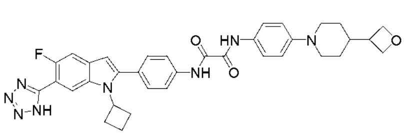 | C | 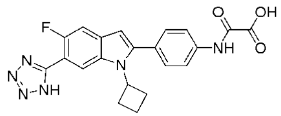 A10 + 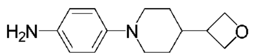 B11 | 1H NMR (400 MHz, DMSO-d6) δ 11.00 (s, 1H), 10.69 (s, 1H), 8.30 (d, J = 6.0 Hz, 1H), 8.04 (d, J = 8.4 Hz, 2H), 7.71 (d, J = 8.8 Hz, 2H), 7.56-7.47 (m, 3H), 6.95 (d, J = 8.8 Hz, 2H), 6.52 (s, 1H), 5.12-5.01 (m, 1H), 4.65-4.59 (m, 2H), 4.37 (d, J = 6.0 Hz, 2H), 3.72-3.65 (m, 2H), 2.79-2.69 (m, 3H), 2.69-2.61 (m, 2H), 2.43-2.34 (m, 2H), 1.94-1.72 (m, 3H), 1.69-1.61 (m, 2H), 1.20-1.08 (m, 2H). MS-ESI [M+H]+: 635.3 |

(continued)

| Ex. | Structure | Syn. method | Raw material 1 | Characterization data |
|---|---|---|---|---|
| 21 | 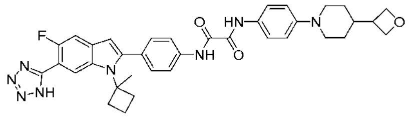 | C | 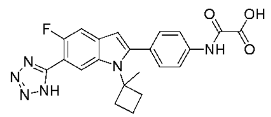 A13 + 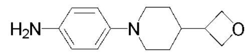 B11 | 1H NMR (400 MHz, DMSO-d6) δ 10.96 (s, 1H), 10.67 (s, 1H), 7.98 (d, J = 8.4 Hz, 2H), 7.89 (d, J = 6.0 Hz, 1H), 7.71 (d, J = 8.8 Hz, 2H), 7.51 (d, J = 8.4 Hz, 2H), 7.44 (d, J = 11.2 Hz, 1H), 6.94 (d, J = 8.8 Hz, 2H), 6.41 (s, 1H), 4.65-4.60 (m, 2H), 4.37 (d, J = 6.0 Hz, 2H), 3.72-3.66 (m, 2H), 2.77-2.70 (m, 1H), 2.68-2.61 (m, 2H), 2.27-2.18 (m, 2H), 2.13-2.04 (m, 2H), 1.97 (s, 3H), 1.91-1.82 (m, 1H), 1.81-1.73 (m, 1H), 1.69-1.61 (m, 3H), 1.20-1.10 (m, 2H). MS-ESI [M+H]+: 649.3 |
| 22 | 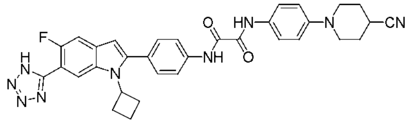 | C | 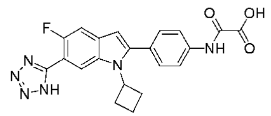 A10 + 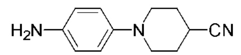 B30 | 1H NMR (400 MHz, DMSO-d6) δ 11.04 (s, 1H), 10.74 (s, 1H), 8.33 (d, J = 5.8 Hz, 1H), 8.05 (d, J = 8.7 Hz, 2H), 7.75 (d, J = 9.1 Hz, 2H), 7.57 (dd, J = 17.1, 10.0 Hz, 3H), 6.98 (d, J = 9.2 Hz, 2H), 6.57 (s, 1H), 5.18-4.98 (m, 1H), 3.37-3.30 (m, 2H), 3.10-3.00 (m, 3H), 2.79-2.66 (m, 2H), 2.46-2.34 (m, 2H), 2.04-1.93 (m, 2H), 1.93-1.74 (m, 4H). 19F NMR (376 MHz, DMSO-d6) δ-124.24. MS-ESI [M+H]+: 604.3 |
| 23 | 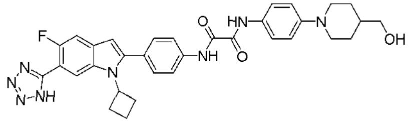 | C | 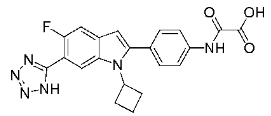 A10 + 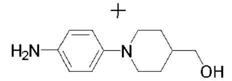 B22 | 1H NMR (400 MHz, DMSO-d6) δ 11.02 (s, 1H), 10.69 (s, 1H), 8.32 (d, J = 5.6 Hz, 1H), 8.05 (d, J = 8.8Hz, 2H), 7.71 (d, J = 9.2 Hz, 2H), 7.57 (d, J = 11.2Hz, 1H), 7.54 (d, J = 8.4Hz, 2H), 6.95 (d, J = 9.2 Hz, 2H), 6.56 (s, 1H), 5.15-5.00 (m, 1H), 4.48 (brs, 1H), 3.74-3.60 (m, 2H), 3.30-3.26 (m, 2H), 2.76-2.55 (m,4H), 2.45-2.32 (m, 2H), 1.95-1.68 (m, 4H), 1.60-1.40 (m, 1H), 1.30-1.10 (m, 2H). MS-ESI [M+H]+: 609.3 |
| 24 | 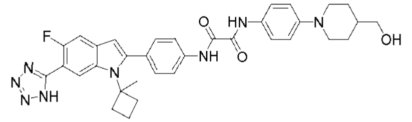 | C | 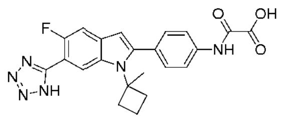 A13 + 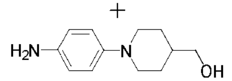 B22 | 1H NMR (400 MHz, DMSO-d6) δ 10.98 (s, 1H), 10.66 (s, 1H), 7.99 (d, J = 8.8 Hz, 2H), 7.96 (d, J = 6.0 Hz, 1H), 7.70 (d, J = 9.2 Hz, 2H), 7.57 (d, J = 11.2 Hz, 1H), 7.52 (d, J = 8.8 Hz, 2H), 6.94 (d, J = 9.2 Hz, 2H), 6.48 (s, 1H), 4.47 (s, 1H), 3.69 (d, J = 12.4 Hz, 2H), 3.29-3.25 (m, 2H), 2.65-2.58 (m, 2H), 2.28-2.19 (m, 2H), 2.16-2.06 (m, 2H), 1.98 (s, 3H), 1.92-1.83 (m, 1H), 1.77-1.71 (m, 2H), 1.69-1.59 (m, 1H), 1.56-1.45 (m, 1H), 1.28-1.18 (m, 2H). MS-ESI [M+H]+: 623.3 |

(continued)

| Ex. | Structure | Syn. method | Raw material 1 | Characterization data |
|---|---|---|---|---|
| 25 | 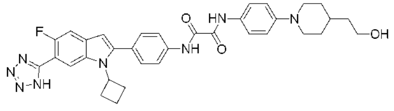 | C | 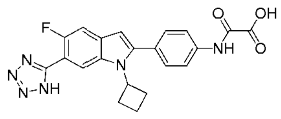 A10 + 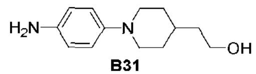 B31 | 1H NMR (400 MHz, DMSO-d6) δ 11.01 (s, 1H), 10.68 (s, 1H), 8.32 (d, J = 5.8 Hz, 1H), 8.04 (d, J = 8.7 Hz, 2H), 7.71 (d, J = 9.1 Hz, 2H), 7.57-7.53 (m, 3H), 6.94 (d, J = 9.2 Hz, 2H), 6.55 (s, 1H), 5.13-5.04 (m, 1H), 4.37 (s, 1H), 3.66 (d, J = 12.2 Hz, 2H), 3.47 (t, J = 6.6 Hz, 2H), 2.77-2.69 (m, 1H), 2.64-2.58 (m, 2H), 2.43-2.36 (m, 2H), 1.90-1.72 (m, 4H), 1.57-1.48 (m, 1H), 1.40 (q, J = 6.6 Hz, 2H), 1.28-1.19 (m, 2H). MS-ESI [M+H]+: 623.4 |
| 26 | 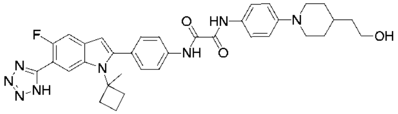 | C | 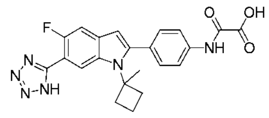 A13 + 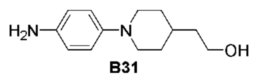 B31 | 1H NMR (400 MHz, DMSO-d6) δ 10.96 (s, 1H), 10.66 (s, 1H), 7.98 (d, J = 8.8 Hz, 2H), 7.89 (d, J = 6.0 Hz, 1H), 7.70 (d, J = 9.2 Hz, 2H), 7.51 (d, J = 8.8 Hz, 2H), 7.44 (d, J = 11.2 Hz, 1H), 6.94 (d, J = 9.2 Hz, 2H), 6.41 (s, 1H), 4.37 (s, 1H), 3.69-3.62 (m, 2H), 3.49-3.45 (m, 2H), 2.65-2.57 (m, 2H), 2.27-2.19 (m, 2H), 2.13-2.04 (m, 2H), 1.97 (s, 3H), 1.91-1.82 (m, 1H), 1.77-1.69 (m, 2H), 1.68-1.59 (m, 1H), 1.57-1.46 (m, 1H), 1.43-1.37 (m, 2H), 1.28-1.18 (m, 2H). MS-ESI [M+H]+: 637.4 |
| 27 | 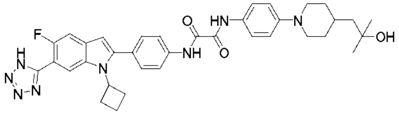 | C | 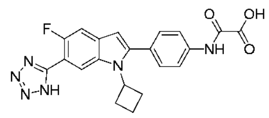 A10 + 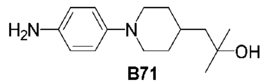 B71 | 1H NMR (400 MHz, DMSO-d6) δ 11.01 (s, 1H), 10.68 (s, 1H), 8.31 (d, J = 5.8 Hz, 1H), 8.04 (d, J = 8.7 Hz, 2H), 7.71 (d, J = 9.1 Hz, 2H), 7.57-7.50 (m, 3H), 6.93 (d, J = 9.2 Hz, 2H), 6.54 (s, 1H), 5.13-5.03 (m, 1H), 4.09 (s, 1H), 3.66-3.58 (m, 2H), 2.76-2.60 (m, 4H), 2.45-2.35 (m, 2H), 1.91-1.75 (m, 4H), 1.64-1.55 (m, 1H), 1.35-1.31 (m, 2H), 1.29-1.23 (m, 2H), 1.12 (s, 6H). 19F NMR (376 MHz, DMSO-d6) δ-123.95. MS-ESI [M+H]+: 651.3 |
| 28 | 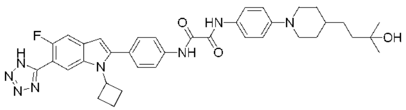 | C | 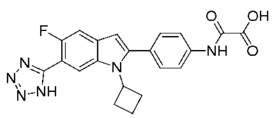 A10 + 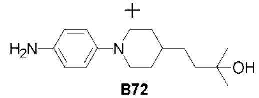 B72 | 1H NMR (400 MHz, DMSO-d6) δ 11.00 (s, 1H), 10.68 (s, 1H), 8.28 (d, J = 5.9 Hz, 1H), 8.03 (d, J = 8.7 Hz, 2H), 7.71 (d, J = 9.1 Hz, 2H), 7.52 (d, J = 8.7 Hz, 2H), 7.43 (d, J = 11.1 Hz, 1H), 6.94 (d, J = 9.3 Hz, 2H), 6.48 (s, 1H), 5.06 (p, J = 8.9 Hz, 1H), 4.08 (s, 1H), 3.71-3.63 (m, 2H), 2.81-2.70 (m, 2H), 2.66-2.56 (m, 2H), 2.43-2.34 (m, 2H), 1.94-1.71 (m, 4H), 1.42-1.33 (m, 2H), 1.30-1.17 (m, 5H), 1.07 (s, 6H). 19F NMR (376 MHz, DMSO-d6) δ -123.34. MS-ESI [M+H]+: 665.3 |

(continued)

| Ex. | Structure | Syn. method | Raw material 1 | Characterization data |
|---|---|---|---|---|
| 29 | 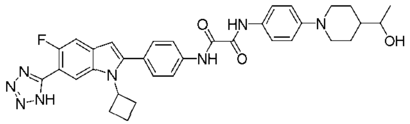 | C | 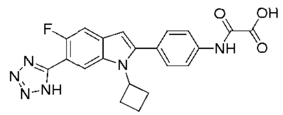 A10 + 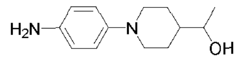 B26 | 1H NMR (400 MHz, DMSO-d6) δ 11.01 (s, 1H), 10.68 (s, 1H), 8.32 (d, J =6.0 Hz, 1H), 8.04 (d, J = 8.4 Hz, 2H), 7.71 (d, J = 8.8 Hz, 2H), 7.58-7.52 (m, 3H), 6.94 (d, J = 9.2 Hz, 2H), 6.55 (s, 1H), 5.11-5.04 (m, 1H), 4.39 (brs, 1H), 3.74-3.70 (m, 2H), 3.41-3.32 (m, 1H), 2.74-2.66 (m, 2H), 2.62-2.22 (m, 2H), 2.45-2.35 (m, 2H), 1.91-1.78 (m, 3H), 1.64-1.61 (m, 1H), 1.35-1.29 (m, 3H), 1.06 (d, J = 6.0 Hz, 3H). MS-ESI [M+H]+: 623.4 |
| 30 | 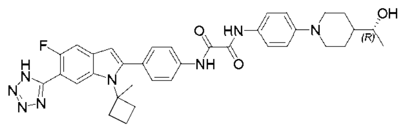 | C | 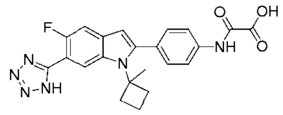 A13 + 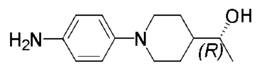 B25 | 1H NMR (400 MHz, DMSO-d6) δ 10.98 (s, 1H), 10.67 (s, 1H), 8.05-7.92 (m, 3H), 7.71 (d, J = 9.1 Hz, 2H), 7.59-7.47 (m, 3H), 6.94 (d, J = 9.2 Hz, 2H), 6.47 (s, 1H), 4.39 (s, 1H), 3.72 (d, J = 11.7 Hz, 2H), 3.40-3.38 (m, 1H), 2.64-2.53 (m, 2H), 2.29-2.18 (m, 2H), 2.16-2.04 (m, 2H), 1.98 (s, 3H), 1.92-1.81 (m, 2H), 1.69-1.58 (m, 2H), 1.36-1.24 (m, 3H), 1.06 (d, J = 6.3 Hz, 3H). 19F NMR (376 MHz, DMSO-d6) δ -124.54. MS-ESI [M+H]+: 637.3 |
| 31 | 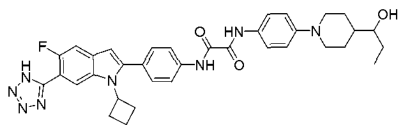 | C | 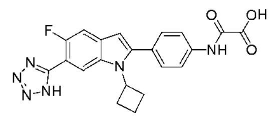 A10 + 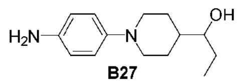 B27 | 1H NMR (400 MHz, DMSO-d6) δ 11.02 (s, 1H), 10.68 (s, 1H), 8.33 (d, J = 5.8 Hz, 1H), 8.05 (d, J = 8.6 Hz, 2H), 7.71 (d, J = 9.1 Hz, 2H), 7.63-7.46 (m, 3H), 6.94 (d, J = 9.2 Hz, 2H), 6.56 (s, 1H), 5.16-5.03 (m, 1H), 4.32 (s, 1H), 3.77-3.68 (m, 2H), 3.16-3.11 (m, 1H), 2.78-2.66 (m, 2H), 2.63-2.53 (m, 2H), 2.44-2.36 (m, 2H), 1.93-1.75 (m, 3H), 1.64-1.55 (m, 1H), 1.49-1.43 (m, 1H), 1.40-1.24 (m, 4H), 0.89 (t, J = 7.3 Hz, 3H). 19F NMR (376 MHz, DMSO-d6) δ -124.17. MS-ESI [M+H]+: 637.3 |
| 32 | 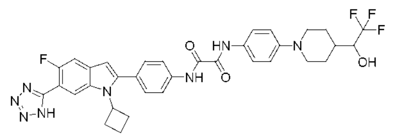 | C | 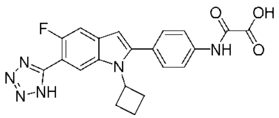 A10 + 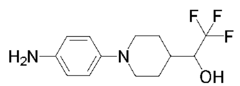 B28 | 1H NMR (400 MHz, DMSO-d6) δ 11.00 (s, 1H), 10.69 (s, 1H), 8.29 (d, J = 6.0 Hz, 1H), 8.03 (d, J = 8.4 Hz, 2H), 7.72 (d, J = 8.8 Hz, 2H), 7.53 (d, J = 8.4 Hz, 2H), 7.45 (d, J = 11.2 Hz, 1H), 6.95 (d, J = 8.8 Hz, 2H), 6.50 (s, 1H), 6.18 (d, J = 6.8 Hz, 1H), 5.11-5.01 (m, 1H), 3.85-3.79 (m, 1H), 3.77-3.71 (m, 2H), 2.80-2.69 (m, 2H), 2.68-2.58 (m, 2H), 2.43-2.34 (m, 2H), 1.93-1.85 (m, 1H), 1.83-1.64 (m, 4H), 1.62-1.49 (m, 2H). MS-ESI [M+H]+: 677.3 |

(continued)

| Ex. | Structure | Syn. method | Raw material 1 | Characterization data |
|---|---|---|---|---|
| 33 | 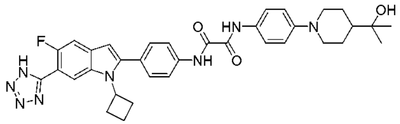 | C | 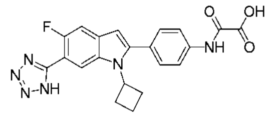 A10 + 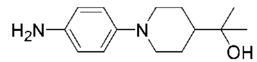 B29 | 1H NMR (400 MHz, DMSO-d6) δ 11.00 (s, 1H), 10.68 (s, 1H), 8.29 (d, J = 5.9 Hz, 1H), 8.04 (d, J = 8.6 Hz, 2H), 7.72 (d, J = 9.1 Hz, 2H), 7.50 (dd, J = 23.5, 9.8 Hz, 3H), 6.95 (d, J = 9.1 Hz, 2H), 6.51 (s, 1H), 5.11-5.02 (m, 1H), 4.12 (s, 1H), 3.76 (d, J = 11.7 Hz, 2H), 2.81-2.68 (m, 2H), 2.60-2.51 (m, 2H), 2.43-2.35 (m, 2H), 1.93-1.74 (m, 4H), 1.38-1.28 (m, 3H), 1.06 (s, 6H). 19F NMR (376 MHz, DMSO-d6) δ -123.57. MS-ESI [M+H]+: 637.3 |
| 34 | 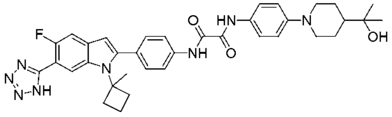 | C | 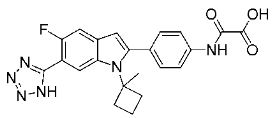 A13 + 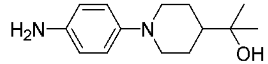 B29 | 1H NMR (400 MHz, DMSO-d6) δ 10.97 (s, 1H), 10.67 (s, 1H), 7.98 (d, J = 8.4 Hz, 2H), 7.91 (d, J = 5.9 Hz, 1H), 7.71 (d, J = 8.8 Hz, 2H), 7.51 (d, J = 8.4 Hz, 2H), 7.46 (d, J = 11.2 Hz, 1H), 6.94 (d, J = 9.1 Hz, 2H), 6.43 (s, 1H), 4.12 (s, 1H), 3.76 (d, J = 11.6 Hz, 2H), 2.57-2.54 (m, 2H), 2.28-2.20 (m, 2H), 2.13-2.07 (m, 2H), 1.97 (s, 3H), 1.91-1.84 (m, 1H), 1.79-1.76 (m, 2H), 1.68-1.60 (m, 1H), 1.38-1.30 (m, 3H), 1.06 (s, 6H). MS-ESI [M+H]+: 651.4 |
| 35 | 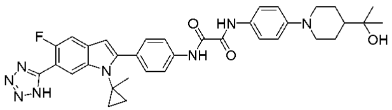 | C | 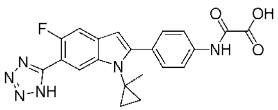 A12 + 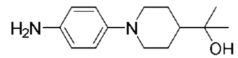 B29 | 1H NMR (400 MHz, DMSO-d6) δ 10.99 (s, 1H), 10.68 (s, 1H), 8.19 (d, J = 5.9 Hz, 1H), 8.04 (d, J = 8.8 Hz, 2H), 7.73-7.66 (m, 4H), 7.47 (d, J = 11.2 Hz, 1H), 6.95 (d, J = 9.2 Hz, 2H), 6.55 (s, 1H), 4.12 (s, 1H), 3.76 (d, J = 11.5 Hz, 2H), 2.57-2.54 (m, 2H), 1.81 (s, 3H), 1.78-1.75 (m, 2H), 1.38-1.32 (m, 3H), 1.17-1.10 (m, 0H), 1.06 (s, 6H), 0.93-0.81 (m, 2H), 0.72-0.63 (m, 1H). MS-ESI [M+H]+: 637.3 |
| 36 | 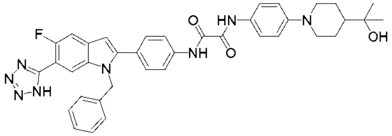 | C | 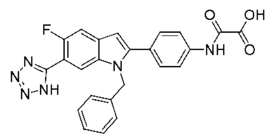 A17 + 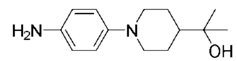 B29 | 1H NMR (400 MHz, DMSO-d6) δ 10.98 (s, 1H), 10.67 (s, 1H), 7.99 (d, J = 8.5 Hz, 2H), 7.93 (d, J = 5.8 Hz, 1H), 7.70 (d, J = 8.8 Hz, 2H), 7.55 (dd, J = 9.8, 6.3 Hz, 3H), 7.31-7.16 (m, 3H), 6.94 (d, J = 8.9 Hz, 4H), 6.73 (s, 1H), 5.55 (s, 2H), 4.13 (s, 1H), 3.79-3.71 (m, 2H), 2.58-2.51 (m, 2H), 1.80-1.71 (m, 2H), 1.38-1.26 (m, 3H), 1.06 (s, 6H). 19F NMR (376 MHz, DMSO-d6) δ -123.40. MS-ESI [M+H]+: 673.3 |

(continued)

| Ex. | Structure | Syn. method | Raw material 1 | Characterization data |
|---|---|---|---|---|
| 37 | 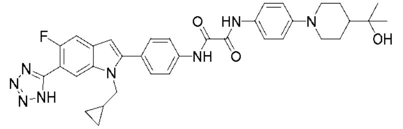 | C | 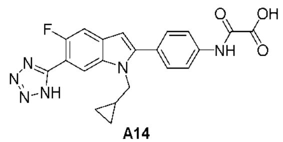 A14 + 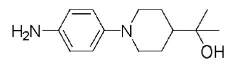 B29 | 1H NMR (400 MHz, DMSO-d6) δ 11.01 (s, 1H), 10.68 (s, 1H), 8.17 (d, J = 6.0 Hz, 1H), 8.05 (d, J = 8.4 Hz, 2H), 7.71 (d, J = 8.8 Hz, 2H), 7.61 (d, J = 8.4 Hz, 2H), 7.50 (d, J = 11.2 Hz, 1H), 6.94 (d, J = 8.8 Hz, 2H), 6.60 (s, 1H), 4.21 (d, J = 6.8 Hz, 2H), 4.12 (s, 1H), 3.76 (d, J = 11.6 Hz, 2H), 2.59-2.53 (m, 2H), 1.80-1.74 (m, 2H), 1.37-1.28 (m, 3H), 1.06 (s, 6H), 0.98-0.91 (m, 1H), 0.35-0.29 (m, 2H), 0.06 (t, J = 5.1 Hz, 2H). MS-ESI [M+H]+: 637.3 |
| 38 | 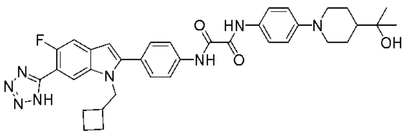 | C | 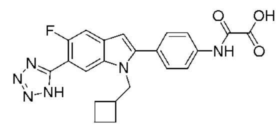 A16 + 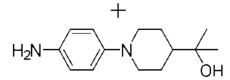 B29 | 1H NMR (400 MHz, DMSO-d6) δ 11.00 (s, 1H), 10.68 (s, 1H), 8.11 (d, J = 6.0 Hz, 1H), 8.05 (d, J = 8.4 Hz, 2H), 7.71 (d, J = 9.2 Hz, 2H), 7.60 (d, J = 8.4 Hz, 2H), 7.46 (d, J = 11.2 Hz, 1H), 6.94 (d, J = 9.2 Hz, 2H), 6.55 (s, 1H), 4.36 (d, J = 7.2 Hz, 2H), 4.12 (s, 1H), 3.76 (d, J = 11.6 Hz, 2H), 2.60-2.53 (m, 2H), 1.82-1.53 (m, 7H), 1.51-1.43 (m, 2H), 1.36-1.28 (m, 3H), 1.06 (s, 6H). MS-ESI [M+H]+: 651.3 |
| 39 | 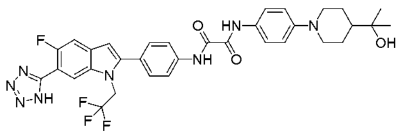 | C | 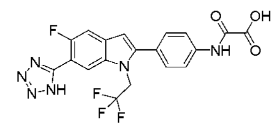 A15 + 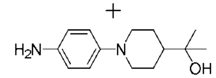 B29 | 1H NMR (400 MHz, DMSO-d6) δ 11.03 (s, 1H), 10.68 (s, 1H), 8.43 (d, J = 6.0 Hz, 1H), 8.06 (d, J = 8.4 Hz, 2H), 7.71 (d, J = 8.8 Hz, 2H), 7.65-7.59 (m, 3H), 6.94 (d, J = 8.8 Hz, 2H), 6.74 (s, 1H), 5.37-5.26 (m, 2H), 4.13 (s, 1H), 3.76 (d, J = 11.6 Hz, 2H), 2.59-2.53 (m, 2H), 1.80-1.73 (m, 2H), 1.37-1.28 (m, 3H), 1.06 (s, 6H), MS-ESI [M+H]+: 665.3 |
| 40 | 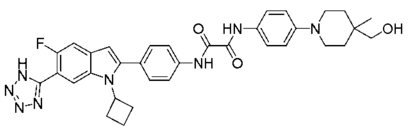 | C | 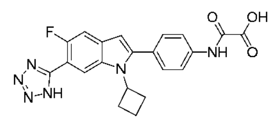 A10 + 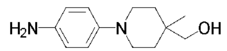 B24 | 1H NMR (400 MHz, DMSO-d6) δ 11.02 (s, 1H), 10.69 (s, 1H), 8.33 (d, J = 5.8 Hz, 1H), 8.05 (d, J = 8.8 Hz, 2H), 7.71 (d, J = 9.1 Hz, 2H), 7.57 (dd, J = 18.7, 10.0 Hz, 3H), 6.95 (d, J = 9.2 Hz, 2H), 6.57 (s, 1H), 5.17-5.01 (m, 1H), 4.54 (s, 1H), 3.35-3.28 (m, 2H), 3.20 (s, 2H), 3.03-2.92 (m, 2H), 2.79-2.64 (m, 2H), 2.46-2.34 (m, 2H), 1.95-1.74 (m, 2H), 1.63-1.52 (m, 2H), 1.37-1.27 (m, 2H), 0.91 (s, 3H). 19F NMR (376 MHz, DMSO-d6) δ -124.27. MS-ESI [M+H]+: 623.3 |

(continued)

| Ex. | Structure | Syn. method | Raw material 1 | Characterization data |
|---|---|---|---|---|
| 41 | 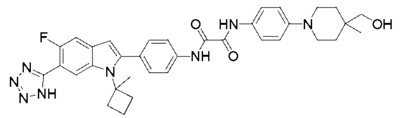 | C | 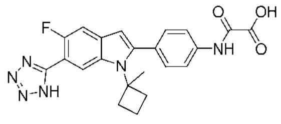 A13 + 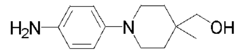 B24 | 1H NMR (400 MHz, DMSO-d6) δ 10.98 (s, 1H), 10.66 (s, 1H), 7.99 (d, J = 8.8 Hz, 2H), 7.95 (d, J = 5.6 Hz, 1H), 7.70 (d, J = 8.8 Hz, 2H), 7.57-7.50 (m, 3H), 6.94 (d, J = 9.2 Hz, 2H), 6.47 (s, 1H), 4.53 (brs, 1H), 3.36-3.28 (m, 4H), 3.19 (s, 2H), 3.01-2.94 (m, 2H), 2.28-2.19 (m, 2H), 2.15-2.05 (m, 2H), 1.98 (s, 3H), 1.91-1.86 (m, 1H), 1.68-1.53 (m, 3H), 1.34-1.28 (m, 2H), 0.91 (s, 3H). MS-ESI [M+H]+: 637.4 |
| 42 | 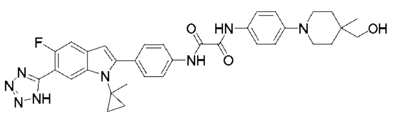 | C | 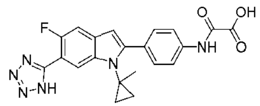 A12 + 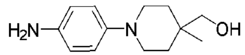 B24 | 1H NMR (400 MHz, DMSO-d6) δ 11.00 (s, 1H), 10.67 (s, 1H), 8.23 (d, J = 5.9 Hz, 1H), 8.05 (d, J = 8.8 Hz, 2H), 7.73-7.67 (m, 4H), 7.54 (d, J = 11.3 Hz, 1H), 6.94 (d, J = 9.2 Hz, 2H), 6.59 (s, 1H), 4.53 (s, 1H), 3.32-3.29 (m, 2H), 3.20 (s, 2H), 3.01-2.95 (m, 2H), 1.82 (s, 3H), 1.61-1.54 (m, 2H), 1.34-1.29 (m, 2H), 1.18-1.1 (m, 1H), 0.95-0.83 (s, 5H), 0.73-0.64 (m, 1H). MS-ESI [M+H]+: 623.3 |
| 43 | 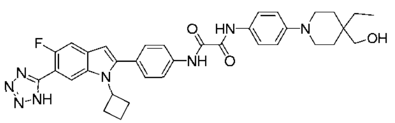 | C | 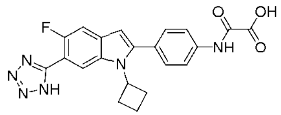 A10 + 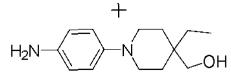 B51 | 1H NMR (400 MHz, DMSO-d6) δ 11.01 (s, 1H), 10.67 (s, 1H), 8.32 (d, J = 6.0 Hz, 1H), 8.04 (d, J = 8.8 Hz, 2H), 7.71 (d, J = 9.2 Hz, 2H), 7.60-7.52 (m, 3H), 6.93 (d, J = 9.2 Hz, 2H), 6.56 (s, 1H), 5.14-5.03 (m, 1H), 4.43 (s, 1H), 3.27 (s, 2H), 3.18-3.13 (m, 2H), 3.11-3.04 (m, 2H), 2.75-2.66 (m, 2H), 2.44-2.35 (m, 2H), 1.92-1.75 (m, 2H), 1.56-1.48 (m, 2H), 1.43-1.34 (m, 4H), 0.79 (d, J = 7.6 Hz, 3H). MS-ESI [M+H]+: 637.4 |
| 44 | 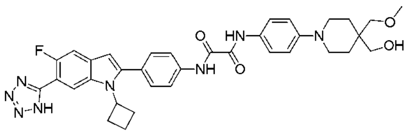 | C | 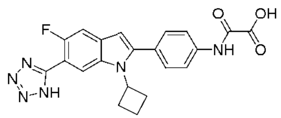 A10 + 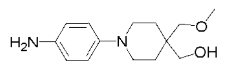 B63 | 1H NMR (400 MHz, DMSO-d6) δ 11.02 (s, 1H), 10.69 (s, 1H), 8.32 (d, J = 6.0 Hz, 1H), 8.05 (d, J = 8.8 Hz, 2H), 7.71 (d, J = 9.2 Hz, 2H), 7.59-7.52 (m, 3H), 6.93 (d, J = 9.2 Hz, 2H), 6.56 (s, 1H), 5.14-5.03 (m, 1H), 4.49 (s, 1H), 3.31-3.29 (m, 2H), 3.26-3.24 (m, 5H), 3.17-3.10 (m, 4H), 2.76-2.66 (m, 2H), 2.45-2.36 (m, 2H), 1.92-1.75 (m, 2H), 1.55-1.47 (m, 4H). MS-ESI [M+H]+: 653.7 |

(continued)

| Ex. | Structure | Syn. method | Raw material 1 | Characterization data |
|---|---|---|---|---|
| 45 | 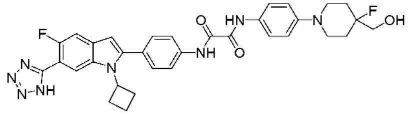 | C | 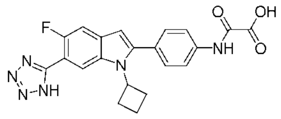 A10 + 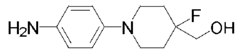 B23 | 1H NMR (400 MHz, DMSO-d6) δ 11.01 (s, 1H), 10.69 (s, 1H), 8.33 (d, J = 5.8 Hz, 1H), 8.09-8.01 (m, 2H), 7.77-7.69 (m, 2H), 7.62-7.50 (m, 3H), 7.03-6.94 (m, 2H), 6.57 (s, 1H), 5.14-5.05 (m, 1H), 4.97 (s, 1H), 3.48-3.43 (m, 4H), 3.04-2.89 (m, 2H), 2.77-2.61 (m, 2H), 2.44-2.31 (m, 2H), 1.95-1.66 (m, 6H). 19F NMR (376 MHz, DMSO-d6) δ -124.26, 163.90. MS-ESI [M+H]+: 627.3 |
| 46 | 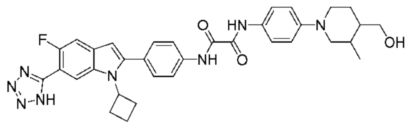 | C | 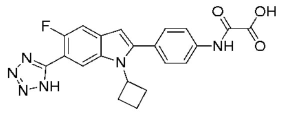 A10 + 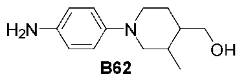 B62 | 1H NMR (400 MHz, DMSO-d6) δ 10.98 (s, 1H), 10.68 (s, 1H), 8.25 (d, J = 6.0 Hz, 1H), 8.02 (d, J = 8.6 Hz, 2H), 7.71 (d, J = 9.0 Hz, 2H), 7.51 (d, J = 8.6 Hz, 2H), 7.35 (d, J = 11.0 Hz, 1H), 6.93 (d, J = 9.2 Hz, 2H), 6.44 (s, 1H), 5.08-5.0 (m, 1H), 4.43 (s, 1H), 4.26 (t, J = 7.0 Hz, 1H), 3.55-3.52 (m, 1H), 3.45-3.38 (m, 3H), 2.81-2.75 (m, 3H), 2.67-2.62 (m, 1H), 2.40-2.32 (m, 2H), 2.06-1.99 (m, 1H), 1.91-1.79 (m, 2H), 1.71-1.65 (m, 1H), 1.58-1.44 (m, 2H), 0.93 (d, J = 7.0 Hz, 3H). MS-ESI [M+H]+: 623.4 |
| 47 | 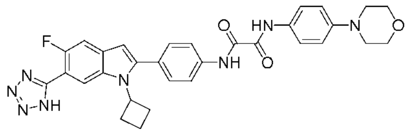 | C | 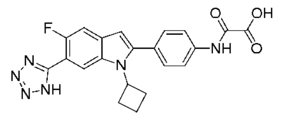 A10 + 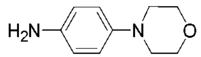 | 1H NMR (400 MHz, DMSO-d6) δ 11.00 (s, 1H), 10.73 (s, 1H), 8.27 (d, J = 6.0 Hz, 1H), 8.03 (d, J = 8.8 Hz, 2H), 7.76 (d, J = 9.1 Hz, 2H), 7.52 (d, J = 8.6 Hz, 2H), 7.41 (d, J = 11.1 Hz, 1H), 6.97 (d, J = 9.2 Hz, 2H), 6.48 (s, 1H), 5.10-5.01 (m, 1H), 3.75 (t, J = 4.8 Hz, 4H), 3.10 (t, J = 4.8 Hz, 4H), 2.81-2.71 (m, 2H), 2.42-2.34 (m, 2H), 1.94-1.76 (m, 2H). MS-ESI [M+H]+: 581.3 |
| 48 | 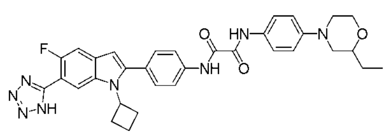 | C | 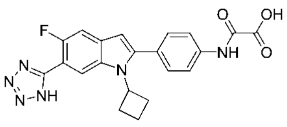 A10 + 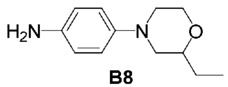 B8 | 1H NMR (400 MHz, DMSO-d6) δ 11.04 (s, 1H), 10.73 (s, 1H), 8.35 (d, J = 5.7 Hz, 1H), 8.05 (d, J = 8.7 Hz, 2H), 7.75 (d, J = 9.1 Hz, 2H), 7.64 (d, J = 11.3 Hz, 1H), 7.55 (d, J = 8.6 Hz, 2H), 6.98 (d, J = 9.2 Hz, 2H), 6.59 (s, 1H), 5.24-4.93 (m, 1H), 4.00-3.87 (m, 1H), 3.66-3.56 (m, 2H), 3.53-3.40 (m, 4H), 2.72-2.63 (m, 2H), 2.43-2.32 (m, 2H), 2.06-1.63 (m, 2H), 1.55-1.45 (m, 2H), 0.95 (t, J = 7.4 Hz, 3H). 19F NMR (376 MHz, DMSO-d6) δ -124.51. MS-ESI [M+H]+: 609.3 |

(continued)

| Ex. | Structure | Syn. method | Raw material 1 | Characterization data |
|---|---|---|---|---|
| 49 | 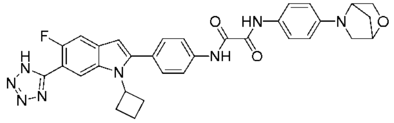 | C | 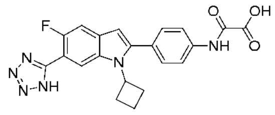 A10 + 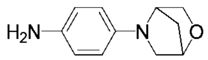 B9 | 1H NMR (400 MHz, DMSO-d6) δ 11.00 (s, 1H), 10.64 (s, 1H), 8.30 (d, J = 5.9 Hz, 1H), 8.04 (d, J = 8.7 Hz, 2H), 7.70 (d, J = 9.0 Hz, 2H), 7.59-7.44 (m, 3H), 6.64 (d, J = 9.1 Hz, 2H), 6.52 (s, 1H), 5.12-5.02 (m, 1H), 4.64-4.58 (m, 1H), 4.57-4.52 (m, 1H), 3.76-3.72 (m, 1H), 3.68-3.65 (m, 1H), 3.51-3.48 (m, 1H), 2.96 (d, J = 9.4 Hz, 1H), 2.79-2.69 (m, 2H), 2.44-2.31 (m, 2H), 1.94-1.80 (m, 4H). 19F NMR (376 MHz, DMSO-d6) δ -123.68. MS-ESI [M+H]+: 593.3 |
| 50 | 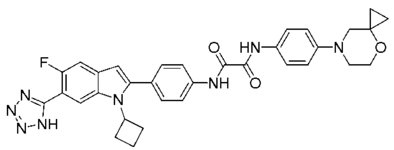 | C | 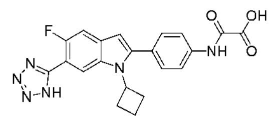 A10 + 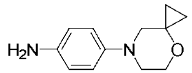 B10 | 1H NMR (400 MHz, DMSO-d6) δ 11.02 (s, 1H), 10.72 (s, 1H), 8.32 (d, J = 5.6 Hz, 1H), 8.04 (d, J = 8.7 Hz, 2H), 7.75 (d, J = 9.1 Hz, 2H), 7.56-7.53 (m, 3H), 6.95 (d, J = 9.2 Hz, 2H), 6.55 (s, 1H), 5.13-5.04 (m, 1H), 3.80 (t, J = 4.9 Hz, 2H), 3.17 (t, J = 4.9 Hz, 2H), 3.09 (s, 2H), 2.75-2.70 (m, 2H), 2.43-2.36 (m, 2H), 1.90-1.76 (m, 2H), 0.75-0.72 (m, 2H), 0.65-0.62 (m, 2H). MS-ESI [M+H]+: 607.3 |
| 51 | 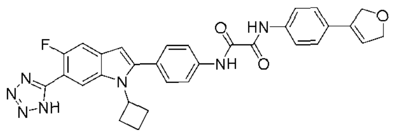 | C | 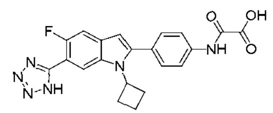 A10 + 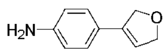 B4 | 1H NMR (400 MHz, DMSO-d6) δ 11.08 (s, 1H), 10.98 (s, 1H), 8.33 (d, J = 5.8 Hz, 1H), 8.05 (d, J = 8.8 Hz, 2H), 7.92 (d, J = 8.8 Hz, 2H), 7.60-7.55 (m, 3H), 7.46 (d, J = 8.6 Hz, 2H), 6.57 (s, 1H), 6.47 (t, J = 2.0 Hz, 1H), 5.14-5.05 (m, 1H), 4.92-4.89 (m, 2H), 4.75-4.72 (m, 2H), 2.77-2.69 (m, 2H), 2.44-2.37 (m, 2H), 1.93-1.76 (m, 2H). MS-ESI [M+H]+: 564.3 |
| 52 | 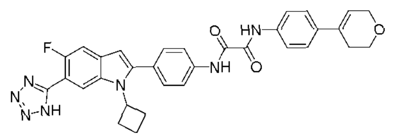 | C | 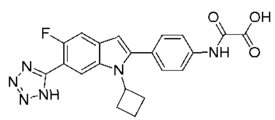 A10 + 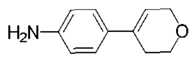 B5 | 1H NMR (400 MHz, DMSO-d6) δ 11.06 (s, 1H), 10.92 (s, 1H), 8.31 (d, J = 5.8 Hz, 1H), 8.04 (d, J = 8.8 Hz, 2H), 7.88 (d, J = 8.8 Hz, 2H), 7.56-7.48 (m, 5H), 6.54 (s, 1H), 6.28-6.26 (m, 1H), 5.13-5.04 (m, 1H), 4.24-4.22 (m, 2H), 3.83 (t, J = 5.5 Hz, 2H), 2.78-2.66 (m, 2H), 2.47-2.37 (m, 4H), 1.93-1.76 (m, 2H)。MS-ESI [M+H]+: 578.2 |

(continued)

| Ex. | Structure | Syn. method | Raw material 1 | Characterization data |
|---|---|---|---|---|
| 53 | 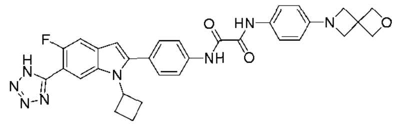 | C | 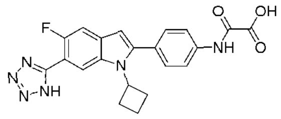 A10 + 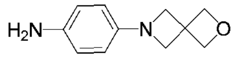 B33 | 1H NMR (400 MHz, DMSO-d6) δ 11.01 (s, 1H), 10.65 (s, 1H), 8.34 (d, J = 5.7 Hz, 1H), 8.05 (d, J = 8.8 Hz, 2H), 7.69 (d, J = 8.9 Hz, 2H), 7.63 (d, J = 11.3 Hz, 1H), 7.55 (d, J = 8.7 Hz, 2H), 6.59 (s, 1H), 6.46 (d, J = 8.9 Hz, 2H), 5.17-5.03 (m, 1H), 4.72 (s, 4H), 3.96 (s, 4H), 2.76-2.63 (m, 2H), 2.46-2.34 (m, 2H), 1.93-1.73 (m, 2H). 19F NMR (376 MHz, DMSO-d6) δ -124.50. MS-ESI [M+H]+: 593.3 |
| 54 | 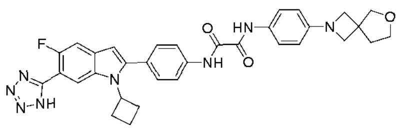 | C | 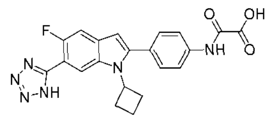 A10 + 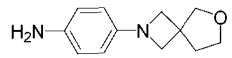 B34 | 1H NMR (400 MHz, DMSO-d6) δ 11.00 (s, 1H), 10.65 (s, 1H), 8.33 (d, J = 5.6 Hz, 1H), 8.03 (d, J = 8.4 Hz, 2H), 7.68 (d, J = 8.4 Hz, 2H), 7.60 (d, J = 11.2 Hz, 1H), 7.54 (d, J = 8.8 Hz, 2H), 6.57(s, 1H), 6.47 (d, J = 8.4 Hz, 2H), 5.15-5.00 (m,1H), 3.80 (s, 2H), 3.78 (s, 4H), 3.75-3.70 (m, 2H), 2.75-2.60 (m, 2H), 2.42-2.30 (m, 2H), 2.15-2.10 (m, 2H), 1.95-1.70 (m, 2H). MS-ESI [M+H]+: 607.3 |
| 55 | 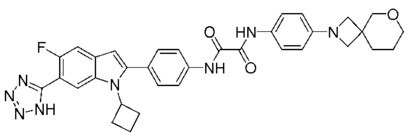 | C | 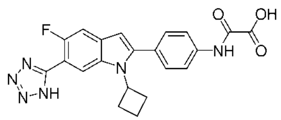 A10 + 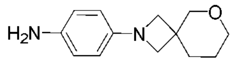 B35 | 1H NMR (400 MHz, DMSO-d6) δ 11.01 (s, 1H), 10.64 (s, 1H), 8.33 (d, J = 5.6 Hz, 1H), 8.04 (d, J = 8.4 Hz, 2H), 7.68 (d, J = 8.8 Hz, 2H), 7.59-7.53 (m, 3H), 6.56 (s, 1H), 6.45 (d, J = 8.8 Hz, 2H), 5.14-5.04 (m, 1H), 3.64 (s, 2H), 3.58 (d, J = 7.2 Hz, 2H), 3.53 (t, J = 5.2 Hz, 2H), 3.46 (d, J = 7.2 Hz, 2H), 2.77-2.66 (m, 2H), 2.43-2.36 (m, 2H), 1.91-1.75 (m, 4H), 1.55-1.49 (m, 2H). MS-ESI [M+H]+: 621.4 |
| 56 | 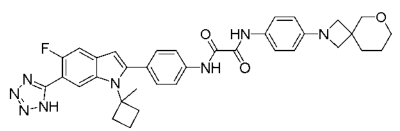 | C | 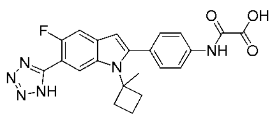 A13 + 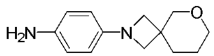 B35 | 1H NMR (400 MHz, DMSO-d6) δ 10.97 (s, 1H), 10.62 (s, 1H), 8.03-7.93 (m, 3H), 7.68 (d, J = 8.9 Hz, 2H), 7.55 (dd, J = 20.7, 10.0 Hz, 3H), 6.50-6.40 (m, 3H), 3.64 (s, 2H), 3.58 (d, J = 7.2 Hz, 2H), 3.56-3.51 (m, 2H), 3.46 (d, J = 7.2 Hz, 2H), 2.29-2.19 (m, 2H), 2.16-2.04 (m, 2H), 1.98 (s, 3H), 1.93-1.84 (m, 1H), 1.83-1.76 (m, 2H), 1.69-1.60 (m, 1H), 1.56-1.49 (m, 2H). 19F NMR (376 MHz, DMSO-d6) δ -124.69. MS-ESI [M+H]+: 635.3 |

(continued)

| Ex. | Structure | Syn. method | Raw material 1 | Characterization data |
|---|---|---|---|---|
| 57 | 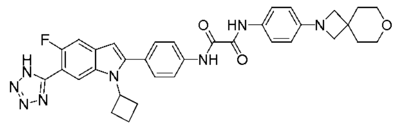 | C | 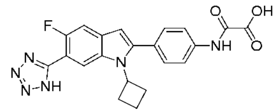 A10 + 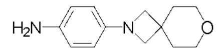 B36 | 1H NMR (400 MHz, DMSO-d6) δ 11.01 (s, 1H), 10.64 (s, 1H), 8.34 (d, J = 5.8 Hz, 1H), 8.05 (d, J = 8.6 Hz, 2H), 7.69 (d, J = 8.9 Hz, 2H), 7.61 (d, J = 11.3 Hz, 1H), 7.55 (d, J = 8.6 Hz, 2H), 6.58 (s, 1H), 6.45 (d, J = 9.0 Hz, 2H), 5.10 (t, J = 8.7 Hz, 1H), 3.59 (s, 4H), 3.59-3.52 (m, 4H), 2.78-2.64 (m, 2H), 2.46-2.35 (m, 2H), 1.99-1.76 (m, 2H), 1.78-1.71 (m, 4H). 19F NMR (376 MHz, DMSO-d6) δ -124.36. MS-ESI [M+H]+: 621.3 |
| 58 | 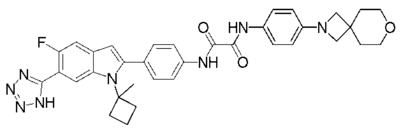 | C | 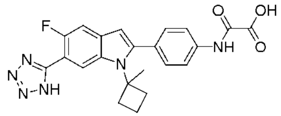 A13 + 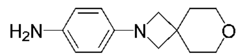 B36 | 1H NMR (400 MHz, DMSO-d6) δ 10.97 (s, 1H), 10.62 (s, 1H), 7.97 (dd, J = 14.8, 7.2 Hz, 3H), 7.68 (d, J = 8.9 Hz, 2H), 7.59-7.49 (m, 3H), 6.50-6.41 (m, 3H), 3.57 (d, J = 14.4 Hz, 8H), 2.30-2.18 (m, 2H), 2.15-2.07 (m, 2H), 1.98 (s, 3H), 1.93-1.82 (m, 1H), 1.78-1.71 (m, 4H), 1.68-1.61 (m, 1H). 19F NMR (376 MHz, DMSO-d6) δ -124.62. MS-ESI [M+H]+: 635.3 |
| 59 | 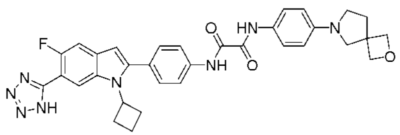 | C | 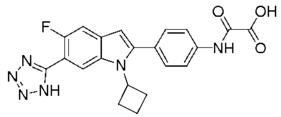 A10 + 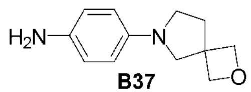 B37 | 1H NMR (400 MHz, DMSO-d6) δ 10.99 (s, 1H), 10.61 (s, 1H), 8.30 (d, J = 5.6 Hz, 1H), 8.04 (d, J = 8.4 Hz, 2H), 7.70 (d, J = 9.2 Hz, 2H), 7.55-7.48 (m, 3H), 6.55 (d, J = 8.8 Hz, 2H), 6.52 (s, 1H), 5.12-5.02 (m, 1H), 4.59 (d, J = 5.6 Hz, 2H), 4.53 (d, J = 6.0 Hz, 2H), 3.50 (s, 2H), 3.25 (t, J = 6.8 Hz, 2H), 2.80-2.66 (m, 2H), 2.54-2.51 (m, 2H), 2.43-2.32 (m, 2H), 2.26 (t, J = 6.8 Hz, 2H), 1.91-1.75 (m, 2H). MS-ESI [M+H]+: 607.3 |
| 60 | 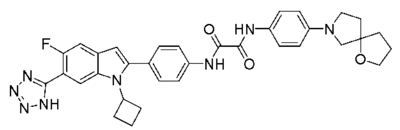 | C | 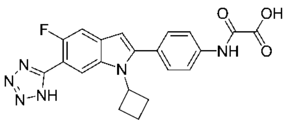 A10 + 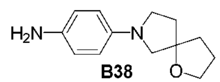 B38 | 1H NMR (400 MHz, DMSO-d6) δ 11.00 (s, 1H), 10.60 (s, 1H), 8.33 (d, J = 5.6 Hz, 1H), 8.05 (d, J = 8.4 Hz, 2H), 7.69 (d, J = 8.4 Hz, 2H), 7.59 (d, J = 11.2 Hz, 1H), 7.54 (d, J = 8.0 Hz, 2H), 6.57 (s, 1H), 6.50 (d, J = 8.4 Hz, 2H), 5.15-5.00 (m, 1H), 3.80-3.70 (m, 2H), 3.35-3.27 (m, 2H), 3.25 (s, 2H), 2.75-2.60 (m, 2H), 2.42-2.30 (m, 2H), 2.05-1.70 (m, 8H). MS-ESI [M+H]+: 621.3 |

(continued)

| Ex. | Structure | Syn. method | Raw material 1 | Characterization data |
|---|---|---|---|---|
| 61 | 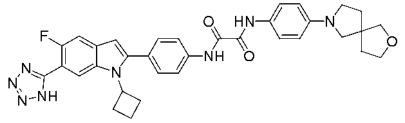 | C | 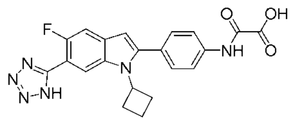 A10 + 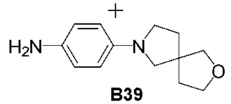 B39 | 1H NMR (400 MHz, DMSO-d6) δ 10.99 (s, 1H), 10.60 (s, 1H), 8.30 (d, J = 6.0 Hz, 1H), 8.04 (d, J = 8.4Hz, 2H), 7.69 (d, J = 8.8 Hz, 2H), 7.58 (d, J = 11.2 Hz, 1H), 7.54 (d, J = 8.4Hz, 2H), 6.56 (s, 1H), 6.54 (d, J = 8.8 Hz, 2H), 5.15-5.00 (m, 1H), 3.85-3.75 (m, 2H), 3.59 (s, 2H), 3.29-3.25 (m, 2H), 3.24 (s, 2H), 2.78-2.62 (m, 2H), 2.45-2.30 (m, 2H), 2.02-1.94 (m, 2H), 1.93-1.70 (m, 4H). MS-ESI [M+H]+: 621.3 |
| 62 | 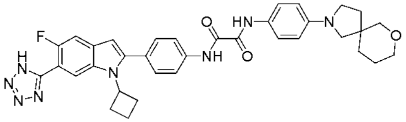 | C | 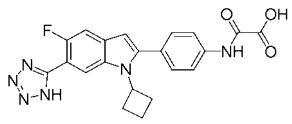 A10 + 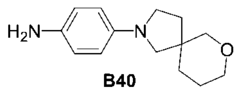 B40 | 1H NMR (400 MHz, DMSO-d6) δ 11.00 (s, 1H), 10.60 (s, 1H), 8.34 (d, J = 5.8 Hz, 1H), 8.05 (d, J = 8.7 Hz, 2H), 7.69 (d, J = 8.7 Hz, 2H), 7.57 (dd, J = 22.2, 9.9 Hz, 3H), 6.61-6.50 (m, 3H), 5.17-5.02 (m, 1H), 3.64-3.59 (m, 1H), 3.56-3.51 (m, 1H), 3.36-3.30 (m, 5H), 3.00 (d, J = 9.7 Hz, 1H), 2.77-2.66 (m, 2H), 2.45-2.35 (m, 2H), 1.96-1.85 (m, 2H), 1.84-1.55 (m, 6H). 19F NMR (376 MHz, DMSO-d6) δ -124.24. MS-ESI [M+H]+: 635.4 |
| 63 | 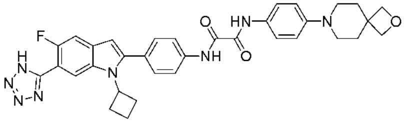 | C | 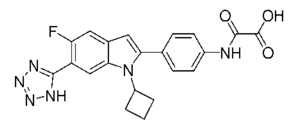 A10 + 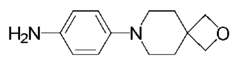 B41 | 1H NMR (400 MHz, DMSO-d6) δ 11.01 (s, 1H), 10.69 (s, 1H), 8.33 (d, J = 5.8 Hz, 1H), 8.05 (d, J = 8.7 Hz, 2H), 7.72 (d, J = 9.1 Hz, 2H), 7.57 (dd, J = 19.6, 10.0 Hz, 3H), 6.96 (d, J = 9.2 Hz, 2H), 6.57 (s, 1H), 5.16-5.03 (m, 1H), 4.34 (s, 4H), 3.11-3.04 (m, 4H), 2.78-2.64 (m, 2H), 2.46-2.34 (m, 2H), 1.93-1.73 (m, 6H). 19F NMR (376 MHz, DMSO-d6) δ-124.29. MS-ESI [M+H]+: 621.3 |
| 64 | 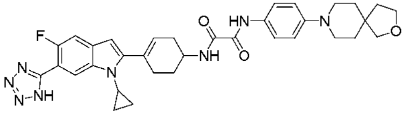 | D | 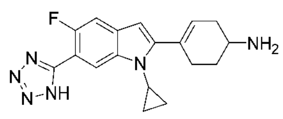 A6 + 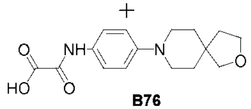 B76 | 1H NMR (400 MHz, DMSO-d6) δ 10.45 (s, 1H), 8.87 (d, J = 8.4 Hz, 1H), 8.13 (d, J = 5.9 Hz, 1H), 7.70-7.61 (m, 2H), 7.46 (d, J = 11.3 Hz, 1H), 6.96-6.88 (m, 2H), 6.41 (s, 1H), 6.16 (t, J = 3.7 Hz, 1H), 4.06-4.01 (m,, 1H), 3.76 (t, J = 7.1 Hz, 2H), 3.57-3.43 (m, 3H), 3.19-3.06 (m, 4H), 2.68-2.55 (m, 2H), 2.49-2.45 (m, 2H), 1.98-1.82 (m, 2H), 1.73 (t, J = 7.1 Hz, 2H), 1.65-1.58 (m, 4H), 1.23-1.17 (m, 2H), 0.87-0.80 (m, 2H)。 19F NMR (376 MHz, DMSO-d6) δ 124.57. MS-ESI [M+H]+: 625.4 |

(continued)

| Ex. | Structure | Syn. method | Raw material 1 | Characterization data |
|---|---|---|---|---|
| 65 | 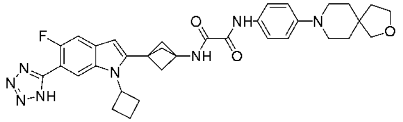 | D | 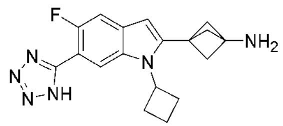 **A7** + 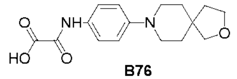 **B76** | 1H NMR (400 MHz, DMSO-d6) δ 10.41 (s, 1H), 9.69 (s, 1H), 8.35 (d, J = 5.8 Hz, 1H), 7.64 (d, J = 9.1 Hz, 2H), 7.46 (d, J = 11.2 Hz, 1H), 6.93 (d, J = 9.2 Hz, 2H), 6.29 (s, 1H), 5.26-5.17 (m, 1H), 3.76 (t, J = 7.1 Hz, 2H), 3.47 (s, 2H), 3.19-3.06 (m, 4H), 3.03-2.93 (m, 2H), 2.56 (s, 6H), 2.45-2.41 (m, 2H), 2.07-1.93 (m, 2H), 1.73 (t, J = 7.1 Hz, 2H), 1.62 (t, J = 6.0 Hz, 4H)。MS-ESI [M+H]+: 625.4 |
| 66 | 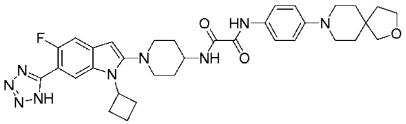 | D | 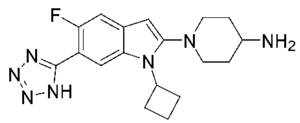 **A8** + 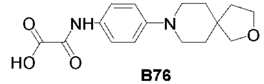 **B76** | 1H NMR (400 MHz, DMSO-d6) δ 10.43 (s, 1H), 8.91 (d, J = 8.4 Hz, 1H), 8.10 (d, J = 5.9 Hz, 1H), 7.66 (d, J= 9.1 Hz, 2H), 7.29 (d, J = 11.3 Hz, 1H), 6.93 (d, J = 9.2 Hz, 2H), 5.94 (s, 1H), 5.01-4.92 (m, 1H), 3.89-3.74 (m, 1H), 3.76 (t, J = 7.1 Hz, 2H), 3.47 (s, 2H), 3.23 (d, J = 12.0 Hz, 2H), 3.18-3.08 (m, 4H), 2.92-2.76 (m, 4H), 2.46-2.42 (m, 2H), 2.00-1.83 (m, 6H), 1.73 (t, J = 7.1 Hz, 2H), 1.62 (t, J = 5.9 Hz, 4H). MS-ESI [M+H]+: 642.4 |
| 67 | 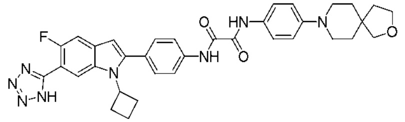 | C | 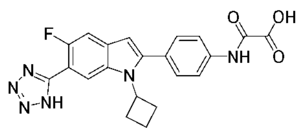 **A10** + 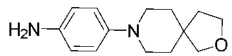 **B42** | 1H NMR (400 MHz, DMSO-d6) δ 11.03 (s, 1H), 10.70 (s, 1H), 8.34 (d, J = 5.6 Hz, 1H), 8.05 (d, J = 8.8 Hz, 2H), 7.72 (d, J = 9.2 Hz, 2H), 7.63 (d, J = 11.2 Hz, 1H), 7.55 (d, J= 8.8 Hz, 2H), 6.96 (d, J = 9.2 Hz, 2H), 6.59 (s, 1H), 5.15-5.00 (m, 1H), 3.80-3.70 (m, 2H), 3.48 (s, 2H), 3.25-3.05 (m, 4H), 2.75-2.60 (m, 2H), 2.45-2.30 (m, 2H), 1.92-1.78 (m, 2H), 1.75-1.70 (m, 2H), 1.65-1.58 (m, 4H)。MS-ESI $[M+H]^+$: 635.3 |

(continued)

| Ex. | Structure | Syn. method | Raw material 1 | Characterization data |
|---|---|---|---|---|
| 68 | 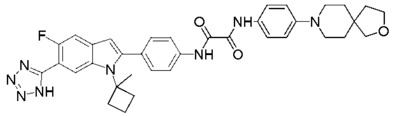 | C/ D | 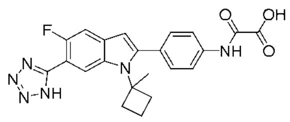 A13 + 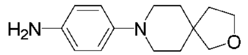 B42 or 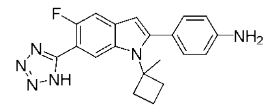 A4 + 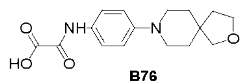 B76 | 1H NMR (400 MHz, DMSO-d6) δ 10.97 (s, 1H), 10.67 (s, 1H), 7.98 (d, J = 8.4 Hz, 2H), 7.94 (d, J = 6.0 Hz, 1H), 7.71 (d, J = 9.2 Hz, 2H), 7.55-7.45 (m, 3H), 6.95 (d, J = 8.8Hz, 2H), 6.46 (s, 1H), 3.80-3.70 (m, 2H), 3.47 (s, 2H), 3.25-3.05 (m, 4H), 2.30-2.17 (m, 2H), 2.12-2.00 (m, 2H), 1.97 (s, 3H), 1.90-1.70 (m, 2H), 1.70-1.72 (m, 2H), 1.65-1.55 (m, 4H). MS-ESI [M+H]+: 649.3 |
| 69 | 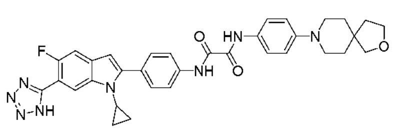 | D | 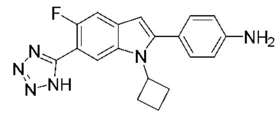 A1 + 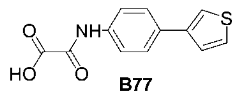 B77 | 1H NMR (400 MHz, DMSO-d6) δ 11.00 (s, 1H), 10.69 (s, 1H), 8.24 (d, J = 5.9 Hz, 1H), 8.08-8.00 (m, 2H), 7.82-7.77 (m, 2H), 7.75-7.69 (m, 2H), 7.57 (d, J = 11.3 Hz, 1H), 6.99-6.92 (m, 2H), 6.65 (s, 1H), 3.83-3.73 (m, 3H), 3.48 (s, 2H), 3.20-3.09 (m, 4H), 1.73 (t, J = 7.1 Hz, 2H), 1.62 (t, J = 6.0 Hz, 4H), 1.08 (dd, J = 7.4, 5.4 Hz, 2H), 0.63-0.52 (m, 2H)。19F NMR (376 MHz, DMSO-d6) δ -124.35. MS-ESI [M+H]+: 621.3 |
| 70 | 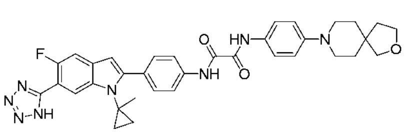 | C | 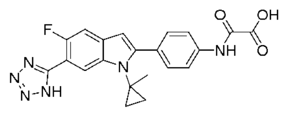 A12 + 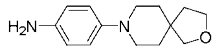 B42 | 1H NMR (400 MHz, DMSO-d6) δ 11.00 (s, 1H), 10.68 (s, 1H), 8.23 (d, J = 5.7 Hz, 1H), 8.05 (d, J = 8.8 Hz, 2H), 7.72 (d, J = 9.1 Hz, 2H), 7.68 (d, J = 8.8 Hz, 2H), 7.54 (d, J = 11.0 Hz, 1H), 6.96 (d, J = 9.3 Hz, 1H), 6.58 (s, 1H), 3.76 (t, J = 7.1 Hz, 2H), 3.48 (s, 2H), 3.22-3.09 (m, 4H), 1.82 (s, 3H), 1.74 (t, J = 7.1 Hz, 2H), 1.63 (t, J = 5.9 Hz, 4H), 1.18-1.11 (m, 1H), 0.94-0.84 (m, 2H), 0.73-0.64 (m, 1H). MS-ESI [M+H]+: 635.4 |

(continued)

| Ex. | Structure | Syn. method | Raw material 1 | Characterization data |
|---|---|---|---|---|
| 71 | 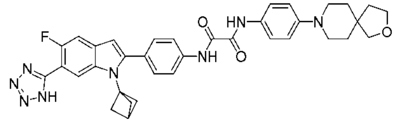 | D | 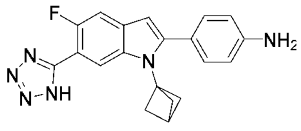 A5 + 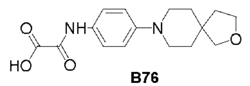 B76 | 1H NMR (400 MHz, DMSO-d6) δ 11.00 (s, 1H), 10.68 (s, 1H), 8.27 (d, J = 6.0 Hz, 1H), 8.00 (d, J = 8.0 Hz, 2H), 7.71 (d, J = 8.8 Hz, 2H), 7.60-7.50 (m, 3H), 6.96 (d, J = 8.8 Hz, 2H), 6.48 (s, 1H), 3.80-3.70 (m, 2H), 3.48 (s, 2H), 3.25-3.05 (m, 4H), 2.22 (s, 6H), 2.07 (s, 1H), 1.77-1.70 (m, 2H), 1.65-1.55 (m, 4H). MS-ESI [M+H]+: 647.3 |
| 72 | 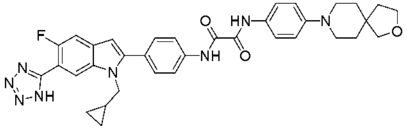 | C | 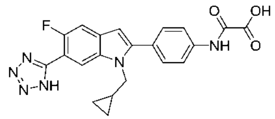 A14 + 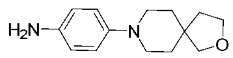 B42 | 1H NMR (400 MHz, DMSO-d6) δ 11.00 (s, 1H), 10.69 (s, 1H), 8.14 (d, J = 6.0 Hz, 1H), 8.04 (d, J = 8.8 Hz, 2H), 7.72 (d, J = 9.2 Hz, 2H), 7.61 (d, J = 8.8 Hz, 2H), 7.46 (d, J = 11.2 Hz, 1H), 6.96 (d, J = 9.2 Hz, 2H), 6.58 (s, 1H), 4.20 (d, J = 6.8 Hz, 2H), 3.76 (d, J = 7.2 Hz, 2H), 3.48 (s, 2H), 3.19-3.11 (m, 4H), 1.74 (d, J = 7.2 Hz, 2H), 1.65-1.59 (m, 4H), 0.98-0.90 (m, 1H), 0.35-0.29 (m, 2H), 0.09-0.04 (m, 2H). MS-ESI [M+H]+: 635.3 |
| 73 | 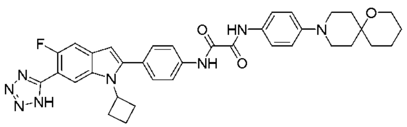 | C | 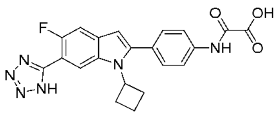 A10 + 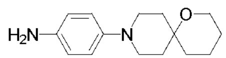 B43 | 1H NMR (400 MHz, DMSO-d6) δ 11.00 (s, 1H), 10.66 (s, 1H), 8.32 (d, J = 6.0 Hz, 1H), 8.03 (d, J = 8.4 Hz, 2H), 7.70 (d, J = 9.2 Hz, 2H), 7.60-7.50 (m, 3H), 6.94 (d, J = 9.2Hz, 2H), 6.55 (s, 1H), 5.12-5.00 (m, 1H), 3.60-3.54 (m, 2H), 3.35-3.25 (m, 2H), 3.02-2.92 (m, 2H), 2.80-2.64 (m, 2H), 2.45-2.34 (m, 2H), 1.93-1.74 (m, 4H), 1.63-1.38 (m, 8H). MS-ESI [M+H]+: 649.3 |
| 74 | 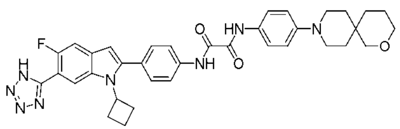 | C | 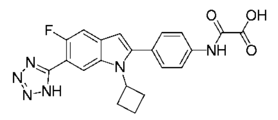 A10 + 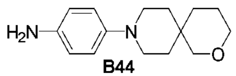 B44 | 1H NMR (400 MHz, DMSO-d6) δ 11.02 (s, 1H), 10.69 (s, 1H), 8.32 (d, J = 5.8 Hz, 1H), 8.05 (d, J = 8.3 Hz, 2H), 7.72 (d, J = 8.7 Hz, 2H), 7.60-7.51 (m, 3H), 6.95 (d, J = 8.8 Hz, 2H), 6.56 (s, 1H), 5.16-5.02 (m, 1H), 3.56-3.52 (m, 2H), 3.37 (s, 2H), 3.13 (t, J = 5.8 Hz, 4H), 2.77-2.67 (m, 2H), 2.44-2.36 (m, 2H), 1.93-1.75 (m, 2H), 1.56-1.49 (m, 8H). 19F NMR (376 MHz, DMSO-d6) δ-124.10. MS-ESI [M+H]+: 649.3 |

(continued)

| Ex. | Structure | Syn. method | Raw material 1 | Characterization data |
|---|---|---|---|---|
| 75 | 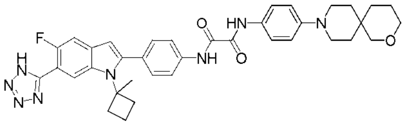 | C | 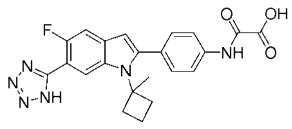 A13 + 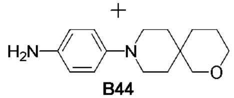 B44 | 1H NMR (400 MHz, DMSO-d6) δ 10.98 (s, 1H), 10.67 (s, 1H), 7.98 (dd, J = 10.7, 7.0 Hz, 2H), 7.71 (d, J = 8.6 Hz, 2H), 7.56 (dd, J = 27.8, 9.8 Hz, 3H), 6.94 (d, J = 8.7 Hz, 2H), 6.50 (s, 1H), 3.56-3.50 (m, 2H), 3.38-3.37 (m, 2H), 3.15-3.07 (m, 4H), 2.30-2.19 (m, 2H), 2.18-2.07 (m, 2H), 1.98 (s, 3H), 1.91-1.83 (m, 1H), 1.69-1.62 (m, 1H), 1.59-1.40 (m, 8H). 19F NMR (376 MHz, DMSO) δ -124.81. MS-ESI [M+H]+: 663.4 |
| 76 | 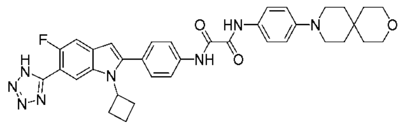 | C | 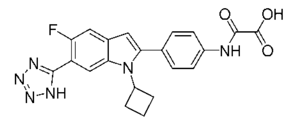 A10 + 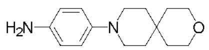 B45 | 1H NMR (400 MHz, DMSO-d6) δ 11.02 (s, 1H), 10.68 (s, 1H), 8.33 (d, J = 5.8 Hz, 1H), 8.05 (d, J = 8.7 Hz, 2H), 7.72 (d, J = 9.2 Hz, 2H), 7.57 (dd, J = 20.5, 9.9 Hz, 3H), 6.95 (d, J = 9.2 Hz, 2H), 6.57 (s, 1H), 5.15-5.05 (m, 1H), 3.60-3.56 (m, 4H), 3.16-3.12 (m, 4H), 2.76-2.65 (m, 2H), 2.44-2.36 (m, 2H), 1.93-1.76 (m, 2H), 1.63-1.58 (m, 4H), 1.48-1.43 (m, 4H). 19F NMR (376 MHz, DMSO-d6) δ -124.30. MS-ESI [M+H]+: 649.3 |
| 77 | 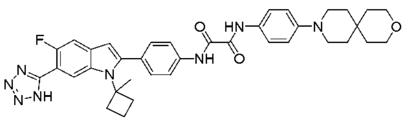 | C | 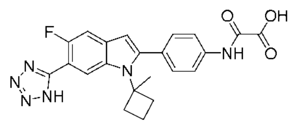 A13 + 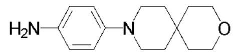 B45 | 1H NMR (400 MHz, DMSO-d6) δ 10.96 (s, 1H), 10.67 (s, 1H), 7.97 (d, J = 8.4 Hz, 2H), 7.88 (d, J = 6.0 Hz, 1H), 7.71 (d, J = 8.4 Hz, 2H), 7.50 (d, J = 8.4 Hz, 2H), 7.41 (d, J = 11.2 Hz, 1H), 6.94 (d, J = 8.8 Hz, 2H), 6.39 (s, 1H), 3.57 (t, J = 5.6 Hz, 4H), 3.13 (t, J = 5.6 Hz, 4H), 2.26-2.18 (m, 2H), 2.11-2.05 (m, 2H), 1.96 (s, 3H), 1.89-1.84 (m, 1H), 1.65-1.62 (m, 1H), 1.59 (t, J = 5.6 Hz, 4H), 1.44 (t, J = 5.6 Hz, 4H). MS-ESI [M+H]+: 663.4 |
| 78 | 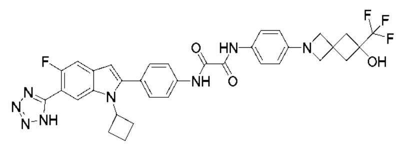 | C | 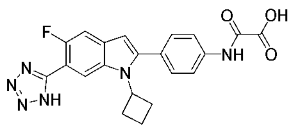 A10 + 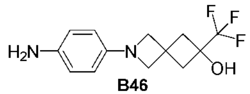 B46 | 1H NMR (400 MHz, DMSO-d6) δ 11.01 (s, 1H), 10.64 (s, 1H), 8.33 (d, J = 5.8 Hz, 1H), 8.04 (d, J = 8.5 Hz, 2H), 7.68 (d, J = 8.5 Hz, 2H), 7.61-7.54 (m, 3H), 6.58 (s, 1H), 6.54 (s, 1H), 6.48-6.39 (m, 2H), 5.14-5.05 (m, 1H), 3.85 (s, 2H), 3.80 (s, 2H), 2.76-2.61 (m, 4H), 2.45-2.31 (m, 4H), 1.95-1.67 (m, 2H)。19F NMR (376 MHz, DMSO-d6) δ -83.12, -124.32. MS-ESI [M+H]+: 675.3 |

(continued)

| Ex. | Structure | Syn. method | Raw material 1 | Characterization data |
|---|---|---|---|---|
| 79 | 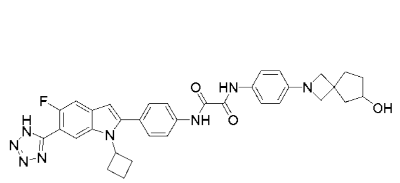 | C | 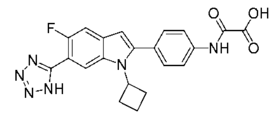 A10 + 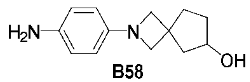 B58 | 1H NMR (400 MHz, DMSO-d6) δ 11.01 (s, 1H), 10.63 (s, 1H), 8.33 (d, J = 5.8 Hz, 1H), 8.05 (d, J = 8.4 Hz, 2H), 7.68 (d, J = 8.6 Hz, 2H), 7.57 (dd, J = 21.5, 9.8 Hz, 3H), 6.57 (s, 1H), 6.42 (d, J = 8.7 Hz, 2H), 5.15-5.04 (m, 1H), 4.52 (s, 1H), 4.18-4.12 (m, 1H), 3.77-3.74 (m, 1H), 3.70-3.62 (m, 3H), 2.76-2.66 (m, 2H), 2.45-2.36 (m, 2H), 2.05-1.95 (m, 2H), 1.93-1.75 (m, 5H), 1.53 (td, J = 11.4, 10.3, 6.0 Hz, 1H). 19F NMR (376 MHz, DMSO-d6) δ -124.31. MS-ESI [M+H]+: 621.3 |
| 80 | 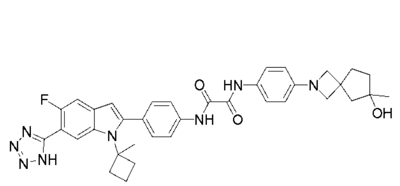 | C | 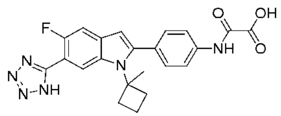 A13 + 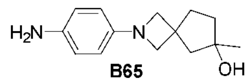 B65 | 1H NMR (400 MHz, DMSO-d6) δ 10.97 (s, 1H), 10.62 (s, 1H), 7.99 (d, J = 8.4 Hz, 2H), 7.95 (d, J = 6.0 Hz, 1H), 7.66 (d, J = 8.4 Hz, 2H), 7.58-7.49 (m, 3H), 6.47 (s, 1H), 6.41 (d, J = 8.8 Hz, 2H), 4.29 (s, 1H), 3.76 (d, J = 7.2 Hz, 1H), 3.70-3.63 (m, 3H), 2.29-2.19 (m, 2H), 2.15-2.05 (m, 3H), 2.00-1.95 (m, 3H), 1.94-1.83 (m, 3H), 1.78 (d, J = 13.2 Hz, 1H), 1.70-1.54 (m, 3H), 1.23 (s, 3H). MS-ESI [M+H]+: 649.7 |
| 81 | 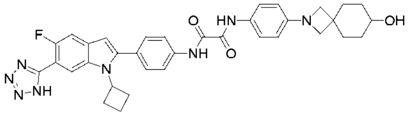 | C | 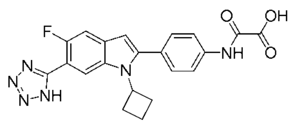 A10 + 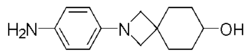 B60 | 1H NMR (400 MHz, DMSO-d6) δ 10.99 (s, 1H), 10.62 (s, 1H), 8.31 (d, J = 6.0 Hz, 1H), 8.04 (d, J = 8.8 Hz, 2H), 7.67 (d, J = 8.8 Hz, 2H), 7.56-7.50 (m, 3H), 6.54 (s, 1H), 6.42 (d, J = 8.8 Hz, 2H), 5.12-5.03 (m,1H), 4.50 (s, 1H), 3.52 (s, 2H), 3.48 (s, 2H), 3.46-3.42 (m, 1H), 2.76-2.67 (m, 2H), 2.43-2.35 (m, 2H), 1.91-1.77 (m, 4H), 1.72-1.65 (m, 2H), 1.54-1.46 (m, 2H), 1.28-1.22 (m, 2H). MS-ESI [M+H]+: 635.4 |
| 82 | 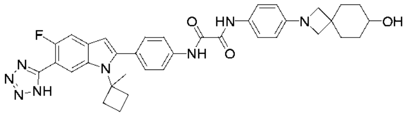 | C | 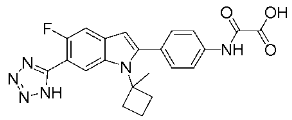 A13 + 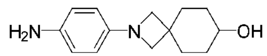 B60 | 1H NMR (400 MHz, DMSO-d6) δ 10.96 (s, 1H), 10.60 (s, 1H), 7.99 (d, J = 8.8 Hz, 2H), 7.95 (d, J = 6.0 Hz, 1H), 7.66 (d, J = 8.8 Hz, 2H), 7.57-7.49 (m, 3H), 6.47 (s, 1H), 6.42 (d, J = 8.8 Hz, 2H), 4.50 (s, 1H), 3.52 (s, 2H), 3.49-3.44 (m, 3H), 2.28-2.18 (m, 2H), 2.16-2.05 (m, 2H), 1.98 (s, 3H), 1.90-1.81 (m, 3H), 1.72-1.61 (m, 3H), 1.54-1.45 (m, 2H), 1.30-1.21 (m, 2H). MS-ESI [M+H]+: 649.4 |

(continued)

| Ex. | Structure | Syn. method | Raw material 1 | Characterization data |
|---|---|---|---|---|
| 83 | 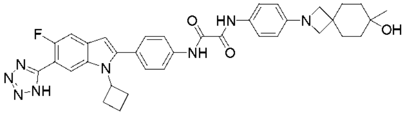 | C | 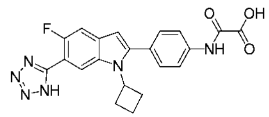 A10 + 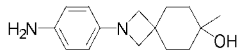 B66 | 1H NMR (400 MHz, DMSO-d6) δ 11.00 (s, 1H), 10.62 (s, 1H), 8.33 (d, J = 6.0 Hz, 1H), 8.04 (d, J = 8.8 Hz, 2H), 7.67 (d, J = 8.8 Hz, 2H), 7.59 (d, J = 11.2 Hz, 1H), 7.54 (d, J = 8.4Hz, 2H), 6.57 (s, 1H), 6.41 (d, J = 8.8 Hz, 2H), 5.14-5.04 (m, 1H), 4.11 (brs, 1H), 3.51 (s, 2H), 3.48 (s, 2H), 2.74-2.65 (m, 2H), 2.43-2.32 (m, 2H), 1.90-1.75 (m, 4H), 1.62-1.58 (m, 2H), 1.48-1.43(m, 2H), 1.39-1.32 (m, 2H), 1.10 (s, 3H). MS-ESI [M+H]+: 649.4 |
| 84 | 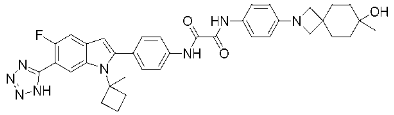 | C | 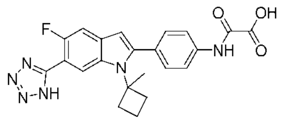 A13 + 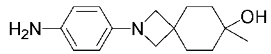 B66 | 1H NMR (400 MHz, DMSO-d6) δ 10.95 (s, 1H), 10.60 (s, 1H), 7.98 (d, J = 8.8 Hz, 2H), 7.94 (d, J = 6.0 Hz, 1H), 7.66 (d, J = 8.8 Hz, 2H), 7.56-7.49 (m, 3H), 6.46 (s, 1H), 6.41 (d, J = 8.8 Hz, 2H), 4.11 (s, 1H), 3.50 (s, 2H), 3.48 (s, 2H), 2.28-2.19 (m, 2H), 2.15-2.06 (m, 2H), 1.98 (s, 3H), 1.91-1.78 (m, 3H), 1.69-1.56 (m, 3H), 1.50-1.41 (m, 2H), 1.40-1.30 (m, 2H), 1.10 (s, 3H). MS-ESI [M+H]+: 663.4 |
| 85 | 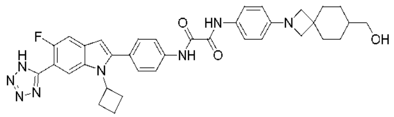 | C | 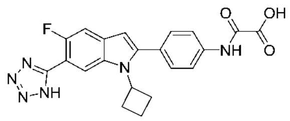 A10 + 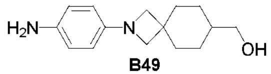 B49 | 1H NMR (400 MHz, DMSO-d6) δ 11.00 (s, 1H), 10.62 (s, 1H), 8.32 (d, J = 5.8 Hz, 1H), 8.04 (d, J = 8.6 Hz, 2H), 7.67 (d, J = 9.0 Hz, 2H), 7.58-7.51 (m, 3H), 6.54 (s, 1H), 6.42 (d, J = 9.0 Hz, 2H), 5.14-5.03 (m, 1H), 4.40 (s, 1H), 3.49 (d, J = 18.3 Hz, 4H), 3.22 (d, J = 6.2 Hz, 2H), 2.78-2.68 (m, 2H), 2.44-2.36 (m, 2H), 1.92-1.76 (m, 4H), 1.70-1.62 (m, 2H), 1.50-1.41 (m, 2H), 1.37-1.29 (m, 1H), 1.02-0.91 (m, 2H). 19F NMR (376 MHz, DMSO-d6) δ -123.98. MS-ESI [M+H]+: 649.3 |
| 86 | 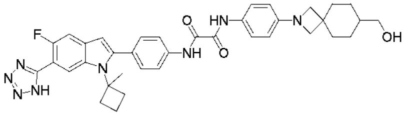 | C | 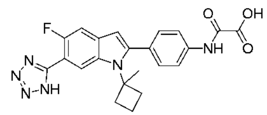 A13 + 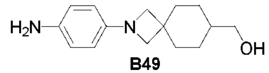 B49 | 1H NMR (400 MHz, DMSO-d6) δ 10.97 (s, 1H), 10.60 (s, 1H), 7.98 (dd, J = 11.8, 7.2 Hz, 3H), 7.66 (d, J = 8.9 Hz, 2H), 7.58 (d, J = 11.3 Hz, 1H), 7.52 (d, J = 8.6 Hz, 2H), 6.49 (s, 1H), 6.42 (d, J = 8.9 Hz, 2H), 4.40 (s, 1H), 3.49 (d, J = 18.4 Hz, 4H), 3.22 (d, J = 6.2 Hz, 2H), 2.29-2.19 (m, 2H), 2.17-2.05 (m, 2H), 1.98 (s, 3H), 1.92-1.83 (m, 3H), 1.70-1.61 (m, 3H), 1.50-1.41 (m, 2H), 1.37-1.29 (m, 1H), 1.02-0.91 (m, 2H). 19F NMR (376 MHz, DMSO-d6) δ -124.72. MS-ESI [M+H]+: 663.4 |

(continued)

| Ex. | Structure | Syn. method | Raw material 1 | Characterization data |
|---|---|---|---|---|
| 87 | 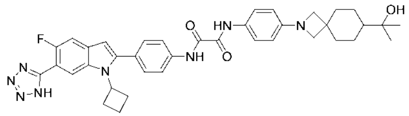 | C | 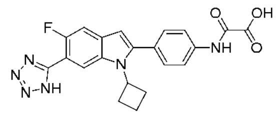 A10 + 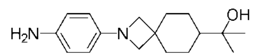 B73 | 1H NMR (400 MHz, DMSO-d6) δ 11.00 (s, 1H), 10.62 (s, 1H), 8.32 (d, J = 5.8 Hz, 1H), 8.04 (d, J = 8.2 Hz, 2H), 7.67 (d, J = 8.7 Hz, 2H), 7.57-7.53 (m, 3H), 6.55 (s, 1H), 6.42 (d, J = 8.5 Hz, 2H), 5.13-5.04 (m, 1H), 4.04 (s, 1H), 3.52 (s, 2H), 3.45 (s, 2H), 2.75-2.67 (m, 2H), 2.43-2.34 (m, 2H), 1.95-1.71 (m, 6H), 1.45-1.39 (m, 2H), 1.27-1.13 (m, 2H), 1.03 (s, 6H), 0.97-0.84 (m, 1H). MS-ESI [M+H]+: 677.4 |
| 88 | 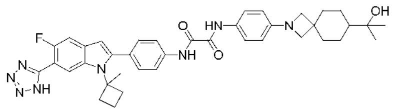 | C | 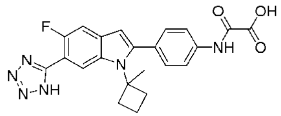 A13 + 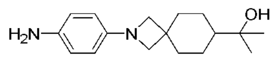 B73 | 1H NMR (400 MHz, DMSO-d6) δ 10.97 (s, 1H), 10.60 (s, 1H), 7.99 (d, J = 8.8 Hz, 2H), 7.96 (d, J = 5.7 Hz, 1H), 7.66 (d, J = 9.0 Hz, 2H), 7.57 (d, J = 11.3 Hz, 1H), 7.52 (d, J = 8.7 Hz, 2H), 6.48 (s, 1H), 6.42 (d, J = 9.0 Hz, 2H), 4.04 (s, 1H), 3.52 (s, 2H), 3.45 (s, 2H), 2.28-2.20 (m, 2H), 2.14-2.10 (m, 2H), 1.98 (s, 3H), 1.92-1.84 (m, 3H), 1.74-1.63 (m, 3H), 1.45-1.39 (m, 2H), 1.19-1.13 (m, 2H), 1.03 (s, 6H), 1.00-0.97 (m, 1H). MS-ESI [M+H]+: 691.4 |
| 89 | 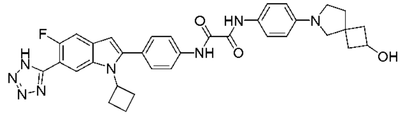 | C | 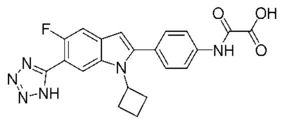 A10 + 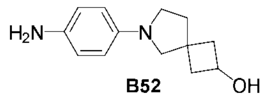 B52 | 1H NMR (400 MHz, DMSO-d6) δ 10.99 (s, 1H), 10.59 (s, 1H), 8.30 (d, J = 5.9 Hz, 1H), 8.04 (d, J = 8.7 Hz, 2H), 7.68 (d, J = 8.6 Hz, 2H), 7.52 (t, J = 10.1 Hz, 3H), 6.55-6.45 (m, 3H), 5.20-4.88 (m, 2H), 4.20-4.09 (m, 1H), 3.26-3.16 (m, 4H), 2.81-2.66 (m, 2H), 2.45-2.35 (m, 2H), 2.30-2.18 (m, 2H), 1.97-1.76 (m, 6H). 19F NMR (376 MHz, DMSO-d6) δ -123.76. MS-ESI [M+H]+: 621.4 |
| 90 | 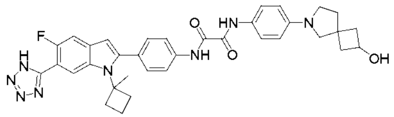 | C | 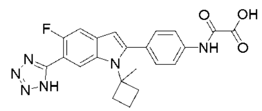 A13 + 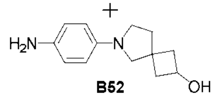 B52 | 1H NMR (400 MHz, DMSO-d6) δ 10.94 (s, 1H), 10.57 (s, 1H), 7.98 (d, J = 8.2 Hz, 2H), 7.87 (d, J = 5.9 Hz, 1H), 7.67 (d, J = 8.5 Hz, 2H), 7.50 (d, J = 8.2 Hz, 2H), 7.40 (d, J = 11.1 Hz, 1H), 6.55-6.45 (m, 2H), 6.39 (s, 1H), 5.05 (s, 1H), 4.20-4.11 (m, 1H), 3.58-3.52 (m, 1H), 3.21-3.18 (m, 2H), 3.09-3.02 (m, 1H), 2.30-2.16 (m, 4H), 2.14-2.02 (m, 2H), 2.01-1.77 (m, 8H), 1.69-1.59 (m, 1H). 19F NMR (376 MHz, DMSO-d6) δ -123.81. MS-ESI [M+H]+: 635.3 |

(continued)

| Ex. | Structure | Syn. method | Raw material 1 | Characterization data |
|---|---|---|---|---|
| 91 | 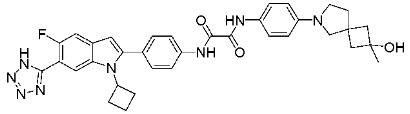 | C | 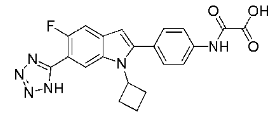 A10 + 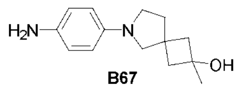 B67 | 1H NMR (400 MHz, DMSO-d6) δ 10.99 (s, 1H), 10.59 (s, 1H), 8.31 (d, J = 5.8 Hz, 1H), 8.04 (d, J = 8.7 Hz, 2H), 7.68 (d, J = 9.0 Hz, 2H), 7.57-7.50 (m, 3H), 6.56-6.48 (m, 3H), 5.14-5.01 (m, 1H), 4.88 (s, 1H), 3.22 (t, J = 6.8 Hz, 4H), 2.80-2.68 (m, 2H), 2.45-2.34 (m, 2H), 2.11-1.98 (m, 6H), 1.93-1.76 (m, 2H), 1.30 (s, 3H). 19F NMR (376 MHz, DMSO-d6) δ -123.91. MS-ESI [M+H]+: 635.3 |
| 92 | 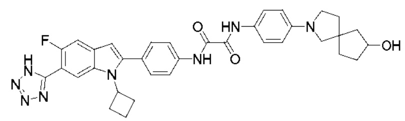 | C | 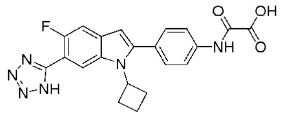 A10 + 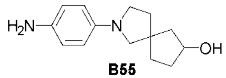 B55 | 1H NMR (400 MHz, DMSO-d6) δ 11.00 (s, 1H), 10.59 (s, 1H), 8.33 (d, J = 5.8 Hz, 1H), 8.05 (d, J = 8.7 Hz, 2H), 7.67 (d, J = 8.7 Hz, 2H), 7.60-7.51 (m, 3H), 6.56 (s, 1H), 6.49 (d, J = 9.2 Hz, 2H), 5.15-5.03 (m, 1H), 4.56 (s, 1H), 4.24-4.17 (m, 1H), 3.30-3.25 (m, 2H), 3.12-3.02 (m, 2H), 2.79-2.66 (m, 2H), 2.44-2.36 (m, 2H), 1.99-1.72 (m, 7H), 1.61-1.50 (m, 3H). 19F NMR (376 MHz, DMSO-d6) δ -124.11. MS-ESI [M+H]+: 635.4 |
| 93 | 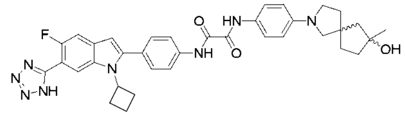 | C | 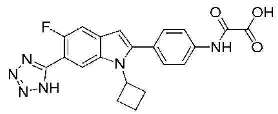 A10 + 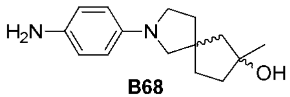 B68 | 1H NMR (400 MHz, DMSO-d6) δ 10.97 (s, 1H), 10.58 (s, 1H), 8.29 (d, J = 5.9 Hz, 1H), 8.03 (d, J = 8.7 Hz, 2H), 7.67 (d, J = 9.0 Hz, 2H), 7.53 (d, J = 8.6 Hz, 2H), 7.47 (d, J = 11.1 Hz, 1H), 6.51-6.47 (m, 3H), 5.11-5.02 (m, 1H), 4.29 (s, 1H), 3.27-3.18 (m, 4H), 2.80-2.70 (m, 2H), 2.42-2.36 (m, 2H), 1.91-1.57 (m, 10H), 1.26 (s, 3H). MS-ESI [M+H]+: 649.3 |
| 94 | 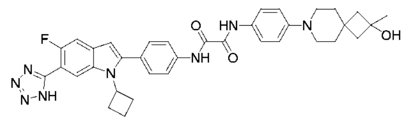 | C | 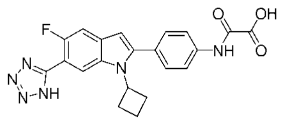 A10 + 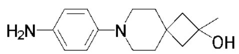 B47 | 1H NMR (400 MHz, DMSO-d6) δ 11.01 (s, 1H), 10.67 (s, 1H), 8.33 (d, J = 5.8 Hz, 1H), 8.09-7.99 (m, 2H), 7.73-7.66 (m, 2H), 7.64-7.51 (m, 3H), 6.98-6.86 (m, 2H), 6.57 (s, 1H), 5.11-5.07 (m, 1H), 4.75 (s, 1H), 3.05-3.03 (m, 4H), 2.76-2.62 (m, 2H), 2.43-2.34 (m, 2H), 1.93-1.73 (m, 6H), 1.68-1.61 (m, 2H), 1.60-1.58 (m, 2H), 1.25 (s, 3H). 19F NMR (376 MHz, DMSO-d6) δ 124.37. MS-ESI [M+H]+: 649.4 |

(continued)

| Ex. | Structure | Syn. method | Raw material 1 | Characterization data |
|---|---|---|---|---|
| 95 | 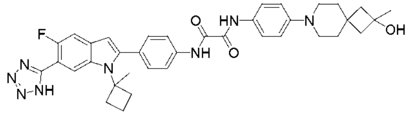 | C | 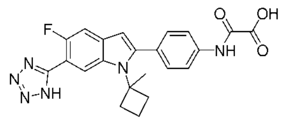 A13 + 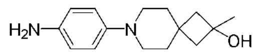 B47 | 1H NMR (400 MHz, DMSO-d6) δ 10.99 (s, 1H), 10.67 (s, 1H), 8.04-7.92 (m, 3H), 7.74-7.65 (m, 2H), 7.59-7.51 (m, 3H), 7.00-6.85 (m, 2H), 6.49 (s, 1H), 4.77 (s, 1H), 3.06-3.03 (m, 4H), 2.25-2.20 (m, 2H), 2.12 (s, 2H), 1.98 (s, 3H), 1.91-1.79 (m, 5H), 1.71-1.59 (m, 5H), 1.26 (s, 3H)。19F NMR (376 MHz, DMSO-d6) δ 124.69. MS-ESI [M+H]+: 663.4 |
| 96 | 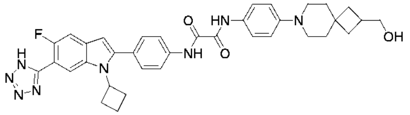 | C | 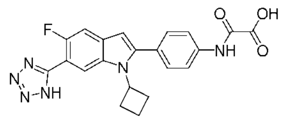 A10 + 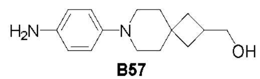 B57 | 1H NMR (400 MHz, DMSO-d6) δ 11.00 (s, 1H), 10.68 (s, 1H), 8.29 (d, J = 5.9 Hz, 1H), 8.04 (d, J = 8.6 Hz, 2H), 7.71 (d, J = 8.9 Hz, 2H), 7.51 (dd, J = 20.2, 9.8 Hz, 3H), 6.94 (d, J = 9.0 Hz, 2H), 6.51 (s, 1H), 5.12-5.04 (m, 1H), 4.45 (s, 1H), 3.38-3.36 (m, 2H), 3.13-3.07 (m, 2H), 3.05-2.98 (m, 2H), 2.79-2.68 (m, 2H), 2.43-2.32 (m, 3H), 1.93-1.76 (m, 4H), 1.69-1.62 (m, 2H), 1.58-1.46 (m, 4H). 19F NMR (376 MHz, DMSO-d6) δ -123.61. MS-ESI [M+H]+: 649.3 |
| 97 | 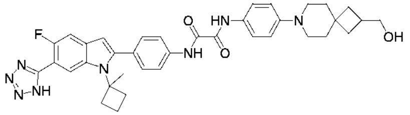 | C | 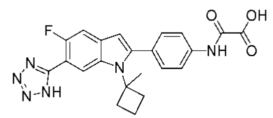 A13 + 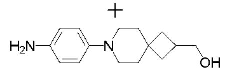 B57 | 1H NMR (400 MHz, DMSO-d6) δ 10.67 (s, 2H), 7.97 (d, J = 8.6 Hz, 2H), 7.84 (d, J = 6.0 Hz, 1H), 7.70 (d, J = 9.1 Hz, 2H), 7.50 (d, J = 8.6 Hz, 2H), 7.31 (d, J = 11.1 Hz, 1H), 6.94 (d, J = 9.1 Hz, 2H), 6.34 (s, 1H), 4.48 (s, 1H), 3.38-3.36 (m, 2H), 3.13-3.07 (m, 2H), 3.04-2.98 (m, 2H), 2.39-2.30 (m, 1H), 2.28-2.17 (m, 2H), 2.15-2.01 (m, 2H), 1.96 (s, 3H), 1.89-1.77 (m, 3H), 1.69-1.59 (m, 3H), 1.58-1.46 (m, 4H). 19F NMR (376 MHz, DMSO-d6) δ-123.42. MS-ESI [M+H]+: 663.4 |
| 98 | 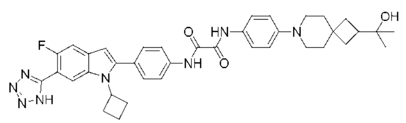 | C | 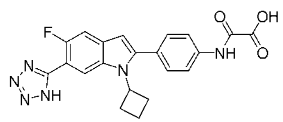 A10 + 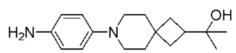 B74 | 1H NMR (400 MHz, DMSO-d6) δ 11.01 (s, 1H), 10.68 (s, 1H), 8.32 (d, J = 5.8 Hz, 1H), 8.04 (d, J = 8.7 Hz, 2H), 7.71 (d, J = 9.0 Hz, 2H), 7.58-7.53 (m, 3H), 6.94 (d, J = 9.1 Hz, 2H), 6.56 (s, 1H), 5.13-5.04 (m, 1H), 4.05 (s, 1H), 3.12 (t, J = 5.6 Hz, 2H), 3.01 (t, J = 5.7 Hz, 2H), 2.74-2.67 (m, 2H), 2.43-2.37 (m, 2H), 2.25-2.16 (m, 1H), 1.91-1.79 (m, 2H), 1.73-1.60 (m, 6H), 1.51 (t, J = 5.6 Hz, 2H), 0.98 (s, 6H). MS-ESI [M+H]+: 677.4 |

(continued)

| Ex. | Structure | Syn. method | Raw material 1 | Characterization data |
|---|---|---|---|---|
| 99 | 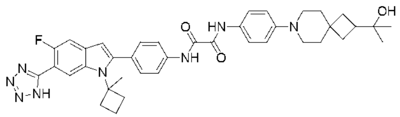 | C | 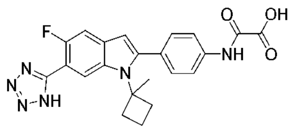 A13 + 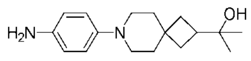 B74 | 1H NMR (400 MHz, DMSO-d6) δ 10.98 (s, 1H), 10.66 (s, 1H), 7.99 (d, J = 8.8 Hz, 2H), 7.96 (d, J = 5.8 Hz, 1H), 7.70 (d, J = 9.1 Hz, 2H), 7.56 (d, J = 11.3 Hz, 1H), 7.52 (d, J = 8.7 Hz, 2H), 6.94 (d, J = 9.2 Hz, 2H), 6.48 (s, 1H), 4.05 (s, 1H), 3.11 (t, J = 5.6 Hz, 2H), 3.01 (t, J = 5.6 Hz, 2H), 2.25-2.18 (m, 3H), 2.13-2.09 (m, 2H), 1.98 (s, 3H), 1.91-1.82 (m, 1H), 1.73-1.60 (m, 7H), 1.51 (t, J = 5.6 Hz, 2H), 0.98 (s, 6H). MS-ESI [M+H]+: 691.4 |
| 100 | 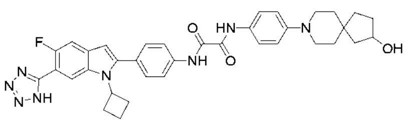 | C | 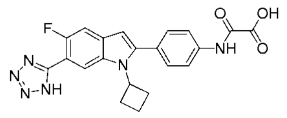 A10 + 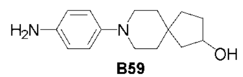 B59 | 1H NMR (400 MHz, DMSO-d6) δ11.00 (s, 1H), 10.68 (s, 1H), 8.28 (d, J = 6.0 Hz,1H), 8.03 (d, J = 8.8 Hz, 2H), 7.71 (d, J = 8.8 Hz, 2H), 7.52 (d, J = 8.4 Hz, 2H), 7.44 (d, J = 11.2Hz, 1H), 6.94 (d, J = 8.8 Hz, 2H), 6.49 (s, 1H), 5.08-5.03 (m, 1H), 4.46 (brs, 1H), 4.16-4.13 (m, 1H), 3.15-3.05 (m, 4H), 2.78-2.72 (m, 2H), 2.40-2.32 (m, 2H), 1.91-1.75 (m, 3H), 1.72-1.51 (m, 5H), 1.50-1.36 (m, 4H). MS-ESI [M+H]+: 649.3 |
| 101 | 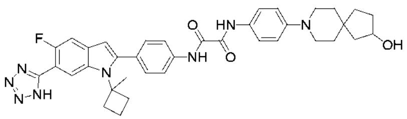 | C | 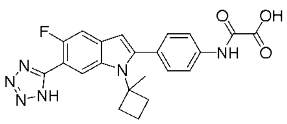 A13 + 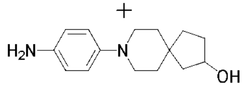 B59 | 1H NMR (400 MHz, DMSO-d6) δ 10.98 (s, 1H), 10.66 (s, 1H), 7.99 (d, J = 8.8 Hz, 2H), 7.95 (d, J = 6.0 Hz, 1H), 7.70 (d, J = 8.8 Hz, 2H), 7.56 (d, J = 11.2 Hz, 1H), 7.52 (d, J = 8.8 Hz, 2H), 6.94 (d, J = 9.2 Hz, 2H), 6.48 (s, 1H), 4.46 (brs, 1H), 4.17-4.11 (m, 1H), 3.18-3.04 (m, 4H), 2.25-2.19 (m, 2H), 2.15-2.05 (m, 2H), 1.98 (s, 3H), 1.71-1.35 (m, 12H). MS-ESI [M+H]+: 663.4 |
| 102 | 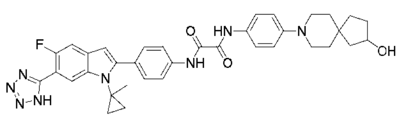 | C | 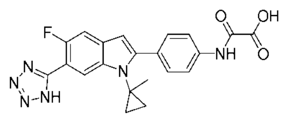 A12 + 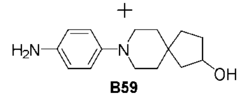 B59 | 1H NMR (400 MHz, DMSO-d6) δ 10.98 (s, 1H), 10.67 (s, 1H), 8.16 (d, J = 6.0 Hz, 1H), 8.04 (d, J = 8.8 Hz, 2H), 7.71 (d, J = 9.1 Hz, 2H), 7.66 (d, J = 8.7 Hz, 2H), 7.42 (d, J = 11.1 Hz, 1H), 6.94 (d, J = 9.2 Hz, 2H), 6.52 (s, 1H), 4.46 (s, 1H), 4.16-4.14 (m, 1H), 3.18-3.05 (m, 4H), 1.81 (s, 3H), 1.79-1.75 (m, 1H), 1.72-1.52 (m, 5H), 1.51-1.45 (m, 2H), 1.44-1.36 (m, 2H), 1.16-1.06 (m, 1H), 0.94-0.80 (m, 2H), 0.73-0.61 (m, 1H). MS-ESI [M+H]+: 649.7 |

(continued)

| Ex. | Structure | Syn. method | Raw material 1 | Characterization data |
|---|---|---|---|---|
| 103 | 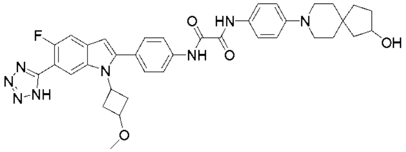 | C | 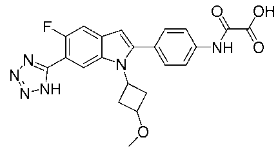 A11 + 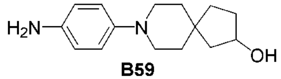 B59 | 1H NMR (400 MHz, DMSO-d6) δ 11.01 (s, 1H), 10.68 (s, 1H), 8.34 (d, J = 6.0 Hz, 0.62H), 8.19 (d, J = 5.6 Hz, 0.31H), 8.04 (d, J = 8.4 Hz, 2H), 7.70 (d, J = 8.8 Hz, 2H), 7.56-7.52 (m, 3H), 6.94 (d, J = 8.8 Hz, 2H), 6.56 (s, 0.31H), 6.55 (s, 0.63H), 5.30-5.25 (m, 0.25H), 4.75-4.75 (m, 0.63H), 4.46 (brs, 1H), 4.14-4.10 (m, 1H), 4.10-4.08 (m, 0.39H), 3.75-3.71 (m, 0.67H), 3.19 (s, 1H), 3.18 (s, 2H), 3.15-3.04 (m, 4H), 2.90-2.70 (m, 2H), 1.81-1.35 (m, 12H). MS-ESI [M+H]+: 679.4 |
| 104 | 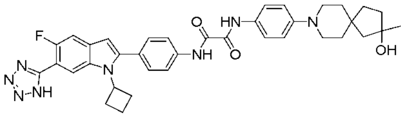 | C | 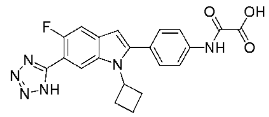 A10 + 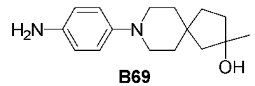 B69 | 1H NMR (400 MHz, DMSO-d6) δ 10.98 (s, 1H), 10.67 (s, 1H), 8.26 (d, J = 6.0 Hz, 1H), 8.02 (d, J = 8.4 Hz, 2H), 7.71 (d, J = 8.8 Hz, 2H), 7.52 (d, J = 8.4 Hz, 2H), 7.38 (d, J = 11.2 Hz, 1H), 6.94 (d, J = 9.2 Hz, 2H), 6.46 (s, 1H), 5.08-4.99 (m, 1H), 4.20 (s, 1H), 3.19-3.13 (m, 2H), 3.07-2.98 (m, 2H), 2.81-2.71 (m, 2H), 2.43-2.33 (m, 2H), 1.92-1.74 (m, 2H), 1.70-1.54 (m, 6H), 1.54-1.46 (m, 3H), 1.44-1.37 (m, 1H), 1.23 (s, 3H), MS-ESI [M+H]+: 663.7 |
| 105 | 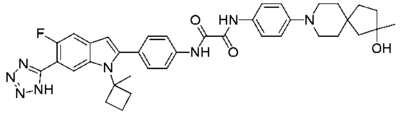 | C | 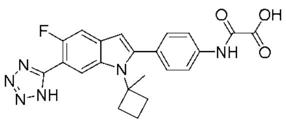 A13 + 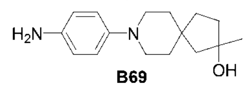 B69 | 1H NMR (400 MHz, DMSO-d6) δ 10.96 (s, 1H), 10.65 (s, 1H), 7.98 (d, J = 8.8 Hz, 2H), 7.91 (d, J = 6.0 Hz, 1H), 7.70 (d, J = 9.2 Hz, 2H), 7.53-7.45 (m, 3H), 6.93 (d, J = 9.2 Hz, 2H), 6.43 (s, 1H), 4.19 (s, 1H), 3.18-3.13 (m, 2H), 3.07-2.99 (m, 2H), 2.27-2.19 (m, 2H), 2.14-2.05 (m, 2H), 1.97 (s, 3H), 1.91-1.83 (m, 1H), 1.69-1.60 (m, 6H), 1.59-1.54 (m, 1H), 1.54-1.46 (m, 3H), 1.43-1.38 (m, 1H), 1.23 (s, 3H). MS-ESI [M+H]+: 677.7 |
| 106 | 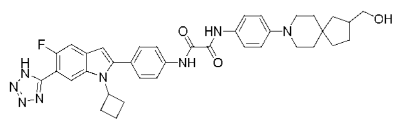 | C | 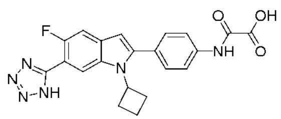 A10 + 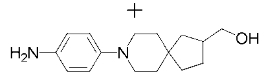 B61 | 1H NMR (400 MHz, DMSO-d6) δ 11.02 (s, 1H), 10.68 (s, 1H), 8.34 (d, J = 5.8 Hz, 1H), 8.05 (d, J = 8.7 Hz, 2H), 7.71 (d, J = 9.1 Hz, 2H), 7.63 (d, J = 11.3 Hz, 1H), 7.55 (d, J = 8.7 Hz, 2H), 6.94 (d, J = 9.2 Hz, 2H), 6.59 (s, 1H), 5.16-5.05 (m, 1H), 4.39 (d, J = 6.9 Hz, 1H), 3.31-3.30 (m, 2H), 3.16-3.06 (m, 4H), 2.76-2.64 (m, 2H), 2.44-2.37 (m, 2H), 2.14-2.06 (m, 1H), 1.93-1.78 (m, 2H), 1.74-1.62 (m, 2H), 1.57-1.49 (m, 4H), 1.48-1.43 (m, 2H), 1.39-1.30 (m, 1H), 1.10-1.03 (m, 1H). 19F NMR (376 MHz, DMSO-d6) δ -124.51. MS-ESI [M+H]+: 663.3 |

(continued)

| Ex. | Structure | Syn. method | Raw material 1 | Characterization data |
|---|---|---|---|---|
| 107 | 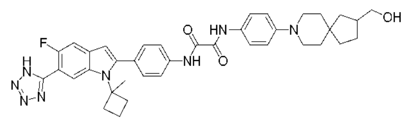 | C | 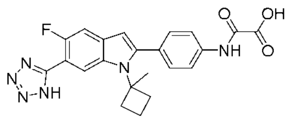 A13 + 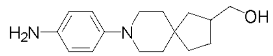 B61 | 1H NMR (400 MHz, DMSO-d6) δ 10.98 (s, 1H), 10.66 (s, 1H), 7.97 (dd, J = 17.3, 7.2 Hz, 3H), 7.71 (d, J = 9.2 Hz, 2H), 7.58-7.48 (m, 3H), 6.94 (d, J = 9.3 Hz, 2H), 6.47 (s, 1H), 4.44 (s, 1H), 3.31 (d, J = 6.6 Hz, 2H), 3.16-3.07 (m, 4H), 2.29-2.19 (m, 2H), 2.17-2.05 (m, 3H), 1.98 (s, 3H), 1.92-1.83 (m, 1H), 1.76-1.62 (m, 3H), 1.57-1.49 (m, 4H), 1.48-1.43 (m, 2H), 1.38-1.30 (m, 1H), 1.09-1.04 (m, 1H). 19F NMR (376 MHz, DMSO-d6) δ -124.56. MS-ESI [M+H]+: 677.3 |
| 108 | 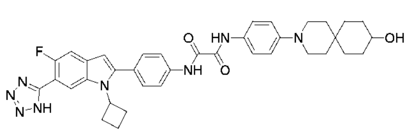 | C | 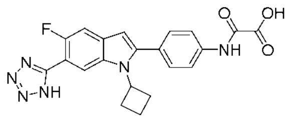 A10 + 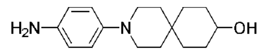 B50 | 1H NMR (400 MHz, DMSO-d6) δ 11.00 (s, 1H), 10.68 (s, 1H), 8.29 (d, J = 6.0 Hz, 1H), 8.03 (d, J = 8.8 Hz, 2H), 7.71 (d, J = 8.8 Hz, 2H), 7.53 (d, J = 8.4 Hz, 2H), 7.48 (d, J = 11.2 Hz, 1H), 6.93 (d, J = 9.2 Hz, 2H), 6.51 (s, 1H), 5.12-5.02 (m, 1H), 4.44 (s, 1H), 3.44 (s, 1H), 3.13-3.09 (m, 4H), 2.79-2.68 (m, 2H), 2.42-2.35 (m, 2H), 1.91-1.74 (m, 2H), 1.64-1.53 (m, 6H), 1.47-1.41 (m, 2H), 1.37-1.27 (m, 2H), 1.19-1.09 (m, 2H). MS-ESI [M+H]+: 663.4 |
| 109 | 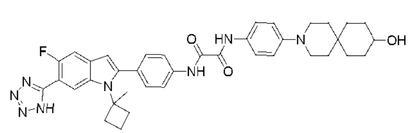 | C | 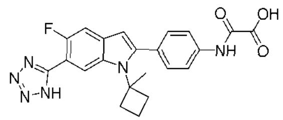 A13 + 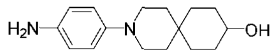 B50 | 1H NMR (400 MHz, DMSO-d6) δ 10.98 (s, 1H), 10.67 (s, 1H), 7.92 (d, J = 8.8 Hz, 2H), 7.91 (d, J = 6.0 Hz, 1H), 7.70 (d, J = 9.2 Hz, 2H), 7.52-7.46 (m, 3H), 7.93 (d, J = 9.2 Hz, 2H), 6.43 (s, 1H), 4.45 (brs, 1H), 3.44-3.41 (m, 1H), 3.11 (t, J = 6.0 Hz, 4H), 2.27-2.19 (m, 2H), 2.15-2.05 (m, 2H), 1.97 (s, 3H), 1.91-1.84 (m, 1H), 1.62-1.54 (m, 7H), 1.45-1.41 (m, 2H), 1.36-1.28 (m, 2H), 1.16-1.10 (m, 2H). MS-ESI [M+H]+: 677.4 |
| 110 | 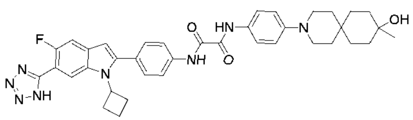 | C | 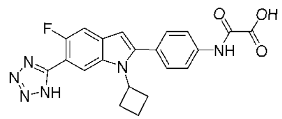 A10 + 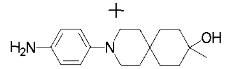 B70 | 1H NMR (400 MHz, DMSO-d6) δ 11.01 (s, 1H), 10.68 (s, 1H), 8.31 (d, J = 6.0 Hz, 1H), 8.04 (d, J = 8.8 Hz, 2H), 7.71 (d, J = 8.8 Hz, 2H), 7.57-7.52 (m, 3H), 6.93 (d, J = 8.8 Hz, 2H), 6.55 (s, 1H), 5.13-5.06 (m, 1H), 4.00 (brs, 1H), 3.14-3.08 (m, 4H), 2.75-2.66 (m, 2H), 2.42-2.37 (m, 2H), 1.93-1.75 (m, 2H), 1.54-1.28 (m, 12H), 1.10 (s, 3H). MS-ESI [M+H]+: 677.4 |

(continued)

| Ex. | Structure | Syn. method | Raw material 1 | Characterization data |
|---|---|---|---|---|
| 111 | 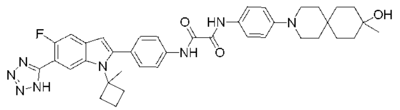 | C | 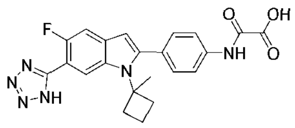 A13 + 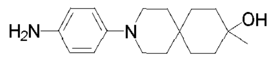 B70 | 1H NMR (400 MHz, DMSO-d6) δ 10.97 (s, 1H), 10.65 (s, 1H), 7.98 (d, J = 8.8 Hz, 2H), 7.93 (d, J = 6.0 Hz, 1H), 7.70 (d, J = 8.8 Hz, 2H), 7.53-7.49 (m, 3H), 6.93 (d, J = 9.2 Hz, 2H), 6.45 (s, 1H), 4.01 (brs, 1H), 3.14-3.08 (m, 4H), 2.27-2.19 (m, 2H), 2.15-2.05 (m, 2H), 1.97 (s, 3H), 1.92-1.83 (m, 1H), 1.68-1.62 (m, 1H), 1.55-1.45 (m, 6H), 1.41-1.37(m, 4H), 1.32-1.28 (m, 2H), 1.09 (s, 3H). MS-ESI [M+H]+: 691.4 |
| 112 | 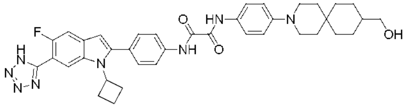 | C | 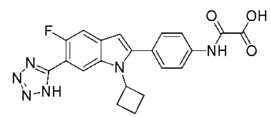 A10 + 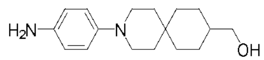 B53 | 1H NMR (400 MHz, DMSO-d6) δ 11.00 (s, 1H), 10.68 (s, 1H), 8.29-8.21 (m, 1H), 8.03 (d, J = 8.2 Hz, 2H), 7.71 (d, J = 8.6 Hz, 2H), 7.52 (d, J = 8.2 Hz, 2H), 7.39 (d, J = 10.9 Hz, 1H), 6.94 (d, J = 8.6 Hz, 2H), 6.46 (s, 1H), 5.10-4.99 (m, 1H), 4.37 (s, 1H), 3.25 (d, J = 6.1 Hz, 2H), 3.17-3.03 (m, 4H), 2.84-2.69 (m, 2H), 2.44-2.33 (m, 2H), 1.93-1.75 (m, 2H), 1.70-1.40 (m, 8H), 1.36-1.29 (m, 1H), 1.16-1.01 (m, 4H). 19F NMR (376 MHz, DMSO-d6) δ -123.06. MS-ESI [M+H]+: 677.3 |
| 113 | 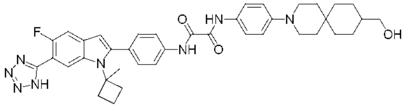 | C | 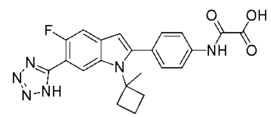 A13 + 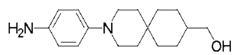 B53 | 1H NMR (400 MHz, DMSO-d6) δ 10.96 (s, 1H), 10.67 (s, 1H), 7.97 (d, J = 8.2 Hz, 2H), 7.85 (d, J = 6.0 Hz, 1H), 7.71 (d, J = 8.6 Hz, 2H), 7.50 (d, J = 8.2 Hz, 2H), 7.36 (d, J = 11.0 Hz, 1H), 6.94 (d, J = 8.7 Hz, 2H), 6.37 (s, 1H), 4.37 (s, 1H), 3.24 (d, J = 6.1 Hz, 2H), 3.11 (dt, J = 12.8, 5.5 Hz, 4H), 2.23 (d, J = 10.6 Hz, 2H), 2.08 (s, 2H), 1.96 (s, 3H), 1.86 (d, J = 9.9 Hz, 1H), 1.73-1.38 (m, 9H), 1.36-1.28 (m, 1H), 1.17-0.99 (m, 4H). 19F NMR (376 MHz, DMSO-d6) δ -123.62. MS-ESI [M+H]+: 691.3 |
| 114 | 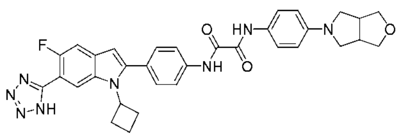 | C | 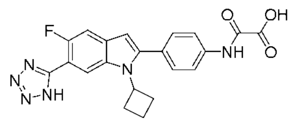 A10 + 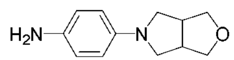 B32 | 1H NMR (400 MHz, DMSO-d6) δ 11.02(s, 1H), 10.65 (s, 1H), 8.35 (d, J = 5.6 Hz, 1H), 8.14-7.99 (m, 2H), 7.77-7.67 (m, 2H), 7.64 (d, J = 11.2 Hz, 1H), 7.58-7.50 (m, 2H), 6.78-6.50 (m, 2H), 6.59 (s, 1H), 5.20-5.0 (m, 2H), 3.90-3.80 (m, 2H), 3.53 (dd, J = 8.8, 3.2 Hz, 2H), 3.16 (dd, J =10, 2.8 Hz, 2H), 3.10-2.80 (m, 2H), 2.77-2.61 (m, 2H), 2.50-2.3 (m, 2H), 1.95-1.75 (m, 2H). MS-ESI [M+H]+: 607.3 |

(continued)

| Ex. | Structure | Syn. method | Raw material 1 | Characterization data |
|---|---|---|---|---|
| 115 | 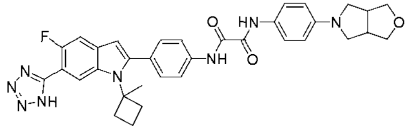 | C | 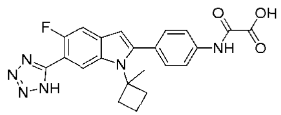 A13 + 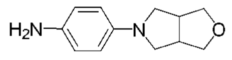 B32 | 1H NMR (400 MHz, DMSO-d6) δ 10.97 (s, 1H), 10.62 (s, 1H), 7.99 (d, J = 8.4 Hz, 2H), 7.96 (d, J = 5.6 Hz, 1H), 7.70 (d, J = 8.8 Hz, 2H), 7.57 (d, J = 11.2 Hz, 1H), 7.52 (d, J = 8.4 Hz, 2H), 6.65 (d, J = 8.8 Hz, 2H), 6.48 (s, 1H), 3.88-3.83 (m, 2H), 3.56-3.52 (m, 2H), 3.19-3.14 (m, 2H), 3.02-2.96 (m, 2H), 2.49-2.47 (m, 2H), 2.28-2.19 (m, 2H), 2.17-2.05 (m, 2H), 1.98 (s, 3H), 1.92-1.83 (m, 1H), 1.69-1.59 (m, 1H). MS-ESI [M+H]+: 621.3 |
| 116 | 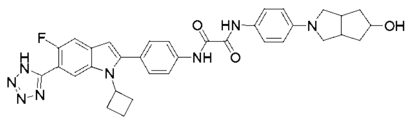 | C | 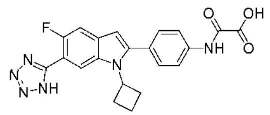 A10 + 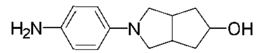 B54 | 1H NMR (400 MHz, DMSO-d6) δ 11.00 (s, 1H), 10.63 (s, 1H), 8.32 (d, J = 5.8 Hz, 1H), 8.05 (d, J = 8.2 Hz, 2H), 7.70 (d, J = 8.5 Hz, 2H), 7.63-7.40 (m, 3H), 6.74-6.47 (m, 3H), 5.15-5.02 (m, 1H), 4.62 (s, 1H), 4.15-3.99 (m, 1H), 3.25-3.05 (m, 4H), 2.84-2.58 (m, 4H), 2.43-2.36 (m, 2H), 2.15-2.00 (m, 2H), 1.91-1.76 (m, 2H), 1.43-1.33 (m, 2H). 19F NMR (376 MHz, DMSO-d6) δ-124.08. MS-ESI [M+H]+: 621.4 |
| 117 | 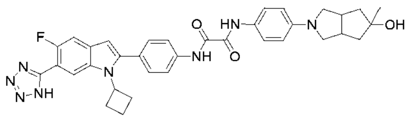 | C | 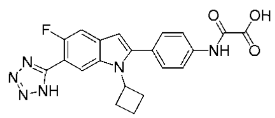 A10 + 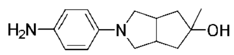 B64 | 1H NMR (400 MHz, DMSO-d6) δ 11.00 (s, 1H), 10.63 (s, 1H), 8.32 (d, J = 5.8 Hz, 1H), 8.05 (d, J = 8.7 Hz, 2H), 7.70 (d, J = 8.9 Hz, 2H), 7.56 (dd, J = 11.6, 9.9 Hz, 3H), 6.66 (d, J = 9.0 Hz, 2H), 6.56 (s, 1H), 5.13-5.04 (m, 1H), 4.47 (s, 1H), 3.27-3.19 (m, 4H), 2.75-2.67 (m, 4H), 2.43-2.37 (m, 2H), 1.91-1.76 (m, 4H), 1.59 (dd, J = 12.6, 5.0 Hz, 2H), 1.20 (s, 3H). MS-ESI [M+H]+: 635.4 |
| 118 | 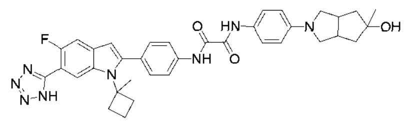 | C | 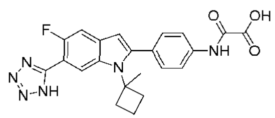 A13 + 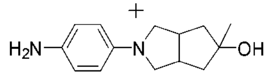 B64 | 1H NMR (400 MHz, DMSO-d6) δ 10.97 (s, 1H), 10.62 (s, 1H), 7.99 (d, J = 8.7 Hz, 2H), 7.93 (d, J = 5.8 Hz, 1H), 7.69 (d, J = 9.0 Hz, 2H), 7.51-7.54 (m, 3H), 6.66 (d, J = 9.1 Hz, 2H), 6.46 (s, 1H), 4.48 (s, 1H), 3.26-3.19 (m, 4H), 2.77-2.72 (m, 2H), 2.28-2.20 (m, 2H), 2.14-2.08 (m, 2H), 1.98 (s, 3H), 1.91-1.81 (m, 3H), 1.68-1.57 (m, 3H), 1.19 (s, 3H). MS-ESI [M+H]+: 649.4 |

(continued)

| Ex. | Structure | Syn. method | Raw material 1 | Characterization data |
|---|---|---|---|---|
| 119 | 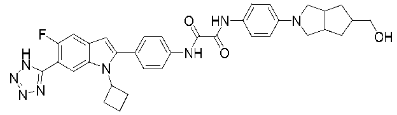 | C | 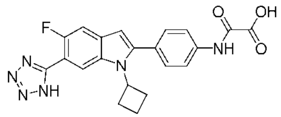 A10 + 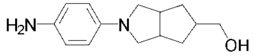 B56 | 1H NMR (400 MHz, DMSO-d6) δ 10.99 (s, 1H), 10.62 (s, 1H), 8.30 (d, J = 5.9 Hz, 1H), 8.04 (d, J = 8.4 Hz, 2H), 7.69 (d, J = 8.7 Hz, 2H), 7.51 (dd, J = 19.6, 9.8 Hz, 3H), 6.61 (d, J = 8.5 Hz, 2H), 6.51 (s, 1H), 5.12-5.03 (m, 1H), 4.46 (s, 1H), 3.38-3.35 (m, 2H), 3.26-3.12 (m, 4H), 2.81-2.68 (m, 4H), 2.43-2.34 (m, 2H), 2.08-1.99 (m, 2H), 1.91-1.77 (m, 2H), 1.61-1.51 (m, 1H), 1.25-1.11 (m, 2H). 19F NMR (376 MHz, DMSO-d6) δ -123.60. MS-ESI [M+H]+: 635.3 |
| 120 | 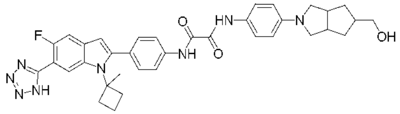 | C | 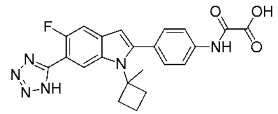 A13 + 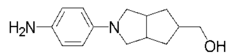 B56 | 1H NMR (400 MHz, DMSO-d6) δ 10.95 (s, 1H), 10.60 (s, 1H), 7.98 (d, J = 8.2 Hz, 2H), 7.87 (d, J = 6.0 Hz, 1H), 7.68 (d, J = 8.5 Hz, 2H), 7.50 (d, J = 8.3 Hz, 2H), 7.39 (d, J = 11.1 Hz, 1H), 6.61 (d, J = 8.5 Hz, 2H), 6.39 (s, 1H), 4.46 (s, 1H), 3.38-3.35 (m, 2H), 3.25-3.12 (m, 4H), 2.80-2.68 (m, 2H), 2.28-2.18 (m, 2H), 2.11-2.00 (m, 4H), 1.97 (s, 3H), 1.90-1.83 (m, 1H), 1.67-1.53 (m, 2H), 1.24-1.10 (m, 2H). 19F NMR (376 MHz, DMSO-d6) δ-123.77. MS-ESI [M+H]+: 649.3 |
| 121 | 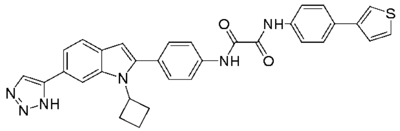 | D | 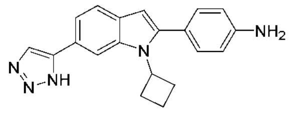 A18 + 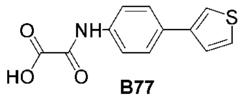 B77 | 1H NMR (400 MHz, DMSO-d6) δ 11.07 (s, 1H), 10.98 (s, 1H), 8.38 (s, 1H), 8.19 (s, 1H), 8.05 (d, J = 8.5 Hz, 2H), 7.96 (d, J = 8.4 Hz, 2H), 7.88 (dd, J = 2.9, 1.3 Hz, 1H), 7.77 (d, J = 8.5 Hz, 2H), 7.67-7.64 (m, 2H), 7.60 (d, J = 6.5 Hz, 2H), 7.54 (d, J = 8.4 Hz, 2H), 6.51 (s, 1H), 5.11-5.06 (m, 1H), 2.80-2.74 (m, 2H), 2.45-2.39 (m, 2H), 1.96-1.90 (m, 1H), 1.84-1.77 (m, 1H). MS-ESI [M+H]+: 559.0 |
| 122 | 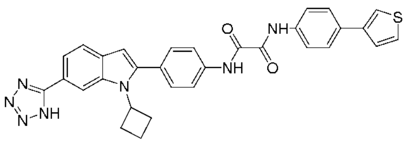 | D | 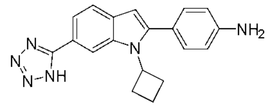 A24 + 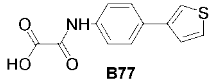 B77 | 1H NMR (400 MHz, DMSO-d6) δ 11.09 (s, 1H), 10.98 (s, 1H), 8.42 (s, 1H), 8.06 (d, J = 8.4 Hz, 2H), 7.95 (d, J = 8.7 Hz, 2H), 7.87 (d, J = 3.1 Hz, 1H), 7.80-7.73 (m, 4H), 7.66-7.64 (m, 1H) 7.59 (dd, J = 5.1, 1.3 Hz, 1H), 7.55 (d, J = 8.4 Hz, 2H), 6.58 (s, 1H), 5.14-5.05 (m, 1H), 2.85-2.74 (m, 2H), 2.45-2.38 (m, 2H), 1.98-1.91 (m, 1H), 1.85-1.78 (m, 1H). MS-ESI [M+H]+: 560.0 |

(continued)

| Ex. | Structure | Syn. method | Raw material 1 | Characterization data |
|---|---|---|---|---|
| 123 | 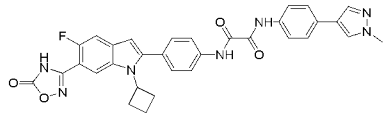 | D | 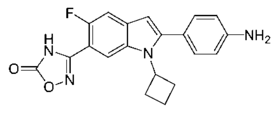 A19 + 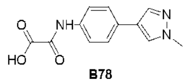 B78 | 1H NMR (400 MHz, DMSO-d6) δ 12.96 (s, 1H), 11.08 (s, 1H), 10.90 (s, 1H), 8.13-8.02 (m, 4H), 7.91-7.83 (m, 3H), 7.61-7.50 (m, 5H), 6.56 (s, 1H), 5.05 (m, 1H), 3.87 (s, 3H), 2.78-2.64 (m, 2H), 2.42-2.30 (m, 2H), 1.93-1.69 (m, 2H). MS-ESI [M+H]+: 592.2 |
| 124 | 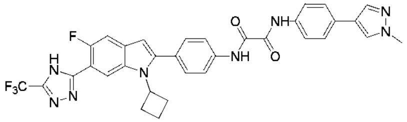 | D | 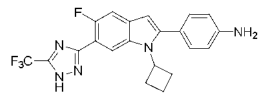 A20 + 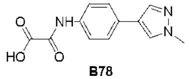 B78 | 1H NMR (400 MHz, DMSO-d6) δ 11.09 (s, 1H), 10.91 (s, 1H), 8.27 (d, J = 5.9 Hz, 1H), 8.12 (s, 1H), 8.06 (d, J = 8.7 Hz, 2H), 7.91-7.86 (m, 3H), 7.63-7.54 (m, 5H), 6.58 (s, 1H), 5.17-5.05 (m, 1H), 3.87 (s, 3H), 2.71-2.61 (m, 2H), 2.44-2.37 (m, 2H), 1.89-1.76 (m, 2H). MS-ESI [M+H]+: 643.2 |
| 125 | 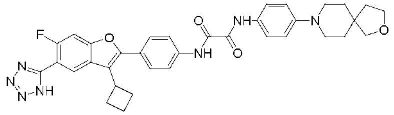 | D | 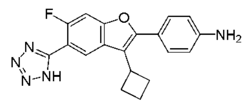 A25 + 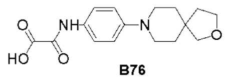 B76 | 1H NMR (400 MHz, DMSO-d6) δ 11.03 (s, 1H), 10.69 (s, 1H), 8.43 (d, J = 7.0 Hz, 1H), 8.07 (d, J = 8.8 Hz, 2H), 7.85 (d, J = 10.4 Hz, 1H), 7.70 (t, J = 9.0 Hz, 4H), 6.96 (d, J = 9.2 Hz, 2H), 4.08-3.99 (m, 1H), 3.76 (t, J = 7.1 Hz, 2H), 3.48 (s, 2H), 3.20-3.09 (m, 4H), 2.47-2.40 (m, 4H), 2.14-2.07 (m, 1H), 1.99-1.93 (m, 1H), 1.74 (t, J = 7.1 Hz, 2H), 1.62 (t, J = 5.8 Hz, 4H). MS-ESI [M+H]+: 636.3 |
| 126 | 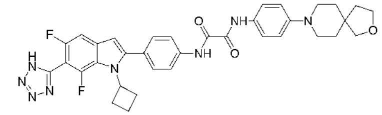 | D | 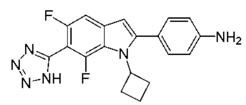 A23 + 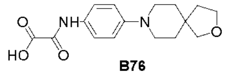 B76 | 1H NMR (400 MHz, DMSO-d6) δ 11.04 (s, 1H), 10.69 (s, 1H), 8.06 (d, J = 8.8 Hz, 2H), 7.72 (d, J = 9.2 Hz, 2H), 7.54 (d, J = 8.8 Hz, 2H), 7.45 (d, J = 9.6 Hz, 1H), 6.96 (d, J = 9.2 Hz, 2H), 6.67 (d, J = 2.4 Hz, 1H), 5.11-4.99 (m, 1H), 3.76 (t, J = 7.2 Hz, 2H), 3.48 (s, 2H), 3.19-3.09 (m, 4H), 2.59-2.52 (m, 2H), 2.44-2.34 (m, 2H), 1.82-1.68 (m, 4H), 1.67-1.57 (m, 4H). MS-ESI [M+H]+: 653.9 |

(continued)

| Ex. | Structure | Syn. method | Raw material 1 | Characterization data |
|---|---|---|---|---|
| 127 | 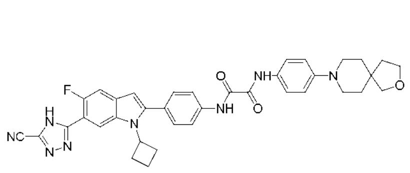 | D | 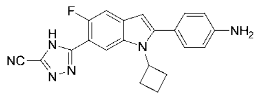 A21 + 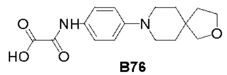 B76 | 1H NMR (400 MHz, DMSO-d6) δ 11.01 (s, 1H), 10.69 (s, 1H), 8.28 (d, J = 5.9 Hz, 1H), 8.04 (d, J = 8.6 Hz, 2H), 7.72 (d, J = 9.2 Hz, 2H), 7.54 (d, J = 8.7 Hz, 3H), 6.96 (d, J = 9.1 Hz, 2H), 6.55 (s, 1H), 5.12-5.03 (m, J = 8.7 Hz, 1H), 3.76 (t, J = 7.1 Hz, 2H), 3.48 (s, 2H), 3.20-3.09 (m, 4H), 2.75-2.64 (m, 2H), 2.43-2.35 (m, 2H), 1.90-1.77 (m, 2H), 1.74 (t, J = 7.1 Hz, 2H), 1.62 (t, J = 5.9 Hz, 4H). MS-ESI [M+H]+: 659.4 |
| 128 | 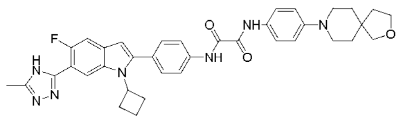 | D | 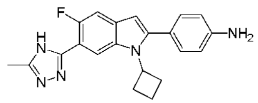 A22 + 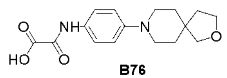 B76 | 1H NMR (400 MHz, DMSO-d6) δ 13.83-13.58 (m, 1H), 11.01 (s, 1H), 10.70 (s, 1H), 8.28 (d, J = 5.9 Hz, 1H), 8.04 (d, J = 8.2 Hz, 2H), 7.72 (d, J = 8.6 Hz, 2H), 7.62-7.37 (m, 3H), 6.96 (d, J = 8.6 Hz, 2H), 6.51 (d, J = 16.5 Hz, 1H), 5.18-4.97 (m, 1H), 3.76 (t, J = 7.1 Hz, 2H), 3.48 (s, 2H), 3.22-3.09 (m, 4H), 2.74-2.64 (m, 2H), 2.46-2.31 (m, 5H), 1.91-1.78 (m, 2H), 1.77-1.71 (m, 2H), 1.66-1.58 (m, 4H). MS-ESI [M+H]+: 648.4 |
| 129 | 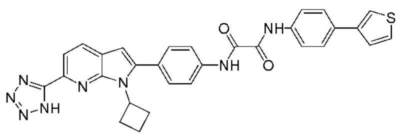 | D | 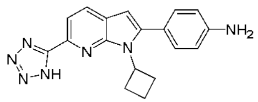 A26 + 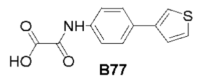 B77 | 1H NMR (400 MHz, DMSO-d6) δ 11.12 (s, 1H), 10.98 (s, 1H), 8.16 (d, J = 8.0 Hz, 1H), 8.11-8.06 (m, 2H), 8.00-7.92 (m, 3H), 7.89-7.85 (m, 1H), 7.77 (d, J = 8.8 Hz, 2H), 7.67-7.63 (m, 1H), 7.62-7.54 (m, 3H), 6.62 (s, 1H), 5.03-4.92 (m, 1H), 2.70-2.53 (m, 2H), 2.38-2.25 (m, 2H), 2.04-1.93 (m, 1H), 1.76-1.63 (m, 1H). MS-ESI [M+H]+: 562.1 |
| 130 | 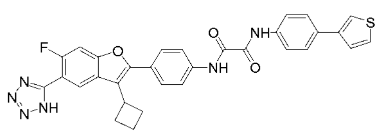 | D | 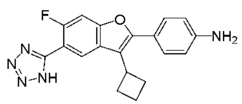 A25 + 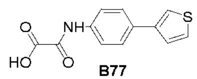 B77 | 1H NMR (400 MHz, DMSO-d6) δ 11.10 (s, 1H), 10.96 (s, 1H), 8.41 (d, J = 7.0 Hz, 1H), 8.08 (d, J = 8.4 Hz, 2H), 7.98-7.83 (m, 3H), 7.82-7.52 (m, 7H), 4.06-3.99 (m, 1H), 2.45-2.25 (m, 4H), 2.14-2.07 m, 1H), 1.97-1.93 (m, 1H). MS-ESI [M+H]+: 578.7 |

(continued)

| Ex. | Structure | Syn. method | Raw material 1 | Characterization data |
|---|---|---|---|---|
| 131 | 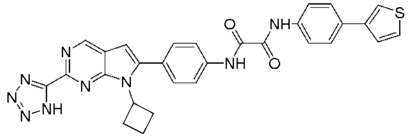 | D | 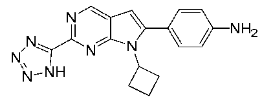 A27 + 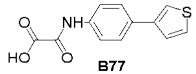 B77 | 1H NMR (400 MHz, DMSO-d6) δ 11.14 (s, 0.5H), 10.99 (s, 0.5H), 9.14 (s, 1H), 8.10 (d, J = 8.6 Hz, 2H), 7.96 (d, J = 8.8 Hz, 2H), 7.87 (dd, J = 2.9, 1.4 Hz, 1H), 7.77 (d, J = 8.7 Hz, 2H), 7.65 (dd, J = 5.0, 2.9 Hz, 1H), 7.62-7.58 (m, 3H), 6.72 (s, 1H), 5.02-4.93 (m, 1H), 2.38-2.33 (m, 2H), 1.99-1.91 (m, 1H), 1.81-1.72 (m, 1H). MS-ESI [M+H]+: 562.0 |
| 132 | 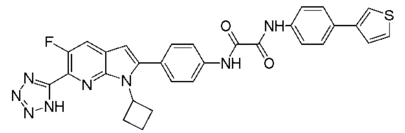 | D | 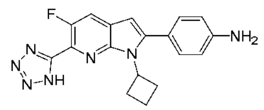 A28 + 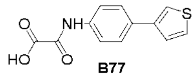 B77 | 1H NMR (400 MHz, DMSO-d6) δ 11.12 (s, 1H), 10.99 (s, 1H), 8.11-8.07 (m, 2H), 8.04 (d, J = 10.8 Hz, 1H), 7.98-7.93 (m, 2H), 7.89-7.85 (m, 1H), 7.79-7.74 (m, 2H), 7.65 (dd, J = 5.2, 2.8 Hz, 1H), 7.61-7.57 (m, 3H), 6.59 (s, 1H), 4.95 (td, J = 8.8 Hz, 1H), 3.26-3.21 (m, 2H), 2.35-2.26 (m, 2H), 1.90 (q, J = 10.0 Hz, 1H), 1.76-1.65 (m, 1H). MS-ESI [M+H]+: 579.7 |
| 133 | 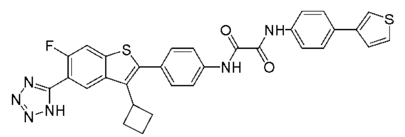 | D | 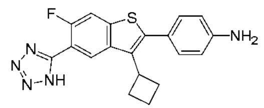 A29 + 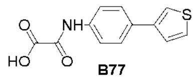 B77 | 1H NMR (400 MHz, DMSO-d6) δ 11.08 (s, 1H), 10.97 (s, 1H), 8.54 (d, J = 6.7 Hz, 1H), 8.12 (d, J = 10.5 Hz, 1H), 8.02 (d, J = 8.7 Hz, 2H), 7.95 (d, J = 8.8 Hz, 2H), 7.87 (dd, J = 2.9, 1.4 Hz, 1H), 7.76 (d, J = 8.7 Hz, 2H), 7.65 (dd, J = 5.0, 2.9 Hz, 1H), 7.59 (dd, J = 5.1, 1.4 Hz, 1H), 7.52 (d, J = 8.6 Hz, 2H), 4.08 (m, 1H), 2.39-2.32 (m, 2H), 2.27-2.17 (m, 2H), 2.08-1.96 (m, 1H), 1.84-1.77 (m, 1H). MS-ESI [M+H]+: 595.1 |
| 134 | 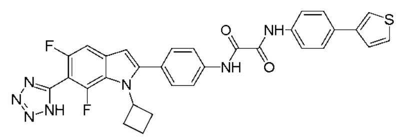 | D | 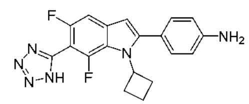 A23 + 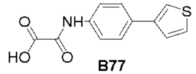 B77 | 1H NMR (400 MHz, DMSO-d6) δ 11.10 (s, 1H), 10.98 (s, 1H), 8.11-8.03 (m, 2H), 7.99-7.92 (m, 2H), 7.87 (d, J = 2.8 Hz, 1H), 7.76 (d, J = 8.8 Hz, 2H), 7.66-7.62 (m, 1H), 7.58 (d, J = 4.8 Hz, 1H), 7.54 (d, J = 8.4 Hz, 2H), 7.34 (d, J = 9.6 Hz, 1H), 6.62 (d, J = 2.4 Hz, 1H), 5.32 (d, J = 4.8 Hz, 1H), 5.09-4.98 (m, 1H), 2.60-2.55 (m, 2H), 2.43-2.34 (m, 2H), 1.83-1.62 (m, 2H). MS-ESI [M+H]+: 596.1 |

## 3. Activity Examples

It should be understood that the dosages referred to herein when describing the compounds of the present invention, pharmaceutical compositions comprising them, pharmaceutical combinations, cassettes, and related uses and methods are based on weight in free form and do not include any salts, hydrates, or solvates thereof, unless otherwise stated.

### Example 1: Inhibitory effect of the compound of the present invention on SHP2 enzyme

This experiment evaluates the inhibitory effect of the test compounds on SHP2 enzyme activity by detecting the reaction between the human recombinant protein SHP2 enzyme and the substrate.

The stock solutions of the reaction buffer components were thawed and mixed homogeneously at room temperature, to prepare the reaction buffer (55 mM HEPES pH = 7.2 (Sigma, Cat. No. #PHR1428), 100 mM NaCl (Beyotime, Cat. No. #ST374, 5 M solution), 0.5 mM EDTA (Beyotime, Cat. No. #ST066), 1 mM DL-Dithiothreitol (DTT) (Sigma, Cat. No. #D9779), 0.002% BSA (Solarbio, Cat. No. #A8010), 0.001% Brij35 (Brij® L23 solution (Brij35) (Sigma, Cat. No. #B4184)). The SHP2 enzyme (catalytic domain, amino acid sequence 224-528, expressed and purified by GenScript) was thawed slowly on ice. 1 μL of this solution (1.37 mg/mL) was added to 386 μL of the reaction buffer to dilute it to 100 nM. Then, 120 μL of the 100 nM SHP2 enzyme solution was added to 2880 μL of reaction buffer to prepare a 4 nM SHP2 enzyme working solution. The test compounds and the specific inhibitor of SHP2 (11a-1, used as a positive control, Zeng, L. et al., J. Med. Chem. 2014, 57, 6594-6609) were prepared as 10 mM DMSO solutions. 10 μL of the 10 mM compound solution was mixed with 20 μL of DMSO solution, and then serially diluted 3-fold, resulting in 10 concentrations. 1 μL of the serially diluted solution was mixed with 99 μL of reaction buffer to obtain a 10-fold diluted compound working solution.

A 96-well black half-area microplate (Greiner, catalog #675076) was used. 25 μL of 4 nM SHP2 enzyme working solution and 5 μL of 10x compound working solution were added to each well and incubated at room temperature for 30 minutes. 15 μL of DiFMUP substrate (Thermo Fisher Scientific, catalog #D6567) was added to 2985 μL of reaction buffer to prepare a 50 μM substrate working solution. After incubation of the compounds and SHP2 enzyme, 20 μL of the 50 μM substrate working solution was added to each well and incubated at room temperature for 5 minutes. The plate was then read on a multi-mode microplate reader (BioTek, Synergy H1) at 340 nm/450 nm fluorescence.

Positive control compound **(11a-1)**

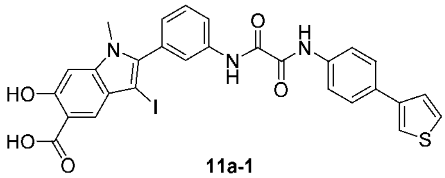

**11a-1**

Data were analyzed using GraphPad Prism 9.0 software. A non-linear S-curve regression dose-response-inhibition equation was used to fit the data and generate the dose-response curve, from which the IC50 value was calculated.

SHP2 enzyme inhibition rate (%) = 1-(RFU $_{\text{test drug)}}$ -RFU $_{\text{buffer control)}}$/(RFU DMSO control) = RFU buffer control) x 100%.

Representative compounds disclosed herein exhibited excellent inhibitory properties against SHP2 enzymes, as shown in the table below.

| Example | S HP2 | Example | S HP2 | Example | S HP2 |
|---|---|---|---|---|---|
| | I C50 (μM) | | I C50 (μM) | | I C50 (μM) |
| **11a-1** | 0.108 | **11a-1** | 0.108 | **11a-1** | 0.108 |
| **1** | 0.066 | **2** | 0.064 | **3** | 0.278 |

(continued)

| Example | S HP2 I C50 (μM) | Example | S HP2 I C50 (μM) | Example | S HP2 I C50 (μM) |
|---|---|---|---|---|---|
| **4** | 0.029 | **5** | 0.105 | **6** | 0.073 |
| **7** | 0.049 | **8** | 0.130 | **9** | 0.087 |
| **10** | 0.067 | **11** | 0.030 | **12** | 0.048 |
| **13** | 0.066 | **14** | 0.045 | **15** | 0.183 |
| **16** | 0.048 | **17** | 0.120 | **18** | 0.074 |
| **19** | 0.152 | **20** | 0.027 | **21** | 0.068 |
| **22** | 0.038 | **23** | 0.102 | **24** | 0.271 |
| **25** | 0.059 | **26** | 0.061 | **27** | 0.111 |
| **28** | 0.092 | **29** | 0.048 | **30** | 0.086 |
| **31** | 0.088 | **32** | 0.061 | **33** | 0.041 |
| **34** | 0.072 | **35** | 0.174 | **36** | 0.052 |
| **37** | 0.044 | **38** | 0.112 | **39** | 0.043 |
| **40** | 0.060 | **41** | 0.063 | **42** | 0.179 |
| **43** | 0.052 | **44** | 0.057 | **45** | 0.055 |
| **46** | 0.045 | **47** | 0.063 | **48** | 0.050 |
| **49** | 0.133 | **50** | 0.113 | **51** | 0.057 |
| **52** | 0.102 | **53** | 0.064 | **54** | 0.069 |
| **55** | 0.045 | **56** | 0.074 | **57** | 0.068 |
| **58** | 0.031 | **59** | 0.062 | **60** | 0.064 |
| **61** | 0.041 | **62** | 0.065 | **63** | 0.038 |
| **64** | 0.088 | **65** | 0.198 | **66** | 0.291 |
| **67** | 0.034 | **68** | 0.048 | **69** | 0.046 |
| **70** | 0.050 | **71** | 0.050 | **72** | 0.046 |
| **73** | 0.048 | **74** | 0.065 | **75** | 0.034 |
| **76** | 0.040 | **77** | 0.020 | **78** | 0.074 |
| **79** | 0.108 | **80** | 0.036 | **81** | 0.070 |
| **82** | 0.030 | **83** | 0.058 | **84** | 0.060 |
| **85** | 0.047 | **86** | 0.064 | **87** | 0.089 |
| **88** | 0.148 | **89** | 0.157 | **90** | 0.139 |
| **91** | 0.093 | **92** | 0.051 | **93** | 0.056 |
| **94** | 0.031 | **95** | 0.061 | **96** | 0.058 |
| **97** | 0.105 | **98** | 0.083 | **99** | 0.077 |
| **100** | 0.048 | **101** | 0.023 | **102** | 0.049 |
| **103** | 0.141 | **104** | 0.070 | **105** | 0.086 |
| **106** | 0.103 | **107** | 0.138 | **108** | 0.030 |
| **109** | 0.061 | **110** | 0.077 | **111** | 0.094 |
| **112** | 0.105 | **113** | 0.114 | **114** | 0.072 |
| **115** | 0.036 | **116** | 0.063 | **117** | 0.071 |

(continued)

| Example | S HP2 | Example | S HP2 | Example | S HP2 |
|---|---|---|---|---|---|
| | I C50 (μM) | | I C50 (μM) | | I C50 (μM) |
| **118** | 0.045 | **119** | 0.055 | **120** | 0.055 |
| **121** | >10 | **122** | 0.066 | **123** | 0.141 |
| **124** | 0.716 | **125** | 0.051 | **126** | 0.023 |
| **127** | 0.144 | **128** | >10 | **129** | 0.059 |
| **130** | 0.025 | **131** | 0.026 | **132** | 0.027 |
| **133** | 0.069 | **134** | 0.069 | | |

The results above show that the compounds disclosed herein exhibit excellent SHP2 enzyme inhibitory activity, with IC50 values < 1 μM, preferably 1-100 nM, and more preferably 1-50 nM. For example, the IC50 values of Examples 4, 11, 20, 22, 58, 63, 67, 75, 77, 80, 82, 94, 101, 108, and 115 are 3-4 times lower than the most active active site inhibitor in the prior art (control compound 11a-1, IC50 = 0.108 μM), meaning the inhibitory activity is increased by 3-4 times.

**Example 2: Inhibitory activity of the disclosed compound against the proliferation of three tumor cell lines: KYSE520, MeWo, and MV4-11.**

In this study, the CellTiter-Glo® Luminescent Cell Viability Assay kit from Promega was used to evaluate the inhibitory effects of the disclosed compounds on the proliferation of cell lines KYSE520 (a human esophageal squamous cell carcinoma cell line dependent on SHP2 for growth), MeWo (a human malignant melanoma cell line), and MV4-11 (an RTK-mutant human acute monocytic leukemia cell line).

KYSE520 cells (Nanjing Kebaio Biotechnology Co., Ltd., Catalog #CBP60658) were thawed by rapidly shaking the cryovial in a 37 °C water bath to thaw within 1 minute. The thawed cell suspension was mixed homogeneously with RPMI 1640 medium (HyClone, Catalog #SH30027.01) containing 10% FBS (ExCell, Catalog #FND500), centrifuged at 1000 rpm for 5 minutes, and the supernatant was discarded. The cell pellet was resuspended in 5 mL of complete culture medium (RPMI 1640 medium containing 10% FBS) and placed in a cell culture flask with 25 $cm^2$ of bottom area, then cultured in a cell culture incubator at 37 °C, 95% humidity, and 5% $CO_2$. Cell passaging was performed when the cell confluence reached approximately 80%. During cell passaging, the old culture medium was discarded, the cells were washed once with DPBS (Thermo Fisher, Catalog #14190-144), and 1 mL of trypsin (Thermo Fisher, Catalog #25200072) was added to digest the cells. When the cells dispersed into single cells and detached from the bottom of the culture flask, 3 mL of fresh complete culture medium was added to stop the digestion. After gently pipetting the cell suspension, 1/5 of the cell suspension was retained, 5 mL of fresh complete culture medium was added, and after mixing, the cells were placed in a cell culture flask for further culture. Cell plating was performed when the cell confluence again reached approximately 80%. During cell plating, following the cell passaging method, 1/5 of the cell suspension was retained for continued culture, and the remaining 4/5 of the cell suspension was placed in a 15 mL centrifuge tube. Cell viability was determined using the trypan blue exclusion method to ensure that cell viability was above 90%. A cell suspension with a density of $2.22 \times 10^4$ cells/mL was prepared using complete culture medium, and 135 μL of the cell suspension was added to a 96-well transparent flat-bottom black-walled cell culture plate (Greiner, Catalog #655090), resulting in a cell density of 3000 viable cells per well. A control group containing only complete culture medium without cells and compounds (i.e., culture medium control) and a control group containing cells but no compound (i.e., cell control) were set up. The cell plates were incubated overnight in a cell culture incubator. First, a 10 mM stock solution of the compound (Example compound and positive control compound 11a-1) in DMSO (Damas-beta, catalog #75927R) was serially diluted 3-fold to the 9th concentration, and a 10th concentration containing only DMSO (without compound) was also prepared as a control. Then, the DMSO solutions containing different concentrations of the compound were diluted 100-fold with complete culture medium, resulting in a 1% DMSO concentration in each solution. Finally, 15 μL of each solution was added to the corresponding wells of the cell culture plate, resulting in a starting compound concentration of 10 μM, with a 3-fold dilution between adjacent concentrations, and a final DMSO concentration of 0.1% in the cell culture plate. The cell plates were then incubated in the cell culture incubator for another 120 hours.

Using essentially the same experimental materials, MeWo cells (ATCC, catalog #HTB-65) were treated similarly: when thawing the cells, the cryovial was rapidly shaken in a 37 °C water bath to thaw the cells within 1 minute. The thawed cell suspension was mixed with MEM/EBSS culture medium containing 10% FBS (HyClone, catalog #SH30024.01), centrifuged at 1000 rpm for 5 minutes, and the supernatant was discarded. The cell pellet was resuspended in 5 mL of complete culture medium (MEM/EBSS medium containing 10% FBS), placed in a cell culture flask with 25 $cm^2$ of bottom

area, and cultured in a cell culture incubator at 37 °C, 95% humidity, and 5% $CO_2$. Cell passaging was performed when the cell confluence reached approximately 80%. During cell passaging, the old culture medium was discarded, the cells were washed once with DPBS, and 1 mL of trypsin was added to digest the cells. When the cells dispersed into single cells and detached from the bottom of the cell culture flask, 3 mL of new complete culture medium was added to stop the digestion. After thoroughly mixing the cell suspension, 1/5 of the cell suspension was retained, 5 mL of new complete culture medium was added, and after thorough mixing, the cells were placed in a cell culture flask for further culture. Cell plating was performed when the cell confluence again reached approximately 80%. During cell plating, following the cell passaging method, 1/5 of the cell suspension was retained for further culture, and the remaining 4/5 of the cell suspension was placed in a 15 mL centrifuge tube. Cell viability was determined using the trypan blue exclusion method to ensure that cell viability was above 90%. A cell suspension with a density of $2.22 \times 10^4$ cells/mL was prepared using MEM/EBSS culture medium containing 5% FBS. 90 μL of the cell suspension was added to a 96-well cell culture plate, resulting in a cell density of 2000 viable cells/well in the cell culture plate. A control group without cells and without compounds, containing only complete culture medium (i.e., culture medium control), and a control group without compounds but containing cells (i.e., cell control) were set up. The cell plate was cultured overnight in the cell culture incubator. First, a 10 mM stock solution of the compound (Example compound and positive control compound 11a-1) in DMSO was serially diluted with DMSO at a 3-fold dilution, down to the 9th concentration. A 10th concentration containing only DMSO (without the compound) was also prepared as a control. Then, the DMSO solutions containing different concentrations of the compound were diluted 100-fold with complete culture medium, resulting in a 1% DMSO concentration in each solution. Finally, 10 μL of each solution was added to the corresponding wells of the cell culture plate, resulting in a starting compound concentration of 10 μM, with a 3-fold dilution between adjacent concentrations, and a 0.1% DMSO concentration in the cell culture plate. The cell plates were then incubated in a cell culture incubator for 72 hours.

Using essentially the same experimental materials, MV4-11 cells (Nanjing Cobio Biotechnology Co., Ltd., Catalog #CBP60522) were treated similarly: when thawing the cells, the cryovial was rapidly shaken in a 37 °C water bath to thaw within 1 minute. The thawed cell suspension was mixed with IMDM culture medium containing 10% FBS (HyClone, Catalog #SH30228.01), centrifuged at 1000 rpm for 7 minutes, and the supernatant was discarded. The cell pellet was resuspended in 5 mL of complete culture medium (IMDM medium containing 10% FBS) placed in a cell culture flask with 25 $cm^2$ of bottom area, and cultured in a cell culture incubator at 37 °C, 95% humidity, and 5% $CO_2$. Cell passaging was performed when the cell density reached approximately $10^6$ cells/mL. During cell passaging, the old cell suspension was thoroughly mixed, 1/5 of the cell suspension was retained, and 4 mL of new complete culture medium was added. After thorough mixing, the cells were placed back in the cell culture incubator for further culture. Cell plating was performed when the cell density again reached approximately $10^6$ cells/mL. During cell plating, following the cell passage method, 1/5 of the cell suspension was retained for continued culture, and the remaining 4/5 of the cell suspension was placed in a 15 mL centrifuge tube. Cell viability was determined using the trypan blue exclusion method to ensure that cell viability was above 90%. A cell suspension with a density of $1.11 \times 10^5$ viable cells/mL was prepared using complete culture medium. 90 μL of the cell suspension was added to a 96-well cell culture plate, resulting in a cell density of 10000 viable cells/well. A control group without cells and without compounds, containing only complete culture medium (i.e., culture medium control) and a control group without compounds but containing cells (i.e., cell control) were set up. The cell plate was incubated overnight in the cell culture incubator. First, a 10 mM stock solution of the compound (Example compound and positive control compound 11a-1) in DMSO was serially diluted with DMSO at a 3-fold dilution, down to the $9^{th}$ concentrations. A $10^{th}$ concentration containing only DMSO (without the compound) was also prepared as a control. Then, the DMSO solutions containing different concentrations of the compound were diluted 100-fold with complete culture medium, resulting in a DMSO concentration of 1% in each solution. Finally, 10 μL of each solution was added to the corresponding wells of a cell culture plate, resulting in an initial compound concentration of 10 μM, with a 3-fold dilution between adjacent concentrations, and a DMSO concentration of 0.1% in the cell culture plate. The cell plate was then incubated in a cell culture incubator for 72 hours.

For endpoint detection, CellTiter-Glo reagent (Promega, catalog number #G7573, CellTiter-Glo® Luminescent Cell Viability Assay Kit) was melt and each cell plate was moved to room temperature for equilibration for 30 minutes. 75 μL (KYSE520 cells) or 50 μL (MeWo cells and MV 4-11 cells) of CellTiter-Glo was added to each well of the corresponding cell plate. The plates were shaken on a track-mounted shaker for 2 minutes to fully lyse the cells. The cell plates were kept at room temperature for 8 minutes to stabilize the luminescence signal. A multi-mode microplate reader (PerkinElmer, Envision 2105 Multimode plate Read) was used to full-wavelength scan the luminescence value of each well.

Use the following formula to calculate the cell survival rate under the action of each concentration of the compound:

$$\text{Cell survival (\%)} = (\text{Lum}_{\text{test drug})} - \text{Lum}_{\text{culture medium control})}/(\text{Lum}_{\text{cell control})} - \text{Lum}_{\text{culture medium control})} \times 100\%$$

Data were analyzed using GmphPad Prism 8.0 software. Nonlinear S-curve regression was used to fit the data to

obtain the dose-response curve, and the IC50 value was calculated from it.

The inhibitory activities of the compounds of Examples on cell proliferation are shown in the table below:

| Example | IC50 (μM) | | | Example | IC50 (μM) | | |
|---|---|---|---|---|---|---|---|
| | Kyse520 | MeWo | M V4-11 | | Kyse520 | MeWo | M V4-11 |
| **11a-1** | 1.23 | 3.10 | 5.13 | **11a-1** | 1.23 | 3.10 | 5.13 |
| **1** | 0.095 | 0.032 | 0.097 | **2** | 0.141 | | 0.216 |
| **6** | 0.065 | 0.113 | 0.358 | **7** | 0.071 | 0.111 | 0.254 |
| **10** | 0.109 | | 0.173 | **12** | 0.121 | 0.082 | 0.262 |
| **13** | 0.182 | | | **14** | 0.113 | | 0.155 |
| **16** | 0.151 | | 0.137 | **17** | 0.185 | | |
| **20** | 0.036 | 0.042 | >10 | **25** | 0.056 | 0.040 | 0.108 |
| **26** | 0.126 | | 0.129 | **27** | 0.170 | | |
| **29** | 0.076 | | 0.130 | **30** | 0.192 | | |
| **31** | 0.116 | | | **32** | 0.182 | | |
| **33** | 0.042 | 0.117 | >10 | **36** | 0.120 | | |
| **40** | 0.168 | 0.073 | 0.269 | **55** | 0.108 | | 0.177 |
| **57** | 0.164 | 0.096 | 0.127 | **58** | 0.125 | | |
| **59** | 0.119 | | 0.213 | **61** | 0.126 | 0.115 | 3.03 |
| **62** | 0.193 | | 0.202 | **67** | 0.086 | 0.038 | >10 |
| **68** | 0.057 | 0.031 | 0.060 | **69** | 0.189 | 0.093 | |
| **70** | 0.085 | 0.213 | 4.25 | **74** | 0.116 | | 0.129 |
| **75** | 0.103 | | | **76** | 0.096 | 0.041 | 0.173 |
| **77** | 0.049 | 0.034 | 0.094 | **82** | 0.097 | | |
| **83** | 0.163 | | 0.133 | **86** | 0.158 | | |
| **89** | 0.062 | 0.036 | 0.095 | **91** | 0.101 | | |
| **92** | 0.130 | 0.098 | 0.123 | **94** | 0.115 | 0.041 | 0.237 |
| **95** | 0.113 | | 0.168 | **96** | 0.059 | 0.103 | >10 |
| **97** | 0.095 | 0.110 | 0.305 | **98** | 0.195 | | |
| **99** | 0.170 | | | **100** | 0.057 | 0.038 | 0.126 |
| **101** | 0.128 | | 0.329 | **102** | 0.116 | | |
| **103** | 0.042 | | 0.124 | **104** | 0.125 | | |
| **106** | 0.110 | | | **107** | 0.101 | | |
| **108** | 0.167 | 0.034 | 0.115 | **109** | 0.107 | | 0.243 |
| **110** | 0.109 | 0.086 | 0.133 | **111** | 0.105 | | 0.122 |
| **113** | 0.116 | | | **114** | 0.118 | 0.078 | 0.156 |
| **115** | 0.110 | | | **116** | 0.092 | 0.067 | 0.263 |
| **117** | 0.104 | | 0.127 | **118** | 0.116 | | 0.131 |
| **119** | 0.105 | | | **120** | 0.115 | | |
| **122** | 4.36 | 0.409 | | **123** | 0.243 | 0.118 | |
| **124** | 0.661 | 1.55 | | **125** | 0.680 | 0.764 | |
| **129** | 2.85 | 0.176 | | **130** | 0.422 | 0.162 | |

(continued)

| Example | IC50 (μM) | | | Example | IC50 (μM) | | |
|---|---|---|---|---|---|---|---|
| | Kyse520 | MeWo | M V4-11 | | Kyse520 | MeWo | M V4-11 |
| **132** | 1.10 | 0.291 | | **133** | 0.688 | 0.686 | |
| **134** | 0.334 | 0.270 | | | | | |

The above results show that the disclosed compounds exhibit excellent proliferation inhibitory activity against KYSE-520 and/or Mewo and/or MV4-11 cell lines, with an IC50 value of <1 μM, preferably < 200 nM, more preferably 50-100 nM, and even more preferably <50 nM, and is significantly superior to the active site inhibitor reference compound **11a-1.** For example, the IC50 values of the representative example compounds tested against KYSE-520 cells were at least 6-fold lower than those against **11a-1 (1.23** μM), and even as much as 34 times lower **(0.036** μM, Ex # **20);** the IC50 values against Mewo cells were at least 15-fold lower than those against **11a-1** (**3.10** μM), and even as much as 100-fold lower **(0.060** μM, Ex # **68);** and the IC50 values against MV4-11 cells were generally significantly lower than those against **11a-1 (5.13** μM), and even as much as 85-fold lower **(0.060** μM, Ex # **68).**

**<u>Example 3:</u> The inhibitory activity of the representative compounds of this disclosure on the proliferation of various tumor cells.**

This experiment used the CellTiter-Glo® Luminescent Cell Viability Assay kit from Promega to evaluate the inhibitory activity of the disclosed compounds on the proliferation of the following cell lines: 4T1 (mouse breast cancer cell line), A20 (mouse B-cell lymphoma cell line), A2780 (human ovarian cancer cell line), AsPC-1 (pancreatic cancer cell line), B16F10 (mouse melanoma cell line), BT549 (human breast cancer cell line), CT26 (mouse colon cancer cell line), EMT6 (mouse breast cancer cell line), HCT116 (colorectal cancer cell line), Jurket (acute leukemia cell line), KP-4 (pancreatic cancer cell line), LL/2 (mouse lung cancer cell line), MCF7 (breast cancer cell line), MKN45 (mouse B-cell lymphoma cell line), NCI-H358 (lung cancer cell line), NCI-H727 (gastric cancer cell line), NCI-1944 (lung cancer cell line), Pan 04.03 (pancreatic cancer cell line), SK-BR-3 (breast cancer cell line), THP-1 (leukemia cell line), TOV21G (ovarian cancer cell line), U87MG (glioblastoma cell line), and UM-UC-a (bladder cancer cell line).

All cell lines were purchased from Nanjing Kebai Biotechnology Co., Ltd. The following materials were also used in the experiment: RPMI-1640 media (HyClone, catalog number #SH30022.01), McCoy's 5A media (Thermo Fisher, catalog number #16600-082), MCDB 105 media (Sigma, catalog number #117-500), Medium 199 (Thermo Fisher, catalog number #11150-059), MEM media (Thermo Fisher, catalog number #11095-080), DMEM media (HyClone, catalog number #SH30027.01), fetal bovine serum (FBS) (ExCell, catalog number # M4530), Sodium Pyruvate (100Mm) (Thermo Fisher, catalog number #11360-070), and MEM NEAA (100X) (Thermo Fisher). Fisher Scientific, catalog number #11140-050), Insulin-Transferrin-Selenium (ITS-G) (100X) (Thermo Fisher Scientific, catalog number #41400045), DPBS (Thermo Fisher Scientific, catalog number #14190-144), trypsin (Thermo Fisher Scientific, catalog number #25200072).

Cells were cultured according to the instructions provided by the cell supplier (Nanjing Kebai Biotechnology Co., Ltd.). Thawing and passaging of adherent and suspension cells were performed by making reference to the above Activity Example 2. Suspension or adherent cells were collected, and cell viability was determined using the trypan blue exclusion method, ensuring that cell viability was above 90%. For adherent cells, a cell suspension with a density of $3.75 \times 10^4$ cells/mL was prepared using complete culture medium. 80 μL of the cell suspension was added to a 96-well transparent flat-bottom black-walled cell culture plate (Greiner, catalog #655090), resulting in a cell density of 3000 viable cells/well. For suspension cells, a cell suspension with a density of $1.25 \times 10^5$ cells/mL was prepared using complete culture medium. 80 μL of the cell suspension was added to a 96-well cell culture plate, resulting in a cell density of 10000 viable cells/well. A control group without cells and compounds but containing only complete culture medium (i.e., culture medium control) and a control group with cells but without compounds (i.e., cell control) were set up. The cell plates were incubated overnight in a cell culture incubator. First, a 2 mM DMSO stock solution of the compound (example compound and positive control compound 11a-1) was serially diluted with DMSO (Damas-beta, catalog #75927R) at a 3-fold dilution, down to the 9th concentration, and a 10th concentration DMSO control without the compound was set up. Then, the DMSO solutions containing different concentrations of the compound were diluted with complete culture medium at a 40-fold dilution, so that the DMSO content in each concentration of the compound solution was 2.5%. Finally, 20 μL of the above solution was added to the corresponding cell culture plate, resulting in a starting compound concentration of 10 μM, with a 3-fold dilution between adjacent concentrations, and a DMSO content of 0.5% in the cell culture plate. The cell plates were incubated in a cell culture incubator for another 72 hours. At the endpoint detection, the CellTiter-Glo reagent (CellTiter-Glo® Luminescent Cell Viability Assay kit, Promega, catalog #G7573) was thawed, and the cell plate was transferred to room temperature for 30 minutes to equilibrate. 50 μL of CellTiter-Glo was added to each well of the cell plate, and the plate was shaken on an orbital shaker for 2 minutes to ensure complete cell lysis. The cell plate was then left at room temperature

for 8 minutes to stabilize the luminescence signal. The luminescence value of each well was measured using a multi-mode plate reader (PerkinElmer Envision 2105 Multimode Plate Reader) at full wavelength.

The cell survival rate at each compound concentration was calculated using the following formula:

$$\text{Cell survival (\%)} = (\text{Lum}_{\text{ test drug)}} - \text{Lum}_{\text{ culture medium control)}}/(\text{Lum}_{\text{ cell control)}} - \text{Lum}_{\text{ culture medium control)}} \times 100\%$$

Data were analyzed using GmphPad Prism 8.0 software. Nonlinear S-curve regression was used to fit the data to obtain the dose-response curve, and the IC50 value was calculated from it.

The inhibitory activities of the representative compounds disclosed herein in various cell lines are shown in the table below:

| Cell lines | Example IC50 (μM) | | | cell lines | Example IC50 (μM) | | |
|---|---|---|---|---|---|---|---|
| | 1 | 68 | 100 | | 1 | 68 | 100 |
| **4T1** | 0.125 | | 0.140 | **MCF7** | 0.083 | | 0.050 |
| **A20** | 0.077 | 0.075 | 0.128 | **MKN45** | 0.326 | | |
| **A2780** | 0.128 | | | **NCI-H358(2D)** | 0.074 | 0.072 | 0.063 |
| **AsPC-1** | 0.121 | | | **NCI-H727** | 0.225 | | |
| **B16F10** | 0.142 | | | **NCI-1944** | 0.112 | | |
| **BT549** | 0.121 | | 0.047 | **Pan 04.03** | 0.340 | | |
| **CT26** | 0.123 | | 0.134 | **SK-BR-3** | 0.140 | 0.057 | 0.044 |
| **EMT6** | 0.131 | | | **THP-1** | 0.086 | 0.063 | 0.070 |
| **HCT116** | 0.111 | 0.078 | 0.044 | **TOV21G** | 0.112 | | 0.106 |
| **Jurket** | 0.165 | | | **U87MG** | 0.276 | | 0.075 |
| **KP-4** | 0.228 | | | **UM-UC-a** | 0.193 | | |
| **LL/2** | 0.207 | | | | | | |

The above results show that the compounds disclosed herein exhibit broad-spectrum anti-tumor cell proliferation activity.

**<u>Example 4:</u> Rat PK results of the representative compounds of this disclosure**

According to standard methods for rat PK studies in this field, the rat PK tests for the representative compounds of this disclosure and the positive control compound (11a-1) were conducted as follows.

Specifically, six male SD rats (6-8 weeks old, weighing 200 ± 20 g, purchased from Vital River Laboratories) were randomly divided into two groups of three rats each, for intravenous (IV) and oral gavage (PO) administration, respectively.

For the intravenous injection group: the test compound was weighed according to the administration dose (1 mg/kg), dissolved using the solvent listed in the table below to obtain a clear solution of 1 mg/mL, and administered to the rats via tail vein injection at a dose of 1 mg/kg.

For the oral administration group: the test compound was weighed according to the administration dose (5 mg/kg), dissolved using the solvent listed in the table below, with a final concentration of 1 mg/mL and an administration volume of 5 mL/kg, for oral administration.

Blood was collected via jugular vein puncture at various time points: 0.083, 0.25, 0.5, 1, 2, 4, 6, 8, and 24 hours. 0.2 mL of whole blood was collected at each time point, placed in a 1.5 mL anticoagulant tube containing EDTA, and placed on a sample plate containing wet ice until centrifugation; after centrifugation at 8000 rpm for 10 minutes at 4°C, 0.1 mL of plasma sample was collected and stored in an 81-well cryovial at -20°C until analysis.

| IV | | P O | |
|---|---|---|---|
| **Example** | **Formula** | **Example** | **Formula** |
| **11a-1** | 5% DMSO/10% Solutol/85% (20% Captisol) | **11a-1** | 5% DMSO/10% Solutol/85% (20% Captisol) |

(continued)

| IV | | P O | |
|---|---|---|---|
| **Example** | **Formula** | **Example** | **Formula** |
| **23** | 5% DMAC + 20% PEG 400 + 75% (30% Solutol in Water) | **23** | 5% DMSO + 10% Soultol + 85% (20% Captisol in Water pH = 8.0) |
| **68** | 5% DMSO + 20% PEG 400 + 75% (20% Captisol in water) | **68** | 10% DMSO + 10% Soultol + 80% (20% Captisol in Water pH = 8.0) |

Preparation of the stock solution of the test compound: 1 mg of the test compound was accurately weighed, dissolved in 1 mL of DMSO to prepare a 1 mg/mL stock solution S1, and stored in a 4°C refrigerator until use.

Preparation of the internal standard working solution: 0.1 mL of tolbutamide (2 mg/mL) internal standard stock solution was pipetted into 1000 mL of acetonitrile to prepare a 200 ng/mL internal standard working solution, which is used as a protein precipitant in sample pretreatment. The prepared internal standard working solution was stored at 4°C and valid for 12 months.

Preparation of the standard curve and quality control sample working solutions: 50 μL of the 1 mg/mL test compound DMSO stock solution S1 was diluted with 950 μL of 100% methanol solution to obtain a working solution with a concentration of 50000 ng/mL. Then, the 50000 ng/mL working solution was serially diluted with 100% methanol solution to obtain a series of standard curve working solutions with concentrations of 10, 20, 50, 100, 500, 1000, 5000, 10000, 20000, and 50000 ng/mL. 40 μL of the 1 mg/mL test compound DMSO stock solution S1 was diluted with 960 μL of 100% methanol solution to obtain a quality control sample working solution with a concentration of 40000 ng/mL. An appropriate amount of this working solution was diluted appropriately to prepare quality control sample working solutions with concentrations of 8000 and 16000 ng/mL. 60 μL of the 50 ng/mL test compound DMSO standard curve working solution was diluted with 40 μL of 100% methanol solution to obtain a quality control sample working solution with a concentration of 30 ng/mL.

Plasma sample treatment: standard curve and quality control samples for the test compound in plasma were prepared by diluting 5 μL of working solutions of different concentrations of the test compound for the standard curve and quality control samples in 45 μL of blank rat plasma, resulting in final concentrations of 1, 2, 5, 10, 50, 100, 500, 1000, 2000, and 5000 ng/mL for the standard curve and final concentrations of 3, 800, 1600, and 4000 ng/mL for the quality control samples.

Plasma sample pretreatment: 50 μL of the plasma sample was transferred to a 96-well plate, added with 500 μL of acetonitrile containing 200 ng/mL tolbutamide as an internal standard for protein precipitation, and centrifuged at 4000 rpm for 20 minutes at 2-8 °C. 300 μL of the supernatant was transferred to a new labeled 96-well plate, mixed thoroughly with 300 μL of 0.1% formic acid in water, and injected 10 μL into LC-MS/MS (API-4000 Qtrap-Shimadzu Controller-CBM20A). Phase A: 0.1% formic acid in water, Phase B: 100% methanol, Gradient: 0-0.3 min, 98%A+2%B, 0.3-0.6 min, B from 98% to 2%/A from 2% to 98%, maintained until 1.5 min, 1.50-1.51 min, to 98%B/2%A, 1.51-2.00 min, maintained 98% B/2%A; Column: Waters Xbridge C18 3.5 μm 2.1 × 50 mm, Injection volume: 10 μL; Flow rate: 0.5 mL/min.

The internal standard method was used for sample analysis. Data acquisition and analysis were performed using Analyst 1.6.3. The theoretical concentration of the standard curve was used as the x-axis, and the ratio of the peak area of the analyte to that of the internal standard was used as the y-axis. Linear regression was performed using the least squares method to fit the standard curve equation (weight 1/X2), R2 > 0.9900. The results are shown in the table below:

| **Example** | **IV** | | | | | **PO** | | | | |
|---|---|---|---|---|---|---|---|---|---|---|
| | **Dose** | **T1/2** | **AUC $_{0->t}$** | **Vz** | **Cl** | **Dose** | **T1/2** | **Cmax** | **AUC $_{0->t}$** | **F** |
| | mpk | h | ng*h/mL | L/kg | mL/min/kg | mpk | h | ng/mL | ng*h/mL | % |
| **11a-1** | 1 | 4.06 | 4233 | 1.42 | 4 | 10 | 1.1 | 44.2 | 30.0 | 0.07 |
| **23** | 1 | 8.49 | 2841 | 4.01 | 5.45 | 5 | 3.17 | 1136 | 8009 | 52.7 |
| **68** | 1 | 1.63 | 1377 | 1.57 | 12.6 | 5 | 4.31 | 150 | 2097 | 30.5 |

The results in the table above show that the oral pharmacokinetic properties of the representative compounds disclosed herein have been greatly improved.

**Example 5: Selectivity study of the compounds of the present invention for tyrosine phosphatase**

This experiment evaluated the selectivity of the disclosed compounds against tyrosine phosphatases, including SHP1, HePTP, Laforin, LMWPTP, LYP, PTP1B, PRL2, SSU72, VHR, FAP1, STEP, CDC14A, CD45, MEG2, and PP5, all expressed and purified from E. coli.

Phosphatase activity was measured using p-nitrophenyl phosphate (pNPP) (Fisher Scientific) as the substrate in 50 mM 3,3-dimethylglutaric acid buffer (pH 7.0, containing 1 mM EDTA), with ionic strength adjusted to 0.15 M with NaCl. In a 384-well plate, 20 μL of each enzyme solution in the aforementioned 3,3-dimethylglutaric acid buffer was added to a 30 μL reaction mixture containing 5 μM of the compound (example compounds and positive control compound 11a-1) and a specific concentration of pNPP (the pNPP concentration used corresponds to the Km value of each enzyme studied) to initiate the reaction. After 10-60 minutes, the reaction was quenched by adding 20 μL of 5 N NaOH. Wells without enzyme were used as controls for correction. The amount of the product, p-nitrophenol, was determined by measuring the absorbance at 405 nm using a Spectra MAX340 microplate spectrophotometer (Molecular Devices), with a molar extinction coefficient of 18000 $M^{-1}cm^{-1}$.

Data were analyzed using GraphPad Prism software. Dose-response curves were obtained by fitting data using the nonlinear S-curve regression Dose-response-inhibition equation, and IC50 values were calculated.

The selectivity of the representative compounds disclosed herein against tyrosine phosphatases is shown in the table below:

| Example | | SHP2 | SHP1 | HePTP | Laforin | LMPTP* | LYP | PTP1B | PRL2 |
|---|---|---|---|---|---|---|---|---|---|
| **11a-1** | IC50 (μM) | 0.108 | 0.80 | 1.03 | 12.2 | 2.34 | 1.05 | 2.29 | |
| | $IC50_{PTP}/IC50_{SHP2}$ | | 7.4 | 9.5 | 112 | 21.6 | 9.7 | 21.2 | |
| **4** | IC50 (μM) | 0.029 | 0.60 | 4.5 | >20 | >20 | 0.5 | 3.2 | >50 |
| | $IC50_{PTP}/IC50_{SHP2}$ | | 20.7 | 155.2 | 689.7 | 689.7 | 17.2 | 110.3 | 1724.1 |
| **47** | IC50 (μM) | 0.063 | 0.12 | 0.80 | 0.61 | 1.09 | 0.189 | 0.37 | >100 |
| | $IC50_{PTP}/IC50_{SHP2}$ | | 1.9 | 12.6 | 9.6 | 17.3 | 3.0 | 5.8 | 1587 |
| **67** | IC50 (μM) | 0.034 | 0.11 | 0.95 | 6.21 | 1.37 | 0.076 | 0.39 | >100 |
| | $IC50_{PTP}/IC50_{SHP2}$ | | 3.2 | 27.9 | 182 | 40.2 | 2.2 | 11.4 | 2941 |
| **68** | IC50 (μM) | 0.048 | 0.383 | 1.17 | >25 | 0.37 | 1.54 | 1.50 | >25 |
| | $IC50_{PTP}/IC50_{SHP2}$ | | 8.0 | 29.3 | 520.8 | 7.7 | 32.1 | 31.3 | 520.8 |
| **114** | IC50 (μM) | 0.072 | 0.100 | 1.06 | 2.44 | 2.13 | 0.100 | 0.470 | >100 |
| | $IC50_{PTP}/IC50_{SHP2}$ | | 1.3 | 14.7 | 33.8 | 29.5 | 1.3 | 6.5 | 1388 |
| **123** | IC50 (μM) | 0.141 | 0.32 | 1.33 | >100 | 2.48 | 1.07 | 1.20 | >100 |
| | $IC50_{PTP}/IC50_{SHP2}$ | | 2.3 | 9.4 | 709.2 | 17.6 | 7.6 | 8.5 | 709.2 |
| **124** | IC50 (μM) | 0.716 | 5.62 | >100 | >100 | >20 | >100 | >100 | >100 |
| | $IC50_{PTP}/IC50_{SHP2}$ | | 7.8 | 139.7 | 139.7 | 27.9 | 139.7 | 139.7 | 139.7 |
| **125** | IC50 (μM) | 0.051 | 0.17 | 35.9 | >100 | 2.24 | 2.50 | 6.28 | >100 |
| | $IC50_{PTP}/IC50_{SHP2}$ | | 3.3 | 703.9 | 1960.8 | 43.9 | 49.0 | 123.1 | 1960.8 |
| **129** | IC50 (μM) | 0.059 | 0.7 | >20 | >20 | >20 | 1.2 | 8.5 | >50 |
| | $IC50_{PTP}/IC50_{SHP2}$ | | 11.9 | 339.0 | 339.0 | 339.0 | 20.3 | 144.1 | 847.5 |
| **130** | IC50 (μM) | 0.025 | 0.6 | >20 | >20 | >20 | 1.2 | 11.6 | >50 |
| | $IC50_{PTP}/IC50_{SHP2}$ | | 24.0 | 800 | 800 | 800 | 48 | 464 | 2000 |
| **131** | IC50 (μM) | 0.026 | 0.8 | 6.1 | >20 | >20 | 0.5 | 4.7 | >50 |
| | $IC50_{PTP}/IC50_{SHP2}$ | | 30.8 | 234.6 | 769.2 | 769.2 | 19.2 | 180.8 | 1923.1 |
| **132** | IC50 (μM) | 0.027 | 0.6 | 5.7 | >20 | >20 | 0.5 | 2.7 | >50 |
| | $IC50_{PTP}/IC50_{SHP2}$ | | 22.2 | 211.1 | 740.7 | 740.7 | 18.5 | 100 | 1851.9 |

(continued)

| Example | | SSU72 | VHR | FAP1 | STEP | CDC14A | CD45 | MEG2 | PP5 |
|---|---|---|---|---|---|---|---|---|---|
| **11a-1** | IC50 (μM) | 1.3 | 3.2 | | 4.0 | 16 | | | |
| | $IC50_{PTP}/IC50_{SHP2}$ | 12.0 | 29.6 | | 37.0 | 148 | | | |
| **4** | IC50 (μM) | <3 | 4.5 | 19.5 | 4.5 | >50 | >50 | 22.1 | >50 |
| | $IC50_{PTP}/IC50_{SHP2}$ | 103.4 | 155.2 | 672.4 | 155.2 | 1724.1 | 1724.1 | 762.1 | 1724.1 |
| **47** | IC50 (μM) | 0.09 | 1.15 | 1.53 | 0.77 | 100 | 1.06 | 0.99 | >100 |
| | $IC50_{PTP}/IC50_{SHP2}$ | 1.4 | 18.2 | 24.2 | 12.2 | 1587 | 16.8 | 15.7 | 1587 |
| **67** | IC50 (μM) | 0.016 | 4.45 | 2.34 | 6.94 | 100 | 2.62 | 1.77 | >100 |
| | $IC50_{PTP}/IC50_{SHP2}$ | 0.47 | 130 | 68.8 | 204.1 | 2941 | 77.0 | 52.0 | 2941 |
| **68** | IC50 (μM) | | >25 | 0.56 | 8.23 | >25 | 3.48 | 0.89 | >25 |
| | $IC50_{PTP}/IC50_{SHP2}$ | | 520.8 | 11.7 | 171.5 | 520.8 | 72.5 | 18.5 | 520.8 |
| **114** | IC50 (μM) | 0.09 | 2.14 | 1.9 | 1.83 | 100 | 1.81 | 1.66 | >100 |
| | $IC50_{PTP}/IC50_{SHP2}$ | 1.2 | 29.7 | 26.3 | 25.4 | 1388 | 25.1 | 23.0 | 1388 |
| **123** | IC50 (μM) | >100 | 2.44 | 7.30 | 15.5 | >100 | 4.27 | 2.41 | >100 |
| | $IC50_{PTP}/IC50_{SHP2}$ | 709.2 | 17.3 | 51.8 | 109.9 | 709.2 | 30.3 | 17.1 | 709.2 |
| **124** | IC50 (μM) | >100 | >100 | >100 | >100 | >100 | >100 | >100 | >100 |
| | $IC50_{PTP}/IC50_{SHP2}$ | 139.7 | 139.7 | 139.7 | 139.7 | 139.7 | 139.7 | 139.7 | 139.7 |
| **125** | IC50 (μM) | >100 | 88.2 | 7.44 | 6.62 | >100 | 16.1 | 8.54 | >100 |
| | $IC50_{PTP}/IC50_{SHP2}$ | 1960.8 | 1729.4 | 145.9 | 129.8 | 1960.8 | 315.7 | 167.5 | 1960.8 |
| **129** | IC50 (μM) | 35.8 | <3 | 4.2 | <3 | 34.6 | 41.5 | 7.2 | >50 |
| | $IC50_{PTP}/IC50_{SHP2}$ | 606.8 | 50.8 | 71.2 | 50.8 | 586.4 | 703.4 | 122.0 | 847.5 |
| **130** | IC50 (μM) | <3 | <3 | <3 | <3 | >50 | >50 | <3 | >50 |
| | $IC50_{PTP}/IC50_{SHP2}$ | 120 | 120 | 120 | 120 | 2000 | 2000 | 120 | 2000 |
| **131** | IC50 (μM) | <3 | 9.9 | 11.0 | 8.4 | >50 | 25.2 | 9.9 | >50 |
| | $IC50_{PTP}/IC50_{SHP2}$ | 115.4 | 380.8 | 423.1 | 323.1 | 1923.1 | 969.2 | 380.8 | 1923.1 |
| **132** | IC50 (μM) | <3 | 10.3 | 13.5 | 10.6 | >50 | 23.7 | 15.1 | >50 |
| | $IC50_{PTP}/IC50_{SHP2}$ | 111.1 | 381.5 | 500 | 392.6 | 1851.9 | 877.8 | 559.3 | 1851.9 |

The results above show that the representative compounds of this disclosure exhibit good selectivity for SHP2 on the PTP phosphatase panel.

**Example 6: hERG activity of representative compounds**

This experiment was a non-GLP trial conducted with reference to GLP guidelines and in accordance with the following technical guidelines: National Medical Products Administration: Technical Guidelines for Non-Clinical Studies of Potential Effects of Drugs on QT Interval Prolongation (2014), and ICH S7B Guidelines for Non-Clinical Evaluation of Potential Drug-Induced Delayed Ventricular Repolarization (QT Interval Prolongation) in Humans (2005).

Sample preparation: the weighed test substance was prepared into a 5 mM or 10 mM stock solution with DMSO, then diluted with DMSO to a 3.33 mM diluent. The stock solution was then diluted with extracellular fluid to prepare a 10 μM working solution, ensuring a DMSO concentration of 0.3%. The solution was sonicated for 20 min. The solubility of the test sample was visually inspected; all concentrations dissolved completely without any visible precipitate.

Preparation of positive control: 3.99 mg Cisapride (positive control) was prepared into a 10 mM stock solution using 807.96 μL DMSO. This stock solution was then successively diluted with DMSO to 1000, 333.33, 33.33, 3.33, and 0.33 μM dilutions. Before testing, 30 μL of each of the last four dilutions was added to 10 mL of extracellular fluid, ensuring a DMSO concentration of 0.3%. The final working solution concentrations of Cisapride were 1000, 100, 10, and 1 nM.

Cisapride was visually inspected for solubility; all concentrations dissolved completely without any visible precipitate.

The blank control stock solution (DMSO) should be stored at room temperature. The blank control working solution should be prepared on the same day and stored at room temperature. The test substance and positive control stock solutions should be stored at -20 °C. The test substance working solution should be prepared on the same day and stored at room temperature. The positive control working solution should be prepared on the same day and stored at room temperature.

The HEK-293 cell line stably expressing the hERG potassium channel (purchased from Creacell, catalog number # A-0320) was used. Specifically, the HEK-293 cell line stably expressing the hERG potassium channel was cultured in DMEM medium containing 10% fetal bovine serum and 0.8 mg/mL G418 (37 °C, 5% $CO_2$). The old medium was removed and the cells were washed once with PBS, then 1 mL of TrypLE™ Express solution (Gibco, catalog number # 12604-021) was added, and the cells were incubated at 37 °C for 0.5 min. When the cells detached from the bottom of the dish, approximately 5 mL of the pre-warmed 37 °C DMEM medium containing 10% fetal bovine serum and 0.8 mg/mL G418 was added. The cell suspension was gently pipetted to separate the aggregated cells. The cell suspension was transferred to sterile centrifuge tubes and centrifuged at 1000 rpm for 5 min to collect the cells. For expansion or maintenance culture, cells were seeded into 6 cm cell culture dishes, with a seeding density of $2.5 \times 10^5$ cells per dish (final volume: 5 mL). To maintain cell electrophysiological activity, the cell density must not exceed 80%.

Prior to patch-clamp assays, cells were isolated using TrypLE™ Express, and $4 \times 10^3$ cells were seeded onto coverslips and cultured in 24-well plates (final volume: 500 μL). Patch-clamp assays were performed in 18 hours.

The hERG activity of the representative compounds of this disclosure was detected using conventional manual patch-clamp techniques in the art (Patch-Clamp Methods and Protocols, 2014, 2nd edition, Humana Press, edited by Marzia Martina and Stefano Taverna).

| **Example** | % **hERG Inhibition @ 10 μM** |
|---|---|
| **23** | -0.72 |
| **67** | 16.92 |
| **68** | 8.79 |

The above results show that the representative compounds disclosed herein have no inhibitory activity against hERG at a concentration of 10 μM.

**<u>Example 7:</u> CY P450 inhibitory activity of representative compounds**

According to standard methods in the field for the study of cytochrome P450 enzyme systems, such as the method described in Kerns, Edward H. and Di Li et al., Drug-like Properties: Concepts, Structure Design and Methods: from ADME to Toxicity Optimization (2008), San Diego, Academic Press, and Lin, Tong et al., In Vitro Assessment of Cytochrome P450 Inhibition: Strategies for Increasing LC/MS-Based Assay Throughput Using a One-Point IC50 Method and Multiplexing High-Performance Liquid Chromatography (J. Pharm. Sci. 2007, 96 (9), 2485). The inhibitory effect of the disclosed compounds on the cytochrome P450 enzyme system was similarly examined below.

Human liver microsomes were purchased from Corning or Xenotech. The test compounds were evaluated by detecting the metabolism of substrates for different Cyp450 isoforms by human liver microsomes.

The specific inhibitor solution, substrate solution, and internal standard information are shown in the table below:

| **CYP** | **Specific Inhibitors** | **Stock solution con. (DMSO)** | **Final con. (μM)** | **Substrate** | **Stock solution con.** | **Final con. (μM)** | **Internal Standard** |
|---|---|---|---|---|---|---|---|
| **1A2** | α-Naphthylflavone | 0.3 mM | 0.3 | Phenacetin | 6 mM/ACN | 30 | Acetaminophen-[D4] |
| **2C9** | Sulfaphenazole | 10 mM | 10 | diclofenac | 10 mM / H2O | 10 | 4'-OH-diclofenac-[13C6] |
| **2C19** | Omeprazole | 100 mM | 100 | S-Mefphenytoin | 35 mM/ACN | 35 | OH-Toluene-$[_{D3]}$ |

(continued)

| CYP | Specific Inhibitors | Stock solution con. (DMSO) | Final con. (μM) | Substrate | Stock solution con. | Final con. (μM) | Internal Standard |
|---|---|---|---|---|---|---|---|
| 2D6 | Quinidine | 2.5 mM | 2.5 | Bufuralol | 10 mM / H2O | 10 | 1-OH-Ibuprofen-maleic acid-[D9] |
| 3A4 | Ketoconazole | 2.5 mM | 2.5 | testosterone | 10 mM/ACN | 80 | Salicylic acid |
| | | | | Midazolam | 1 mM/ACN | 5 | 1-OH-Midazolam-[13C3] |
| 2B6 | Clopidogrel | 10 mM | 10 | Bupropion | 50 mM/ACN | 70 | OH-Bupropion-D6 |
| 2C8 | Nicardipine | 100 mM | 100 | Paclitaxel | 10 mM/ACN | 10 | 6α-Hydroxypaclitaxel-[D5] |

0.1 M potassium phosphate buffer (K-buffer, pH 7.4) was pre-warmed.

100 mM K-buffer was prepared by mixing 9.5 mL of stock solution A (1 M potassium dihydrogen phosphate, prepared by dissolving 136.5 g of potassium dihydrogen phosphate in 1 L of Milli-Q water) with 40.5 mL of stock solution B (1 M potassium hydrogen phosphate, prepared by dissolving 174.2 g of potassium hydrogen phosphate in 1 L of Milli-Q water). The total volume was adjusted to 500 mL with Milli-Q water, and the buffer was titrated to pH 7.4 using KOH or $H_3PO_4$.

Test compound solution and specific inhibitor solution (400× dilution) were prepared in a 96-well plate. For the test compound, 8 μL of 10 mM test compound was transferred into 12 μL of ACN. For specific inhibitors of CYP1A2, CYP2C9, and CYP2D6: 12 μL of 1 mM α-naphthoflavone, 10 μL of 40 mM sulfaphenazole, 10 μL of 10 mM quinidine, and 8 μL of DMSO were mixed. For specific inhibitors of CYP3A4, CYP2B6, CYP2C8, and CYP2C19: 8 μL of DMSO stock solution was added to 12 μL of ACN.

A 4× diluted NADPH solution was prepared by dissolving 66.7 mg of NADPH in 10 mL of 0.1 M K-buffer (pH 7.4). Human liver microsome (HLM) solutions were prepared in K-buffer, wherein 0.1 mg/mL for CYP1A2, CYP2C9, CYP2D6, CYP3A4, and CYP2B6, 0.5 mg/mL for CYP2C19, 0.2 mg/mL for CYP2C8. 4× diluted substrate dilution solutions (2 mL per isozyme) were prepared in K-buffer (HLM was added on ice if required) with the following concentrations: 120 μM for CYP1A2, 40 μM for CYP2C9, 140 μM with 1.6 mg/mL HLM for CYP2C19, 40 μM for CYP2D6, 320 μM and 20 μM for CYP3A4 (two concentrations), 280 μM for CYP2B6, 40 μM with 0.4 mg/mL HLM for CYP2C8.

A 0.2 mg/mL HLM solution was prepared on ice (by adding 10 μL of 20 mg/mL stock to 990 μL of 0.1 M K buffer). To the assay wells, 400 μL of the 0.2 mg/mL HLM solution was added, followed by 2 μL of the 400× diluted test compound solution, which was added on ice to the designated wells. To the assay wells, 200 μL of the 0.2 mg/mL HLM solution was added, followed by 1 μL of the specific inhibitor solution, which was added on ice to the designated wells. The following solutions were added in duplicate to the 96-well analysis plate kept on ice: 30 μL of the 2× diluted test compound solution and the specific inhibitor solution were added to the 0.2 mg/mL HLM solution, 15 μL of the 4× diluted substrate solution was added. The 96-well assay plate and the NADPH solution were pre-incubated at 37 °C for 5 min. The reaction was initiated by adding 15 μL of the pre-warmed 8 mM NADPH solution to the assay plate. Reactions were performed at 37 °C for the following durations according to the P450 isoform: 5 min for CYP3A4, 10 min for CYP1A2, CYP2B6, CYP2C8, CYP2C9, and CYP2D6, 45 min for CYP2C19. Reactions were terminated by adding 120 μL of ACN containing internal standards. The final internal standard concentrations were: 0.1 μM for CYP2C19, CYP1A2, CYP3A4 (midazolam), and CYP2D6, 200 ng/mL for CYP3A4 (testosterone), 0.31 μM for CYP2C9, 0.36 μM (100 ng/mL) for CYP2B6, 40 ng/mL for CYP2C8.

After quenching, the plate was shaken on a vibrating shaker (IKA, MTS 2/4) at 600 rpm for 10 min, and then centrifuged at 5594 g for 15 min (Thermo Multifuge × 3R). From each well, 50 μL of supernatant was transferred to a 96-well sample plate containing 50 μL of ultrapure water (Millipore, ZMQS50F01) for LC/MS analysis (Column: ACQUITY UPLC BEH C18 (2.1 × 50 mm, 1.7 μm), Mobile phase A: $H_2O$-0.025% FA-1 mM $NH_4OAc$, Mobile phase B: MeOH-0.025% FA-1 mM $NH_4OAc$, Flow rate: 0.6 mL/min).

The CYP inhibition results of the representative compounds of this invention are shown in the table below:

| Example | % CYP inhibition @ 10 μM | | | | | | | |
|---|---|---|---|---|---|---|---|---|
| | **1A2** | **2B6** | **2C8** | **2C9** | **2C19** | **2D6** | **3A4** (midazolam) | **3A4** (testosterone) |
| **23** | 17.12 | 1.32 | 43.81 | -3.32 | 1.78 | -5.43 | 5.28 | -22.59 |
| **67** | 9.47 | 15.31 | 39.18 | 7.84 | 13.95 | -12.61 | 13.04 | -0.87 |
| **68** | 11.39 | 7.06 | 42.08 | 6.51 | 13.46 | -4.07 | -10.17 | 2.46 |

The results above show that the representative compounds of this disclosure do not have a significant inhibitory effect on major P450 enzymes, which indicates that the risk of drug interactions is greatly reduced and will greatly facilitate the combined use of the compounds of this disclosure with other active agents.

Although the invention has been described in conjunction with specific embodiments thereof, it should be understood that the invention is capable of further modifications, and this application is intended to cover any variations, uses, or alterations to the principles generally followed by the invention and to include such deviations from the disclosure within the scope of known or customary practice in the field to which the invention pertains, which may apply to the basic features described above and within the scope of the claims.

## Claims

1. A compound of formula (I), a pharmaceutically acceptable salt, isomer, solvate, hydrate or stable isotopic variant thereof,

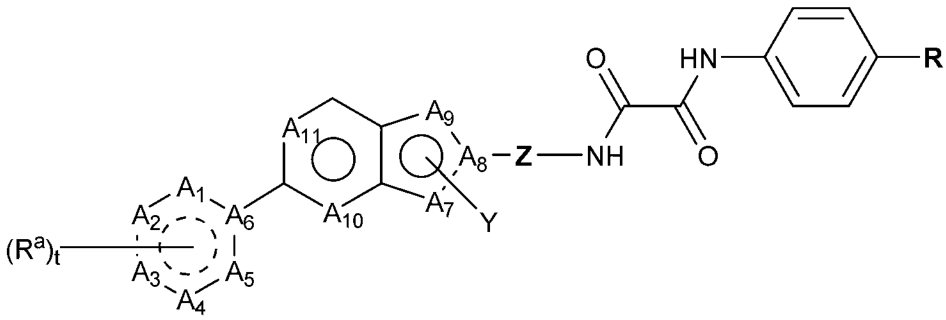

(I)

or a pharmaceutically acceptable salt, isomer, solvate, hydrate or stable isotopic variant thereof, wherein:

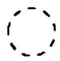 represents a saturated, partially unsaturated, or aromatic ring system;

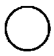 represents an aromatic ring system;

$A_1$, $A_2$, $A_3$, $A_4$, $A_5$ are each independently selected from absence, C, N, O, and S, and A6 is selected from C and N, provided that (1) at most two of $A_1$~$A_5$ are absent, and (2) at most four of $A_1$~$A_6$ are heteroatoms;

$R^a$ is selected from -H, =O, =S, =N-OH, -OH, $-NH_2$, halogen, -CN and $-C_{1-6}$ alkyl optionally substituted by halogen;

$A_7$, $A_8$ and $A_9$ are each independently selected from C, N, O, and S, provided that at least one of $A_7$, $A_8$ and $A_9$ is not C;

$A_{10}$ is selected from C-$R^b$ and N;

$A_{11}$ is selected from C-X and N;

$R^b$ is selected from H, halogen, CN and $-C_{1-6}$ alkyl substituted by halogen;

X is selected from H, halogen, CN, -OH, $-OC_{1-6}$ alkyl and $-C_{1-6}$ alkyl, wherein the $C_{1-6}$ alkyl is optionally substituted;

Y is an optionally substituted group selected from $-C_{1-6}$ alkyl, $-(CH_2)_t$-3-15 membered carbocyclyl, $-(CH_2)_t-C_{6-10}$ aryl, $-(CH_2)_t$-5-12 membered heteroaryl having one or more heteroatom(s) independently selected from N, O and S, and $-(CH_2)_t$-3-15 membered heterocyclyl having one or more heteroatom(s) independently selected from N, O and S;

Z is an optionally substituted divalent group selected from $-C_{6-10}$ aryl-, -3-10 membered carbocyclyl-, -5-12 membered heteroaryl having one or more heteroatom(s) independently selected from N, O and S, and -3-15

membered heterocyclyl having one or more heteroatom(s) independently selected from N, O and S;
R is an optionally substituted group selected from -$C_{6-10}$ aryl, -3-15 membered carbocyclyl, -5-12 membered heteroaryl having one or more heteroatom(s) independently selected from N, O, and S, and -3-15 membered heterocyclyl having one or more heteroatom(s) independently selected from N, O, and S; and
t is each optionally an integer from 0 to 3.

**2.** The compound of claim 1, a pharmaceutically acceptable salt, isomer, solvate, hydrate or stable isotopic variant thereof, wherein:

Y is an optionally substituted group selected from:

- $C_{1-6}$ alkyl,
- $(CH_2)_t$-3-8 membered monocyclic saturated or partially unsaturated carbocycle,
- $(CH_2)_t$-5-10 membered bridged saturated or partially unsaturated carbocycle,
- $(CH_2)_t$-6-15 membered spiro or fused saturated or partially unsaturated carbocycle,
- $(CH_2)_t$-$C_{6-10}$ aryl,
- $(CH_2)_t$-5-12 membered heteroaryl having 1-4 heteroatoms independently selected from N, O, and S,
- $(CH_2)_t$-3-8 membered monocyclic saturated or partially unsaturated heterocycle having 1-3 heteroatoms independently selected from N, O, and S,
- $(CH_2)_t$-5-10 membered bridged saturated or partially unsaturated heterocycle having 1-3 heteroatoms independently selected from N, O, and S, and
- $(CH_2)_t$-6-15 membered spiro or fused saturated or partially unsaturated heterocycle having 1-4 heteroatoms independently selected from N, O and S;

Z is an optionally substituted divalent group selected from:

- $C_{6-10}$ aryl-,
- 3-8 membered monocyclic saturated or partially unsaturated carbocycle-,
- 5-10 membered bridged saturated or partially unsaturated carbocycle-,
- 5-12 membered heteroaryl having 1-4 heteroatoms independently selected from N, O, and S-,
- 3-8 membered monocyclic saturated or partially unsaturated heterocycle having 1-3 heteroatoms independently selected from N, O, and S-,
- 5-10 membered bridged saturated or partially unsaturated heterocycle having 1-3 heteroatoms independently selected from N, O, and S-, and
- 6-15 membered spiro or fused saturated or partially unsaturated heterocycle having 1-4 heteroatoms independently selected from N, O and S-;

R is an optionally substituted group selected from:

- $C_{6-10}$ aryl,
- 3-8 membered monocyclic saturated or partially unsaturated carbocycle,
- 5-10 membered bridged saturated or partially unsaturated carbocycle,
- 6-15 spiro or fused saturated or partially unsaturated carbocycle,
- 5-12 membered heteroaryl having 1-4 heteroatoms independently selected from N, O, and S,
- 3-8 membered monocyclic saturated or partially unsaturated heterocycle having 1-3 heteroatoms independently selected from N, O, and S,
- 5-10 membered bridged saturated or partially unsaturated heterocycle having 1-3 heteroatoms independently selected from N, O, and S, and
- 6-15 membered spiro or fused saturated or partially unsaturated heterocycle having 1-4 heteroatoms independently selected from N, O and S.

**3.** The compound of claim 1 or 2, a pharmaceutically acceptable salt, isomer, solvate, hydrate or stable isotopic variant thereof, wherein:

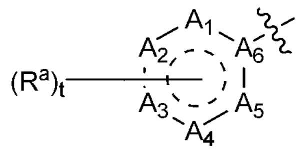

is a five-membered partially unsaturated or aromatic group optionally substituted by 0-1 $R^a$, having 3-4 cyclic heteroatoms selected from N, O, and S, preferably a five-membered aromatic group having 4 nitrogen heteroatoms, and $A_6$ is C; $R^a$ is selected from -H, -CN, halogen, =O and $-C_{1-6}$ alkyl substituted by halogen.

**4.** The compound of any one of claims 1-3, a pharmaceutically acceptable salt, isomer, solvate, hydrate or stable isotopic variant thereof, wherein: one of $A_7$ and $A_9$ is a heteroatom selected from N, O and S, the other is C, and $A_8$ is a carbon atom; preferably $A_7$ is selected from C-Y and N-Y, $A_9$ is selected from C, O and S, and only one of $A_7$ and $A_9$ is a heteroatom, and $A_8$ is a carbon atom.

**5.** The compound of any one of claims 1-4, a pharmaceutically acceptable salt, isomer, solvate, hydrate or stable isotopic variant thereof, wherein: $A_{10}$ is C-$R^b$ and $A_{11}$ is C-X; $R^b$ is selected from H, and X is selected from halogen and -OH.

**6.** The compound of any one of claims 1-5, a pharmaceutically acceptable salt, isomer, solvate, hydrate or stable isotopic variant thereof, wherein: Y is an optionally substituted group selected from $-C_{1-6}$ alkyl, $-(CH_2)_t$-3-8 membered monocyclic saturated or partially unsaturated carbocycle, $-(CH_2)_t$-5-10 membered bridged saturated or partially unsaturated carbocycle, $-(CH_2)_t-C_{6-10}$ aryl; preferably an optionally substituted group selected from $-C_{1-6}$ alkyl, $-(CH_2)_t$-3-6 membered monocyclic saturated carbocycle, $-(CH_2)_t$-5-8 membered bicyclic bridged saturated carbocycle, and $-(CH_2)_t$-phenyl.

**7.** The compound of any one of claims 1-6, a pharmaceutically acceptable salt, isomer, solvate, hydrate or stable isotopic variant thereof, wherein: the $-C_{1-6}$ alkyl of Y is optionally substituted by halogen or CN, and the cyclic group of Y is optionally substituted by 1-3, preferably 1 or 2, more preferably 1 group selected from $-C_{1-6}$ alkyl and $-OC_{1-6}$ alkyl.

**8.** The compound of any one of claims 1-7, a pharmaceutically acceptable salt, isomer, solvate, hydrate or stable isotopic variant thereof, wherein: Z is selected from $-C_{6-10}$ aryl-, -3-8 membered monocyclic saturated or partially unsaturated carbocycle-, -5-10 membered bridged saturated or partially unsaturated carbocycle-, and -3-8 membered monocyclic saturated or partially unsaturated heterocycle having 1-3 heteroatoms independently selected from N, O, and S, which is optionally substituted; preferably Z is selected from phenyl, -3-6 membered monocyclic saturated or partially unsaturated carbocycle-, -5-8 membered bridged saturated carbocycle-, and -4-7 membered monocyclic saturated or partially unsaturated heterocycle having 1-2 heteroatoms independently selected from N, O, and S, which is optionally substituted.

**9.** The compound of any one of claims 1-8, a pharmaceutically acceptable salt, isomer, solvate, hydrate or stable isotopic variant thereof, wherein: Z is optionally substituted by one or more group(s) independently selected from halogen, $-C_{1-6}$ alkyl or $-OC_{1-6}$ alkyl, wherein the $-C_{1-6}$ alkyl is optionally substituted by halogen or CN; preferably Z is not substituted.

**10.** The compound of any one of claims 1-9, a pharmaceutically acceptable salt, isomer, solvate, hydrate or stable isotopic variant thereof, wherein: R is an optionally substituted group selected from -5-6 membered monocyclic heteroaryl or 8-10 membered bicyclic heteroaryl having 1-4 heteroatoms independently selected from N, O, and S, -3-8 membered monocyclic saturated or partially unsaturated heterocycle having 1-3 heteroatoms independently selected from N, O, and S, -5-10 membered bridged saturated or partially unsaturated heterocycle having 1-3 heteroatoms independently selected from N, O, and S, and -6-15 membered spiro or fused saturated or partially unsaturated heterocycle having 1-4 heteroatoms independently selected from N, O, and S; preferably an optionally substituted group selected from -5-6 membered monocyclic heteroaryl having 1-4 heteroatoms independently selected from N, O, and S, -4-7 membered monocyclic saturated or unsaturated heterocycle having 1-2 heteroatoms independently selected from N, O and S, -5-8 membered bicyclic bridged saturated heterocycle having 1-2 heteroatoms independently selected from N, O and S, and 7-11 membered spiro or fused saturated heterocycle having 1-3 heteroatoms independently selected from N, O and S.

**11.** The compound of any one of claims 1-10, a pharmaceutically acceptable salt, isomer, solvate, hydrate or stable isotopic variant thereof, wherein: R is substituted by 0 to 4, preferably 0 to 3, more preferably 0-2 groups independently

selected from: halogen, CN, -OH, $-NH_2$, $-C_{1-6}$ alkyl, $-C_{2-6}$ alkenyl, $-C_{2-6}$ alkynyl, $-OC_{1-6}$ alkyl, $-NHC_{1-6}$ alkyl, $-N(C_{1-6}$ alkyl$)_2$, $-(CH_2)_t$-3-8 membered monocyclic saturated or partially unsaturated carbocycle, $-(CH_2)_t$-5-8 membered bridged saturated or partially unsaturated carbocycle, $-(CH_2)_t$-3-8 membered monocyclic saturated or partially unsaturated heterocycle having 1-3 heteroatoms independently selected from N, O, and S, $-(CH_2)_t$-5-8 membered bridged saturated or partially unsaturated heterocycle having 1-3 heteroatoms independently selected from N, O, and S, wherein the two substituents attached to the same ring carbon atom, together with the carbon atom to which they are attached, may form a 3-8 membered saturated spirocarbocycle, and the $-C_{1-6}$ alkyl, $-C_{2-6}$ alkenyl, $-C_{2-6}$ alkynyl or cyclic group of the substituents is each independently and optionally substituted by halogen, CN, -OH, or $-OC_{1-6}$ alkyl; preferably is substituted by groups selected from: halogen, CN, -OH, $-C_{1-6}$ alkyl, $-OC_{1-6}$ alkyl, $-(CH_2)_t$-4-7 membered monocyclic saturated heterocycle having 1-2 heteroatoms independently selected from N, O, and S, wherein the two substituents attached to the same carbon atom, together with the carbon atom to which they are attached, may form a 3-6 membered saturated spirocarbocycle, and the $C_{1-6}$ alkyl of the substituents is optionally substituted by halogen, CN, -OH, or $-OC_{1-6}$ alkyl.

**12.** The compound of any one of claims 1-10, a pharmaceutically acceptable salt, isomer, solvate, hydrate or stable isotopic variant thereof, wherein: R has formula (A)

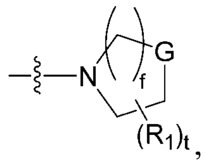

wherein,

f is selected from an integer from 0 to 3, preferably an integer from 0 to 2;
t is selected from an integer from 0 to 3, preferably an integer from 0 to 1;
G is selected from O, N-$R_2$ and $CR_3R_4$; preferably O and $CR_3R_4$, and most preferably $CR_3R_4$.
$R_1$ is selected from H and $-C_{1-6}$ alkyl optionally substituted by halogen, CN, -OH or $-OC_{1-6}$ alkyl, wherein the two $R_1$ attached to the same ring carbon atom, together with the carbon atom to which they are attached, may form 3-8 membered saturated spirocarbocycle; preferably $R_1$ is selected from H and $-C_{1-6}$ alkyl optionally substituted by -OH or $-OC_{1-6}$ alkyl; most preferably $R_1$ is selected from H and $-C_{1-6}$ alkyl;
$R_3$ and $R_4$ are each independently selected from H, halogen, CN, -OH, $-NH_2$ , $-C_{1-6}$ alkyl, $-C_{2-6}$ alkenyl, $-C_{2-6}$ alkynyl, $-OC_{1-6}$ alkyl, $-NHC_{1-6}$ alkyl, $-N(C_{1-6}$ alkyl$)_2$, $-(CH_2)_t$-3-8 membered monocyclic saturated or partially unsaturated carbocycle, $-(CH_2)_t$-5-8 membered bridged saturated or partially unsaturated carbocycle, $-(CH_2)_t$-3-8 membered monocyclic saturated or partially unsaturated heterocycle having 1-3 heteroatoms independently selected from N, O, and S, $-(CH_2)_t$-5-8 membered bridged saturated or partially unsaturated heterocycle having 1-3 heteroatoms independently selected from N, O, and S, or $R_3$ and $R_4$ together with the ring carbon atom to which they are attached form a 3-8 membered saturated spirocarbocycle, and the $-C_{1-6}$ alkyl, $-C_{2-6}$ alkenyl, $-C_{2-6}$ alkynyl or cyclic group in the substituents are each independently and optionally substituted by halogen, CN, -OH or - $OC_{1-6}$ alkyl; preferably $R_3$ and $R_4$ are selected from H, halogen, CN, -OH, $-C_{1-6}$ alkyl, $-OC_{1-6}$ alkyl, - $(CH_2)_t$-4-7 membered monocyclic saturated heterocycle having 1-2 heteroatoms independently selected from N, O and S, or $R_3$ and $R_4$ together with the ring carbon atom to which they are attached form a 3-6 membered saturated spirocarbocycle, and the $C_{1-6}$ alkyl in the substituents is optionally substituted by halogen, CN, -OH or $-OC_{1-6}$ alkyl; more preferably $R_3$ and $R_4$ are selected from H, halogen, CN, $-C_{1-6}$ alkyl, 4-7 membered monocyclic saturated heterocycle having 1-2 heteroatoms independently selected from N, O and S, wherein the $C_{1-6}$ alkyl in the substituent is optionally further substituted by halogen, -OH or $-OC_{1-6}$ alkyl;
$R_2$ is selected from H, $-C_{1-6}$ alkyl, $-(CH_2)_t$-3-8 membered monocyclic saturated or partially unsaturated carbocycle, $-(CH_2)_t$-5-8 membered bridged saturated or partially unsaturated carbocycle, $-(CH_2)_t$-3-8 membered monocyclic saturated or partially unsaturated heterocycle having 1-3 heteroatoms independently selected from N, O, and S, and $-(CH_2)_t$-5-8 membered bridged saturated or partially unsaturated heterocycle having 1-3 heteroatoms independently selected from N, O, and S, wherein the $C_{1-6}$ alkyl or cyclic group in the substituent is optionally substituted by halogen, CN, - OH, or $-OC_{1-6}$ alkyl; preferably $R_2$ is selected fromH, $-C_{1-6}$ alkyl, $-(CH_2)_t$-4-7 membered monocyclic saturated heterocycle having 1-2 heteroatoms independently selected from N, O, and S, wherein the $C_{1-6}$ alkyl in the substituent is optionally substituted by halogen, CN, -OH, or $-OC_{1-6}$ alkyl; more preferably $R_2$ is selected from H, $-C_{1-6}$ alkyl, 4-7 membered monocyclic saturated heterocycle having 1-2 heteroatoms independently selected from N, O and S, wherein the $C_{1-6}$ alkyl in the substituent is optionally

substituted by halogen, -OH or -$OC_{1-6}$ alkyl;
when f is 0, G is -$CH_2$-.

**13.** The compound of any one of claims 1-10, a pharmaceutically acceptable salt, isomer, solvate, hydrate or stable isotopic variant thereof, wherein: R has the following formula:

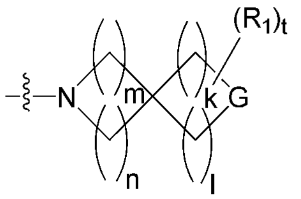 (B) or 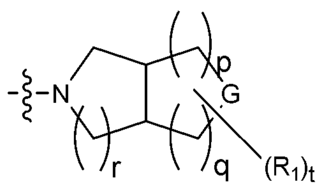 (C)

wherein:

k is an integer from 0-2, m and n are each independently an integer from 1-3, l is an integer from 1-4, preferably k is an integer from 0-2, m and n are each independently an integer from 1-2, and l is an integer from 1-3;
r and p are each independent an integer from 0-3, and q is an integer from 1-4, preferably r is an integer from 0-1, p is an integer from 0-2, and q is an integer from 1-3;
t is selected from an integer from 0 to 3, preferably an integer from 0 to 1;
G is selected from O, N-$R_2$ and $CR_3R_4$, preferably O and $CR_3R_4$;
$R_1$ is selected from H and -$C_{1-6}$ alkyl optionally substituted by halogen, CN, -OH or -$OC_{1-6}$ alkyl, wherein two $R_1$ attached to the same ring carbon atom together with the carbon atom to which they are attached may form 3-8 membered saturated spirocarbocycle; preferably $R_1$ is selected from H and -$C_{1-6}$ alkyl optionally substituted by -OH or -$OC_{1-6}$ alkyl; more preferably $R_1$ is H;
$R_3$ and $R_4$ are each independently selected from H, halogen, CN, -OH, -$NH_2$, -$C_{1-6}$ alkyl, -$C_{2-6}$ alkenyl, -$C_{2-6}$ alkynyl, -$OC_{1-6}$ alkyl, -$NHC_{1-6}$ alkyl, -N($C_{1-6}$ alkyl)$_2$, -$(CH_2)_t$-3-8 membered monocyclic saturated or partially unsaturated carbocycle, -$(CH_2)_t$-5-8 membered bridged saturated or partially unsaturated carbocycle, -$(CH_2)_t$-3-8 membered monocyclic saturated or partially unsaturated heterocycle having 1-3 heteroatoms independently selected from N, O, and S, -$(CH_2)_t$-5-8 membered bridged saturated or partially unsaturated heterocycle having 1-3 heteroatoms independently selected from N, O, and S, or $R_3$ and $R_4$ together with the ring carbon atom to which they are attached form a 3-8 membered saturated spirocarbocycle, wherein the -$C_{1-6}$ alkyl, -$C_{2-6}$ alkenyl, -$C_{2-6}$ alkynyl or cyclic group in the substituents is each independently and optionally substituted by halogen, CN, -OH or - $OC_{1-6}$ alkyl; preferably $R_3$ and $R_4$ are selected from H, halogen, CN, -OH, -$C_{1-6}$ alkyl, -$OC_{1-6}$ alkyl, - $(CH_2)_t$-4-7 membered monocyclic saturated heterocycle having 1-2 heteroatoms independently selected from N, O and S, or $R_3$ and $R_4$ together with the ring carbon atom to which they are attached form a 3-6 membered saturated spirocarbocycle, and the $C_{1-6}$ alkyl in the substituents is optionally substituted by halogen, CN, -OH or -$OC_{1-6}$ alkyl; more preferably $R_3$ and $R_4$ are selected from H, - OH, -$C_{1-6}$ alkyl, wherein the $C_{1-6}$ alkyl in the substituents is optionally further substituted by halogen, -OH or -$OC_{1-6}$ alkyl;
$R_2$ is selected from H, -$C_{1-6}$ alkyl, -$(CH_2)_t$-3-8 membered monocyclic saturated or partially unsaturated carbocycle, -$(CH_2)_t$-5-8 membered bridged saturated or partially unsaturated carbocycle, -$(CH_2)_t$-3-8 membered monocyclic saturated or partially unsaturated heterocycle having 1-3 heteroatoms independently selected from N, O, and S, and -$(CH_2)_t$-5-8 membered bridged saturated or partially unsaturated heterocycle having 1-3 heteroatoms independently selected from N, O, and S, wherein the $C_{1-6}$ alkyl or cyclic group in the substituent is optionally substituted by halogen, CN, - OH, or -$OC_{1-6}$ alkyl; preferably $R_2$ is selected from H, -$C_{1-6}$ alkyl, -$(CH_2)_t$-4-7 membered monocyclic saturated heterocycle having 1-2 heteroatoms independently selected from N, O, and S, wherein the $C_{1-6}$ alkyl or cyclic group in the substituent is optionally substituted by halogen, CN, -OH, or -$OC_{1-6}$ alkyl; more preferably $R_2$ is selected from H, -$C_{1-6}$ alkyl, wherein the $C_{1-6}$ alkyl is optionally further substituted by halogen, -OH or -$OC_{1-6}$ alkyl.

**14.** The compound of claim 1, a pharmaceutically acceptable salt, isomer, solvate, hydrate or stable isotopic variant thereof, having the following formula:

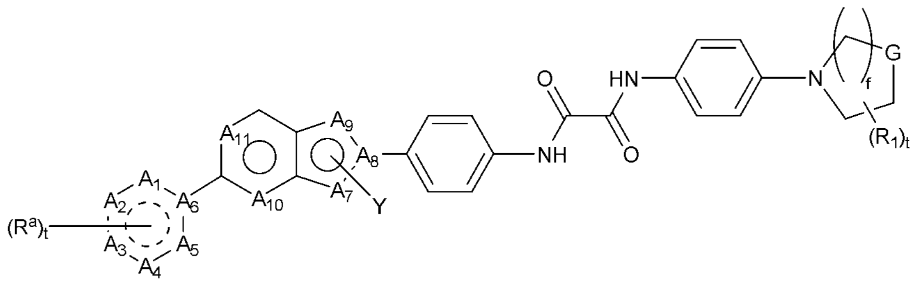

(I-2)

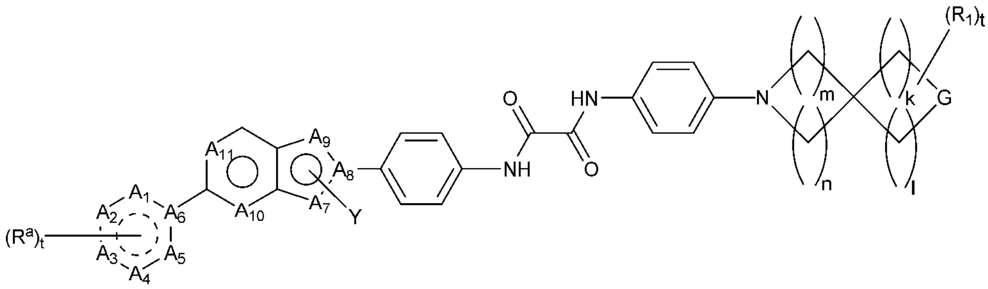

(I-3)

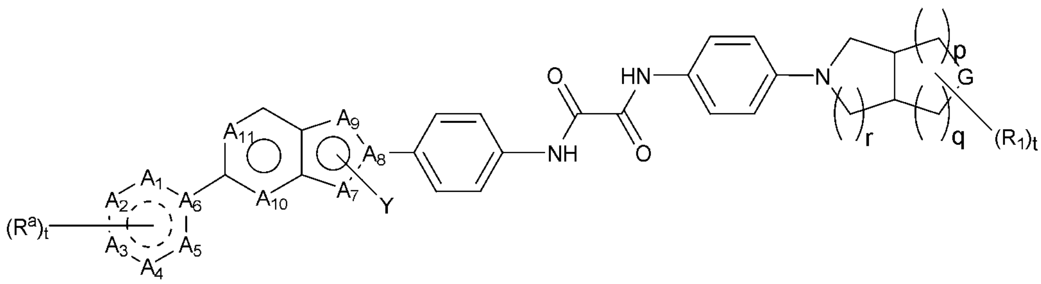

(I-4)

wherein $A_1$~$A_{11}$, $R^a$, $R^b$, Y, G, $R_1$, t, f, m, k, n, l, r, p and q are each as defined in the preceding claims for the compound of formula (I), the structural fragment of formula (A), formula (B) and formula (C);
wherein

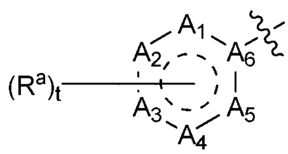

is preferably a five-membered partially unsaturated or aromatic group having 3-4 ring heteroatoms selected from N, O and S, optionally substituted by 0-1 $R^a$; more preferably a five-membered aromatic group having 3-4 nitrogen heteroatoms, optionally substituted by 0-1 $R^a$; most preferably a five-membered aromatic group having 4 nitrogen heteroatoms; $A_{11}$ is preferably C-X, $A_{10}$ is selected from N and C-$R^b$, $A_8$ is C, $R^b$ is preferably H, $A^7$ is selected from C-Y or N-Y, $A_9$ is selected from C, O and S, and only one of $A_7$ and $A_9$ is a heteroatom;
preferably, having the following formula:

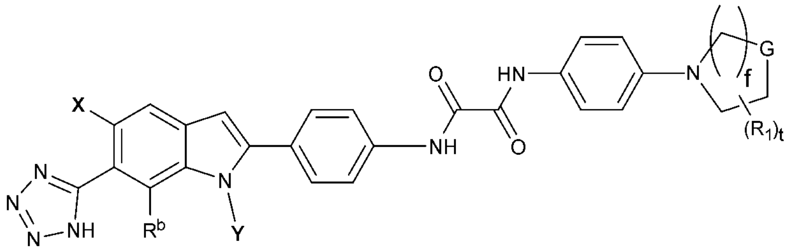

(I-2-1)

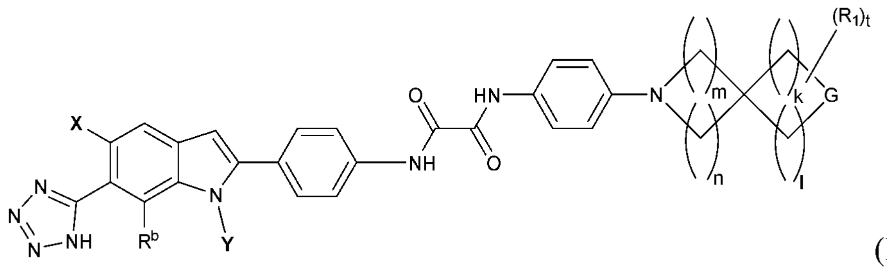

(I-3-1)

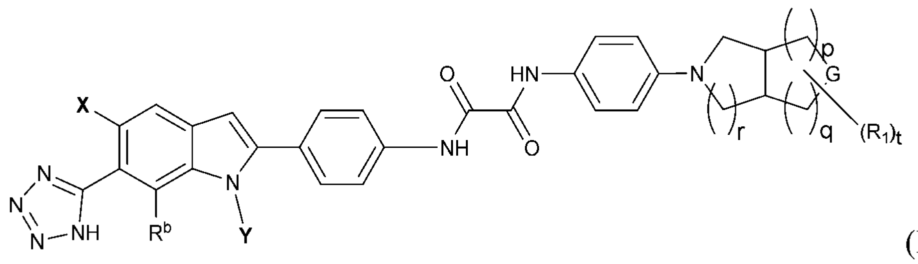

(I-4-1).

**15.** A compound, a pharmaceutically acceptable salt, isomer, solvate, hydrate or stable isotopic variant thereof, which are the compounds of Examples 1-134.

**16.** A pharmaceutical composition, comprising a compound according to any one of claims 1-15, a pharmaceutically acceptable salt, isomer, solvate, hydrate or stable isotopic variant thereof, and a pharmaceutically acceptable excipient.

**17.** The compound of any one of claims 1-15, a pharmaceutically acceptable salt, isomer, solvate, hydrate or stable isotopic variant thereof, or the pharmaceutical composition of claim 16, for use as a medicament for treating and/or preventing a disease mediated by SHP2.

**18.** Use of a compound of any one of claims 1-15, a pharmaceutically acceptable salt, isomer, solvate, hydrate or stable isotopic variant thereof, or the pharmaceutical composition of claim 16 in the preparation of a medicament for preventing or treating a disease mediated by SHP2.

**19.** A method for treating and/or preventing a disease mediated by SHP2, comprising administering to a subject in need a therapeutically effective amount of a compound of any one of claims 1-15, a pharmaceutically acceptable salt, isomer, solvate, hydrate or stable isotopic variant thereof, or a pharmaceutical composition of claim 16.

**20.** The compound of claim 17, the use of claim 18, and the method of claim 19, wherein the disease mediated by SHP2 is cancer or tumor, cardiovascular disease, immune dysregulation, fibrosis, ocular dysregulation, systemic lupus erythematosus, diabetes, neutropenia, or a combination thereof, preferably selected from Noonan syndrome (NS), leopard syndrome (LS), juvenile myelomonocytic leukemia (JMML), myelodysplastic syndrome (MDS), neuroblastoma, melanoma, squamous cell carcinoma of the head and neck, acute myeloid leukemia (AML), B-cell acute lymphoblastic leukemia (B-ALL), breast cancer, esophageal cancer, lung cancer, colon cancer, head cancer, gastric cancer, lymphoma, glioblastoma, stomach cancer, pancreatic cancer, or a combination thereof.

## INTERNATIONAL SEARCH REPORT

International application No. **PCT/CN2024/103967**

**A. CLASSIFICATION OF SUBJECT MATTER**

C07D291/08(2006.01)i; A61K31/16(2006.01)i; A61K31/33(2006.01)i; A61P35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS, CNTXT, ENTXT, ISI web of knowledge, STN: 泰励生物科技, SHP2抑制剂, 癌症, 肿瘤, TYLIGAND BIOSCIENCE, INHIBITORS, CANCER, TUMOR, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| A | WO 2015003094 A2 (INDIANA UNIVERSITY RESEARCH & TECHNOLOGY CORPORATION et al.) 08 January 2015 (2015-01-08)<br>claims 1-20, and embodiments 1-16 | 1-20 |
| A | CN 101437519 A (ABBOTT LABORATORIES) 20 May 2009 (2009-05-20)<br>claims 1-9, embodiments, and Preparation Examples | 1-20 |
| A | CN 105142634 A (INDIANA UNIVERSITY RESEARCH & TECHNOLOGY CORPORATION) 09 December 2015 (2015-12-09)<br>claims 1-20, and embodiments 1-8 | 1-20 |
| A | JP 2002161084 A (SUMITOMO PHARMACEUT CO., LTD.) 04 June 2002 (2002-06-04)<br>claims 1-8, and embodiments | 1-20 |
| A | ZENG, L.F. et al. "Therapeutic Potential of Targeting the Oncogenic SHP2 Phosphatase" *Journal of Medicinal Chemistry*, Vol. 57, No. (15), 08 July 2014 (2014-07-08), 6594-6609<br>tables 1-4 | 1-20 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 August 2024** | **15 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

**INTERNATIONAL SEARCH REPORT**

International application No.
**PCT/CN2024/103967**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17, 19-20**
because they relate to subject matter not required to be searched by this Authority, namely:
Said claims relate to a living human or animal body, which belongs to a method for preventing or treating diseases. The present report is provided on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/103967**

| Patent document cited in search report | Publication date (day/month/year) | Patent family member(s) | Publication date (day/month/year) |
|---|---|---|---|
| WO 2015003094 A2 | 08 January 2015 | US 2018071252 A1 | 15 March 2018 |
| | | US 10092543 B2 | 09 October 2018 |
| | | AU 2014284279 A1 | 18 February 2016 |
| | | CA 2953482 A1 | 08 January 2015 |
| | | EP 3016652 A2 | 11 May 2016 |
| | | EP 3016652 A4 | 08 March 2017 |
| | | US 2019000801 A1 | 03 January 2019 |
| | | US 2016374988 A1 | 29 December 2016 |
| | | US 9844535 B2 | 19 December 2017 |
| CN 101437519 A | 20 May 2009 | US 2012053345 A1 | 01 March 2012 |
| | | JP 2009532370 A | 10 September 2009 |
| | | JP 5255559 B2 | 07 August 2013 |
| | | CA 2644910 A1 | 18 October 2007 |
| | | CA 2644910 C | 28 January 2014 |
| | | WO 2007117465 A2 | 18 October 2007 |
| | | WO 2007117465 A3 | 28 August 2008 |
| | | EP 2001480 A2 | 17 December 2008 |
| | | EP 2001480 A4 | 15 June 2011 |
| | | MX 2008012482 A | 10 October 2008 |
| | | US 2007282101 A1 | 06 December 2007 |
| | | US 8008481 B2 | 30 August 2011 |
| CN 105142634 A | 09 December 2015 | US 2016102054 A1 | 14 April 2016 |
| | | US 9522881 B2 | 20 December 2016 |
| | | CA 2944198 A1 | 30 October 2014 |
| | | CA 2944198 C | 16 November 2021 |
| | | WO 2014176488 A1 | 30 October 2014 |
| | | AU 2014256984 A1 | 22 October 2015 |
| | | AU 2014256984 B2 | 14 February 2019 |
| | | EP 2988741 A1 | 02 March 2016 |
| | | EP 2988741 A4 | 21 December 2016 |
| | | EP 2988741 B1 | 27 November 2019 |
| JP 2002161084 A | 04 June 2002 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023108367770


### Non-patent literature cited in the description


- *J. Med. Chem.*, 2014, vol. 57, 6594-6609
- McGraw-Hill Dictionary of Chemical Terms. McGraw-Hill Book Company, 1984
- **ELIEL, E.** ; **WILEN, S.** Stereochemistry of Organic Compounds. John Wiley & Sons, Inc, 1994
- **SMITH MB**. March's Advanced Organic Chemistry: Reactions, Mechanisms, and Structure. Wiley, 2013
- **E.L. ELIEL** ; **S.H. WILEN** ; **L.N. MANDER**. Stereochemistry of Organic Compounds. Wiley-Interscience, 1994
- **ZENG, L. et al.** *J. Med. Chem.*, 2014, vol. 57, 6594-6609
- Concepts, Structure Design and Methods: from ADME to Toxicity Optimization. **KERNS, EDWARD H.** ; **DI LI et al.** Drug-like Properties. Academic Press, 2008
- **LIN, TONG et al.** In Vitro Assessment of Cytochrome P450 Inhibition: Strategies for Increasing LC/MS-Based Assay Throughput Using a One-Point IC50 Method and Multiplexing High-Performance Liquid Chromatography. *J. Pharm. Sci.*, 2007, vol. 96 (9), 2485